# EUROPEAN PATENT APPLICATION

(11) **EP 4 092 019 A1**
(43) Date of publication of application: **23.11.2022**
(21) Application number: 21741490.3
(22) Date of filing: 12.01.2021
(51) Int. Cl.: C07D 401/14, C07D 471/00, C07D 491/107, C07D 487/04, C07D 405/14, C07D 413/14, C07D 513/10, C07D 519/00, A61K 31/497, A61K 31/675, A61K 31/5377, A61P 35/00, A61P 9/00

(54) **HETEROARYL DERIVATIVE, PREPARATION METHOD THEREFOR, AND USE THEREOF**

(30) Priority: 16.01.2020 CN 202010046221; 10.07.2020 CN 202010660519
(71) Applicant: Zhejiang Hisun Pharmaceutical Co., Ltd., Taizhou, Zhejiang 318000 (CN); Hisun Accuray Therapeutics Co., Ltd., Shanghai 201612 (CN)
(72) Inventor: HUANG, Xiangui, Shanghai 201612 (CN); GUO, Yanghui, Shanghai 201612 (CN); QIU, Zongxing, Shanghai 201612 (CN); BIE, Pingyan, Shanghai 201612 (CN); LIAO, Weiwei, Shanghai 201612 (CN); YAN, Qingyan, Shanghai 201612 (CN); SHEN, Weichao, Shanghai 201612 (CN); CAO, Hai, Shanghai 201612 (CN); XING, Qingna, Shanghai 201612 (CN); WANG, Xin, Shanghai 201612 (CN); CAO, Qi, Shanghai 201612 (CN); MENG, Lichen, Shanghai 201612 (CN); WU, Nuoyi, Shanghai 201612 (CN); LI, Wenpeng, Shanghai 201612 (CN); YE, Cheng, Shanghai 201612 (CN); HU, Taishan, Shanghai 201612 (CN); CHEN, Lei, Zhejiang 318000 (CN)
(74) Representative: Grünecker Patent- und Rechtsanwälte PartG mbB
(86) International application number: PCT/CN2021/071297
(87) International publication number: WO 2021/143680

(57) **Abstract**

The present invention relates to heteroaryl derivatives, preparation methods therefor, and applications thereof in medicine. Specifically, the present invention relates to a heteroaryl derivative represented by general formula (AI), a preparation method therefor, and a pharmaceutically acceptable salt, and use thereof as a therapeutic agent, in partucular, as an SHP2 allosteric inhibitor, wherein the definition of substituents in general formula (AI) is the same as that in the description.

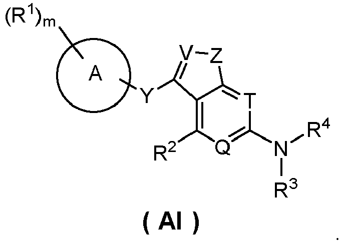

## Description

### TECHNICAL FIELD

The present invention relates to novel heteroaryl derivatives, preparation methods thereof, pharmaceutical compositions containing the derivatives and uses thereof as therapeutic agents, in particular, as a SHP2 allosteric inhibitor.

### BACKGROUND

Src Homology-2 Domain-Containing Protein Tyrosine Phosphatase (SHP2) is an important member of Protein Tyrosine Phosphatase (PTP) family, which is encoded by Protein Tyrosine Phosphatase, Non-Receptor Type 11 (PTPN11) gene and catalyzes the dephosphorylation of tyrosine in protein. An N-terminal of SHP2 contains two SH2 domains, which control the subcellular localization and function regulation of SHP2, and a C-terminal contains a PTP domain with catalytic activity and two tyrosine residues related to activities thereof. Normally, SHP2 is in a state of self-inhibition. When stimulated by growth factors, cytokines or inflammatory factors, such as Platelet-Derived Growth Factors PDGF and FGF, catalytic sites are exposed, leading to the activation of SHP2 enzymes.

SHP2 is widely present in human body, and participates in Rat Sarcoma (RAS)-Extracellular Signal-related Kinase (ERK), Phosphatidylinositol 3 Kinase (PI3K)-protein kinase B and NF-KB, and activates multiple signalling channels like fibroblast growth factors, epidermal growth factors and Mitogen-Activated Protein Kinase (MAPK/ERK) downstream of insulin receptors, so as to regulate cell proliferation, differentiation, migration and apoptosis. At present, it has been found that activating mutation of SHP2 is closely related to the occurrence of Noonan syndrome, Leopard spot syndrome, monocytic leukemia, melanoma, solid tumor, cardiovascular diseases, immune disorder, fibrosis or visual disorder. Over-expression of SHP2 will increase the risks of chronic granulocytic leukemia, mastocytosis, glioblastoma, lung cancer, breast cancer and other cancers, indicating that SHP2 plays an important role in different types of cancers and different stages of the cancers. Due to the multiple functions of SHP2 in tumors, the research on SHP2 target inhibitors also brings new hope and orientation for tumor therapy.

According to different mechanisms of action, SHP2 inhibitors may be divided into competitive inhibitors (including tautomycin, phenylpyrazolyl hydrazine sulfonate and NSC-87877), noncompetitive inhibitors (including indole salicylic acid and fumostone) and irreversible inhibitors (including sodium antimonyl gluconate and cryptotanshinone). As an irreversible SHP2 inhibitor, it is reported that cryptotanshinone can inhibit the proliferation of rhabdomyosarcoma, melanoma, colon cancer and breast cancer in vitro, while in vivo studies have shown that cryptotanshinone can inhibit the proliferation of prostate cancer in mice, and whether cryptotanshinone can further become a clinically effective drug needs many tests to verify.

At present, RMC-4630, a compound developed by REVOLUTION Medicines Inc, has entered clinical phase II for the treatment of solid tumors, and meanwhile, RMC-4550, which is in preclinical phase, is included. Meanwhile, there are also three compounds JAB-3068, JAB-3312 and TNO-155 of clinical phase I, developed by Jacobio (Jacobio Pharmaceuticals Co Ltd) and Novartis (Novartis AG), respectively. Meanwhile, Novartis preclinical drug SHP-099 is included. REVOLUTION Medicines Inc and Novartis AG have disclosed a series of SHP2 inhibitor patents, including WO-2019075265, WO-2018136265, WO-2018136264, WO-2017216706 and WO-2018013597, and the like. Although some progress has been made in SHP2 research, there are still no proven drugs on the market, so it is still necessary to continue research and development of new SHP2 inhibitors.

### SUMMARY

In view of the above technical problems, the present invention provides a novel heteroaryl compound represented by general formula (AI) or a stereoisomer or a tautomer thereof, or a pharmaceutically acceptable salt thereof: 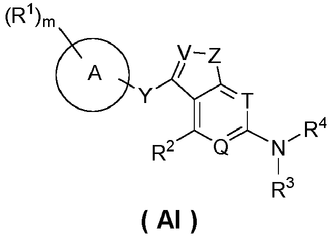 wherein:
Y is selected from a chemical bond or -S-;
when Z is selected from -NH-, V is selected from -N- or -CH-; alternatively, when Z is selected from -O-, V is selected from -N-;
Q and T are each independently selected from N or CH; wherein at least one of Q and T is selected from N;
ring A is selected from aryl, heteroaryl or bicyclic fused ring, wherein the aryl is monocyclic aryl, the heteroaryl is a 5-6 membered monocyclic heteroaryl, and the bicyclic fused ring is preferably a fused ring of aryl or heteroaryl with monocyclic heterocyclyl or monocyclic cycloalkyl;
R¹ are the same or different, and are each independently selected from hydrogen atom, alkyl, alkenyl, alkynyl, cyano, halogen, nitro, cycloalkyl, heterocyclyl, -OR⁵, -C(O)R⁵, -SO₂R⁵, -NR⁶R⁷, -SO₂NR⁶R⁷, -NHSO₂R⁵ or -C(O)NR⁶R⁷, wherein the alkyl, alkenyl, alkynyl, cycloalkyl or heterocyclyl is optionally further substituted by one or more substituents selected from halogen, nitro, cyano, alkyl, cycloalkyl, heterocyclyl, aryl, heteroaryl, -OR⁵, -C(O)R⁵, -C(O)OR⁵, - OC(O)R⁵, -SO₂R⁵, -NR⁶R⁷, -SO₂NR⁶R⁷, -NHSO₂R⁵ or -C(O)NR⁶R⁷;
R² is selected from cyano, tetrazolyl, -C(O)R⁵, -C(O)OR⁵ or -C(O)NR⁶R⁷;
R³ and R⁴ together with the N atom bound therewith form a 4-11 membered heterocyclyl, preferably a 5-11 membered heterocyclyl, wherein the heterocyclyl internally contains one or more N, O, S or SO₂ atoms, and the heterocyclyl is optionally further substituted by one or more substituents selected from halogen, nitro, cyano, alkyl, alkenyl, alkynyl, cycloalkyl, heterocyclyl, aryl, heteroaryl, -CH₂R⁵, -CH(OH)R⁵, -CH₂OR⁵, =O, -OR⁵, -SR⁵, -SOR⁵, -C(O)R⁵, -C(O)OR⁵, - OC(O)R⁵, -SO₂R⁵, -NR⁶R⁷, -SO₂NR⁶R⁷, -NHC(=NH)NH₂, -NHSO₂R⁵ or -C(O)NR⁶R⁷, wherein the alkyl, alkenyl, alkynyl, cycloalkyl, heterocyclyl, aryl or heteroaryl is optionally further substituted by one or more substituents selected from hydroxy, amino, halogen, nitro, cyano, alkyl, haloalkyl, alkoxy, cycloalkyl, heterocyclyl, aryl, heteroaryl, -C(O)R⁸, -C(O)OR⁸, -OC(O)R⁸, - SO₂R⁸, -NR⁹R¹⁰, -C(O)NR⁹R¹⁰, -SO₂NR⁹R¹⁰ or -NR⁹C(O)R¹⁰;
alternatively, R³ and R⁴ together with the N atom bound therewith form a group: 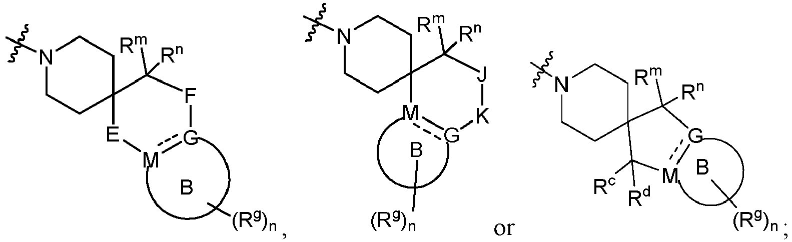 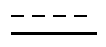
is a single bond or double bond;
when 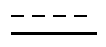 represents a single bond, G and M are each independently selected from N or CR^{j};
when 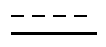 represents a double bond, G and M are each independently selected from C; ring B is selected from cycloalkyl, heterocyclyl aryl or heteroaryl;
E is selected from NR^{k}, (CR^{p}R^{q})ₚ, O or S;
F is selected from (CR^{p}R^{q})_{q};
the condition is that when E is selected from (CR^{p}R^{q})ₚ, p is 1 and q is 1; alternatively, p is 2 and q is 0; and when E is selected from NR^{k}, O or S, q is 1;
J is selected from CR^{p}R^{q};
K is selected from NR^{k}, (CR^{p}R^{q})ᵣ, O or S;
r is 0 or 1;
R^{m}, Rⁿ, R^{p} and R^{q} are the same or different, and are each independently selected from R^{A};
alternatively, R^{p} and R^{q} together with the carbon atom bound therewith form R^{B};
R^{c} and R^{d} are the same or different, and are each independently selected from hydrogen atom, halogen, alkyl or -OR⁵, wherein the alkyl is optionally further substituted by a substituent of hydroxy, halogen, alkoxy, cycloalkyl or -NR⁶R⁷;
alternatively, R^{c} and R^{d} together with the carbon atom bound therewith form R^{B};
R^{g} are the same or different, and are each independently selected from hydrogen atom, halogen, nitro, alkyl, alkenyl, alkynyl, cyano, cycloalkyl, heterocyclyl, aryl, heteroaryl, -OR⁵, - C(O)R⁵, -C(O)OR⁵, -OC(O)R⁵, -SO₂R⁵, -NR⁶R⁷, -SO₂NR⁶R⁷, -NHC(=NH)NH₂, -NHSO₂R⁵ or - C(O)NR⁶R⁷, wherein the alkyl, alkenyl, alkynyl, cycloalkyl, heterocyclyl, aryl or heteroaryl is optionally further substituted by a substituent of hydroxy, halogen, alkyl, alkoxy, cycloalkyl or - NR⁶R⁷;
alternatively, two R^{g} together with the same carbon atom bound therewith form C=O;
R^{j} and R^{k} are the same or different, and are each independently selected from hydrogen atom or alkyl;
R^{A} are the same or different, and are each independently selected from hydrogen atom, halogen, nitro, alkyl, alkenyl, alkynyl, cyano, cycloalkyl, heterocyclyl, aryl, heteroaryl, -OR⁵, - C(O)R⁵, -C(O)OR⁵, -OC(O)R ⁵, -SO₂R⁵, -NR⁶R⁷, -SO₂NR⁶R⁷, -NHC(=NH)NH₂, -NHSO₂R⁵ or - C(O)NR⁶R⁷, wherein the alkyl, alkenyl, alkynyl, cycloalkyl, heterocyclyl, aryl or heteroaryl is optionally further substituted by a substituent of hydroxy, halogen, alkyl, alkoxy, cycloalkyl or - NR⁶R⁷;
R^{B} are the same or different, and are each independently selected from 3-10 membered cycloalkyl or 3-10 membered heterocyclyl, wherein the cycloalkyl or heterocyclyl is optionally further substituted by one or more substituents selected from halogen, cyano, nitro, alkyl, cycloalkyl, heterocyclyl, aryl, heteroaryl, =O, -OR⁵, -C(O)R⁵, -C(O)OR⁵, -OC(O)R⁵, -SO₂R⁵, - NR⁶R⁷, -SO₂NR⁶R⁷, -NHC(=NH)NH₂, -NHSO₂R⁵ or -C(O)NR⁶R⁷;
R⁵, R⁶ and R⁷ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, amino, halogen, nitro, cyano, alkyl, alkoxy, cycloalkyl, heterocyclyl, aryl, heteroaryl, -C(O)R⁸, -C(O)OR⁸, - OC(O)R⁸, -SO₂R⁸, -NR⁹R¹⁰, -C(O)NR⁹R¹⁰, -SO₂NR⁹R¹⁰ or -NR⁹C(O)R¹⁰;
alternatively, R⁶ and R⁷ together with the N atom bound therewith form a 3-8 membered heterocyclyl, wherein the 3-8 membered heterocyclyl internally contains one or more N, O, S or SO₂ atoms, and the 3-8 membered heterocyclyl is optionally further substituted by one or more substituents selected from hydroxy, halogen, amino, alkyl or alkoxy;
R⁸, R⁹ and R¹⁰ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, halogen, nitro, cyano, alkyl, alkoxy, cycloalkyl, heterocyclyl, aryl, heteroaryl, carboxyl or carboxylate;
m is 0, 1, 2, 3, 4 or 5;
n is selected from 0, 1, 2, 3 or 4; and
p is selected from 1 or 2.

In a preferred embodiment of the present invention, the compound represented by general formula (AI) or the stereoisomer or the tautomer thereof, or the pharmaceutically acceptable salt thereof, is a compound represented by general formula (All) or a stereoisomer or a tautomer thereof, or a pharmaceutically acceptable salt thereof: 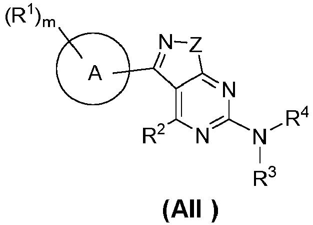 wherein: ring A, m, Z and R¹-R⁴ are defined as in general formula (AI).

In a preferred embodiment of the present invention, the compound represented by general formula (AI) or the stereoisomer or the tautomer thereof, or the pharmaceutically acceptable salt thereof, is a compound represented by general formula (I) or a stereoisomer or a tautomer thereof, or a pharmaceutically acceptable salt thereof: 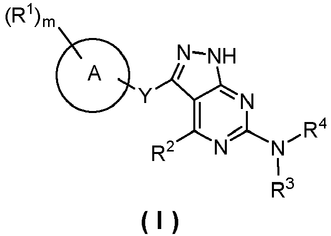 wherein: ring A, Y, m, and R¹-R⁴ are defined as in general formula (AI).

In a preferred embodiment of the present invention, the compound represented by general formula (AI) or the stereoisomer or the tautomer thereof, or the pharmaceutically acceptable salt thereof, is a compound represented by general formula (II) or a stereoisomer or a tautomer thereof, or a pharmaceutically acceptable salt thereof: 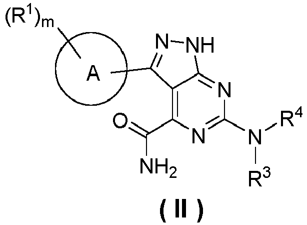 wherein: ring A, m, R¹, R³ and R⁴ are defined as in general formula (AI).

In a preferred embodiment of the present invention, in the compound represented by general formula (AI), (AII), (I) or (II), or the stereoisomer or the tautomer thereof, or the pharmaceutically acceptable salt thereof, wherein:
R³ and R⁴ together with the N atom bound therewith form a 4-8 membered monocyclic heterocyclyl, preferably a 5-6 membered monocyclic heterocyclyl, more preferably piperidinyl, wherein the monocyclic heterocyclyl is optionally further substituted by one or more substituents selected from methyl, amino, cycloalkyl, phenyl, halophenyl, heteroaryl, -CH₂NH₂, -CH₂OH, - NHC(=NH)NH₂, =O or -OR⁵; wherein the methyl, cycloalkyl, phenyl or heteroaryl is optionally further substituted by substituents selected from one or more of mesyl, hydroxy, amino, halogen, haloalkyl, alkoxy, haloalkoxy, pyridinyl, or pyrimidinyl; wherein the heteroaryl is preferably pyridinyl, pyrimidinomethylbenzopyrazolyl, pyrrolyl, furanyl, thienyl, pyrazolyl, imidazolyl, thiazolyl, benzimidazolyl, benzofuranyl or benzoxazolyl; and
R⁵ is defined as in general formula (AI).

In a preferred embodiment of the present invention, in the compound represented by general formula (AI), (AII), (I) or (II), or the stereoisomer or the tautomer thereof, or the pharmaceutically acceptable salt thereof, wherein:
R³ and R⁴ together with the N atom bound therewith form a 7-11 membered spiroheterocyclyl, wherein the spiroheterocyclyl is optionally further substituted by one or more substituents selected from methyl, amino, -CH₂NH₂, -CH₂OH, -NHC(=NH)NH₂, =O or -OR⁵; R⁵ is defined as in general formula (AI); and preferably, the spiroheterocyclyl is selected from: 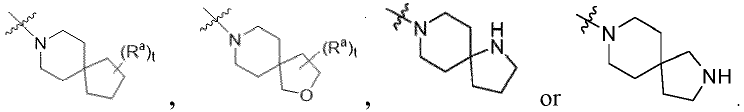
R^{a} are the same or different, and are each independently selected from methyl, amino, - CH₂NH₂, -CH₂OH, -NHC(=NH)NH₂ or -OR⁵; or two R^{a} together with the same carbon atom bound therewith form C=O; t is 1, 2 or 3; and R⁵ is defined as in general formula (AI).

In a preferred embodiment of the present invention, in the compound represented by general formula (AI), (AII), (I) or (II), or the stereoisomer or the tautomer thereof, or the pharmaceutically acceptable salt thereof, R³ and R⁴ together with the N atom bound therewith form a 7-11 membered bridged heterocyclyl, wherein the bridged heterocyclyl is optionally further substituted by one or more substituents selected from methyl, amino, -CH₂NH₂, -CH₂OH, -NHC(=NH)NH₂, =O or - OR⁵; and R⁵ is defined as in general formula (AI).

In a preferred embodiment of the present invention, in the compound represented by general formula (I) or (II), or the stereoisomer or the tautomer thereof, or the pharmaceutically acceptable salt thereof, R³ and R⁴ together with the N atom bound therewith form a 7-11 membered fused heterocyclyl, wherein the fused heterocyclyl is optionally further substituted by one or more substituents selected from methyl, amino, -CH₂NH₂, -CH₂OH, -NHC(=NH)NH₂, =O or -OR⁵; and R⁵ is defined as in general formula (AI).

In a preferred embodiment of the present invention, the compound represented by general formula (I) or the stereoisomer or the tautomer thereof, or the pharmaceutically acceptable salt thereof, is a compound represented by general formula (III) or a stereoisomer or a tautomer thereof, or a pharmaceutically acceptable salt thereof: 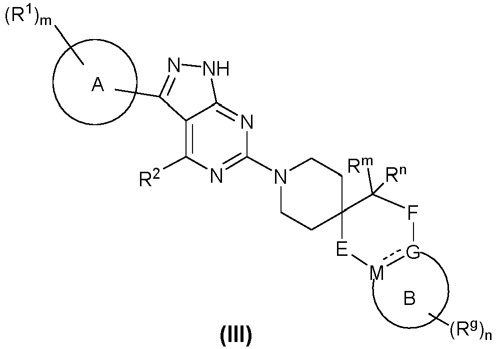 wherein:
ring B is selected from phenyl, 3-8 membered cycloalkyl, 4-8 membered heterocyclyl or 5-6 membered heteroaryl;
E is selected from NR^{k}, (CR^{p}R^{q})ₚ, O or S;
F is selected from ((CR^{p}R^{q})_{q};
the condition is that when E is selected from (CR^{p}R^{q})ₚ, p is 1 and q is 1; alternatively, p is 2 and q is 0; and when E is selected from NR^{k}, O or S, q is 1;
R^{m} is selected from amino, -CH₂NH₂ or -NHC(=NH)NH₂;
Rⁿ is selected from hydrogen atom, methyl or -CH₂OH;
R^{p} and R^{q} are each independently selected from hydrogen atom, halogen, amino, C₁-C₄ alkyls, hydroxy C₁-C₄ alkyls, amino C₁-C₄ alkyls or -OR⁵; and 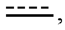
ring A, G, M, m, n, R¹-R², R⁵, R^{k} and R^{g} are defined as in general formula (I).

In a preferred embodiment of the present invention, the compound represented by general formula (I) or the stereoisomer or the tautomer thereof, or the pharmaceutically acceptable salt thereof, is a compound represented by general formula (IV) or a stereoisomer or a tautomer thereof, or a pharmaceutically acceptable salt thereof: 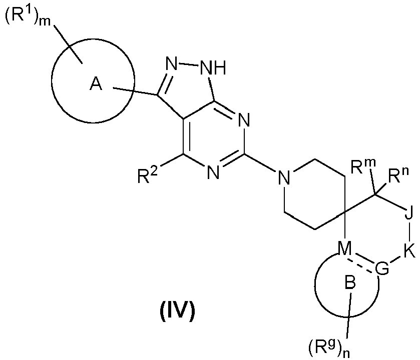 wherein:
ring B is selected from phenyl, 3-8 membered cycloalkyl, 4-8 membered heterocyclyl or 5-6 membered heteroaryl;
J is selected from CR^{p}R^{q};
K is selected from NR^{k}, (CR^{p}R^{q})ᵣ, O or S;
r is 0 or 1;
R^{m} is selected from amino, -CH₂NH₂ or -NHC(=NH)NH₂;
Rⁿ is selected from hydrogen atom, methyl or -CH₂OH;
R^{p} and R^{q} are each independently selected from hydrogen atom, halogen, amino, C₁-C₄ alkyls, hydroxy C₁-C₄ alkyls, amino C₁-C₄ alkyls or -OR⁵; and 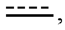
ring A, G, M, m, n, R¹-R², R⁵, R^{k} and R^{g} are defined as in general formula (I).

In a preferred embodiment of the present invention, the compound represented by general formula (I) or the stereoisomer or the tautomer thereof, or the pharmaceutically acceptable salt thereof, is a compound represented by general formula (V) or a stereoisomer or a tautomer thereof, or a pharmaceutically acceptable salt thereof: 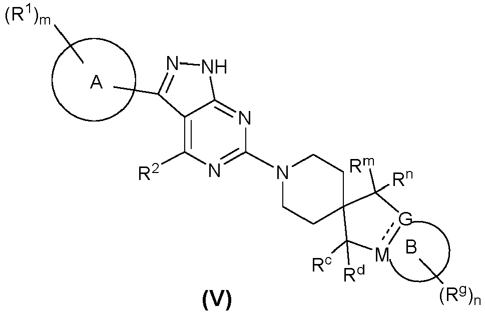 wherein:
ring B is selected from phenyl, 3-8 membered cycloalkyl, 4-8 membered heterocyclyl or 5-6 membered heteroaryl;
R^{c} and R^{d} together with the atom bound therewith form a 3-8 membered cycloalkyl;
R^{m} is selected from amino, -CH₂NH₂ or -NHC(=NH)NH₂;
Rⁿ is selected from hydrogen atom, methyl or -CH₂OH; and 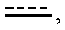
ring A, G, M, m, n, R¹-R² and R^{g} are defined as in general formula (I).

In a preferred embodiment of the present invention, the compound represented by general formula (AI) or the stereoisomer or the tautomer thereof, or the pharmaceutically acceptable salt thereof, is a compound represented by general formula (VI) or a stereoisomer or a tautomer thereof, or a pharmaceutically acceptable salt thereof: 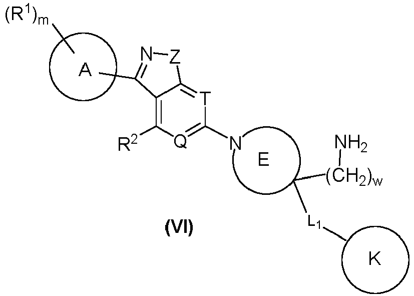 wherein:
L₁ is absent, or selected from -(C=O)- and -(CR^{w}R^{v})ᵤ-, wherein any one of -(CR^{w}R^{v})- is optionally further replaced by -N(R^{z})-, -O-, -S-, -SO- and -SO₂-;
each R^{w} and R^{v} are the same or different, and are each independently selected from hydrogen atom, halogen, hydroxy, alkyl or alkoxy;
each R^{z} are the same or different, and are each independently selected from hydrogen atom or alkyl;
   ring E is selected from 4-11 membered monocyclic heterocyclyl containing N, 4-11 membered fused heterocyclyl containing N or 4-11 membered bridged heterocyclyl containing N, wherein the monocyclic heterocyclyl, fused heterocyclyl or bridged heterocyclyl is optionally further substituted by one or more substituents selected from halogen, alkyl, -OR⁵ or =O;
   ring K is absent, or selected from cycloalkyl, aryl or heteroaryl, wherein the cycloalkyl, aryl or heteroaryl is optionally further substituted by one or more substituents selected from hydroxy, amino, halogen, nitro, cyano, alkyl, alkoxy, haloalkyl, cycloalkyl, heterocyclyl, aryl, heteroaryl, - C(O)R⁸, -C(O)OR⁸, -OC(O)R⁸, -SO₂R⁸, -NR⁹R¹⁰, -C(O)NR⁹R¹⁰, -SO₂NR⁹R¹⁰ or -NR⁹C(O)R¹⁰;
wherein -L₁-ring K and -(CH₂)_{W}-NH₂ are bound to the same carbon atom of ring E;
w is 0, 1 or 2;
u is 0, 1, 2 or 3; and
ring A, Z, Q, T, m, R¹-R², R⁵, and R⁸-R¹⁰ are defined as in general formula (AI).

In a preferred embodiment of the present invention, in the compound represented by general formula (AI), (AII), (I), (II), (III), (IV), (V) or (VI) or the stereoisomer or the tautomer thereof, or the pharmaceutically acceptable salt thereof, R¹ is selected from hydrogen atom, F, Cl, Br, amino, hydroxy, cyano, nitro, methoxy, ethoxy, methyl, ethyl, trifluoromethyl, cyclopropyloxy, ethynyl, ethenyl, -NHCH₃ or -N(CH₃)₂.

In a preferred embodiment of the present invention, in the compound represented by general formula (III), (IV), (V) or (VI) or the stereoisomer or the tautomer thereof, or the pharmaceutically acceptable salt thereof, R² is selected from -C(O)NH₂ or -C(O)OH.

In a preferred embodiment of the present invention, in the compound represented by general formula (AI), (AII), (I), (II), (III), (IV), (V) or (VI) or the stereoisomer or the tautomer thereof, or the pharmaceutically acceptable salt thereof, R⁵ is selected from hydrogen atom or alkyl.

In a preferred embodiment of the present invention, in the compound represented by general formula (AI), (AII), (I), (II), (III), (IV), (V) or (VI) or the stereoisomer or the tautomer thereof, or the pharmaceutically acceptable salt thereof, ring A is selected from phenyl, pyridinyl or pyrimidinyl.

In a preferred embodiment of the present invention, in the compound represented by general formula (III), (IV) or (V) or the stereoisomer or the tautomer thereof, or the pharmaceutically acceptable salt thereof, ring B is selected from:

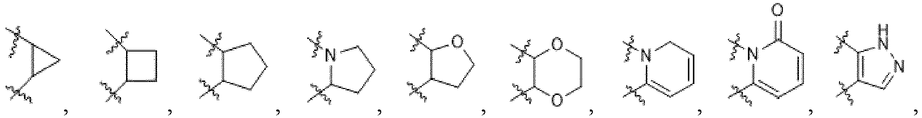

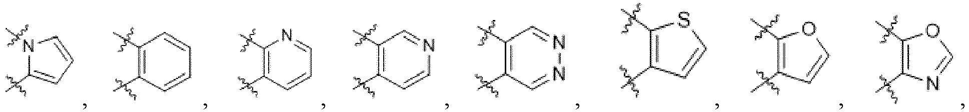

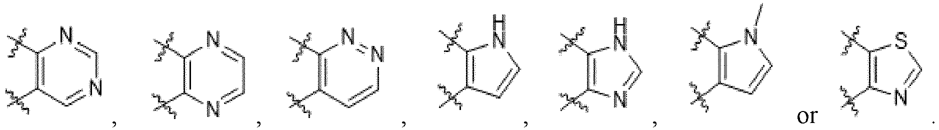

In a preferred embodiment of the present invention, in the compound represented by general formula (III), (IV) or (V) or the stereoisomer or the tautomer thereof, or the pharmaceutically acceptable salt thereof, R^{g} are the same or different, and are each independently selected from hydrogen atom, F, Cl, Br, amino, hydroxy, cyano, nitro, methoxy, ethoxy, methyl, ethyl, ethynyl, ethenyl, -NHCH₃ or -N(CH₃)₂; and
alternatively, two R^{g} together with the same carbon atom bound therewith form C=O.

In a preferred embodiment of the present invention, in the compound represented by general formula (VI) or the stereoisomer or the tautomer thereof, or the pharmaceutically acceptable salt thereof, ring E is selected from:

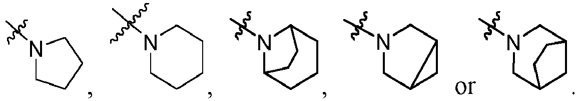

In a preferred embodiment of the present invention, the compound represented by general (AI) is selected from:

| Compound No. | Structure | Name |
|---|---|---|
| Example 1 | 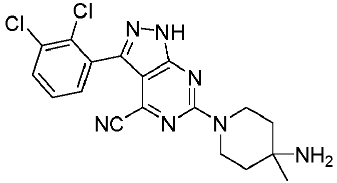 | 6-(4-Amino-4-methylpiperidin-1-yl)-3-(2,3-dichlorophenyl)-1H-pyrazolo[3,4-d]pyrimidine-4-carbonitrile |
| Example 2 | 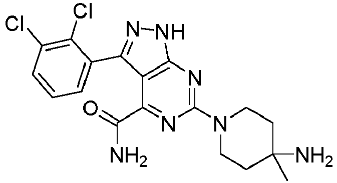 | 6-(4-Amino-4-methylpiperidin-1-yl)-3-(2,3-dichlorophenyl)-1H-pyrazolo[3,4-d]pyrimidine-4-carboxamide |
| Example 3 | 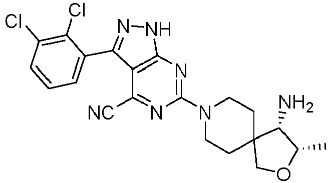 | 6-((3S,4S)-4-Amino-3-methyl-2-oxa-8-azaspiro[4.5]decan-8-yl)-3-(2,3-dichlorophenyl)-1H-pyrazolo[3,4-d]pyrimidine-4-carbonitrile |
| Example 4 | 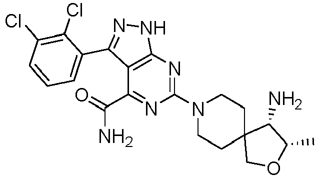 | 6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carboxamide |
| Example 5 | 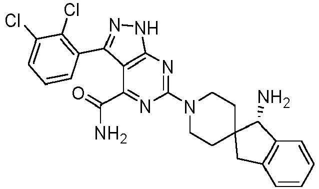 | (S)-6-(1-amino-1,3-dihydrospiro[indene-2,4'-piperidin]-1-yl)-3-(2,3-dichlorophenyl)-1H-pyrazolo[3,4-d]pyrimidine-4-carboxamide |
| Example 6 | 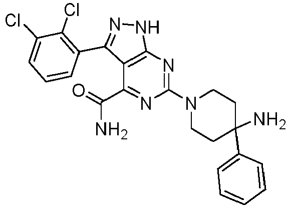 | 6-(4-Amino-4-phenylpiperidin-1-yl)-3 - (2,3-dichlorophenyl)-1H-pyrazolo[3,4-d]pyrimidine-4-carboxamide |
| Example 7 | 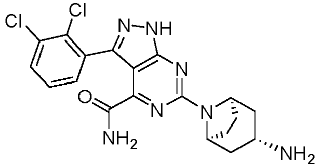 | 6-((Endo)-3-amino-8-azabicyclo[3.2.1]octan-8-yl)-3-(2,3-dichlorophenyl)-1H-pyrazolo[3,4-d]pyrimidine-4-carboxamide |
| Example 8 | 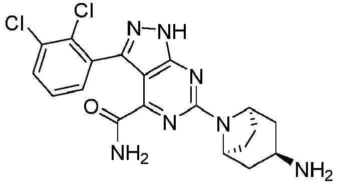 | 6-((Exo)-3-amino-8-azabicyclo[3.2.1]octan-8-yl)-3-(2,3-dichlorophenyl)-1H-pyrazolo[3,4-d]pyrimidine-4-carboxamide |
| Example 9 | 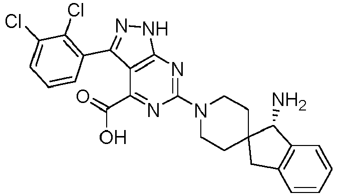 | (S)-6-(1-Amino-1,3-dihydrospiro[indene-2,4'-piperidin]-1'-yl)-3-(2,3-dichlorophenyl)-1H-pyrazolo[3,4-d]pyrimidine-4-carboxylic acid |
| Example 10 | 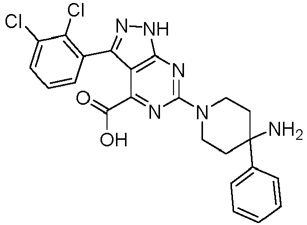 | 6-(4-Amino-4-phenylpiperidin-1-yl)-3 - (2,3-dichlorophenyl)-1H-pyrazolo[3,4-d]pyrimidine-4-carboxylic acid |
| Example 11 | 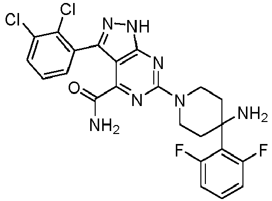 | 6-(4-Amino-4-(2,6-difluorophenyl)piperidin-1-yl)-3-(2,3-dichlorophenyl)-1H-pyrazolo[3, 4-d]pyrimidine-4-carboxamide |
| Example 12 | 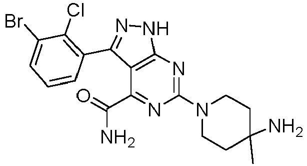 | 6-(4-Amino-4-methylpiperidin-1-yl)-3-(3-bromo-2-chlorophenyl)-1H-pyrazolo[3,4-d]pyrimidine-4-carboxamide |
| Example 13 | 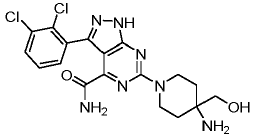 | 6-(4-Amino-4-(hydroxymethyl)piperidin-1-yl)-3-(2,3-dichlorophenyl)-1H-pyrazolo[3,4-d]pyrimidine-4-carboxamide |
| Example 14 | 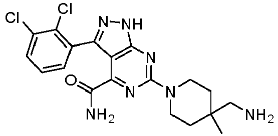 | 6-(4-(Aminomethyl)-4-methylpiperidin-1-yl)-3-(2,3-dichlorophenyl)-1H-pyrazolo[3,4-d]pyrimidine-4-carboxamide |
| Example 15 | 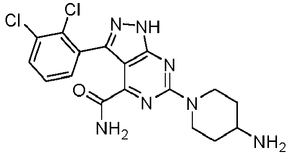 | 6-(4-Aminopiperidin-1-yl)-3-(2,3 - dichlorophenyl)-1H-pyrazolo[3,4-d]pyrimidine-4-carboxamide |
| Example 16 | 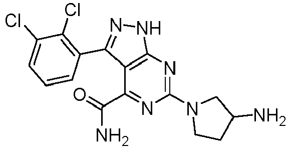 | 6-(3-Aminopyrrolidin-1-yl)-3-(2,3-dichlorophenyl)-1H-pyrazolo[3,4-d]pyrimidine-4-carboxamide |
| Example 17 | 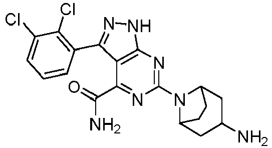 | 6-(3-Amino-8-azabicyclo[3.2.1]octan-8-yl)-3-(2,3-dichlorophenyl)-1H-pyrazolo[3,4-d]pyrimidine-4-carboxamide |
| Example 18 | 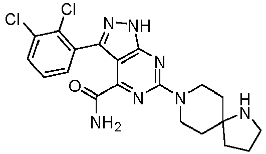 | 3-(2,3-Dichlorophenyl)-6-(1,8-diazaspiro[4.5]decan-8-yl)-1H-pyrazolo[3,4-d]pyrimidine-4-carboxamide |
| Example 19 | 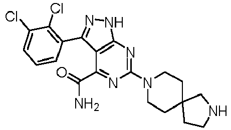 | 3-(2,3-Dichlorophenyl)-6-(2,8-diazaspiro[4.5]decan-8-yl)-1H-pyrazolo[3,4-d]pyrimidine-4-carboxamide |
| Example 20 | 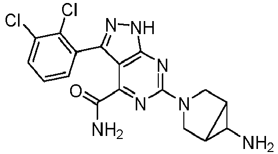 | 6-(6-Amino-3-azabicyclo[3.1.0]hexan-3-yl)-3-(2,3-dichlorophenyl)-1H-pyrazolo[3,4-d]pyrimidine-4-carboxamide |
| Example 21 | 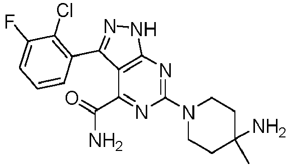 | 6-(4-Amino-4-methylpiperidin-1-yl)-3-(2-chloro-3-fluorophenyl)-1H-pyrazolo[3,4-d]pyrimidine-4-carboxamide |
| Example 22 | 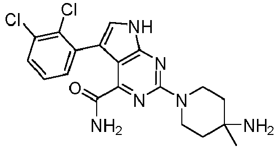 | 2-(4-Amino-4-methylpiperidin-1-yl)-5-(2,3-dichlorophenyl)-7H-pyrrolo[2,3-d]pyrimidine-4-carboxamide |
| Example 23 | 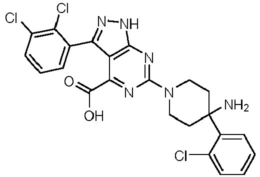 | 6-(4-Amino-4-(2-chlorophenyl)piperidin-1-yl)-3-(2,3-dichlorophenyl)-1H-pyrazolo[3,4-d]pyrimidine-4-carboxylic acid |
| Example 24 | 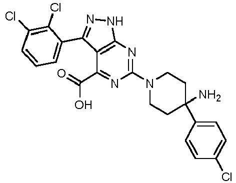 | 6-(4-Amino-4-(4-chlorophenyl)piperidin-1-yl)-3-(2,3-dichlorophenyl)-1H-pyrazolo[3,4-d]pyrimidine-4-carboxylic acid |
| Example 25 | 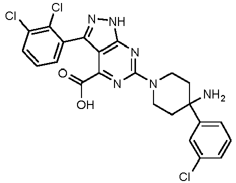 | 6-(4-Amino-4-(3-chlorophenyl)piperidin-1-yl)-3-(2,3-dichlorophenyl)-1H-pyrazolo[3,4-d]pyrimidine-4-carboxylic acid |
| Example 26 | 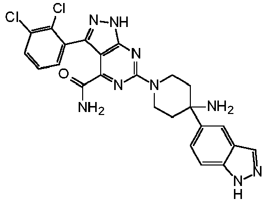 | 6-(4-Amino-4-(1H-indazole-5-yl)piperidin-1-yl)-3-(2,3- dichlorophenyl)-1H-pyrazolo[3,4-d]pyrimidine-4-carboxamide |
| Example 27 | 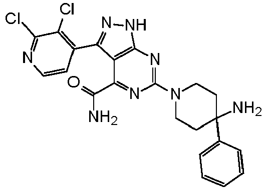 | 6-(4-Amino-4-phenylpiperidin-1-yl)-3 - (2,3-dichloropyridin-4-yl)-1H-pyrazolo[3,4-d]pyrimidine-4-carboxamide |
| Example 28 | 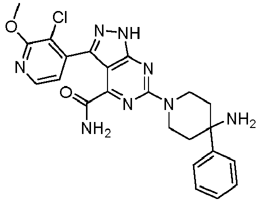 | 6-(4-Amino-4-phenylpiperidin-1-yl)-3 - (3-chloro-2-methoxypyridin-4-yl)-1H-pyrazolo[3,4-d]pyrimidine-4-carboxamide |
| Example 29 | 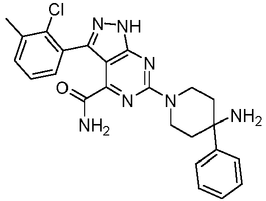 | 6-(4-Amino-4-phenylpiperidin-1-yl)-3 - (2-chloro-3-methylphenyl)-1H-pyrazolo[3,4-d]pyrimidine-4-carboxamide |
| Example 30 | 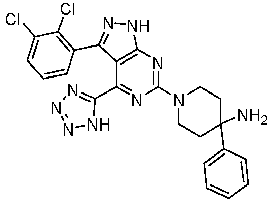 | 1-(3-(2,3-Dichlorophenyl)-4-(1H-tetrazol-5-yl)-1H-pyrazolo[3,4-d]pyrimidin-6-yl)-4-phenylpiperidin-4-amine |
| Example 32 | 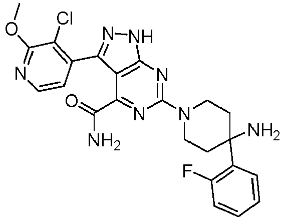 | 6-(4-Amino-4-(2-fluorophenyl)piperi din-1-yl)-3-(3-chloro-2-methoxypyridin-4-yl)-1H-pyrazolo[3,4-d]pyrimidine-4-carboxamide |
| Example 33 | 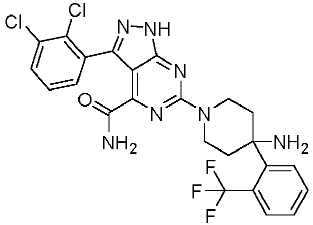 | 6-(4-Amino-4-(2-(trifluoromethyl)phenyl)piperidin-1-yl)-3-(2,3-dichlorophenyl)-1H-pyrazolo[3,4-d]pyrimidine-4-carboxamide |
| Example 34 | 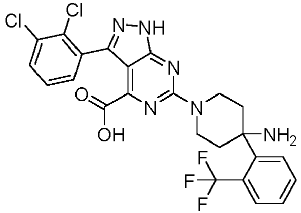 | 6-(4-Amino-4-(2-(trifluoromethyl)phenyl)piperidin-1-yl)-3-(2,3-dichlorophenyl)-1H-pyrazolo [3,4-d]pyrimidine-4-carboxylic acid |
| Example 35 | 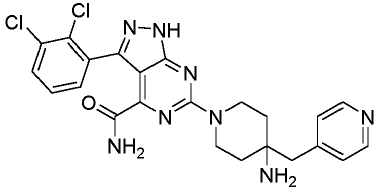 | 6-(4-Amino-4-(pyridin-4-ylmethyl)piperidin-1-yl)-3-(2,3-dichlorophenyl)-1H-pyrazolo[3,4-d]pyrimidine-4-carboxamide |
| Example 36 | 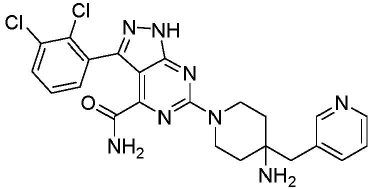 | 6-(4-Amino-4-(pyridin-3-ylmethyl)piperidin-1-yl)-3-(2,3-dichlorophenyl)-1H-pyrazolo[3,4-d]pyrimidine-4-carboxamide |
| Example 37 | 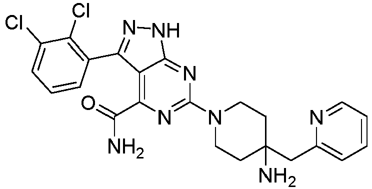 | 6-(4-Amino-4-(pyridin-2-ylmethyl)piperidin-1-yl)-3-(2,3-dichlorophenyl)-1H-pyrazolo[3,4-d]pyrimidine-4-carboxamide |
| Example 38 | 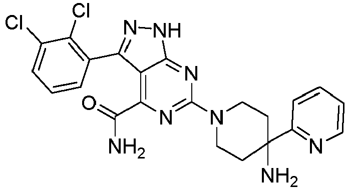 | 6-(4-Amino-4-(pyridin-2-yl)piperidin-1-yl)-3-(2,3-dichlorophenyl)-1H-pyrazolo[3,4-d]pyrimidine-4-carboxamide |
| Example 39 | 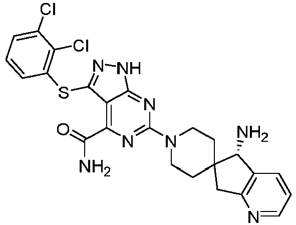 | (S)-6-(5-amino-5,7-dihydrospiro[cyclopenta[b]pyridine-6,4'-piperidin]-1'-yl)-3-((2,3-dichlorophenyl)thio)-1H-pyrazolo[3,4-d]pyrimidine-4-carboxamide |
| Example 40 | 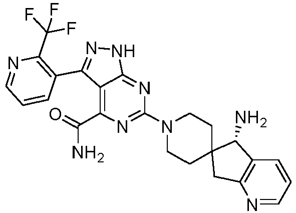 | (S)-6-(5-amino- 5,7-dihydrospiro[cyclopenta[b]pyridine-6,4'-piperidin]-1'-yl)-3-(2-(trifluoromethyl)pyridin-3-yl)-1H-pyrazolo[3,4-d]pyrimidine-4-carboxamide |
| Example 41 | 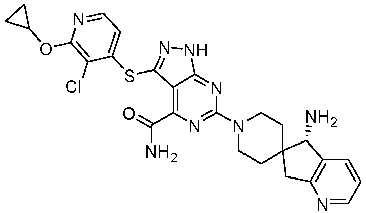 | (S)-6-(5-amino- 5,7-dihydrospiro[cyclopenta[b]pyridine-6,4'-piperidin]-1'-yl)-3-((3-chloro-2-cyclopropoxypyridin-4-yl)thio)-1H-pyrazolo[3,4-d]pyrimidine-4-carboxamide |
| Example 42 | 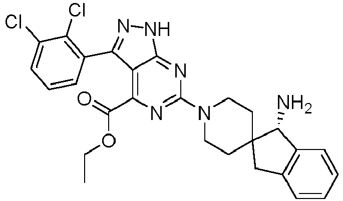 | Ethyl (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxylate |
| Example 43 | 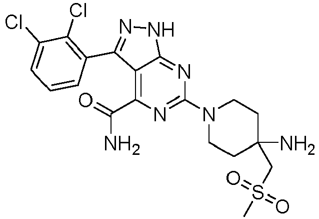 | 6-(4-Amino-4-((methylsulfonyl)methyl)piperidin-1-yl)-3-(2,3-dichlorophenyl)-1H-pyrazolo[3,4-d]pyrimidine-4-carboxamide |
| Example 44 | 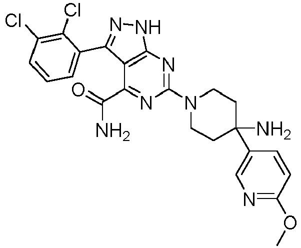 | 6-(4-Amino-4-(6-methoxypyridin-3-yl)piperidin-1-yl)-3-(2,3 - dichlorophenyl)-1H-pyrazolo[3,4-d]pyrimidine-4-carboxamide |
| Example 45 | 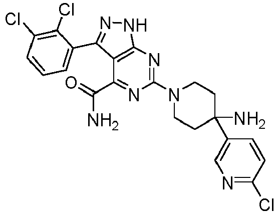 | 6-(4-Amino-4-(6-chloropyridin-3-yl)piperidin-1-yl)-3-(2,3 - dichlorophenyl)-1H-pyrazolo[3,4-d]pyrimidine-4-carboxamide |
| Example 46 | 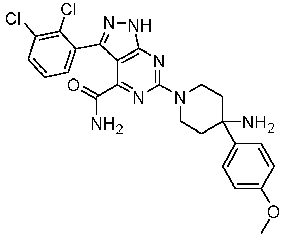 | 6-(4-Amino-4-(4-methoxyphenyl)piperidin-1-yl)-3 -(2,3 - dichlorophenyl)-1H-pyrazolo[3, 4-d]pyrimidine-4-carboxamide |
| Example 47 | 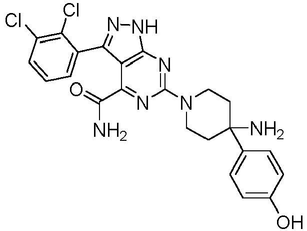 | 6-(4-Amino-4-(4-hydroxyphenyl)piperidin-1-yl)-3-(2,3 - dichlorophenyl)-1H-pyrazolo[3,4-d]pyrimidine-4-carboxamide |
| Example 48 | 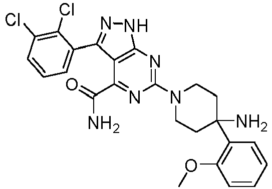 | 6-(4-Amino-4-(2-methoxyphenyl)piperidin-1-yl)-3-(2,3 - dichlorophenyl)-1H-pyrazolo[3,4-d]pyrimidine-4-carboxamide |
| Example 49 | 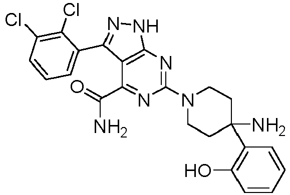 | 6-(4-Amino-4-(2-hydroxyphenyl)piperidin-1-yl)-3-(2,3-dichlorophenyl)-1H-pyrazolo[3,4-d]pyrimidine-4-carboxamide |
| Example 50 | 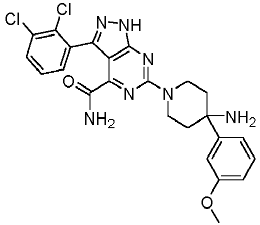 | 6-(4-Amino-4-(3-methoxyphenyl)piperidin-1-yl)-3-(2,3-dichlorophenyl)-1H-pyrazolo[3,4-d]pyrimidine-4-carboxamide |
| Example 51 | 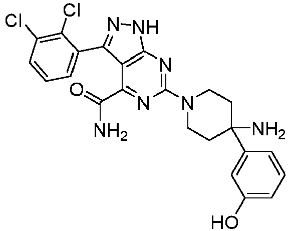 | 6-(4-Amino-4-(3-hydroxyphenyl)piperidin-1-yl)-3-(2,3-dichlorophenyl)-1H-pyrazolo[3,4-d]pyrimidine-4-carboxamide |
| Example 52 | 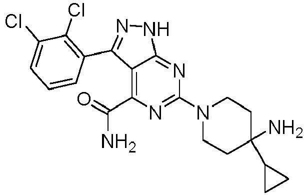 | 6-(4-Amino-4-cyclopropylpiperidin-1-yl)-3-(2,3-dichlorophenyl)-1H-pyrazolo[3,4-d]pyrimidine-4-carboxamide |
| Example 53 | 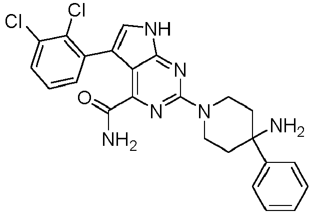 | 2-(4-Amino-4-phenylpiperidin-1-yl)-5-(2,3-dichlorophenyl)-7H-pyrrolo[2,3-d] pyrimi dine-4-carboxamide |
| Example 54 | 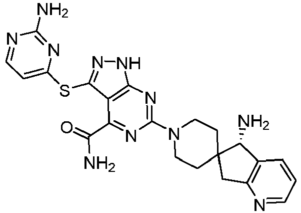 | (S)-6-(5-amino-5,7-dihydrospiro[cyclopenta[b]pyridine-6,4'-piperidin]-1'-yl)-3-((2-aminopyrimidin - 4-yl)thio)-1H-pyrazolo[3,4-d]pyrimidine-4-carboxamide |
| Example 55 | 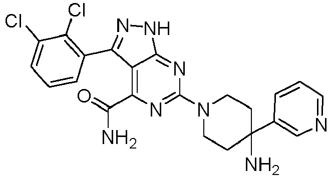 | 6-(4-Amino-4-(pyridin-3-yl)piperidin-1-yl)-3-(2,3-dichlorophenyl)-1H-pyrazolo[3,4-d]pyrimidine-4-carboxamide |
| Example 56 | 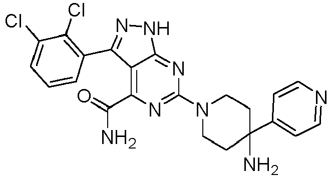 | 6-(4-Amino-4-(pyridin-4-yl)piperidin-1-yl)-3-(2,3-dichlorophenyl)-1H-pyrazolo[3,4-d]pyrimidine-4-carboxamide |
| Example 57 | 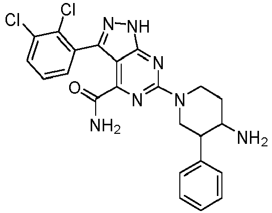 | 6-(4-Amino-3-phenylpiperidin-1-yl)-3 - (2,3-dichlorophenyl)-1H-pyrazolo[3,4-d]pyrimidine-4-carboxamide |
| Example 58 | 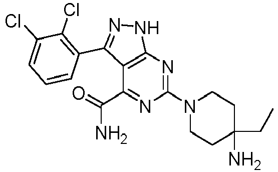 | 6-(4-Amino-4-ethylpiperidin-1-yl)-3-(2,3-dichlorophenyl)-1H-pyrazolo[3,4-d]pyrimidine-4-carboxamide |

or a stereoisomer, tautomer thereof or a pharmaceutically acceptable salt thereof.

Further, the present invention provides a preparation method for the compound represented by general formula (I) or the stereoisomer or the tautomer thereof, wherein the method comprises: 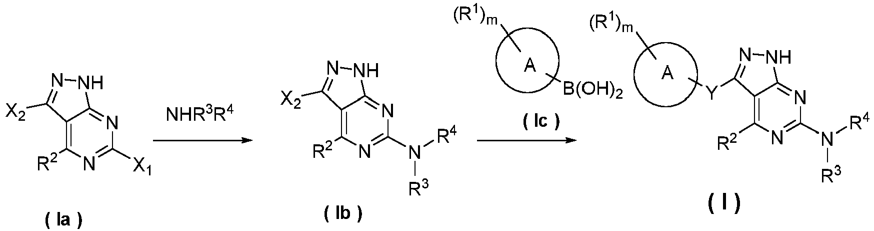 subjecting the compound represented by general formula (Ia) and NHR³R⁴ to a nucleophilic substitution reaction under alkaline condition to obtain the compound represented by general formula (Ib); and subjecting the compound represented by general formula (Ib) and the compound represented by general formula (Ic) to a Suzuki reaction in the presence of palladium catalyst and alkaline condition, and optionally further removing a protecting group of the obtained compound to obtain the compound represented by general formula (I); wherein:
Y is selected from chemical bond;
X₁ is selected from leaving group, wherein the leaving group is selected from halogen or - SO₂R^{t};
X₂ is selected from halogen;
R^{t} is selected from alkyl; and
ring A, m, and R¹-R⁴ are defined as in general formula (I).

Further, the present invention provides a preparation method for the compound represented by general formula (I) or the stereoisomer or the tautomer thereof, wherein the method comprises: 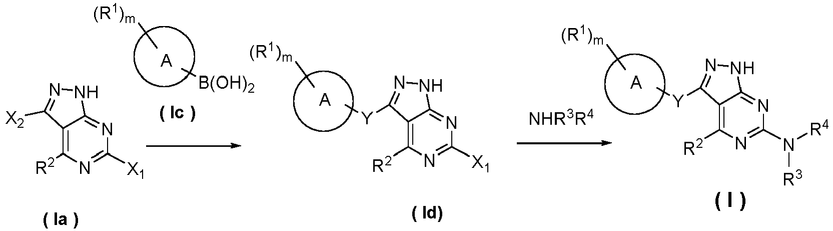 subjecting the compound represented by general formula (Ia) and the compound represented by general formula (Ic) to a Suzuki reaction in the presence of palladium catalyst and alkaline condition, to obtain the compound represented by general formula (Id); and subjecting the compound represented by general formula (Id) and NHR³R⁴ to a nucleophilic substitution reaction under alkaline condition, the protecting group of the obtained compound is removed to obtain the compound represented by general formula (I); wherein:
Y is selected from chemical bond;
X₁ is selected from leaving group, wherein the leaving group is selected from halogen or SO₂R^{t};
X₂ is selected from halogen;
R^{t} is selected from alkyl; and
ring A, m, and R¹-R⁴ are defined as in general formula (I).

Further, the present invention provides a compound represented by general formula (Ia) or a stereoisomer or a tautomer thereof, which is an intermediate for preparing a compound represented by general formula (I): 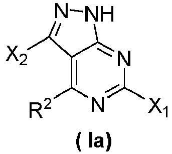 wherein:
X₁ is selected from leaving group, wherein the leaving group is selected from halogen or SO₂R^{t};
X₂ is selected from halogen;
R^{t} is selected from alkyl; and
R² is selected from cyano, tetrazolyl, -C(O)R⁵, -C(O)OR⁵ or -C(O)NR⁶R⁷;
R⁵, R⁶ and R⁷ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, amino, halogen, nitro, cyano, alkyl, alkoxy, cycloalkyl, heterocyclyl, aryl, heteroaryl, -C(O)R⁸, -C(O)OR⁸, - OC(O)R⁸, -SO₂R⁸, -NR⁹R¹⁰, -C(O)NR⁹R¹⁰, -SO₂NR⁹R¹⁰ or -NR⁹C(O)R¹⁰;
alternatively, R⁶ and R⁷ together with the N atom bound therewith form a 3-8 membered heterocyclyl, wherein the 3-8 membered heterocyclyl internally contains one or more N, O, S or SO₂ atoms, and the 3-8 membered heterocyclyl is optionally further substituted by one or more substituents selected from hydroxy, halogen, amino, alkyl or alkoxy; and
R⁸, R⁹ and R¹⁰ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, halogen, nitro, cyano, alkyl, alkoxy, cycloalkyl, heterocyclyl, aryl, heteroaryl, carboxyl or carboxylate.

Further, the present invention provides a preparation method for the compound represented by general formula (Ia) or the stereoisomer or the tautomer thereof, wherein the method comprises: 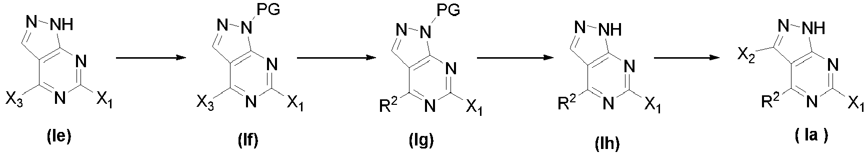 protecting the amino of the compound represented by general formula (Ie) to obtain the compound represented by general formula (If); subjecting the compound represented by general formula (If) to a coupling reaction under the action of palladium catalysts to obtain the compound represented by general formula (Ig); removing the protecting group PG from the compound represented by general formula (Ig) to obtain the compound represented by general formula (Ih); and subjecting the compound represented by general formula (Ih) to a halogenating reaction to obtain the compound represented by general formula (Ia); wherein:
PG is the protecting group, preferably 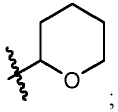
X₃ is selected from halogen; and
X₁, X₂ and R² are as defined in general formula (Ia).

Further, the present invention provides a pharmaceutical composition, wherein the pharmaceutical composition comprises an effective dose of the compound represented by general formula (AI), (AII), (I), (II), (III), (IV), (V) or (VI) or the stereoisomer or the tautomer thereof, or the pharmaceutically acceptable salt thereof, and a pharmaceutically acceptable carrier, an excipient or a combination thereof.

The present invention provides use of the compound represented by general formula (AI), (AII), (I), (II), (III), (IV), (V) or (VI) or the stereoisomer or the tautomer thereof, or the pharmaceutically acceptable salt thereof, or the pharmaceutical composition thereof in preparing a SHP2 allosteric inhibitor.

The present invention also provides use of the compound represented by general formula (AI), (AII), (I), (II), (III), (IV), (V) or (VI) or the stereoisomer or the tautomer thereof, or the pharmaceutically acceptable salt thereof, or the pharmaceutical composition thereof in preparing a medicament for treating a disease mediated by SHP2, wherein the disease mediated by SHP2 is preferably cancer, cancerometastasis, cardiovascular disease, immune disorder, fibrosis or visual disorder; wherein the disease mediated by SHP2 is preferably selected from Noonan syndrome, Leopard spot syndrome, juvenile myelomonocytic leukemia, neuroblastoma, melanoma, acute myeloid leukemia, breast cancer, esophagus cancer, lung cancer, colon cancer, head cancer, neuroblastoma, squamous cell carcinoma of head and neck, gastric cancer, anaplastic large cell lymphoma and glioblastoma.

The present invention further provides use of the compound represented by general formula (AI), (AII), (I), (II), (III), (IV), (V) or (VI) or the stereoisomer or the tautomer thereof, or the pharmaceutically acceptable salt thereof, or the pharmaceutical composition thereof in preparing a medicament for treating cancer, cancerometastasis, cardiovascular disease, immune disorder, fibrosis or visual disorder.

The present invention provides use of the compound represented by general formula (AI), (AII), (I), (II), (III), (IV), (V) or (VI) or the stereoisomer or the tautomer thereof, or the pharmaceutically acceptable salt thereof, or the pharmaceutical composition thereof in preparing a medicament for treating Noonan syndrome, Leopard spot syndrome, juvenile myelomonocytic leukemia, neuroblastoma, melanoma, acute myeloid leukemia, breast cancer, esophagus cancer, lung cancer, colon cancer, head cancer, neuroblastoma, squamous cell carcinoma of head and neck, gastric cancer, anaplastic large cell lymphoma and glioblastoma.

The present invention provides a method for inhibiting a SHP2 receptor in vitro, wherein the method comprises the step of contacting the SHP2 receptor with the compound represented by general formula (AI), (AII), (I), (II), (III), (IV), (V) or (VI) or the stereoisomer or the tautomer thereof, or the pharmaceutically acceptable salt thereof, or the pharmaceutical composition thereof.

The present invention provides a method for treating a disease mediated by SHP2, wherein the method comprises the steps of administering to a patient in need of treatment an effective dose of the compound represented by general formula (AI), (AII), (I), (II), (III), (IV), (V) or (VI) or the stereoisomer or the tautomer thereof, or the pharmaceutically acceptable salt thereof, or the pharmaceutical composition thereof; wherein the disease mediated by SHP2 is preferably cancer, cancerometastasis, cardiovascular disease, immune disorder, fibrosis or visual disorder; wherein the disease mediated by SHP2 is more preferably selected from Noonan syndrome, Leopard spot syndrome, juvenile myelomonocytic leukemia, neuroblastoma, melanoma, acute myeloid leukemia, breast cancer, esophagus cancer, lung cancer, colon cancer, head cancer, neuroblastoma, squamous cell carcinoma of head and neck, gastric cancer, anaplastic large cell lymphoma and glioblastoma.

### Detailed description of the invention

Unless stated to the contrary, some terms used in the specification and claims of the present invention are defined as follows:
"Alkyl", when regarded as a group or a part of a group, means to include C₁-C₂₀ linear chain or branched aliphatic hydrocarbon groups. It is preferably C₁-C₁₀ alkyl, and more preferably C₁-C₆ alkyl. Examples of alkyls include, but are not limited to, methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, or the like. The alkyl may be substituted or unsubstituted.
"Alkenyl" refers to an alkyl as defined above consisting of at least two carbon atoms and at least one carbon-carbon double bond, representative examples of which comprise but are not limited to ethenyl, 1-propenyl, 2-propenyl, 1-, 2- or 3-butenyl, or the like. The alkenyl may also be substituted or unsubstituted.
"Alkynyl" refers to an aliphatic hydrocarbon group with one carbon-carbon triple bond, which may be a linear chain or branched chain. Preferably, C₂-C₁₀ alkynyl, more preferably C₂-C₆ alkynyl, and most preferably C₂-C₄ alkynyl. Examples of alkynyl groups comprise, but are not limited to, ethynyl, 1-propynyl, 2-propynyl, 1-, 2- or 3-butynyl, or the like. The alkynyl may be substituted or unsubstituted
"Cycloalkyl" refers to saturated or partially saturated monocyclic, fused, bridged and spirocyclic carbocycles. Preferably, C₃-C₁₂ cycloalkyl, more preferably C₃-C₈ cycloalkyl, and most preferably C₃-C₆ cycloalkyl. Examples of monocyclic cycloalkyl comprise but are not limited to cyclopropyl, cyclobutyl, cyclopentyl, cyclopentenyl, cyclohexyl, cyclohexenyl, cyclohexadienyl, cycloheptyl, cyclohepttrienyl, cyclooctyl, or the like, and cyclopropyl and cyclohexenyl are preferred. The cycloalkyl may be optionally substituted or unsubstituted.
"Spirocycloalkyl" refers to a 5-18 membered polycyclic group with two or more cyclic structures, and single rings share one carbon atom (called spiro atom) with each other. The ring contains one or more double bonds, but none of the rings has a completely conjugated π electron aromatic system. Preferably, 6-14 membered, and more preferably 7-10 membered. According to the number of spiro atoms shared between rings, the spirocycloalkyl may be classified into mono-spiro, di-spiro or multi-spiro-cycloalkyls, preferably mono-spiro and di-spiro-cycloalkyls, and preferably 4 membered /5 membered, 4 membered /6 membered, 5 membered /5 membered, or 5 membered /6 membered. Non-limiting examples of "spirocycloalkyl" comprise, but are not limited to, spiro[4.5]decyl, spiro[4.4]nonyl, spiro[3.5]nonyl, and spiro[2.4]heptyl.
"Fused cycloalkyl" refers to a 5-18 membered all-carbon polycyclic group with two or more cyclic structures sharing a pair of carbon atoms, and one or more rings may contain one or more double bonds, but none of the rings has a completely conjugated π electron aromatic system. The fused acycloalkyl is preferably 6-12 membered, and more preferably 7-10 membered. According to the number of constituent rings, fused cycloalkyl may be classified into bicyclic, tricyclic, tetracyclic or polycyclic fused cycloalkyls, preferably bicyclic or tricyclic, and more preferably 5 membered /5 membered or 5 membered /6 membered bicycloalkyl.Non-limiting examples of "fused cycloalkyl" comprise, but are not limited to, bicyclo[3.1.0]hexyl, bicyclo[3.2.0]heptyl-1-alkenyl, bicyclo[3.2.0]heptyl, decalinyl or tetradecahydrophenanthryl.
"Bridged cycloalkyl" refers to a 5-18 membered all-carbon polycyclic group with two or more cyclic structures sharing two carbon atoms that are not directly bound with each other, one or more rings may contain one or more double bonds, but none of the rings has a completely conjugated π electron aromatic system. The bridged cycloalkyl is preferably 6-12 membered, and more preferably 7-10 membered. It is preferably 6-14 membered, and more preferably 7-10 membered. According to the number of constituent rings, it may be classified into bicyclic, tricyclic, tetracyclic or polycyclic bridged cycloalkyls, preferably bicyclic, tricyclic or tetracyclic, and more preferably bicyclic or tricyclic. Non-limiting examples of "bridged cycloalkyl" comprise, but are not limited to, (1s,4s)-bicyclo[2.2.1]heptyl, bicyclo[3.2.1]octyl, (1s,5s)-bicyclo[3.3.1]nonyl, bicyclo[2.2.2]octyl, and (1r,5r)-bicyclo[3.3.2]decyl.
"Heterocyclyl", "heterocycle" or "heterocyclic" are used interchangeably in this application, and all refer to non-aromatic heterocyclyls, wherein one or more ring-forming atoms are heteroatoms, such as oxygen, nitrogen, sulfur atoms, or the like, comprising monocyclic ring, polycyclic ring, fused ring, bridged ring and spiro. Preferably having a 5-7 membered monocyclic ring or a 7-10 membered bicyclic or tricyclic ring, which may contain 1, 2 or 3 atoms selected from nitrogen, oxygen and/or sulfur. Examples of "heterocyclyl" comprise but are not limited to morpholinyl, oxetanyl, thiomorpholinyl, tetrahydropyranyl, 1,1-dioxo-thiomorpholinyl, piperidinyl, 2-oxo-piperidinyl, pyrrolidinyl, 2-oxo-pyrrolidinyl, piperazine-2-one, 8-oxa-3-aza-bicyclo[3.2.1]octyl, piperazinyl, 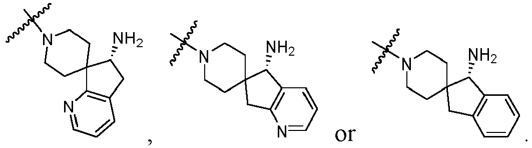 The heterocyclyl may be substituted or unsubstituted.
"Spiroheterocyclyl" refers to a 5-18 membered polycyclic group with two or more cyclic structures, and single rings share one atom with each other. The ring contains one or more double bonds, but none of the rings has a completely conjugated π electron aromatic system, wherein one or more ring atoms are selected from heteroatoms of nitrogen, oxygen or S(O)ₙ (wherein n is selected from 0, 1 or 2), and the remaining ring atoms are carbon. It is preferably 6-14 membered, and more preferably 7-10 membered. According to the number of spiro atoms shared between rings, the spirocycloalkyl may be classified into mono-spiroheterocyclyl, bi-spiroheterocyclyl or multi-spiroheterocyclyl, preferably mono-spiroheterocyclyl and bi-spiroheterocyclyl. Preferably a 4 membered /4 membered, 4 membered /5 membered, 4 membered /6 membered, 5 membered/5 membered, or 5 membered /6 membered mono-spiroheterocyclyl. Non-limiting examples of "spiroheterocyclyl" comprise, but are not limited to: 1,7-dioxaspiro[4.5]decyl, 2-oxa-7-azaspiro[4.4]nonyl, 7-oxaspiro[3.5]nonyl, 5-oxaspiro[2.4]heptyl, 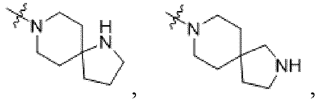 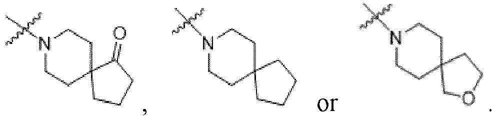
"Fused heterocyclyl" refers to an all-carbon polycyclic group with two or more cyclic structures sharing a pair of atoms, and one or more rings may contain one or more double bonds, but none of the rings has a completely conjugated π electron aromatic system, wherein one or more ring atoms are selected from heteroatoms of nitrogen, oxygen or S(O)ₙ (wherein n is selected from 0, 1 or 2), and the remaining ring atoms are carbon. Preferably 6-14 membered, and more preferably 7-10 membered. According to the number of constituent rings, fused heterocyclyl may be classified into bicyclic, tricyclic, tetracyclic or polycyclic fused heterocyclyls, preferably bicyclic or tricyclic, and more preferably 5 membered /5 membered or 5 membered /6 membered bicyclic fused heterocyclyl. Non-limiting examples of "fused heterocyclyl" comprise, but are not limited to: octahydropyrrolo[3,4-c]pyrrolyl, octahydro-1H-isoindolyl, 3-azabicyclo[3.1.0]hexyl, octahydrobenzo[b][1,4]dioxine (dioxine) and 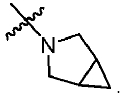
"Bridged heterocyclyl" refers to a 5-14 membered or 5-18 membered polycyclic group with two or more cyclic structures sharing two atoms that are not directly bound with each other. One or more rings may contain one or more double bonds, but none of the rings has a completely conjugated π electron aromatic system, wherein one or more ring atoms are selected from heteroatoms of nitrogen, oxygen or S(O)ₙ (wherein n is selected from 0, 1 or 2), and the remaining ring atoms are carbon. It is preferably 6-14 membered, and more preferably 7-10 membered. According to the number of constituent rings, bridged heterocyclyl may be classified into bicyclic, tricyclic, tetracyclic or polycyclic bridged heterocyclyls, preferably bicyclic, tricyclic or tetracyclic, and more preferably bicyclic or tricyclic. Non-limiting examples of "bridged heterocyclyl" comprise, but are not limited to, 2-azabicyclo[2.2.1]heptyl, 2-azabicyclo[2.2.2]octyl, 2-azabicyclo[3.3.2]decyl, 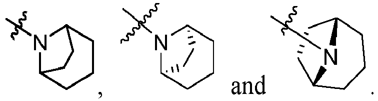
"Aryl" refers to a carbocyclic aromatic system containing one or two rings, wherein the rings may be bound together in a fused manner. The term "aryl" comprises monocyclic or bicyclic aryls, such as aromatic groups of phenyl, naphthyl, and tetrahydronaphthyl. The aryl may be substituted or unsubstituted.
"Heteroaryl" refers to a 5-6 membered monocyclic ring or a 8-10 membered bicyclic ring, which may contain 1 to 4 atoms selected from nitrogen, oxygen and/or sulfur. Examples of "heteroaryl" comprise but are not limited to, furanyl, pyridinyl, 2-oxo-1,2-dihydropyridyl, pyridazinyl, pyrimidinyl, pyrazinyl, thienyl, isoxazolyl, oxazolyl, oxadiazolyl, imidazolyl, pyrrolyl, pyrazolyl, triazolyl, tetrazolyl, thiazolyl, isothiazolyl, 1,2,3-thiadiazolyl, benzodioxolyl, benzothienyl, benzimidazolyl, indolyl, isoindolyl, 1,3-dioxo-isoindolyl, quinolinyl, indazolyl, benzisothiazolyl, benzoxazolyl, and benzisoxazolyl, 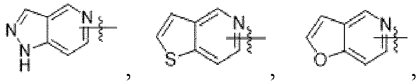 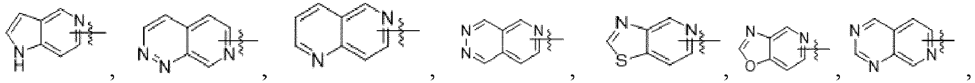 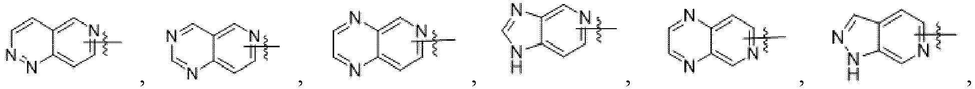 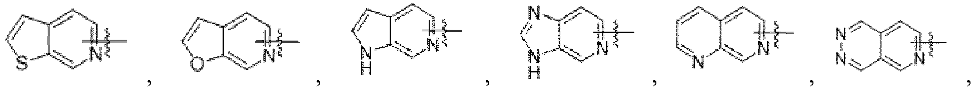 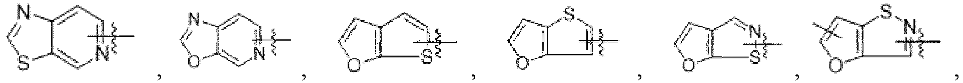 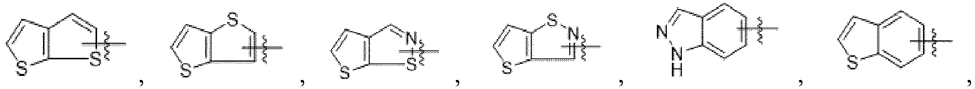 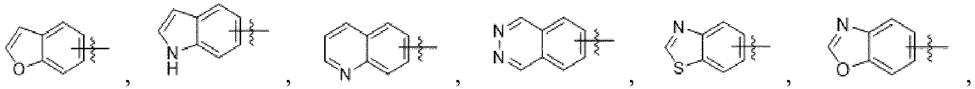 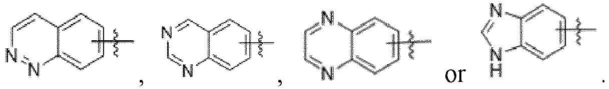 The heteroaryl may be substituted or unsubstituted.
"Fused ring" refers to a polycyclic group with two or more cyclic structures sharing a pair of atoms with each other. One or more rings may contain one or more double bonds, but at least one ring does not have a completely conjugated π electron aromatic system, and meanwhile, at least one ring has a completely conjugated π electron aromatic system, wherein zero, one or more ring atoms are selected from heteroatoms of nitrogen, oxygen or S(O)ₙ (wherein n is selected from 0, 1 or 2), and the remaining ring atoms are carbon. The fused ring is preferably a bicyclic or tricyclic fused ring, wherein the bicyclic fused ring is preferably a fused ring of aryl or heteroaryl and monocyclic heterocyclyl or monocyclic cycloalkyl. Preferably 7-14 membered, and more preferably 8-10 membered. Examples of "fused ring" comprise, but are not limited to: 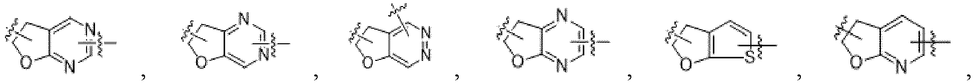 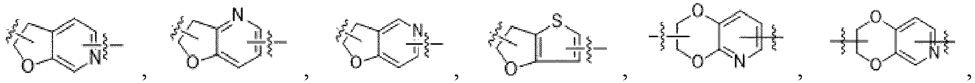 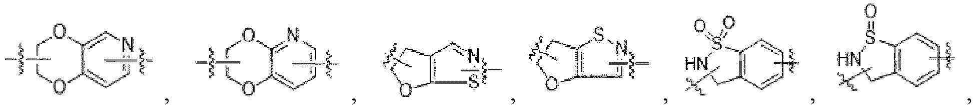 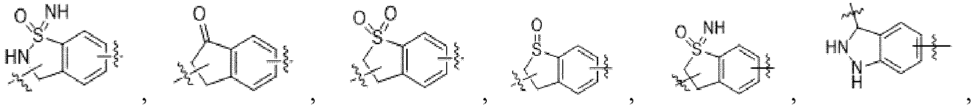 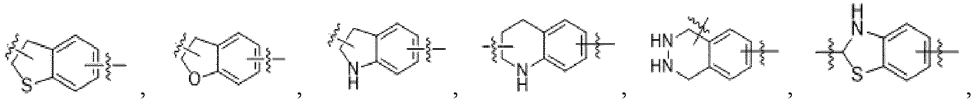 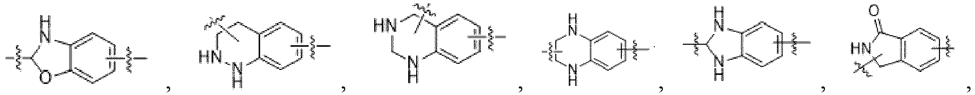 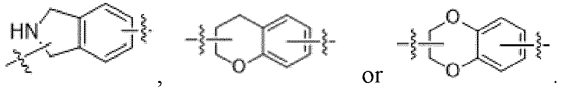
"Alkoxy" refers to a group of (alkyl -O-), wherein, the alkyl is defined herein. C₁-C₆ alkoxy is preferred. Examples of such alkoxy comprise, but are not limited to, methoxy, ethoxy, n-propoxy, isopropoxy, n-butoxy, isobutoxy, tert-butoxy, and the like.
"Hydroxy" refers to an -OH group.
"Halogen" refers to fluorine, chlorine, bromine and iodine.
"Amino" refers to -NH₂.
"Cyano" refers to -CN.
"Nitro" refers to -NO₂.
"Benzyl" and "Bn" refer to -CH₂- phenyl.
"Carboxyl" refers to -C(O)OH.
"Carboxylate" refers to -C(O)O-alkyl or -C(O)O-cycloalkyl, wherein the definitions of the alkyl and the cycloalkyl are as above.
"DMSO" refers to dimethyl sulfoxide.
"BOC" refers to tert-butoxycarbonyl.
"TFA" refers to trifluoroacetic acid.
"Ts" refers to p-toluenesulfonyl.
"Hydroxy C₁-C₄ alkyl" refers to a C₁-C₄ alkyl substituted by hydroxy.
"Amino C₁-C₄ alkyl" refers to a C₁-C₄ alkyl substituted by amino.
"Leaving group", is an atom or functional group separated from a larger molecule in chemical reaction, which is a term used in nucleophilic substitution reaction and elimination reaction. In nucleophilic substitution reaction, a reactant attacked by a nucleophilic reagent is called substrate, while an atom or atomic group broken away with a pair of electrons in the substrate molecule is called leaving group. A group that accepts electrons easily and has strong ability of bearing negative charges is a good leaving group. When the pKa of a conjugate acid of the leaving group is smaller, it is easier for the leaving group to separate from other molecules. The reason is that when the pKa of the conjugated acid of the leaving group is smaller, the corresponding leaving group does not need to be combined with other atoms, and the tendency to exist in the form of anions (or electrically neutral leaving group) is enhanced. Common leaving groups comprise but are not limited to, halogen, mesyl, -OTs or -OH.
"Substituted" means that one or more hydrogen atoms in a group, preferably at most 5, more preferably 1 to 3 hydrogen atoms, are independently replaced by a corresponding number of substituents. Obviously, substituents are only in their possible chemical positions, and those skilled in the art can determine (through experiments or theories) possible or impossible substitutions without going through much effort. For example, amino or hydroxy with free hydrogen may be unstable when combined with carbon atoms with unsaturated (e.g., olefinic) bonds.

As used in this specification, "substitute" or "substituted", unless otherwise specified, means that a group may be substituted by one or more groups selected from the following: alkyl, alkenyl, alkynyl, alkoxy, alkylthio, alkylamino, halogen, mercapto, hydroxy, nitro, cyano, cycloalkyl, heterocyclic, aryl, heteroaryl, cycloalkoxy, heterocyclic alkoxy, cycloalkylthio, heterocycloalkylthio, amino, haloalkyl, hydroxyalkyl, carboxyl, carboxylate, =O, -OR⁵, -C(O)R⁵, -C(O)OR⁵, -OC(O)R ⁵, -SO₂R⁵, -NR⁶R⁷, -SO₂NR⁶R⁷, -NHC(=NH)NH₂, -NHSO₂R⁵ or - C(O)NR⁶R⁷;
R⁵, R⁶ and R⁷ are each independently selected from hydrogen atom, alkyl, cycloalkyl or heterocyclyl, wherien the alkyl, cycloalkyl or heterocyclyl is optionally further substituted by one or more substituents selected from hydroxy, amino, halogen, nitro, cyano, alkyl, alkoxy, cycloalkyl, heterocyclyl, aryl, heteroaryl, -C(O)R⁸, -C(O)OR⁸, -OC(O)R⁸, -SO₂R⁸, -NR⁹R¹⁰, -C(O)NR⁹R¹⁰, - SO₂NR⁹R¹⁰ or -NR⁹C(O)R¹⁰;
alternatively, R⁶ and R⁷ together with the N atom bound therewith form a 3-8 membered heterocyclyl, wherein the 3-8 membered heterocyclyl internally contains one or more N, O, S or SO₂ atoms, and the 3-8 membered heterocyclyl is further substituted by one or more substituents selected from hydroxy, halogen, amino, alkyl or alkoxy; and
R⁸, R⁹ and R¹⁰ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, halogen, nitro, cyano, alkyl, alkoxy, cycloalkyl, heterocyclyl, aryl, heteroaryl, carboxyl or carboxylate.
"Pharmaceutically acceptable salts" refers to some salts of the above-mentioned compounds which can keep the original biological activity and are suitable for medical use. The pharmaceutically acceptable salt of a compound represented by general formula (I) may be a metal salt, an amine salt formed by a suitable acid.
"Pharmaceutical composition" represents a mixture containing one or more compounds described herein or physiologically acceptable salts or prodrugs thereof and other chemical components, as well as other components such as physiologically acceptable carriers and excipients. The object of the pharmaceutical composition is to promote the administration to organisms and facilitate the absorption of active ingredients to exert biological activity.

### Synthesis methods of the compounds of the present invention

In order to achieve the objects of the present invention, the following technical solutions are adopted by the present invention.

The present invention provides a preparation method for a compound represented by general formula (I) or a stereoisomer or a tautomer thereof, or a pharmaceutically acceptable salt thereof, wherein the method comprises: 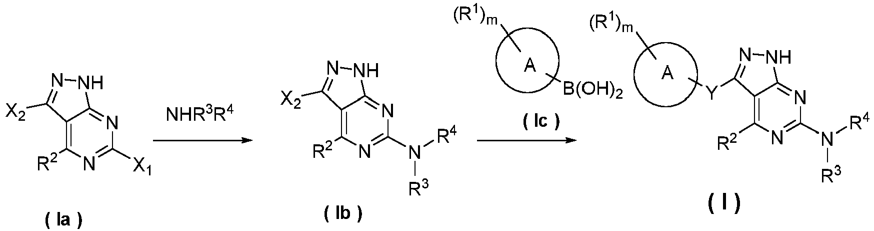 subjecting the compound represented by general formula (Ia) and NHR³R⁴ to a nucleophilic substitution reaction under alkaline condition to obtain the compound represented by general formula (Ib); and subjecting the compound represented by general formula (Ib) and the compound represented by general formula (Ic) to a Suzuki reaction in the presence of palladium catalyst and alkaline condition, and optionally further removing a protecting group of the obtained compound to obtain the compound represented by general formula (I);
wherein:
Y is selected from chemical bond;
X₁ is selected from leaving group, wherein the leaving group is selected from halogen or - SO₂R^{t};
X₂ is selected from halogen;
R^{t} is selected from alkyl; and
ring A, m, and R¹-R⁴ are defined as in general formula (I).

The present invention provides a preparation method for a compound represented by general formula (I) or a stereoisomer or a tautomer thereof, or a pharmaceutically acceptable salt thereof, wherein the method comprises: 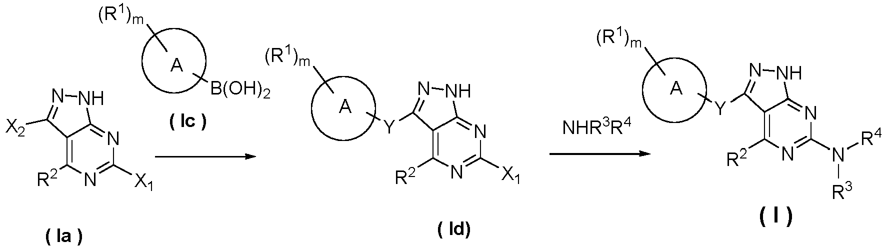 subjecting the compound represented by general formula (Ia) and the compound represented by general formula (Ic) to a Suzuki reaction in the presence of palladium catalyst and alkaline condition, to obtain the compound represented by general formula (Id); and subjecting the compound represented by general formula (Id) and NHR³R⁴ to a nucleophilic substitution reaction under alkaline condition, and further removing a protecting group from the obtained compound to obtain the compound represented by general formula (I);
wherein:
Y is selected from chemical bond;
X₁ is selected from leaving group, wherein the leaving group is selected from halogen or - SO₂R^{t};
X₂ is selected from halogen;
R^{t} is selected from alkyl; and
ring A, m, and R¹-R⁴ are defined as in general formula (I).

The present invention provides a preparation method for a compound of general formula (II) or a stereoisomer or a tautomer thereof or a pharmaceutically acceptable salt thereof, wherein the method comprises: 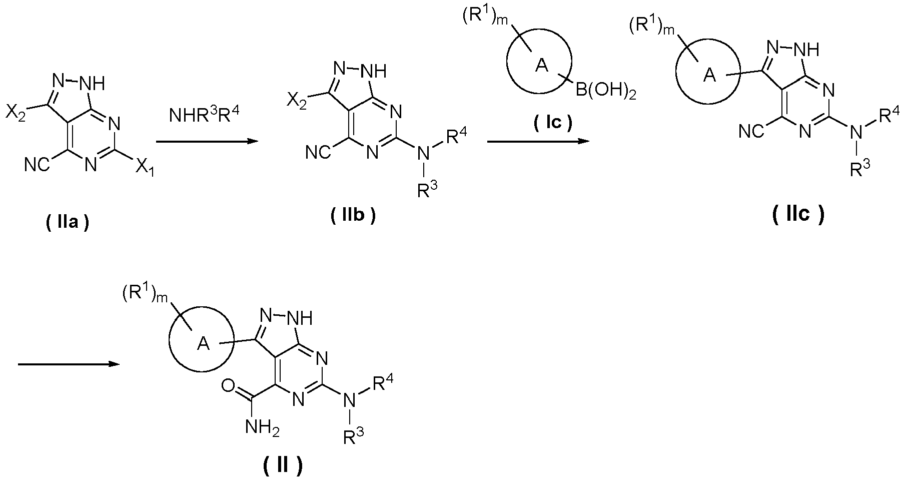 subjecting the compound represented by general formula (IIa) and NHR³R⁴ to a nucleophilic substitution reaction under alkaline condition to obtain the compound represented by general formula (IIb); subjecting the compound represented by general formula (IIb) and the compound represented by general formula (Ic) to a Suzuki reaction in the presence of palladium catalyst and alkaline condition, and optionally further removing a protecting group from the obtained compound to obtain the compound represented by general formula (IIc); and hydrolyzing the compound represented by general formula (IIc) under the condition of a sodium hydroxide solution to obtain the compound represented by general formula (II);
wherein:
X₁ is selected from leaving group, wherein the leaving group is selected from halogen or - SO₂R^{t};
X₂ is selected from halogen;
R^{t} is selected from alkyl; and
ring A, m, R¹, R³ and R⁴ are defined as in general formula (II).

The present invention provides a preparation method for a compound of general formula (II) or a stereoisomer or a tautomer thereof or a pharmaceutically acceptable salt thereof, wherein the method comprises: 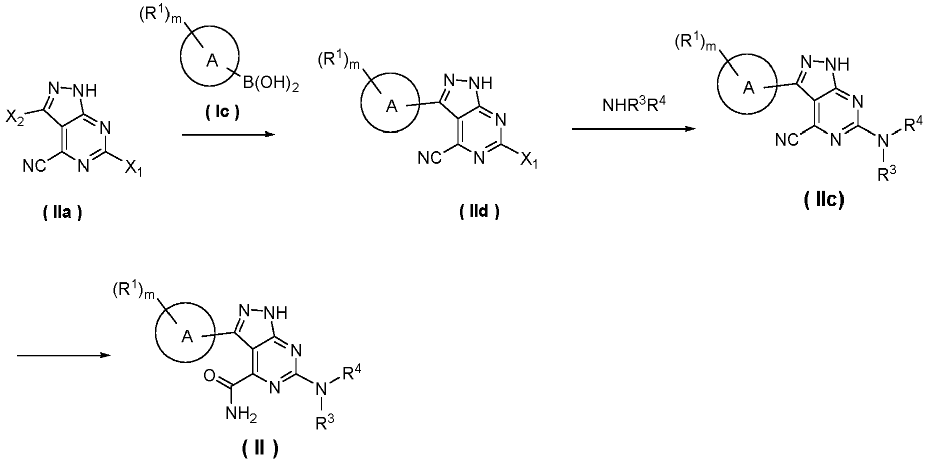 subjecting the compound represented by general formula (IIa) and the compound represented by general formula (Ic) to a Suzuki reaction in the presence of palladium catalyst and alkaline condition, to obtain the compound represented by general formula (IId); subjecting the compound represented by general formula (IId) and NHR³R⁴ to a nucleophilic substitution reaction under alkaline condition to obtain the compound represented by general formula (IIc); and hydrolyzing the compound represented by general formula (IIc) under the condition of a sodium hydroxide solution to obtain the compound represented by general formula (II);
wherein:
X₁ is selected from leaving group, wherein the leaving group is selected from halogen or - SO₂R^{t};
X₂ is selected from halogen;
R^{t} is selected from alkyl; and
ring A, m, R¹, R³ and R⁴ are defined as in general formula (II).

### EMBODIMENTS

The following examples are used to further describe the present invention, but these examples do not limit the scope of the present invention.

### Examples

The examples show the preparation of representative compounds represented by formula (I) and related structural identification data. It should be noted that the following examples are only used to illustrate the present invention, but not to limit the present invention. ¹H NMR spectrum was measured by Bruker instrument (400 MHz), and chemical shift was expressed in ppm. Tetramethylsilane internal standard (0.00 ppm) was employed. ¹H NMR was expressed as follows: s = singlet, d = doublet, t = triplet, m = multiplet, br = broadened, dd = doublet of doublets, and dt = doublet of triplets. If a coupling constant was provided, it was in the unit of Hz.

A mass spectrum was determined by LC/MS, and an ionization method may be ESI or APCI.

Yantai Huanghai HSGF254 or Qingdao GF254 silica gel plates were used as silica gel plates for thin layer chromatography. The silica gel plates used for thin layer chromatography (TLC) had a specification of 0.15 mm to 0.2 mm, and products separated and purified by TLC had a specification of 0.4 mm to 0.5 mm.

In general, Yantai Huanghai silica gel with 200-300 meshes was used as a carrier for column chromatography.

In the following examples, unless otherwise specified, all temperatures are in Celsius. Unless otherwise specified, various starting materials and reagents are commercially available or synthesized according to known methods, and the commercially available materials and reagents are directly used without further purification. Unless otherwise specified, the commercially available manufacturers include but are not limited to Aldrich Chemical Company, ABCR GmbH & Co.KG, Acros Organics, Guangzan Chemical Science and Technology Ltd., Jingyan Chemical Science and Technology Ltd., and the like.
CD₃OD: Methanol-d4.
CDCl₃: Chloroform-d.
DMSO-*d₆*: Dimethyl sulfoxide-d6.

Argon atmosphere refers to that a reaction flask is connected with an argon balloon with a volume of about 1 L.

Unless otherwise specified in the examples, a solution in the reaction refers to an aqueous solution.

The compounds were purified by a silica gel column chromatography eluent system and thin layer chromatography, wherein the eluent system was selected from A: petroleum ether and ethyl acetate system; B: dichloromethane and methanol system; and C: dichloromethane and ethyl acetate system. The volume ratios of the solvents varied according to the polarity of the compounds, and may also be adjusted by adding a small amount of acidic or basic reagents, such as acetic acid or triethylamine, or the like.

### Example 1

### 6-(4-amino-4-methylpiperidin-1-yl)-3-(2,3-dichlorophenyl)-1H-pyrazolo[3,4-d]pyrimidine-4-carbonitrile

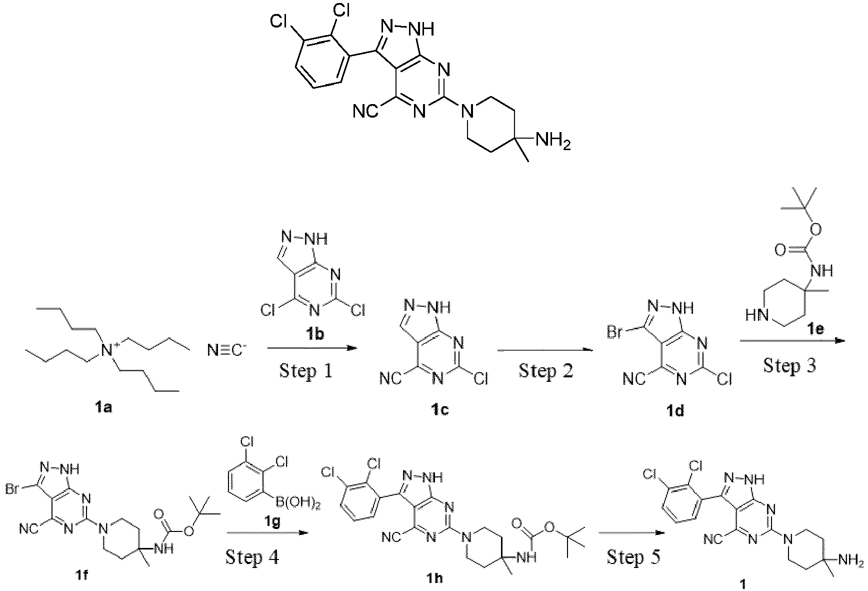

### Step 1

### 6-chloro-1H-pyrazolo[3,4-d]pyrimidine-4-carbonitrile

Tetrabutylammonium cyanide **1a** (2.81 g, 10.48 mmol), 4,6-dichloro-1H-pyrazolo[3,4-d]pyrimidine **1b** (1.8 g, 9.52 mmol) and triethylene diamine (213.65 mg, 1.90 mmol) were added to dichloromethane (30 mL) in turn, and continuously stirred for 2 hours at room temperature. After the reaction was completed, the reaction solution was concentrated under reduced pressure. The obtained residue was further analyzed and purified by silica gel column chromatography (eluent: system B), and a small amount of triethylamine was added to the system to obtain 6-chloro-1H-pyrazolo[3,4-d]pyrimidine-4-carbonitrile **1c** (741 mg) with a yield of 43.33%.
MS m/z (ESI): 179.9 [M+1]

### Step 2

### 3-bromo-6-chloro-1H-pyrazolo[3,4-d]pyrimidine-4-carbonitrile

6-chloro-1H-pyrazolo[3,4-d]pyrimidine-4-carbonitrile **1c** (200 mg, 1.11 mmol) and N-bromosuccinimide (218.06 mg, mmol) were added to acetonitrile (4 mL) in turn, heated to 90°C, and reacted for 4 hours. After the reaction was completed, the reaction solution was concentrated under reduced pressure. The obtained residue was further analyzed and purified by silica gel column chromatography (eluent: system B) to obtain 3-bromo-6-chloro-1H-pyrazolo[3,4-d]pyrimidine-4-carbonitrile **1d** (280 mg) with a yield of 97.26%.
MS m/z (ESI): 257.7 [M+1]

### Step 3

### tert-butyl N-[1-(3-bromo-4-cyano-1H-pyrazolo[3,4-d]pyrimidin-6-yl)-4-methyl-4-piperidinyl] carbamate

3-bromo-6-chloro-1H-pyrazolo[3,4-d]pyrimidine-4-carbonitrile **1d** (200 mg, 773.81 µmol), tert-butyl N-(4-methyl-4-piperidinyl) carbamate **1e** (248.74 mg, 1.16 mmol) and diisopropylethylamine (600.04 mg, 4.64 mmol, 810.87 uL) were added to N-methyl pyrrolidone (3 mL) in turn, heated to 110°C, and reacted for 6 hours. After the reaction was completed, the reaction solution was added with 30 mL of ethyl acetate and 15 mL of water for liquid separation and extraction to separate the aqueous layer, then organic phases were washed with a saturated sodium chloride solution (10 mL×2) in turn, and concentrated under reduced pressure. The obtained residue was separated and purified by silica gel column chromatography (eluent: system B) to obtain tert-butyl N-[1-(3-bromo-4-cyano-1H-pyrazolo[3,4-d]pyrimidin-6-yl)-4-methyl-4-piperidinyl] carbamate **1f** (230 mg) with a yield of 68.12%.
MS m/z (ESI): 436.1 [M+1]

### Step 4

### Tert-butyl N-[1-(4-cyano-3-(2,3-dichlorophenyl)-1H-pyrazolo[3,4-d]pyrimidin-6-yl)-4-methyl-4-piperidinyl) carbamate

Under the protection of argon gas, tert-butyl N-[1-(3-bromo-4-cyano-1H-pyrazolo[3,4-d]pyrimidin-6-yl)-4-methyl-4-piperidinyl] carbamate **1f** (230 mg, 527.15 µmol), (2,3-dichlorophenyl)boronic acid **1g** (150.89 mg, 790.73 µmol), methanesulfonato(2-dicyclohexylphosphino-2',6'-di-isopropoxy-1,1'-biphenyl)(2'-amino-1,1'-biphenyl-2-yl)palladium (88.25 mg, 105.43 µmol), potassium phosphate (335.27 mg, 1.58 mmol) and 2-dicyclohexylphosphino-2',6'-di-isopropoxy-1,1'-biphenyl (98.26 mg, 210.86 µmol) were added to 6 mL of mixed solution (1,4-dioxane: water = 5: 1) in turn, heated to 120°C, and reacted for 6 hours. After the reaction was completed, the reaction solution was added with 15 mL of water and 30 mL of ethyl acetate for liquid separation and extraction, then organic phases were washed with 10 mL of saturated salt solution, and concentrated under reduced pressure. The obtained residue was further separated and purified by silica gel column chromatography (eluent: system A) to obtain tert-butyl N-[1-[4-cyano-3-(2,3-dichlorophenyl)-1H-pyrazolo[3,4-d]pyrimidin-6-yl)-4-methyl-4-piperidinyl] carbamate **1h** (143 mg) with a yield of 53.99%.
MS m/z (ESI): 502.0 [M+1]

### Step 5

### 6-(4-amino-4-methylpiperidin-1-yl)-3-(2,3-dichlorophenyl)-1H-pyrazolo[3,4-d]pyrimidine-4-carbonitrile

tert-butyl N-[1-[4-cyano-3-(2,3-dichlorophenyl)-1H-pyrazolo[3,4-d]pyrimidin-6-yl)-4-methyl-4-piperidinyl] carbamate **1h** (140 mg, 278.67 µmol) and trifluoroacetic acid (1.53 g, 13.42 mmol, 1 mL) were added to dichloromethane (3 mL) in turn, and reacted at room temperature for 6 hours. After the reaction was completed, a saturated sodium carbonate solution was added slowly dropwise to the reaction solution to adjust the pH to be 8, and then concentrated under reduced pressure. 30 mL of ethyl acetate and 15 mL of water were added to the residue for liquid separation and extraction, then organic phases were washed with 10 mL of saturated salt water, and concentrated under reduced pressure. The obtained residue was further separated and purified by silica gel column chromatography (eluent: system A) to obtain the target product 6-(4-amino-4-methylpiperidin-1-yl)-3-(2,3-dichlorophenyl)-1H-pyrazolo[3,4-d]pyrimidine-4-carbonitrile 1 (70 mg) with a yield of 47.92%.
MS m/z (ESI): 401.9 [M+1]

### Example 2

### 6-(4-amino-4-methylpiperidin-1-yl)-3-(2,3-dichlorophenyl)-1H-pyrazolo[3,4-d]pyrimidine-4-carboxamide

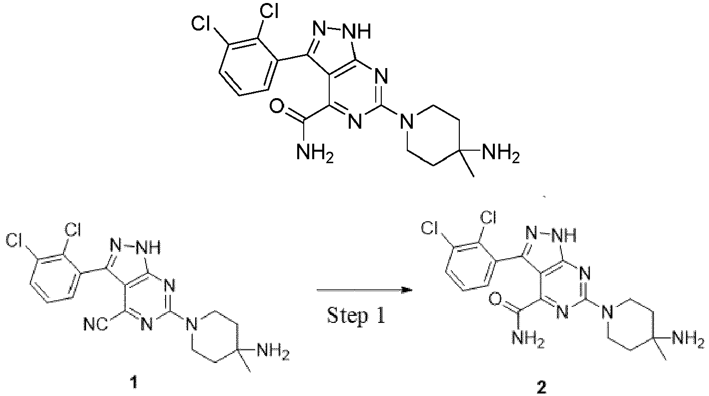

### Step 1

### 6-(4-amino-4-methylpiperidin-1-yl)-3-(2,3-dichlorophenyl)-1H-pyrazolo[3,4-d]pyrimidine-4-carboxamide

6-(4-amino-4-methylpiperidin-1-yl)-3-(2,3-dichlorophenyl)-1H-pyrazolo[3,4-d]pyrimidine-4-carbonitrile **1** (65 mg, 125.90 µmol) and 6 M sodium hydroxide solution (0.5 mL) were added to 2 mL of ethanol in turn, heated to 80°C, and reacted for 3 hours. After the reaction was completed, trifluoroacetic acid was slowly added dropwise in the reaction solution to adjust the pH to be 5, and then concentrated under reduced pressure. The obtained residue was subjected to liquid phase separation (separation column AKZONOBEL Kromasil; 250×21.2 mm I.D.; 5 µm, 20 mL/min; mobile phase A: 0.05% TFA+ H₂O, mobile phase B: CH₃CN) to obtain the target product 6-(4-amino-4-methylpiperidin-1-yl)-3-(2,3 -dichlorophenyl)-1H-pyrazolo [3,4-d]pyrimidine-4-carboxamide **2** (10 mg) with a yield of 13.80%.
MS m/z (ESI): 421.9 [M+1]
¹H NMR (400 MHz, CD₃OD) δ7.56 (dd, J = 7.6, 2.0 Hz, 1H), 7.38 - 7.31 (m, 2H), 4.64 - 4.60 (m, 2H), 3.59-3.52 (m, 2H), 1.89-1.82 (m, 4H), 1.51 (s, 3H).

### Example 3 and Example 4

### 6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carbonitrile 3

### 6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carboxamide 4

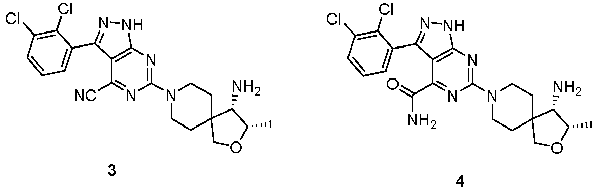

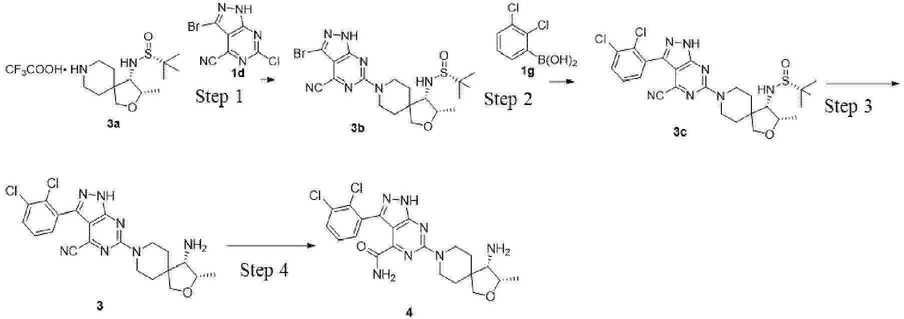

### Step 1

### (R)-N-((3S,4S)-8-(3-bromo-4-cyano-1H-pyrazolo[3,4-d]pyrimidin-6-yl)-3-methyl-2-oxa-8-azaspiro[4.5]decan-4-yl)-2-methylpropane-2-sulfinamide

3-bromo-6-chloro-1H-pyrazolo[3,4-d]pyrimidine-4-carbonitrile **1d** (300 mg, 1.16 mmol), diisopropylethylamine (750 mg, 5.8 mmol, 1.0 mL) and (R)-2-methyl-N-((3S,4S)-3-methyl-2-oxa-8-azaspiro[4.5]decan-4-yl)propane-2-sulfinamide 2,2,2-trifluoroacetate **3a** (478 mg, 1.74 mmol) were added to N-methyl pyrrolidone (5 mL) in turn, heated to 110°C, and reacted for 2 hours. After the reaction was completed, the reaction solution was added with 30 mL of ethyl acetate and 15 mL of water for liquid separation and extraction to separate the aqueous layer, then organic phases were washed with a saturated sodium chloride solution (10 mL×2) in turn, and concentrated under reduced pressure. The obtained residue was separated and purified by silica gel column chromatography (eluent: system B) to obtain the product (R)-N-((3S,4S)-8-(3-bromo-4-cyano-1H-pyrazolo[3,4-d]pyrimidin-6-yl)-3-methyl-2-oxa-8-azaspiro[4.5]decan-4-yl)-2-methylpropane-2-sulfinamide **3b** (230 mg) with a yield of 40%.
MS m/z (ESI): 495.9 [M+1]

### Step 2

### (R)-N-((3S,4S)-8-(3-(2,3-dichlorophenyl)-4-cyano-1H-pyrazolo[3,4-d]pyrimidin-6-yl)-3-methyl-2-oxa-8-azaspiro[4.5]decan-4-yl)-2-methylpropane-2-sulfinamide

Under the protection of argon gas, (R)-N-((3S,4S)-8-(3-bromo-4-cyano-1H-pyrazolo[3,4-d]pyrimidin-6-yl)-3-methyl-2-oxa-8-azaspiro[4.5]decan-4-yl)-2-methylpropane-2-sulfinamide **3b** (230 mg, 463 µmol), (2,3-dichlorophenyl)boronic acid **1g** (142 mg, 745 µmol), methanesulfonato(2-dicyclohexylphosphino-2',6'-di-isopropoxy-1,1'-biphenyl)(2-amino-1,1'-biphenyl-2-yl)palladium (77 mg, 93 µmol), potassium phosphate (295 mg, 1.39 mmol) and 2-dicyclohexylphosphino-2',6'-di-isopropoxy-1,1'-biphenyl (87 mg, 186 µmol) were added to 6 mL of mixed solution of 1,4-dioxane and water (V_{1,4-dioxane}: V_{water} = 5: 1) in turn, heated to 100°C, and reacted for 6 hours. After the reaction was completed, the reaction solution was added with 15 mL of water and 30 mL of ethyl acetate for liquid separation and extraction, then organic phases were washed with 10 mL of saturated salt water, and concentrated under reduced pressure. The obtained residue was further separated and purified by silica gel column chromatography (eluent: system A) to obtain the product (R)-N-((3S,4S)-8-(3-(2,3-dichlorophenyl)-4-cyano-1H-pyrazolo[3,4-d]pyrimidin-6-yl)-3-methyl-2-oxa-8-azaspiro[4.5]decan-4-yl)-2-methylpropane-2-sulfinamide **3c** (74 mg) with a yield of 28%.
MS m/z (ESI): 561.9 [M+1]

### Step 3

### 6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carbonitrile

(R)-N-((3S,4S)-8-(3-(2,3-dichlorophenyl)-4-cyano-1H-pyrazolo[3,4-d]pyrimidin-6-yl)-3-methyl-2-oxa-8-azaspiro[4.5]decan-4-yl)-2-methylpropane-2-sulfinamide **3c** (74 mg, 132 µmol) and bromosuccinimide (26 mg, 145 µmol) were added to 1 mL of N,N-dimethylformamide in turn, and reacted for 2 hours at room temperature. After the reaction was completed, the reaction solution was concentrated under reduced pressure, and the obtained residue was further separated and purified by silic agel column chromatography (eluent: system A) to obtain the product 6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carbonitrile **3** (20 mg) with a yield of 33%.
MS m/z (ESI): 457.9 [M+1]

### Step 4

### 6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carboxamide

6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carbonitrile **3** (20 mg, 43.6 µmol) and 6 M sodium hydroxide solution (0.5 mL) were added to 2 mL of ethanol in turn, heated to 80°C, and reacted for 1 hour. After the reaction was completed, trifluoroacetic acid was slowly added dropwise to the reaction solution to adjust the pH to be 5, and then concentrated under reduced pressure. The obtained residue was subjected to liquid phase separation (separation column AKZONOBEL Kromasil; 250×21.2 mm I.D.; 5 µm, 20 mL/min; mobile phase A: 0.05% TFA + H₂O, mobile phase B: CH₃CN) to obtain the target product 6-((3S,4S)-4-amino-3-methyl-2-oxa-8-azaspiro[4.5]decan-8-yl)-3-(2,3-dichlorophenyl)-1H-pyrazolo[3,4-d]pyrimidine-4-carboxamide **4** (4.5 mg) with a yield of 22%.
MS m/z (ESI): 475.9 [M+1]

### Example 5

### (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxamide

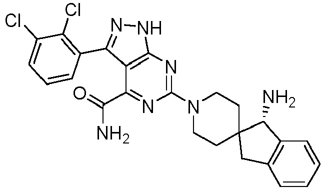

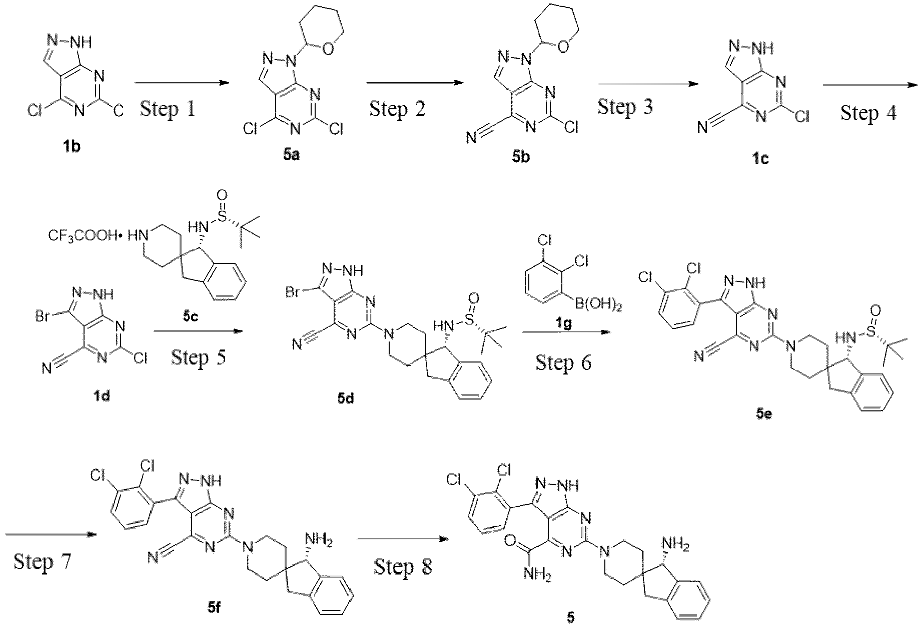

### Step 1

### 4,6-dichloro-1-(tetrahydro-2H-pyran-2-yl)-1H-pyrazolo[3,4-d]pyrimidine

3,4-dihydro-2H-pyrane (14.69 g, 174.60 mmol), 4,6-dichloro-1H-pyrazolo[3,4-d]pyrimidine **1b** (11 g, 58.20 mmol) and p-toluenesulfonic acid (1.00 g, 5.82 mmol) were added to tetrahydrofuran (100 mL), heated to 60°C, and reacted for 2 hours. After the reaction was completed, the reaction solution was concentrated under reduced pressure. The obtained residue was further analyzed and purified by silica gel column chromatography (eluent: system A) to obtain 4,6-dichloro-1-(tetrahydro-2H-pyran-2-yl)-1H-pyrazolo[3,4-d]pyrimidine **5a** (14.2 g) with a yield of 89.3%.
MS m/z (ESI): 272.9 [M+1]

### Step 2

### 6-chloro-1-(tetrahydro-2H-pyran-2-yl)-1H-pyrazolo[3,4-d]pyrimidine-4-carbonitrile

Under the protection of argon gas, 4,6-dichloro-1-(tetrahydro-2H-pyran-2-yl)-1H-pyrazolo[3,4-d]pyrimidine **5a** (2 g, 7.32 mmol), tetrakis(triphenylphosphine)palladium (845.79 mg,732.28 µmol) and zinc cyanide (1.72 g,14.65 mmol) were added to N,N-dimethylformamide (20 mL), heated to 110°C, and reacted for 2 hours. After the reaction was completed, the reaction solution was concentrated under reduced pressure, and added with ethyl acetate (50 mL) for fully dissolved to filter insolubles. The filtrate was concentrated under reduced pressure. The obtained residue was further analyzed and purified by silica gel column chromatography (eluent: system A) to obtain 6-chloro-1-(tetrahydro-2H-pyran-2-yl)-1H-pyrazolo[3,4-d]pyrimidine-4-carbonitrile **5b** (0.8 g) with a yield of 41.4%.

### Step 3

### 6-chloro-1H-pyrazolo[3,4-d]pyrimidine-4-carbonitrile

At room temperature, 6-chloro-1-(tetrahydro-2H-pyran-2-yl)-1H-pyrazolo[3,4-d]pyrimidine-4-carbonitrile **5b** (1 g, 3.79 mmol) was added to trifluoroacetic acid (10 mL) and water (1 mL), and reacted for 4 hours at room temperature. After the reaction was completed, the reaction solution was concentrated under reduced pressure, and subjected to liquid phase separation (separation column AKZONOBEL Kromasil; 250×21.2 mm I.D.; 5 µm, 20 mL/min; mobile phase A: 0.05% TFA + H₂O, mobile phase B: CH₃CN) to obtain 6-chloro-1H-pyrazolo[3,4-d]pyrimidine-4-carbonitrile **1c** (321 mg) with a yield of 47.1% .
MS m/z (ESI): 179.9 [M+1]

### Step 4

### 3-bromo-6-chloro-1H-pyrazolo[3,4-d]pyrimidine-4-carbonitrile

Bromosuccinimide (475.77 mg, 2.67 mmol) and 6-chloro-1H-pyrazolo[3,4-d]pyrimidine-4-carbonitrile **1c** (320 mg, 1.78 mmol) was added to acetonitrile (10 mL) for heated and refluxed for 2 hours. After the reaction was completed, the reaction solution was concentrated under reduced pressure, and subjected to liquid phase separation (separation column AKZONOBEL Kromasil; 250×21.2 mm I.D.; 5 µm, 20 mL/min; mobile phase A: 0.05% TFA + H₂O, mobile phase B: CH₃CN) to obtain 3-bromo-6-chloro-1H-pyrazolo[3,4-d]pyrimidine-4-carbonitrile **1d** (380 mg) with a yield of 82.5%.
MS m/z (ESI): 259.8 [M+1]

### Step 5

### (R)-N-((S)-1'-(3-bromo-4-cyano-1H-pyrazolo[3,4-d]pyrimidin-6-yl)-1,3-dihydrospiro[indene-2,4'-piperidin]-1-yl)-2-methylpropane-2-sulfinamide

At room temperature, (R)-N-((S)-1,3-dihydrospiro[indene-2,4'-piperidin]-1-yl)-2-methylpropane-2-sulfinamide 2,2,2,-trifluoroacetate **5c** (110.87 mg, 361.75 µmol), N,N-diisopropylethylamine (127.51 mg, 986.60 µmol, 162.93 µL) and 3-bromo-6-chloro-1H-pyrazolo[3,4-d]pyrimidine-4-carbonitrile **1d** (85 mg, 328.87 µmol) were added to N-methyl pyrrolidone (5 mL), heated to 100°C and reacted for 3 hours. After the reaction was completed, the reaction solution was concentrated under reduced pressure, and subjected to liquid phase separation (separation column AKZONOBEL Kromasil; 250×21.2 mm I.D.; 5 µm, 20 mL/min; mobile phase A: 0.05% TFA + H₂O, mobile phase B: CH₃CN) to obtain (R)-N-((S)-1'-(3-bromo-4-cyano-1H-pyrazolo[3,4-d]pyrimidine-6-yl)-1,3-dihydrospiro[indene-2,4'-piperidin]-1-yl)-2-methylpropane-2-sulfinamide **5d** (151 mg) with a yield of 86.9%.
MS m/z (ESI): 527.8 [M+1].

### Step 6

### (R)-N-((S)-1'-(4-cyano-3-(2,3-dichlorophenyl)-1H-pyrazolo[3,4-d]pyrimidin-6-yl)-1,3-dihydrospiro[indene-2,4'-piperidin]-1-yl)-2-methylpropane-2-sulfinamide

Under the protection of argon gas, (R)-N-((S)-1'-(3-bromo-4-cyano-1H-pyrazolo[3,4-d]pyrimidin-6-yl)-1,3-dihydrospiro[indene-2,4'-piperidin]-1-yl)-2-methylpropane-2-sulfinamide **5d** (150 mg, 283.84 µmol), (2,3-dichlorophenyl)boronic acid **1g** (162.49 mg, 851.52 µmol), 2-dicyclohexylphosphino-2',6'-di-isopropoxy-1,1'-biphenyl (52.98 mg, 113.54 µmol), methanesulfonato(2-dicyclohexylphosphino-2',6'-di-isopropoxy-1,1'-biphenyl)(2-amino-1,1'-biphenyl-2-yl)palladium (47.54 mg, 56.77 µmol) and potassium phosphate (180.75 mg, 851.52 µmol) were added to 12 mL of mixed solution (1,4-dioxane: water = 5: 1) in turn, heated to 100°C, and reacted for 16 hours. After the reaction was completed, the reaction solution was concentrated under reduced pressure, and added with ethyl acetate (10 mL) and water (10 mL) for extraction and liquid separation, and then aqueous phases were extracted with ethyl acetate (20 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain (R)-N-((S)-1'-(4-cyano-3-(2,3-dichlorophenyl)-1H-pyrazolo[3,4-d]pyrimidin-6-yl)-1,3-dihydrospiro[indene-2,4'-piperidin]-1-yl)-2-methylpropane-2-sulfinamide **5e** (115 mg) with a yield of 68.14%.
MS m/z (ESI): 593.8 [M+1]

### Step 7

### (S)-6-(1-amino-1,3-dihydrospiro[indene-2,4'-piperidin]-1'-yl)-3-(2,3-dichlorophenyl)-1H-pyrazolo[3,4-d]pyrimidine-4-carbonitrile

(R)-N-((S)-1'-(4-cyano-3-(2,3-dichlorophenyl)-1H-pyrazolo[3,4-d]pyrimidin-6-yl)-1,3-dihydrospiro[indene-2,4'-piperidin]-1-yl)-2-methylpropane-2-sulfinamide **5e** (115 mg, 193.42 µmol) was added to a hydrochloric acid dioxane solution (5 mL), and reacted for 1 hour at room temperature. After the reaction was completed, the reaction solution was concentrated under reduced pressure to obtain crude product (S)-6-(1-amino-1,3-dihydrospiro[indene-2,4'-piperidin]-1'-yl)-3-(2,3-dichlorophenyl)-1H-pyrazolo[3,4-d]pyrimidine-4-carbonitrile **5f** (94 mg) with a yield of 92.2%, which was directly used for the next reaction without purification.
MS m/z (ESI): 472.9 [M-16]

### Step 8

### (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxamide

At room temperature, (S)-6-(1-amino-1,3-dihydrospiro[indene-2,4'-piperidin]-1'-yl)-3-(2,3-dichlorophenyl)-1H-pyrazolo[3,4-d]pyrimidine-4-carbonitrile **5f** (94 mg, 155.52 µmol) was added to a mixed solution of aqueous sodium hydroxide (5 M, 1 mL), 30% hydrogen peroxide (1 mL) and methanol (1 mL), and reacted for 3 hours at room temperature. After the reaction was completed, the reaction solution was concentrated under reduced pressure, and subjected to liquid phase separation (separation column AKZONOBEL Kromasil; 250×21.2 mm I.D.; 5 µm, 20 mL/min; mobile phase A: 0.05% TFA + H₂O, mobile phase B: CH₃CN) to obtain (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxamide **5** (21 mg) with a yield of 20.4%.
MS m/z (ESI): 508.1 [M+1]
¹HNMR (400 MHz, DMSO-*d₆*) δ 8.24 (s, 3H), 8.12 (s, 1H), 7.65-7.70 (m, 2H), 7.50-7.55 (m, 1H), 7.30-7.42 (m, 5H), 4.52-5.00 (m, 2H), 4.39 (s, 1H), 3.18-3.35 (m, 3H), 3.00-3.10 (m, 1H), 1.65-1.82 (m, 2H), 1.47-1.60 (m, 2H).

### Example 6

### 6-(4-amino-4-phenylpiperidin-1-yl)-3-(2,3-dichlorophenyl)-1H-pyrazolo[3,4-d]pyrimidine-4-carboxamide

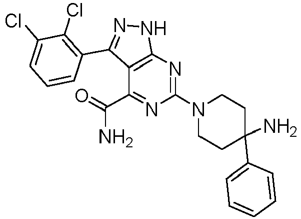

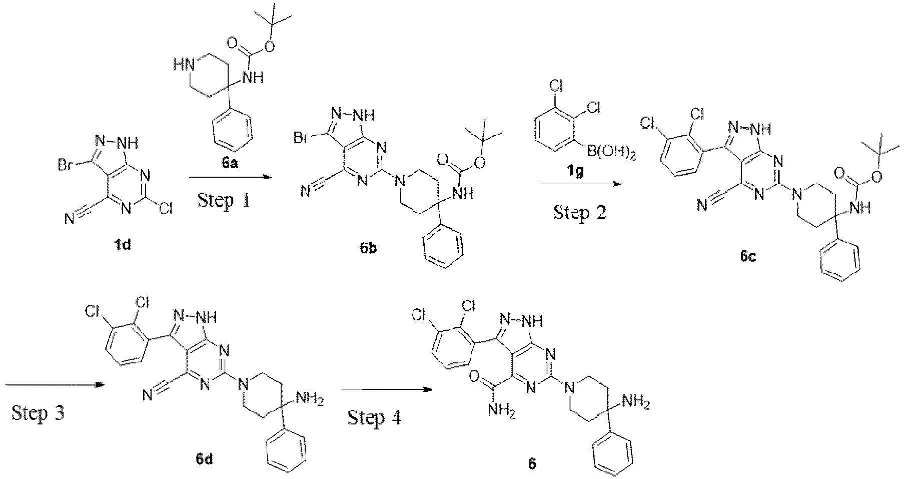

### Step 1

### Tert-butyl (1-(3-bromo-4-cyano-1H-pyrazolo[3,4-d]pyrimidin-6-yl)-4-phenylpiperidine -4-yl)carbamate

At room temperature, tert-butyl (4-phenylpiperidin-4-yl)carbamate **6a** (117.62 mg, 425.59 µmol), 3-bromo-6-chloro-1H-pyrazolo[3,4-d]pyrimidine-4-carbonitrile **1d** (100 mg, 386.90 µmol) and N,N-diisopropylethylamine (150.01 mg, 1.16 mmol, 191.68 µL) were added to N-methyl pyrrolidone (5 mL), heated to 110°C, and reacted for 1 hour. After the reaction was completed, the reaction solution was concentrated under reduced pressure, and subjected to liquid phase separation (separation column: AKZONOBEL Kromasil; 250×21.2 mm I.D.; 5µm, 20 mL/min; mobile phase A: 0.1%TFA+ H₂O, mobile phase B: CH₃CN) to obtain tert-butyl (1-(3-bromo-4-cyano-1H-pyrazolo[3,4-d]pyrimidin-6-yl)-4-phenylpiperidin-4-yl)carbamate **6b** (153 mg) with a yield of 79.35%.
MS m/z (ESI): 497.8 [M+1].

### Step 2

### tert-butyl (1-(3-(2,3-dichlorophenyl)-4-cyano-1H-pyrazolo[3,4-d]pyrimidin-6-yl)-4-phenylpiperidin-4-yl) carbamate

Under the protection of argon gas, tert-butyl (1-(3-bromo-4-cyano-1H-pyrazolo[3,4-d]pyrimidin-6-yl)-4-phenylpiperidin -4-yl)carbamate **6b** (153 mg, 307.00 µmol), (2,3-dichlorophenyl)boronic acid **1g** (175.74 mg, 920.99 µmol), 2-dicyclohexylphosphino-2',6'-di-isopropoxy-1,1'-biphenyl (57.30 mg, 122.80 µmol), methanesulfonato(2-dicyclohexylphosphino-2',6'-di-isopropoxy-1,1'-biphenyl)(2-amino-1,1'-biphenyl-2-yl)palladium (51.41 mg, 61.40 µmol) and potassium phosphate (195.50 mg, 920.99 µmol) were added to 11 mL of mixed solution (1,4-dioxane: water = 10: 1) in turn, heated to 100°C, and reacted for 16 hours. After the reaction was completed, the reaction solution was concentrated under reduced pressure, and added with ethyl acetate (10 mL) and water (10 mL) for extraction and separation, and then aqueous phases were extarcted with ethyl acetate (10 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain tert-butyl (1-(3-(2,3-dichlorophenyl)-4-cyano-1H-pyrazolo[3,4-d]pyrimidin-6-yl)-4-phenylpiperidin-4-yl) carbamate **6c** (128 mg) with a yield of 73.86%.
MS m/z (ESI): 563.8 [M+1]

### Step 3

### 6-(4-amino-4-phenylpiperidin-1-yl)-3-(2,3-dichlorophenyl)-1H-pyrazolo[3,4-d]pyrimidine-4-carbonitrile

Tert-butyl (1-(3-(2,3-dichlorophenyl)-4-cyano-1H-pyrazolo[3,4-d]pyrimidin-6-yl)-4-phenylpiperidin-4-yl) carbamate **6c** (120 mg, 212.59 µmol) and trifluoroacetic acid (1 mL) were added to dichloromethane (3 mL), and reacted at room temperature for 1 hour. The raction solutoin was concentrated under reduced pressure to obtain crude product 6-(4-amino-4-phenylpiperidin-1-yl)-3-(2,3-dichlorophenyl)-1H-pyrazolo[3,4-d]pyrimidine-4-carbonitrile **6d** (89.94 mg) with a yield of 91.3%, which was directly used for the next reaction without purification.
MS m/z (ESI): 447.1 [M-16]

### Step 4

### 6-(4-amino-4-phenylpiperidin-1-yl)-3-(2,3-dichlorophenyl)-1H-pyrazolo[3,4-d]pyrimidine-4-carboxamide

6-(4-amino-4-phenylpiperidin-1-yl)-3-(2,3-dichlorophenyl)-1H-pyrazolo[3,4-d]pyrimidine-4-carbonitrile **6d** (89.94 mg, 193.68 µmol) was added to a mixed solution of methanol (1.00 mL), aqueous sodium hydroxide (5 M, 1.00 mL) and 30% hydrogen peroxide (0.5 mL), and reacted for 3 hours at room temperature. After the reaction was completed, the reaction solution was concentrated under reduced pressure, and subjected to liquid phase separation (separation column: AKZONOBEL Kromasil; 250×21.2 mm I.D.; 5µm, 20 mL/min; mobile phase A: 0.05% TFA + H₂O, mobile phase B: CH₃CN) to obtain 6-(4-amino-4-phenylpiperidin-1-yl)-3-(2,3-dichlorophenyl)-1H-pyrazolo[3,4-d]pyrimidine-4-carboxamide **6** (18 mg) with a yield of 14.65%. MS m/z (ESI): 481.9 [M+1]
¹HNMR (400 MHz, DMSO-*d₆*) δ 8.25-8.50 (m, 3H), 8.05- 8.23 (m, 1H), 7.60-7.80 (m, 4H), 7.50-7.60 (m, 2H), 7.38-7.50 (m, 3H), 4.15-4.60 (m, 4H), 1.93-2.18 (m, 2H), 1.20-1.50 (m, 2H).

### Example 7

### 6-((endo)-3-amino-8-azabicyclo[3.2.1]octan-8-yl)-3-(2,3-dichlorophenyl)-1H-pyrazolo[3,4-d]pyrimidine-4-carboxamide

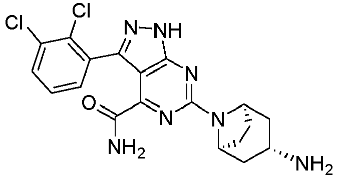

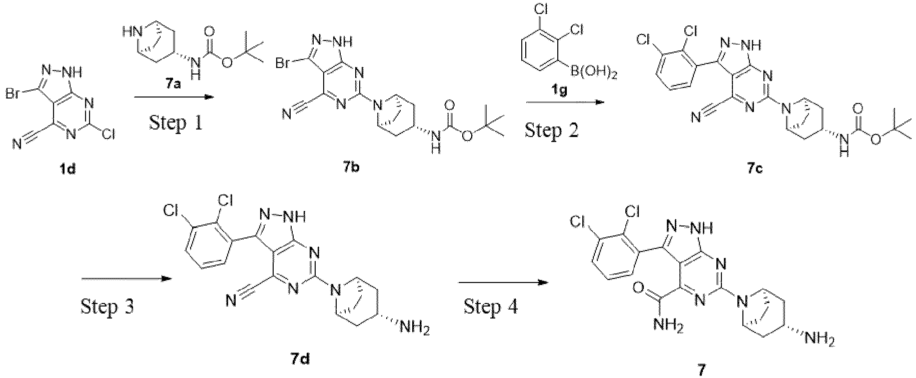

### Step 1

### Tert-butyl ((endo)-8-(3-bromo-4-cyano-1H-pyrazolo[3,4-d]pyrimidin-6-yl)-8-azabicyclo[3.2.1]octan-3-yl)carbamate

At room temperature, tert-butyl((endo)-8-azabicyclo[3.2.1]octan-3-yl)carbamate **7a** (144 mg, 636 µmol), 3-bromo-6-chloro-1H-pyrazolo[3,4-d]pyrimidine-4-carbonitrile **1d** (150 mg, 580 µmol) and N,N-diisopropylethylamine (225 mg, 1.74 mmol) were added to N-methyl pyrrolidone (5 mL), heated to 110°C, and reacted for 16 hours. After the reaction was completed, the reaction solution was concentrated under reduced pressure, and separated on a C₁₈ reversed phase column (C₁₈ separation column 20-45 µm; mobile phase A: H₂O, mobile phase B: CH₃CN) to obtain tert-butyl ((endo)-8-(3-bromo-4-cyano-1H-pyrazolo[3,4-d]pyrimidin-6-yl)-8-azabicyclo[3.2.1]octan-3-yl)carbamate **7b** (150 mg) with a yield of 57.7%.
MS m/z (ESI): 448.0 [M+1].

### Step 2

### Tert-butyl ((endo)-8-(4-cyano-3-(2,3-dichlorophenyl)-1H-pyrazolo[3,4-d]pyrimidin-6-yl)-8-azabicyclo[3.2.1]octan-3-yl) carbamate

Under the protection of argon gas, tert-butyl((endo)-8-(3-bromo-4-cyano-1H-pyrazolo[3,4-d]pyrimidin-6-yl)-8-azabicyclo[3.2.1]octan-3-yl) carbamate **7b** (150 mg, 335 µmol), (2,3-dichlorophenyl)boronic acid **1g** (255 mg, 1.34 mmol), 2-dicyclohexylphosphino-2',6'-di-isopropoxy-1.1'-biphenyl (62.5 mg, 134 µmol), methanesulfonato(2-dicyclohexylphosphino-2',6'-di-isopropoxy-1,1'-biphenyl)(2-amino-1,1'-biphenyl-2-yl)palladium (56 mg, 67 µmol) and potassium phosphate (213 mg, 1.00 mmol) were added to 12 mL of mixed solution (1,4-dioxane: water = 5: 1) in turn, heated to 100°C, and reacted for 16 hours. After the reaction was completed, the reaction solution was concentrated under reduced pressure, and added with ethyl acetate (10 mL) and water (10 mL) for extraction and separation, and then aqueous phases were extracted with ethyl acetate (10 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain tert-butyl((endo)-8-(4-cyano-3-(2,3-dichlorophenyl)-1H-pyrazolo[3,4-d]pyrimidin-6-yl)-8-azabicyclo[3.2.1]octan-3-yl) carbamate 7c (45 mg) with a yield of 26.1%.
MS m/z (ESI): 513.8 [M+1]

### Step 3

### 6-((endo)-3-amino-8-azabicyclo[3.2.1]octan-8-yl)-3-(2,3-dichlorophenyl)-1H-pyrazolo[3,4-d]pyrimidine-4-carbonitrile

Tert-butyl((endo)-8-(4-cyano-3-(2,3-dichlorophenyl)-1H-pyrazolo[3,4-d]pyrimidin-6-yl)-8-azabicyclo[3.2.1]octan-3-yl) carbamate 7c (45 mg, 87.75 µmol) and trifluoroacetic acid (1 mL) were added to dichloromethane (3 mL), and reacted at room temperature for 1 hour. The raction solutoin was concentrated under reduced pressure to obtain crude product 6-((endo)-3-amino-8-azabicyclo[3.2.1]octan-8-yl)-3-(2,3-dichlorophenyl)-1H-pyrazolo[3,4-d]pyrimidine-4-carbonitrile **7d** (35.9 mg) with a yield of 99%, which was directly used for the next reaction without purification.
MS m/z (ESI): 413.6 [M+1]

### Step 4

### 6-((endo)-3-amino-8-azabicyclo[3.2.1]octan-8-yl)-3-(2,3-dichlorophenyl)-1H-pyrazolo[3,4-d]pyrimidine-4-carboxamide

6-((endo)-3-amino-8-azabicyclo[3.2.1]octan-8-yl)-3-(2,3-dichlorophenyl)-1H-pyrazolo[3,4-d]pyrimidine-4-carbonitrile **7d** (35.9 mg, 86.6 µmol) was added to a mixed solution of methanol (1.00 mL), aqueous sodium hydroxide (5 M, 1.00 mL) and 30% hydrogen peroxide (0.5 mL), and reacted for 3 hours at room temperature. After the reaction was completed, the reaction solution was concentrated under reduced pressure, and subjected to liquid phase separation (separation column: AKZONOBEL Kromasil; 250×21.2 mm I.D.; 5µm, 20 mL/min; mobile phase A: 0.05% TFA + H₂O, mobile phase B: CH₃CN) to obtain 6-((endo)-3-amino-8-azabicyclo[3.2.1]octan-8-yl)-3-(2,3-dichlorophenyl)-1H-pyrazolo[3,4-d]pyrimidine-4-carboxamide 7 (18 mg) with a yield of 48%.
MS m/z (ESI): 431.9 [M+1]

### Example 8

### 6-((exo)-3-amino-8-azabicyclo[3.2.1]octan-8-yl)-3-(2,3-dichlorophenyl)-1H-pyrazolo[3,4-d]pyrimidine-4-carboxamide

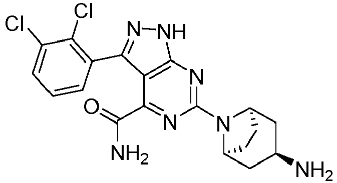

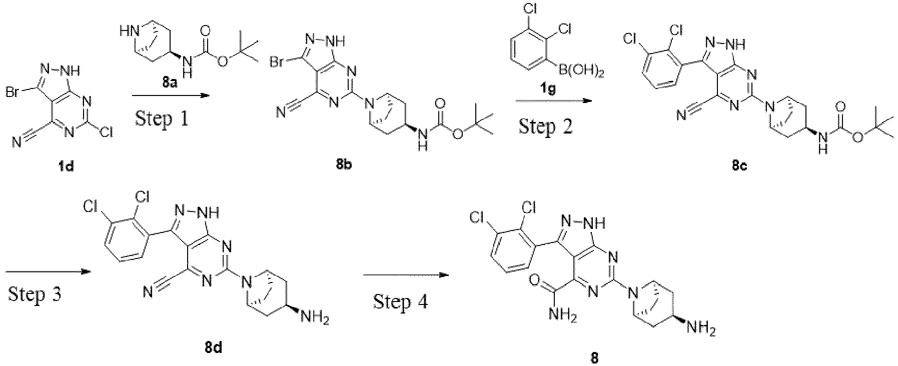

### Step 1

### Tert-butyl ((exo)-8-(3-bromo-4-cyano-1H-pyrazolo[3,4-d]pyrimidin-6-yl)-8-azabicyclo[3.2.1]octan-3-yl)carbamate

At room temperature, tert-butyl((exo)-8-azabicyclo[3.2.1]octan-3-yl)carbamate **8a** (144 mg, 636 µmol), 3-bromo-6-chloro-1H-pyrazolo[3,4-d]pyrimidine-4-carbonitrile **1d** (150 mg, 580 µmol) and N,N-diisopropylethylamine (225 mg, 1.74 mmol) were added to N-methyl pyrrolidone (5 mL), heated to 110°C, and reacted for 16 hours. After the reaction was completed, the reaction solution was concentrated under reduced pressure and separated on a C₁₈ reversed phase column (C₁₈ separation column 20-45 µm; mobile phase A: H₂O, mobile phase B: CH₃CN) to obtain tert-butyl ((exo)-8-(3-bromo-4-cyano-1H-pyrazolo[3,4-d]pyrimidin-6-yl)-8-azabicyclo[3.2.1]octan-3-yl) carbamate **8b** (90 mg) with a yield of 34.6%.
MS m/z (ESI): 448.0 [M+1]

### Step 2

### Tert-butyl ((exo)-8-(4-cyano-3-(2,3-dichlorophenyl)-1H-pyrazolo[3,4-d]pyrimidin-6-yl)-8-azabicyclo[3.2.1]octan-3-yl) carbamate

Under the protection of argon gas, tert-butyl ((exo)-8-(3-bromo-4-cyano-1H-pyrazolo[3,4-d]pyrimidin-6-yl)-8-azabicyclo[3.2.1]octan-3-yl)carbamate **8b** (90 mg, 200 µmol), (2,3-dichlorophenyl)boronic acid **1g** (153 mg, 0.8 mmol), 2-dicyclohexylphosphino-2',6'-di-isopropoxy-1,1'-biphenyl (37 mg, 80 µmol), methanesulfonato(2-dicyclohexylphosphino-2',6'-di-isopropoxy-1,1'-biphenyl)(2-amino-1,1'-biphenyl-2-yl)palladium (33 mg, 40 µmol) and potassium phosphate (127 mg, 0.6 mmol) were added to 12 mL of mixed solution (1,4-dioxane: water = 5: 1) in turn, heated to 100°C, and reacted for 16 hours. After the reaction was completed, the reaction solution was concentrated under reduced pressure, and added with ethyl acetate (10 mL) and water (10 mL) for extraction and separation, and then aqueous phases were extracted with ethyl acetate (10 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain tert-butyl ((exo)-8-(4-cyano-3-(2,3-dichlorophenyl)-1H-pyrazolo[3,4-d]pyrimidin-6-yl)-8-azabicyclo[3.2.1]octan-3-yl) carbamate **8c** (25 mg) with a yield of 24.3%.
MS m/z (ESI): 513.8 [M+1]

### Step 3

### 6-((exo)-3-amino-8-azabicyclo[3.2.1]octan-8-yl)-3-(2,3-dichlorophenyl)-1H-pyrazolo[3,4-d]pyrimidine-4-carbonitrile

Tert-butyl ((exo)-8-(4-cyano-3-(2,3-dichlorophenyl)-1H-pyrazolo[3,4-d]pyrimidin-6-yl)-8-azabicyclo[3.2.1]octan-3-yl)carbamate **8c** (25 mg, 49 µmol) and trifluoroacetic acid (1 mL) were added to dichloromethane (3 mL), and reacted at room temperature for 1 hour. The raction solutoin was concentrated under reduced pressure to obtain crude product 6-((exo)-3-amino-8-azabicyclo[3.2.1]octan-8-yl)-3-(2,3-dichlorophenyl)-1H-pyrazolo[3,4-d]pyrimidine-4-carbonitrile **8d** (20 mg) with a yield of 98%, which was directly used for the next reaction without purification.
MS m/z (ESI): 413.6 [M+1]

### Step 4

### 6-((exo)-3-amino-8-azabicyclo[3.2.1]octan-8-yl)-3-(2,3-dichlorophenyl)-1H-pyrazolo[3,4-d]pyrimidine-4-carboxamide

6-((exo)-3-amino-8-azabicyclo[3.2.1]octan-8-yl)-3-(2,3-dichlorophenyl)-1H-pyrazolo[3,4-d]pyrimidine-4-carbonitrile **8d** (20 mg, 48 µmol) was added to a mixed solution of methanol (1.00 mL), aqueous sodium hydroxide (5 M, 1.00 mL) and 30% hydrogen peroxide (0.5 mL), and reacted for 3 hours at room temperature. After the reaction was completed, the reaction solution was concentrated under reduced pressure, and subjected to liquid phase separation (separation column: AKZONOBEL Kromasil; 250×21.2 mm I.D.; 5µm, 20 mL/min; mobile phase A: 0.05% TFA + H₂O, mobile phase B: CH₃CN) to obtain 6-((exo)-3-amino-8-azabicyclo[3.2.1]octan-8-yl)-3-(2,3-dichlorophenyl)-1H-pyrazolo[3,4-d]pyrimidine-4-carboxamide **8** (9 mg) with a yield of 43.5%.
MS m/z (ESI): 431.9 [M+1]
¹H NMR (400 MHz, DMSO-*d₆*) δ 8.02-8.14 (m, 1H), 7.87 (br, 3H), 7.55-7.73 (m, 2H), 7.30-7.47 (m, 2H), 4.90-5.16 (br, 1H), 4.49-4.76 (br, 1H), 3.16-3.27 (m, 1H), 2.29-2.48 (m, 2H), 2.06-2.23 (m, 2H), 1.83-2.01 (m, 2H), 1.56-1.80 (m, 2H).

### Example 9

### (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxylic acid

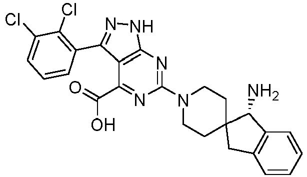

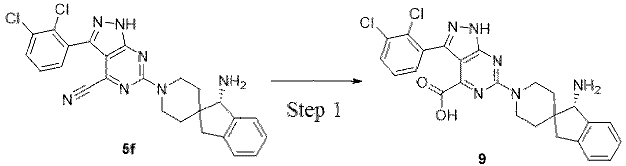

### Step 1

(S)-6-(1-amino-1,3-dihydrospiro[indene-2,4'-piperidin]-1'-yl)-3-(2,3-dichlorophenyl)-1H-pyrazolo[3,4-d]pyrimidine-4-carbonitrile **5f** (63 mg, 128.47 µmol) was added to 5 mL of aqueous hydrochloric acid solution, and heated and refluxed for 3 hours.After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxylic acid 9 (21 mg) with a yield of 25.43%.
MS m/z (ESI): 509.1 [M+1]
¹HNMR (400 MHz, DMSO-*d₆*) δ 13.64 (s, 1H), 8.24 (s, 3H), 7.63-7.75 (m, 1H), 7.49-7.54 (m, 1H), 7.40-7.48 (m, 2H), 7.28-7.40 (m, 3H), 4.47-4.88 (m, 2H), 4.39 (s, 1H), 3.18-3.40 (m, 3H), 3.00-3.10 (m, 1H), 1.61-1.82 (m, 2H), 1.44-1.60 (m, 2H).

### Example 10

### 6-(4-amino-4-phenylpiperidin-1-yl)-3-(2,3-dichlorophenyl)-1H-pyrazolo[3,4-d]pyrimidine-4-carboxylic acid

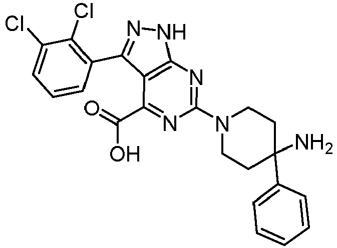

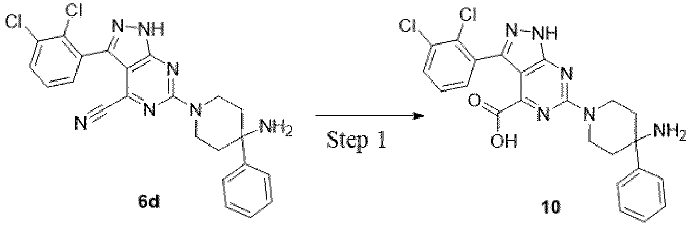

### Step 1

6-(4-amino-4-phenylpiperidin-1-yl)-3-(2,3-dichlorophenyl)-1H-pyrazolo[3,4-d]pyrimidine-4-carbonitrile **6d** (50 mg, 107.68 µmol) was added to 3 mL of concentrated hydrochloric acid, and heated and refluxed for 1 hour. After the reaction was completed, the reaction solution was added with a potassium carbonate solution to adjust the pH to be 9-10, concentrated under reduced pressure, and then subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-phenylpiperidin-1-yl)-3-(2,3-dichlorophenyl)-1H-pyrazolo[3,4-d]pyrimidine-4-carboxylic acid **10** (21 mg) with a yield of 32.65%.
MS m/z (ESI): 465.8 [M-16]
¹HNMR (400 MHz, DMSO-*d₆*) δ 13.70 (s, 1H), 8.25-8.45 (m, 3H), 7.68-7.76 (m, 3H), 7.50-7.60 (m, 2H), 7.40-7.50 (m, 3H), 4.15-4.50 (m, 2H), 3.43-3.60 (m, 2H), 2.54-2.64 (m, 2H), 2.00-2.15 (m, 2H).

### Example 11

### 6-(4-amino-4-(2,6-difluorophenyl)piperidin-1-yl)-3-(2,3-dichlorophenyl)-1H-pyrazolo[3,4-d]pyrimidine-4-carboxamide

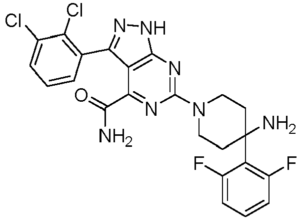

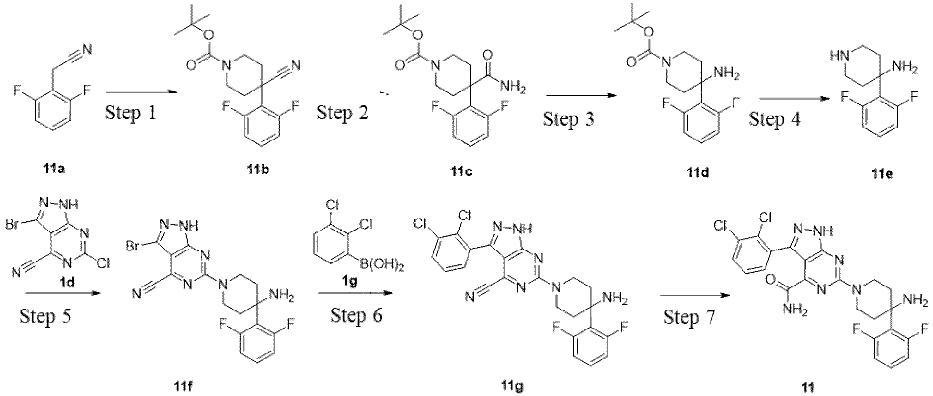

### Step 1

### Tert-butyl 4-cyano-4-(2,6-difluorophenyl)piperidine-l-carboxylate

At 0°C, sodium hydride (2.40 g, 59.92 mmol), 2-(2,6-difluorophenyl)acetonitrile **11a** (1.53 g, 9.99 mmol) and tert-butyl N,N-bis(2-chloroethyl)carbamate (2.66 g, 10.99 mmol) were added to 15 mL of N,N-dimethylformamide, stirred for 1 hour in an ice bath, heated to 60°C, and then stirred for 16 hours. After the reaction was completed, the reaction solution was quenched by adding a saturated aqueous ammonium chloride solution (30 mL), and added with ethyl acetate (30 mL) for extraction and separation, and then aqueous phases were extracted with ethyl acetate (30 mL×2), and organic phases were combined, and washed with saturated brine, dried by anhydrous sodium sulfate, and concentrated under reduced pressure. The obtained residue was further separated and purified by silica gel column chromatography (eluent: system A) to obtain tert-butyl 4-cyano-4-(2,6-difluorophenyl)piperidine-1-carboxylate **11b** (0.67 g) with a yield of 20.81%.
MS m/z (ESI): 222.8 [M-99]

### Step 2

### Tert-butyl 4-carbamoyl-4-(2,6-difluorophenyl)piperidine-1-carboxylate

Potassium hydroxide (233.25 mg, 4.16 mmol) and tert-butyl 4-cyano-4-(2,6-difluorophenyl)piperidine-1-carboxylate **11b** (0.67 g, 2.08 mmol) were added to a solution of dimethyl sulfoxide (5 mL), hydrogen peroxide (2 mL, 30%) was slowly added to the reaction solution in an ice bath. After the dropwise addition was completed, the reaction solution was heated to room temperature, and stirred for 30 minutes. After the reaction was completed, the reaction solution was added with water (100 mL) to precipitate a white solid, and filtered, then the filter cake was washed with water, and dried in vacuum to obtain tert-butyl 4-carbamoyl-4-(2,6-difluorophenyl)piperidine-1-carboxylate **11c** (0.64 g) with a yield of 90.47%.
MS m/z (ESI): 285.1 [M-55]

### Step 3

Tert-butyl 4-amino-4-(2,6-difluorophenyl)piperidine-1-carboxylate [Bis(trifluoroacetoxy)iodo]benzene (985.51 mg, 2.29 mmol) was added to 20 mL of mixed solution (acetonitrile: water = 1: 1) containing tert-butyl 4-carbamoyl-4-(2,6-difluorophenyl)piperidine-1-carboxylate **11c** (0.64 g, 1.88 mmol), and stirred for 16 hours at room temperature. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain tert-butyl 4-amino-4-(2,6-difluorophenyl)piperidine-1-carboxylate **11d** (251 mg) with a yield of 30.83%.
MS m/z (ESI): 239.9 [M-72]

### Step 4

### 4-(2,6-difluorophenyl)piperidin-4-amine

Tert-butyl 4-amino-4-(2,6-difluorophenyl)piperidine-1-carboxylate 1**1d** (270 mg, 691.61 µmol) and trifluoroacetic acid (1 mL) were added to dichloromethane (3 mL), and reacted at room temperature for 1 hour. The reaction solutoin was concentrated under reduced pressure to obtain 4-(2,6-difluorophenyl)piperidin-4-amine **11e** (138.85 mg) with a yield of 94.7%, which was directly used for the next reaction without purification.
MS m/z (ESI): 160.1 [M-52]

### Step 5

### 6-(4-amino-4-(2,6-difluorophenyl)piperidin-1-yl)-3-bromo-1H-pyrazolo[3,4-d]pyrimidine-4-carbonitrile

At room temperature, 4-(2,6-difluorophenyl)piperidin-4-amine **11e** (138.85 mg, 425.59 µmol), 3-bromo-6-chloro-1H-pyrazolo[3,4-d]pyrimidine-4-carbonitrile **1d** (100 mg, 386.90 µmol) and N,N-diisopropylethylamine (150 mg, 1.16 mmol) were added to N-methyl pyrrolidone (5 mL), heated to 110°C, and stirred for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2,6-difluorophenyl)piperidin-1-yl)-3-bromo-1H-pyrazolo[3,4-d]pyrimidine-4-carbonitrile **11f** (181 mg) with a yield of 85.33%. MS m/z (ESI): 417.0 [M-15]

### Step 6

### 6-(4-amino-4-(2,6-difluorophenyl)piperidin-1-yl)-3-(2,3-dichlorophenyl)-1H-pyrazolo[3,4-d]pyrimidine-4-carbonitrile

Under the protection of argon gas, 6-(4-amino-4-(2,6-difluorophenyl)piperidin-1-yl)-3-bromo-1H-pyrazolo[3,4-d]pyrimidine-4-carbonitrile **11f** (150 mg, 345.43 µmol), (2,3-dichlorophenyl)boronic acid **1g** (263.66 mg, 1.38 mmol), 2-dicyclohexylphosphino-2',6'-di-isopropoxy-1,1'-biphenyl (64.48 mg, 138.17 µmol), methanesulfonato(2-dicyclohexylphosphino-2',6'-di-isopropoxy-1,1'-biphenyl)(2-amino-1,1'-biphenyl-2-yl)palladium (57.85 mg, 69.09 µmol) and potassium phosphate (219.97 mg, 1.04 mmol) were added to 11 mL of mixed solution (1,4-dioxane: water = 10: 1) in turn, heated to 100°C, and reacted for 16 hours. After the reaction was completed, the reaction solution was concentrated under reduced pressure, added with ethyl acetate (10 mL) and water (10 mL) for extraction and separation, and then aqueous phases were extrated with ethyl acetate (10 mL×2), and organic phases were combined, and washed with saturated salt water, dried by anhydrous sodium sulfate, concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2,6-difluorophenyl)piperidin-1-yl)-3-(2,3-dichlorophenyl)-1H-pyrazolo[3,4-d]pyrimidine-4-carbonitrile **11g** (130 mg) with a yield of 61.26%.
MS m/z (ESI): 483.0 [M-15]

### Step 7

### 6-(4-amino-4-(2,6-difluorophenyl)piperidin-1-yl)-3-(2,3-dichlorophenyl)-1H-pyrazolo[3,4-d]pyrimidine-4-carboxamide

6-(4-amino-4-(2,6-difluorophenyl)piperidin-1-yl)-3-(2,3-dichlorophenyl)-1H-pyrazolo[3,4-d]pyrimidine-4-carbonitrile **11g** (50 mg, 81.39 µmol) was added to a mixed solution of methanol (1.00 mL), aqueous sodium hydroxide (5 M, 1.00 mL) and 30% hydrogen peroxide (0.5 mL), and reacted for 3 hours at room temperature. After the reaction was completed, the reaction solution was concentrated under reduced pressure, and subjected to liquid phase separation (separation column AKZONOBEL Kromasil; 250×21.2 mm I.D.; 5 µm, 20 mL/min; mobile phase A: 0.05% TFA+ H₂O, mobile phase B: CH₃CN) to obtain 6-(4-amino-4-(2,6-difluorophenyl)piperidin-1-yl)-3-(2,3-dichlorophenyl)-1H-pyrazolo[3,4-d]pyrimidine-4-carboxamide 11 (15 mg) with a yield of 35.7%.
MS m/z (ESI): 517.8 [M+1]
¹HNMR (400 MHz, DMSO-*d₆*) δ 8.50-8.70 (m, 3H), 8.16 (s, 1H), 7.55-7.70 (m, 3H), 7.37-7.42 (m, 2H), 7.22-7.33 (m, 2H), 4.00-4.60 (m, 2H), 3.60-3.90 (m, 2H), 2.62-2.81 (m, 2H), 2.00-2.25 (m, 2H).

### Example 12

### 6-(4-amino-4-methylpiperidin-1-yl)-3-(3-bromo-2-chlorophenyl)-1H-pyrazolo[3,4-d]pyrimidine-4-carboxamide

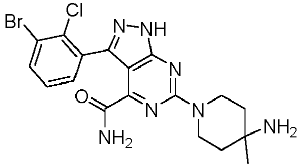

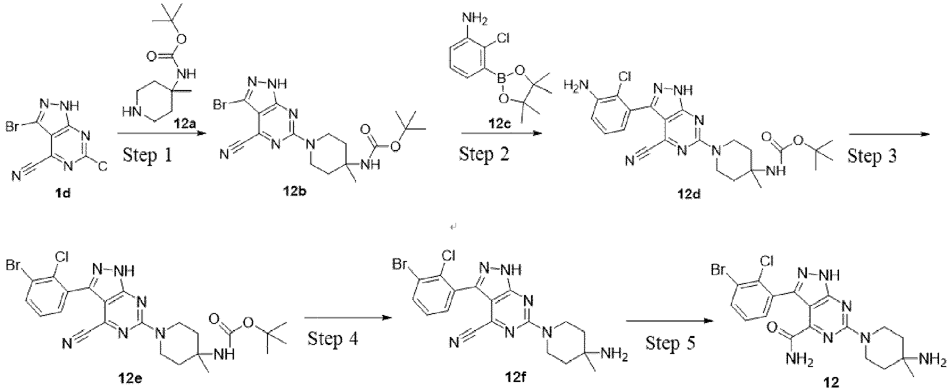

### Step 1

### Tert-butyl(1-(3-bromo-4-cyano-1H-pyrazolo[3,4-d]pyrimidin-6-yl)-4-methylpiperidin-4-yl)carbamate

3-bromo-6-chloro-1H-pyrazolo[3,4-d]pyrimidine-4-carbonitrile **1d** (800 mg, 3.10 mmol), tert-butyl (4-methylpiperidin-4-yl)carbamate 12a (729.65 mg, 3.40 mmol) and diisopropylethylamine (1.20 g, 9.29 mmol, 1.53 mL) were added to N-methyl pyrrolidone (5 mL) in turn, heated to 110°C, and reacted for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain tert-butyl (1-(3-bromo-4-cyano-1H-pyrazolo[3,4-d]pyrimidin-6-yl)-4-methylpiperidin-4-yl)carbamate 12b (810 mg) with a yield of 59.98%.
MS m/z (ESI): 435.9 [M+1]

### Step 2

### Tert-butyl(1-(3-(3-amino-2-chlorophenyl)-4-cyano-1H-pyrazolo[3,4-d]pyrimidin-6-yl)-4-methylpiperidin-4-yl] carbamate

Under the protection of argon gas, tert-butyl (1-(3-bromo-4-cyano-1H-pyrazolo[3,4-d]pyrimidin-6-yl)-4-methylpiperidin-4-yl)carbamate **12b** (100 mg, 181.71 µmol), 2-chloro-3-(4,4,5,5,-tetramethyl-1,3,2-dioxaborolan-2-yl)aniline **12c** (138.21 mg, 545.13 µmol), methanesulfonato(2-dicyclohexylphosphino-2',6'-di-isopropoxy-1,1'-biphenyl)(2-amino-1,1'-biphenyl-2-yl)palladium (30.42 mg, 36.34 µmol), potassium phosphate (55.43 mg, 261.15 µmol) and 2-dicyclohexylphosphino-2',6'-di-isopropoxy-1,1'-biphenyl (33.92 mg, 72.68 µmol)were added to 11 mL of mixed solution (1,4-dioxane: water = 10: 1) in turn, and heated and refluxed for 16 hours. After the reaction was completed, the reaction solution was added with 10 mL of water and 10 mL of ethyl acetate for liquid separation and extraction, and then aqueous phases were extracted with ethyl acetate (10 mL×2), and organic phases were combined, and washed with 10 mL of saturated salt water, and concentrated under reduced pressure. The obtained residue was further separated and purified by silica gel column chromatography (eluent: system A) to obtain tert-butyl (1-(3-(3-amino-2-chlorophenyl)-4-cyano-1H-pyrazolo[3,4-d]pyrimidin-6-yl)-4-methylpiperidin-4-yl]carbamate **12d** (51 mg) with a yield of 58.11%.
MS m/z (ESI): 482.9 [M+1]

### Step 3

### Tert-butyl(1-(3-(3-bromo-2-chlorophenyl)-4-cyano-1H-pyrazolo[3,4-d]pyrimidin-6-yl)-4-methylpiperidin-4-yl]carbamate

Under the protection of argon gas, tert-butyl (1-(3-(3-amino-2-chlorophenyl)-4-cyano-1H-pyrazolo[3,4-d]pyrimidin-6-yl)-4-methylpiperidin-4-yl]carbamate **12d** (51 mg, 105.60 µmol) and cuprous bromide (30.30 mg, 211.20 µmol) were added to 5 mL of acetonitrile, cooled to 0-10°C in an ice water bath, then added with tert-butyl nitrite (22.0 mg, 211.20 µmol), and reacted for 2 hours. After the reaction was completed, the reaction solution was extracted with ethyl acetate (10 mL×2), then organic phases were combined, and organic phases were washed with 10 mL of saturated salt water, and concentrated under reduced pressure. The obtained residue was further separated and purified by silica gel column chromatography (eluent: system A) to obtain tert-butyl (1-(3-(3-bromo-2-chlorophenyl)-4-cyano-1H-pyrazolo[3,4-d]pyrimidin-6-yl)-4-methylpiperidin-4-yl]carbamate **12e** (42 mg) with a yield of 72.73%.
MS m/z (ESI): 545.8 [M+1]

### Step 4

### 6-(4-amino-4-methylpiperidin-1-yl)-3-(3-bromo-2-chlorophenyl)-1H-pyrazolo[3,4-d]pyrimidine-4-carbonitrile

Tert-butyl(1-(3-(3-bromo-2-chlorophenyl)-4-cyano-1H-pyrazolo[3,4-d]pyrimidin-6-yl)-4-methylpiperidin-4-yl]carbamate 12e (42 mg, 76.80 µmol) and trifluoroacetic acid (1 mL) were added to dichloromethane (3 mL), and reacted at room temperature for 1 hour. The raction solutoin was concentrated under reduced pressure to obtain crude product 6-(4-amino-4-methylpiperidin-1-yl)-3-(3-bromo-2-chlorophenyl)-1H-pyrazolo[3,4-d]pyrimidine-4-carbonitrile **12f,** which was directly used for the next reaction without purification.
MS m/z (ESI): 446.0 [M+1]

### Step 5

### 6-(4-amino-4-methylpiperidin-1-yl)-3-(3-bromo-2-chlorophenyl)-1H-pyrazolo[3,4-d]pyrimidine-4-carboxamide

6-(4-amino-4-methylpiperidin-1-yl)-3-(3-bromo-2-chlorophenyl)-1H-pyrazolo[3,4-d]pyrimidine-4-carbonitrile **12f** (50 mg, 81.39 µmol) was added to a mixed solution of methanol (1.00 mL), aqueous sodium hydroxide (5 M, 1.00 mL) and 30% hydrogen peroxide (0.5 mL), and reacted for 3 hours at room temperature. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA+ H₂O, mobile phase B: CH₃CN) to obtain 6-(4-amino-4-methylpiperidin-1-yl)-3-(3-bromo-2-chlorophenyl)-1H-pyrazolo[3,4-d]pyrimidine-4-carboxamide **12** (2.1 mg) with a yield of 4.4%. MS m/z (ESI): 463.8 [M+1]
¹HNMR (400 MHz, DMSO-*d₆*) δ 8.11 (s, 1H), 8.01 (s, 3H), 7.79 (d, *J* = 8.0Hz, 1H), 7.65 (s, 1H), 7.35-7.48 (m, 1H), 7.23-7.35 (m, 1H), 4.30-4.55 (m, 2H), 3.48-3.55 (m, 2H), 1.68- 1.79 (m, 4H), 1.41(s, 3H).

### Examples 13-21

Referring to the operation steps of step 1 to step 4 of Example 6, different starting materials were used to obtain the compounds of Examples 13-21.

| Example No. | Product structure | MS m/z (ESI) | ¹HNMR (400MHz, DMSO-*d₆*) |
|---|---|---|---|
| 13 | 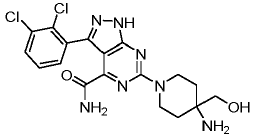 | 436.1 [M+1] | δ 8.13(s, 1H), 8.01-7.76(m, 3H), 7.70-7.61(m, 2H), 7.40(d, *J*=8.0Hz, 2H), 5.59(s, 1H), 4.40-4.12(m, 2H), 3.70-3.60(m, 4H), 1.90-1.74(m, 2H), 1.74-1.60(m, 2H) |
| 14 | 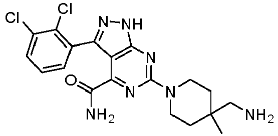 | 434.1 [M+1] | δ 8.06(s, 1H), 7.72(s, 3H), 7.67-7.61(m, 2H), 7.38(d, *J*=8.0Hz, 2H), 4.40-4.20(m, 2H), 3.62-3.45(m, 2H), 2.80(s, 2H), 1.58-1.47(m, 2H), 1.47-1.36(m, 2H), 1.10(s, 3H) |
| 15 | 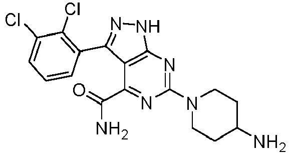 | 405.9 [M+1] | δ 8.12(s, 1H), 7.95(s, 3H), 7.75-7.55(m, 2H), 7.45-7.35(m, 2H), 5.0-4.60(m, 2H), 3.42-3.32(m, 1H), 3.06(t, J=16Hz, 2H), 2.08-1.91(m, 2H), 1.60-1.38(m, 2H) |
| 16 | 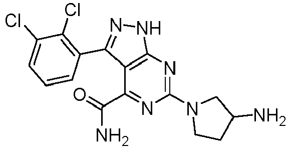 | 391.9 [M+1] | δ 8.08(s, 1H), 7.77(s, 3H), 7.68-7.60(m, 2H), 7.38(d, *J*=4.0Hz, 2H), 4.08-3.56(m, 6H), 2.41-2.26(m, 1H) |
| 17 | 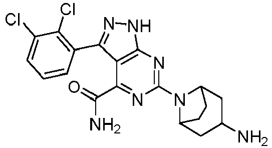 | 431.1 [M+1] | δ 8.12(s, 1H), 7.80-7.60(m, 5H), 7.45-7.35(m, 2H), 5.07(s, 1H), 4.67(s, 1H), 3.42-3.32(m, 1H), 2.06(s, 2H), 2.0-1.73(m, 5H), 1.73-1.58(m, 1H) |
| 18 | 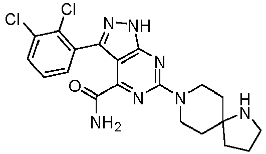 | 445.9 [M+1] | δ 8.88(s, 2H), 8.12(s, 1H), 7.70-7.60(m, 2H), 7.45-7.35(m, 2H), 4.60-4.20(m, 2H), 3.42-3.32(m, 3H), 2.13-1.93(m, 5H), 1.93-1.78(m, 4H) |
| 19 | 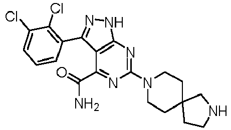 | 445.9 [M+1] | δ 8.88(s, 2H), 7.94(s, 1H), 7.53-7.45(m, 2H), 7.33-7.16(m, 2H), 3.90-3.80(m, 2H), 3.20-3.0(m, 4H), 3.0-2.80(m, 2H), 1.73(t, *J*=4.0Hz, 2H), 1.57-1.36(m, 4H) |
| 20 | 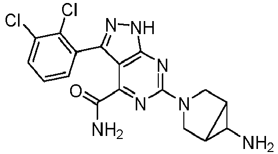 | 403.9 [M+1] | δ 8.25-8.20(m, 1H), 8.0-7.80(m, 3H), 7.75-7.61(m, 2H), 7.48-7.36(m, 2H), 4.0-3.77(m, 4H), 2.15-2.0(m, 3H) |
| 21 | 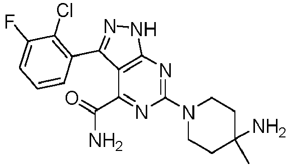 | 403.9 [M+1] | δ 8.11(s, 1H), 8.01(s, 3H), 7.65(s, 1H), 7.48-7.35(m, 2H), 7.25-7.33(m, 1H), 4.55-4.30(m, 2H), 4.0-3.7(m, 2H), 1.74(s, 4H), 1.41(s, 3H) |

### Example 22

### 2-(4-amino-4-methylpiperidin-1-yl)-5-(2,3-dichlorophenyl)-7H-pyrrolo[2,3-d]pyrimidine-4-carboxamide

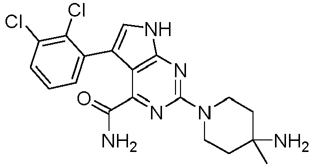

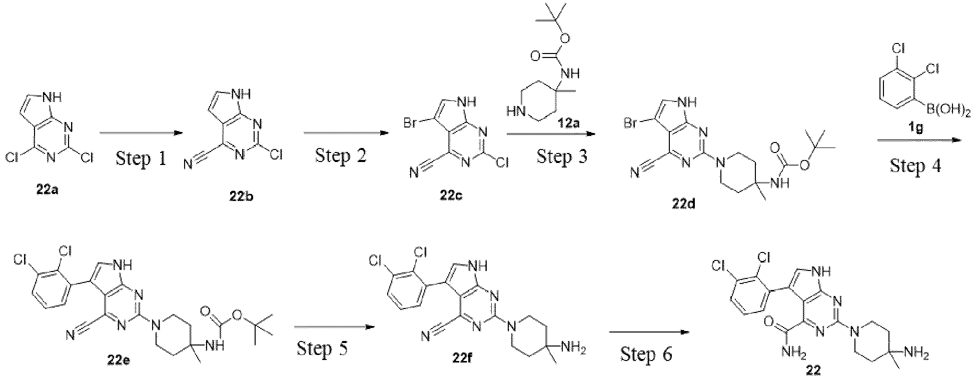

### Step 1

### 2-chloro-7H-pyrrolo[2,3-d]pyrimidine-4-carbonitrile

2,4-dichloro-7H-pyrrolo[2,3-d]pyrimidine **22a** (1 g, 5.32 mmol), tetrakis(triphenylphosphine)palladium (614.32 mg, 531.88 µmol) and zinc cyanide (1.25 g, 10.64 mmol) were added to N,N-dimethylformamide (20 mL), heated to 110°C and stirred for 4 hours after argon gas displacement. After the reaction was completed, the filtrate was concentrated under reduced pressure, and the obtained residue was further separated and purified by silica gel column chromatography (eluent: system A) to obtain 2-chloro-7H-pyrrolo[2,3-d]pyrimidine-4-carbonitrile **22b** (850 mg) with a yield of 89.49%.
MS m/z (ESI): 178.9 [M+1]

### Step 2

### 5-bromo-2-chloro-7H-pyrrolo[2,3-d]pyrimidine-4-carbonitrile

2-chloro-7H-pyrrolo[2,3-d]pyrimidine-4-carbonitrile **22b** (850 mg, 4.76 mmol) was added to a solution of acetonitrile (20 mL), added with bromosuccinimide (1,270 mg, 7.14 mmol), heated and refluxed, and stirred for 2 hours. After the reaction was completed, the filtrate was concentrated under reduced pressure, and the obtained residue was further separated and purified by silica gel column chromatography (eluent: system A) to obtain 5-bromo-2-chloro-7H-pyrrolo[2,3-d]pyrimidine-4-carbonitrile **22c** (1.05 g) with a yield of 85.68%. MS m/z (ESI): 256.8 [M+1]

### Step 3

### Tert-butyl(1-(5-bromo-4-cyano-7H-pyrrolo[2,3-d]pyrimidin-2-yl)-4-methylpiperidin-4-yl)carbamate

At room temperature, tert-butyl (4-methylpiperidin-4-yl)carbamate **12a** (174.79 mg, 815.61 µmol), N-methyl pyrrolidone (10 mL) and N,N-diisopropylethylamine (301.17 mg, 2.33 mmol, 384.83 µL) were added to a 100 mL single-necked round-bottom flask and shaken for 1 minute, then added with 5-bromo-2-chloro-7H-pyrrolo[2,3-d]pyrimidine-4-carbonitrile **22c** (200 mg, 776.78 µmol), heated to 110°C, and stirred for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA+ H₂O, mobile phase B: CH₃CN) to obtain tert-butyl (1-(5-bromo-4-cyano-7H-pyrrolo[2,3-d]pyrimidin-2-yl)-4-methylpiperidin-4-yl) carbamate **22d** (186 mg) with a yield of 55.01%.
MS m/z (ESI): 434.9 [M+1]

### Step 4

### Tert-butyl (1-(5-(2,3-dichlorophenyl)-4-cyano-7H-pyrrolo[2,3-d]pyrimidin-2-yl)-4-methylpiperidin-4-yl) carbamate

At room temperature, tert-butyl(1-(5-bromo-4-cyano-7H-pyrrolo[2,3-d]pyrimidin-2-yl)-4-methylpiperidin-4-yl) carbamate **22d** (186 mg, 427.27 µmol), (2,3-dichlorophenyl)boronic acid **1g** (326.13 mg, 1.71 mmol), 2-dicyclohexylphosphino-2',6'-di-isopropoxy-1,1'-biphenyl (79.75 mg, 170.91 µmol), methanesulfonato(2-dicyclohexylphosphino-2',6'-di-isopropoxy-1,1'-biphenyl)(2-amino-1,1'-biphenyl-2-yl)palladium (71.56 mg, 85.45 µmol) and potassium phosphate (272.09 mg, 1.28 mmol) were added to a 50 mL double-necked round-bottom flask, and finally added with 11 mL of mixed solution (1,4-dioxane: water = 10: 1), subjected to argon gas displacement thrice, heated to 100°C, and reacted for 16 hours. After the reaction was completed, the reaction solution was concentrated under reduced pressure, and then added with ethyl acetate (10 mL) and water (10 mL) for extraction and separation, and then aqueous phases were extracted with ethyl acetate (10 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain tert-butyl (1-(5-(2,3-dichlorophenyl)-4-cyano-7H-pyrrolo[2,3-d]pyrimidin-2-yl)-4-methylpiperidin-4-yl)carbamate 22e (159 mg) with a yield of 74.22%.
MS m/z (ESI): 500.9 [M+1]

### Step 5

### 2-(4-amino-4-methylpiperidin-1-yl)-5-(2,3-dichlorophenyl)-7H-pyrrolo[2,3-d]pyrimidine-4-carbonitrile

Tert-butyl (1-(5-(2,3-dichlorophenyl)-4-cyano-7H-pyrrolo[2,3-d]pyrimidin-2-yl)-4-methylpiperidin-4-yl)carbamate **22e** (159 mg, 317.11 µmol) and trifluoroacetic acid (1 mL) were added to a solution of dichloromethane (3 mL), and stirred at room temperature for 1 hour. After the reaction was completed, the reaction solution was concentrated under reduced pressure to obtain 2-(4-amino-4-methylpiperidin-1-yl)-5-(2,3-dichlorophenyl)-7H-pyrrolo[2,3-d]pyrimidine-4-carbonitrile **22f,** which was directly used for the next reaction without purification.
MS m/z (ESI): 400.7 [M+1]

### Step 6

### 2-(4-amino-4-methylpiperidin-1-yl)-5-(2,3-dichlorophenyl)-7H-pyrrolo[2,3-d]pyrimidine-4-carboxamide

2-(4-amino-4-methylpiperidin-1-yl)-5-(2,3-dichlorophenyl)-7H-pyrrolo[2,3-d]pyrimidine-4-carbonitrile **22f** (160 mg, 310.49 µmol) was dissolved in a mixed solution of methanol (1.00 mL), aqueous sodium hydroxide (5 M, 1.00 mL) and 30% hydrogen peroxide (0.5 mL), and the reaction solution was stirred for 0.5 hour at room temperature. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 2-(4-amino-4-methylpiperidin-1-yl)-5-(2,3-dichlorophenyl)-7H-pyrrolo[2,3-d]pyrimidine-4-carboxamide **22** (48 mg) with a yield of 28.99%.
MS m/z (ESI): 418.8 [M+1]
¹HNMR (400 MHz, DMSO-*d₆*) δ 8.0-7.80 (m, 4H), 7.54-7.48 (m, 1H), 7.48-7.43 (m, 1H), 7.33-7.28 (m, 3H), 4.45-4.35 (m, 2H), 3.45-3.35 (m, 2H), 1.78-1.65 (m, 4H), 1.41(s, 3H).

### Example 23

### 6-(4-amino-4-(2-chlorophenyl)piperidin-1-yl)-3-(2,3-dichlorophenyl)-1H-pyrazolo[3,4-d]pyrimidine-4-carboxylic acid

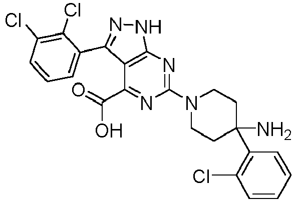

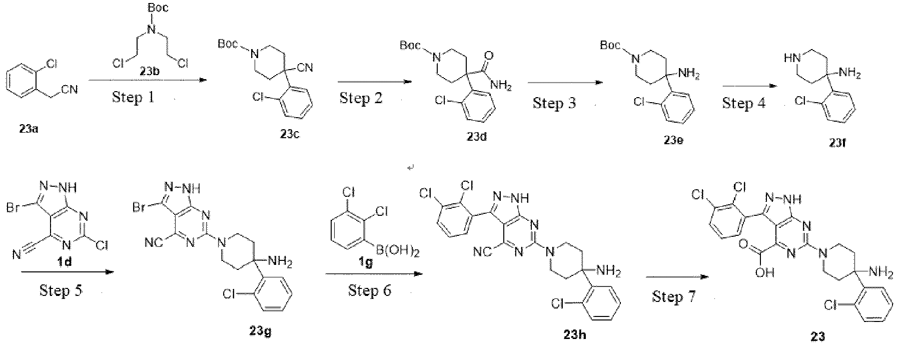

### Step 1

### Tert-butyl 4-(2-chlorophenyl)-4-cyanopiperidine-1-carboxylate

In an ice water bath, sodium hydride (2.40 g, 60 mmol, 60%) was added to a solution of N,N-dimethylformamide (15 mL) containing 2-(2-chlorophenyl)acetonitrile **23a** (1.52 g, 10 mmol) and tert-butyl bis(2-chloroethyl)carbamate **23b** (2.66 g, 11 mmol), stirred for 1 hour, heated to 60°C, and then stirred overnight. After the reaction was completed, the reaction solution was cooled to room temperature, quenched with a saturated aqueous ammonium chloride solution (30 mL), and added with ethyl acetate (30 mL) for extraction and separation, then aqueous phases were extracted with ethyl acetate (30 mL×2), and organic phases were combined, and washed with saturated salt water, dried by anhydrous sodium sulfate, and concentrated under reduced pressure to obtain crude product. The crude product was beaten with 100 mL of mixed solvent (ethyl acetate: petroleum ether = 10: 90), filtered and dried to obtain the product tert-butyl 4-(2-chlorophenyl)-4-cyanopiperidine-1-carboxylate **23c** (2.1 g) with a yield of 65.5%.
MS m/z (ESI): 338.1 [M+18]

### Step 2

### Tert-butyl 4-carbamoyl-4-(2-chlorophenyl)piperidine-1-carboxylate

Potassium hydroxide (735 mg, 13.1 mmol) and tert-butyl 4-(2-chlorophenyl)-4-cyanopiperidine-1-carboxylate **23c** (2.1 g, 6.55 mmol) were added to a solution of dimethylsulphoxide (15 mL), and hydrogen peroxide (30%, 6.5 mL) was slowly added dropwise to the reaction solution. After the dropwise addition was completed, the reaction solution was stirred for 1 hour. After the reaction was completed, the reaction solution was added with 50 mL of water to precipitate a yellow solid, and filtered, then the filter cake was washed with water, and dried in vacuum to obtain the product tert-butyl 4-carbamoyl-4-(2-chlorophenyl)piperidine-1-carboxylate **23d** (1.9 g) with a yield of 85.7%.
MS m/z (ESI): 282.9 [M-55]

### Step 3

### Tert-butyl 4-amino-4-(2-chlorophenyl)piperidine-1-carboxylate

Tert-butyl 4-carbamoyl-4-(2-chlorophenyl)piperidine-1-carboxylate **23d** (1.9 g, 5.61 mmol) was added to a mixed solution of acetonitrile (10 mL) and water (40 mL) containing potassium hydroxide (1.42 g, 25.23 mmol), and then added with 1,3-dibromo-5,5-dimethylhydantoin (882 mg, 3.08 mmol) in batches, and stirred at room temperature for 1 hour. After the reaction was completed, the reaction solution was added with sodium sulfite (70.6 mg, 0.56 mmol) and stirred for 15 minutes, then added with ethyl acetate (20 mL) and potassium phosphate (1.31 g, 6.17 mmol) for liquid separation, aqueous phases were extracted with ethyl acetate (50 mL×2), organic phases were combined and washed with a saturated brine solution, dried, and concentrated to obtain tert-butyl 4-amino-4-(2-chlorophenyl)piperidine-1-carboxylate **23e** (1.62 g) with a yield of 93.0%. MS m/z (ESI): 311.0 [M+1]

### Step 4

### 4-(2-chlorophenyl)piperidin-4-amine

Trifluoroacetic acid (1 mL) was dropwise added to 3 mL of dichloromethane solution containing tert-butyl 4-amino-4-(2-chlorophenyl)piperidine-1-carboxylate **23e** (200 mg, 643 µmol), and reacted at room temperature for 1 hour. The reaction solution was concentrated under reduced pressure to obtain 4-(2-chlorophenyl)piperidine-4-amine **23f,** which was directly used for the next reaction without purification.
MS m/z (ESI): 211.0 [M+1]

### Step 5

### 6-(4-amino-4-(2-chlorophenyl)piperidin-1-yl)-3-bromo-1H-pyrazolo[3,4-d]pyrimidine-4-carbonitrile

Diisopropylethylamine (91.4 mg, 707 µmol) and the above-mentioned crude product 4-(2-chlorophenyl)piperidine-4-amine **23f** were added to a solution of N-methyl pyrrolidone (5 mL) containing 3-bromo-6-chloro-1H-pyrazolo[3,4-d]pyrimidine-4-carbonitrile **1d** (150 mg, 580 µmol), heated to 100°C, and stirred for 1 hour. After the reaction was completed, the reaction solution was separated on a C₁₈ reversed phase column (C₁₈ separation column 20-45 µm; mobile phase A: H₂O, mobile phase B: CH₃CN) to obtain the product 6-(4-amino-4-(2-chlorophenyl)piperidin-1-yl)-3-bromo-1H-pyrazolo[3,4-d]pyrimidine-4-carbonitrile **23g** (200 mg) with a yield of 65.4%.
MS m/z (ESI): 414.8 [M-16]

### Step 6

### 6-(4-amino-4-(2-chlorophenyl)piperidin-1-yl)-3-(2,3-dichlorophenyl)-1H-pyrazolo[3,4-d]pyrimidine-4-carbonitrile

6-(4-amino-4-(2-chlorophenyl)piperidin-1-yl)-3-bromo-1H-pyrazolo[3,4-d]pyrimidine-4-carbonitrile **23g** (200 mg, 462 µmol), (2,3-dichlorophenyl)boronic acid **1g** (353 mg, 1.85 mmol), methanesulfonato(2-dicyclohexylphosphino-2',6'-di-isopropoxy-1,1'-biphenyl)(2-amino-1,1'-biphenyl-2-yl)palladium (77 mg, 92 µmol), 2-dicyclohexylphosphino-2',6'-di-isopropoxy-1,1'-biphenyl (86 mg, 185 µmol) and potassium phosphate (294 mg, 1.39 mmol) were added to a mixed solution of 1,4-dioxane (5 mL) and water (1 mL), subjected to argon gas displacement thrice, then heated to 100°C, and reacted overnight. After the reaction was completed, the reaction solution was concentrated under reduced pressure, and added with ethyl acetate (10 mL) and water (10 mL) for extraction and separation, then aqueous phases were extracted with ethyl acetate (10 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain 6-(4-amino-4-(2-chlorophenyl)piperidin-1-yl)-3-(2,3-dichlorophenyl)-1H-pyrazolo[3,4-d]pyrimidine-4-carbonitrile **23h** (90 mg) with a yield of 39.0%.
MS m/z (ESI): 480.8 [M-16]

### Step 7

### 6-(4-amino-4-(2-chlorophenyl)piperidin-1-yl)-3-(2,3-dichlorophenyl)-1H-pyrazolo[3,4-d]pyrimidine-4-carboxylic acid

6-(4-amino-4-(2-chlorophenyl)piperidin-1-yl)-3-(2,3-dichlorophenyl)-1H-pyrazolo[3,4-d]pyrimidine-4-carbonitrile **23h** (90 mg, 180 µmol) was added to 3 mL of concentrated hydrochloric acid, and heated and refluxed for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2-chlorophenyl)piperidin-1-yl)-3-(2,3-dichlorophenyl)-1H-pyrazolo[3,4-d]pyrimidine-4-carboxylic acid 23 (12 mg) with a yield of 10%.
MS m/z (ESI): 516.8 [M+1]
¹HNMR (400 MHz, CD₃OD) δ 7.72-7.82 (m 1H), 7.57-7.67 (m, 2H), 7.46-7.56 (m, 2H), 7.33-7.45 (m, 2H), 4.21-4.52 (m, 2H), 3.78-4.03 (m, 2H), 2.87-3.04 (m, 2H), 2.23-2.39 (m, 2H).

### Example 24

### 6-(4-amino-4-(4-chlorophenyl)piperidin-1-yl)-3-(2,3-dichlorophenyl)-1H-pyrazolo[3,4-d]pyrimidine-4-carboxylic acid

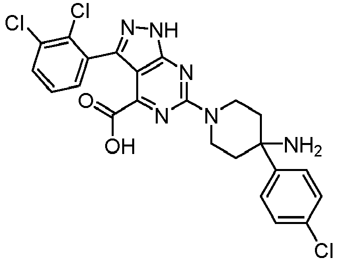

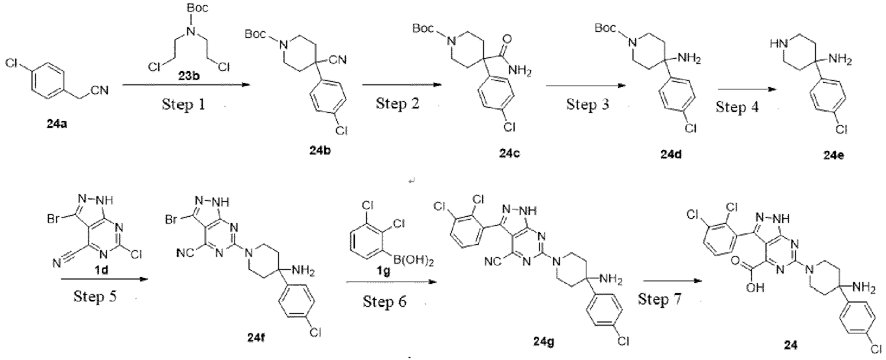

### Step 1

### Tert-butyl 4-(4-chlorophenyl)-4-cyanopiperidine-1-carboxylate

In an ice water bath, sodium hydride (2.41 g, 60.2 mmol, 60%) was added to a solution of N,N-dimethylformamide (15 mL) containing 2-(4-chlorophenyl)acetonitrile **24a** (1.52 g, 10 mmol) and tert-butyl bis(2-chloroethyl)carbamate **23b** (2.67 g, 11 mmol), stirred for 1 hour, heated to 60°C, and then stirred overnight. After the reaction was completed, the reaction solution was cooled to room temperature, quenched with a saturated aqueous ammonium chloride solution (30 mL), then added with ethyl acetate (30 mL) for extraction and separation, then aqueous phases were washed with ethyl acetate (30 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain the product tert-butyl 4-(4-chlorophenyl)-4-cyanopiperidine-1-carboxylate **24b** (2.4 g) with a yield of 74.6%.
MS m/z (ESI): 338.1 [M+18]

### Step 2

### Tert-butyl 4-carbamoyl-4-(4-chlorophenyl)piperidine-1-carboxylate

Potassium hydroxide (840 mg, 15.0 mmol) and tert-butyl 4-(4-chlorophenyl)-4-cyanopiperidine-1-carboxylate **24b** (2.40 g, 7.48 mmol) were added to a solution of dimethylsulphoxide (15 mL), and hydrogen peroxide (30%, 6.5 mL) was slowly added dropwise to the reaction solution. After the dropwise addition was completed, the reaction solution was stirred for 1 hour. After the reaction was completed, the reaction solution was added with 50 mL of water to precipitate a yellow solid, and filtered, then the filter cake was washed with water, and dried in vacuum to obtain the product tert-butyl 4-carbamoyl-4-(4-chlorophenyl)piperidine-1-carboxylate **24c** (2.2 g) with a yield of 86.8%.
MS m/z (ESI): 283.1 [M-55]

### Step 3

### Tert-butyl 4-amino-4-(4-chlorophenyl)piperidine-1-carboxylate

Tert-butyl 4-carbamoyl-4-(4-chlorophenyl)piperidine-1-crboxylate **24c** (1.0 g, 2.95 mmol) was added to a mixed solution of acetonitrile (2.5 mL) and water (10 mL) containing potassium hydroxide (745 mg, 13.3 mmol), and then added with dibromohydantoin (464 mg, 1.62 mmol) in batches, and stirred at room temperature for 1 hour. After the reaction was completed, the reaction solution was added with sodium sulfite (37.8 mg, 0.3 mmol) and stirred for 15 minutes, then added with ethyl acetate (5 mL) and potassium phosphate (688 mg, 3.25 mmol) for liquid separation, aqueous phases were extracted with ethyl acetate (5 mL×2), organic phases were combined and washed with a saturated salt water, dried, and concentrated to obtain tert-butyl 4-amino-4-(4-chlorophenyl)piperidine-1-carboxylate **24d** (0.85 g) with a yield of 92.7%.
MS m/z (ESI): 237.9 [M-72]

### Step 4

### 4-(4-chlorophenyl)piperidin-4-amine

Trifluoroacetic acid (1 mL) was dropwise added to 3 mL of dichloromethane solution containing tert-butyl 4-amino-4-(4-chlorophenyl)piperidine-1-carboxylate **24d** (200 mg, 643 µmol), and reacted at room temperature for 1 hour. The reaction solution was concentrated under reduced pressure to obtain 4-(4-chlorophenyl)piperidin-4-amine **24e,** which was directly used for the next reaction without purification.
MS m/z (ESI): 211.0 [M+1]

### Step 5

### 6-(4-amino-4-(4-chlorophenyl)piperidin-1-yl)-3-bromo-1H-pyrazolo[3,4-d]pyrimidine-4-carbonitrile

Diisopropylethylamine (91.4 mg, 707 µmol) and the above-mentioned crude product 4-(4-chlorophenyl)piperidin-4-amine **24e** were added to a solution of N-methyl pyrrolidone (5 mL) containing 3-bromo-6-chloro-1H-pyrazolo[3,4-d]pyrimidine-4-carbonitrile **1d** (150 mg, 580 µmol), heated to 100°C, and stirred for 1 hour. After the reaction was completed, the reaction solution was separated on a C₁₈ reversed phase column (C₁₈ separation column 20-45 µm; mobile phase A: H₂O, mobile phase B: CH₃CN) to obtain the product 6-(4-amino-4-(4-chlorophenyl)piperidin-1-yl)-3-bromo-1H-pyrazolo[3,4-d]pyrimidine-4-carbonitrile **24f** (225 mg) with a yield of 73.5%.
MS m/z (ESI): 414.8 [M-16]

### Step 6

### 6-(4-amino-4-(4-chlorophenyl)piperidin-1-yl)-3-(2,3-dichlorophenyl)-1H-pyrazolo[3,4-d]pyrimidine-4-carbonitrile

6-(4-amino-4-(4-chlorophenyl)piperidin-1-yl)-3-bromo-1H-pyrazolo[3,4-d]pyrimidine-4-carbonitrile **24f** (225 mg, 519.99 µmol), (2,3-dichlorophenyl)boronic acid **1g** (396.89 mg, 2.08 mmol), methanesulfonato(2-dicyclohexylphosphino-2',6'-di-isopropoxy-1,1'-biphenyl)(2-amino-1,1'-biphenyl-2-yl)palladium (87.08 mg, 104.00 µmol), 2-dicyclohexylphosphino-2',6'-di-isopropoxy-1.1'-biphenyl (97.06 mg, 207.99 µmol) and potassium phosphate (331 mg, 1.56 mmol) were added to a mixed solution of 1,4-dioxane (5 mL) and water (1 mL), subjected to argon gas displacement thrice, then heated to 100°C, and reacted overnight. After the reaction was completed, the reaction solution was concentrated under reduced pressure, and added with ethyl acetate (10 mL) and water (10 mL) for extraction and separation, then aqueous phases were extracted with ethyl acetate (10 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain 6-(4-amino-4-(4-chlorophenyl)piperidin-1-yl)-3-(2,3-dichlorophenyl)-1H-pyrazolo[3,4-d]pyrimidine-4-carbonitrile **24g** (95 mg) with a yield of 36.6%.
MS m/z (ESI): 480.8 [M-16]

### Step 7

### 6-(4-amino-4-(4-chlorophenyl)piperidin-1-yl)-3-(2,3-dichlorophenyl)-1H-pyrazolo[3,4-d]pyrimidine-4-carboxylic acid

6-(4-amino-4-(4-chlorophenyl)piperidin-1-yl)-3-(2,3-dichlorophenyl)-1H-pyrazolo[3,4-d]pyrimidine-4-carbonitrile **24g** (95 mg, 190 µmol) was added to 3 mL of concentrated hydrochloric acid, and heated and refluxed for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(4-chlorophenyl)piperidin-1-yl)-3-(2,3-dichlorophenyl)-1H-pyrazolo[3,4-d]pyrimidine-4-carboxylic acid **24** (15 mg) with a yield of 8.8%.
MS m/z (ESI): 516.8 [M+1]

### Example 25

### 6-(4-amino-4-(3-chlorophenyl)piperidin-1-yl)-3-(2,3-dichlorophenyl)-1H-pyrazolo[3,4-d]pyrimidine-4-carboxylic acid

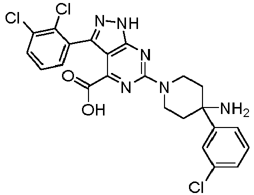

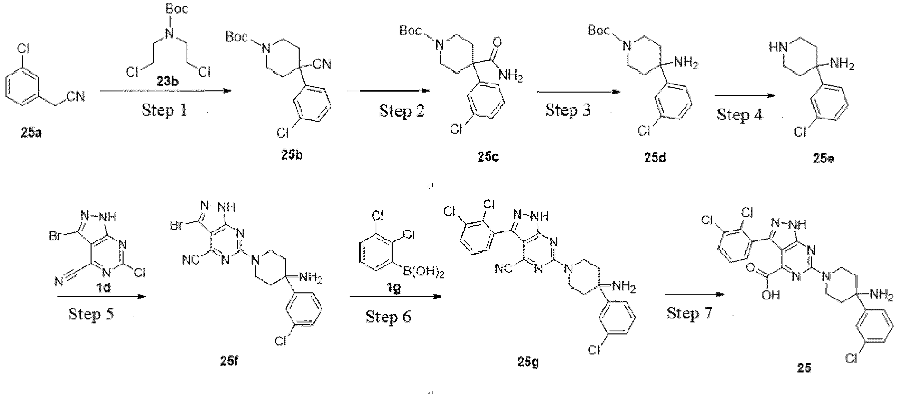

### Step 1

### Tert-butyl 4-(3-chlorophenyl)-4-cyanopiperidine-1-carboxylate

In an ice water bath, sodium hydride (2.4 g, 60 mmol, 60%) was added to a solution of N,N-dimethylformamide (15 mL) containing 2-(3-chlorophenyl)acetonitrile **25a** (1.52 g, 10 mmol) and tert-butyl bis(2-chloroethyl)carbamate **23b** (2.66 g, 11 mmol), stirred for 1 hour, heated to 60°C, and then stirred overnight. After the reaction was completed, the reaction solution was cooled to room temperature, quenched with a saturated aqueous ammonium chloride solution (30 mL), then added with ethyl acetate (30 mL) for extraction and separation, then aqueous phases were washed with ethyl acetate (30 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain the product tert-butyl 4-(3-chlorophenyl)-4-cyanopiperidine-1-carboxylate **25b** (2.19 g) with a yield of 68.3%.
MS m/z (ESI): 338.1 [M+18]

### Step 2

### Tert-butyl 4-carbamoyl-4-(3-chlorophenyl)piperidine-1-carboxylate

Potassium hydroxide (770 mg, 13.7 mmol) and tert-butyl 4-(3-chlorophenyl)-4-cyanopiperidine-1-carboxylate **25b** (2.2 g, 6.86 mmol) were added to a solution of dimethylsulphoxide (15 mL), and hydrogen peroxide (30%, 6.5 mL) was slowly added dropwise to the reaction solution. After the dropwise addition was completed, the reaction solution was stirred for 1 hour. After the reaction was completed, the reaction solution was added with 50 mL of water to precipitate a yellow solid, and filtered, then the filter cake was washed with water, and dried in vacuum to obtain the product tert-butyl 4-carbamoyl-4-(3-chlorophenyl)piperidine-1-carboxylate **25c** (2.28 g) with a yield of 98.1%.
MS m/z (ESI): 260.9 [M+23]

### Step 3

### Tert-butyl 4-amino-4-(3-chlorophenyl)piperidine-1-carboxylate

Tert-butyl 4-carbamoyl-4-(3-chlorophenyl)piperidine-1-carboxylate **25c** (2.28 g, 6.73 mmol) and [Bis(trifluoroacetoxy)iodo]benzene (3.18 g, 7.40 mmol) were added to a mixed solution of acetonitrile (15 mL) and water (15 mL) containing potassium hydroxide (566 mg, 10 mmol), and reacted at room temperature overnight. After the reaction was completed, the reaction solution was separated on a C₁₈ reversed phase column (C₁₈ separation column 20-45 µm; mobile phase A: H₂O, mobile phase B: CH₃CN) to obtain tert-butyl 4-amino-4-(3-chlorophenyl)piperidine-1-carboxylate **25d** (0.9 g) with a yield of 43.1%.
MS m/z (ESI): 237.9 [M-72]

### Step 4

### 4-(3-chlorophenyl)piperidin-4-amine

Trifluoroacetic acid (1 mL) was dropwise added to 3 mL of dichloromethane solution containing tert-butyl 4-amino-4-(3-chlorophenyl)piperidine-1-carboxylate **25d** (200 mg, 643 µmol), and reacted at room temperature for 1 hour. The reaction solution was concentrated under reduced pressure to obtain 4-(3-chlorophenyl)piperidin-4-amine **25e,** which was directly used for the next reaction without purification.
MS m/z (ESI): 211.0 [M+1]

### Step 5

### 6-(4-amino-4-(3-chlorophenyl)piperidin-1-yl)-3-bromo-1H-pyrazolo[3,4-d]pyrimidine-4-carbonitrile

Diisopropylethylamine (91.4 mg, 707 µmol) and the above-mentioned crude product 4-(3-chlorophenyl)piperidin-4-amine **25e** were added to a solution of N-methyl pyrrolidone (5 mL) containing 3-bromo-6-chloro-1H-pyrazolo[3,4-d]pyrimidine-4-carbonitrile **1d** (150 mg, 580 µmol), heated to 100°C, and stirred for 1 hour. After the reaction was completed, the reaction solution was separated on a C₁₈ reversed phase column (C₁₈ separation column 20-45 µm; mobile phase A: H₂O, mobile phase B: CH₃CN) to obtain the product 6-(4-amino-4-(3-chlorophenyl)piperidin-1-yl)-3-bromo-1H-pyrazolo[3,4-d]pyrimidine-4-carbonitrile **25f** (230 mg) with a yield of 75.2%.
MS m/z (ESI): 414.8 [M-16]

### Step 6

### 6-(4-amino-4-(3-chlorophenyl)piperidin-1-yl)-3-(2,3-dichlorophenyl)-1H-pyrazolo[3,4-d]pyrimidine-4-carbonitrile

6-(4-amino-4-(3-chlorophenyl)piperidin-1-yl)-3-bromo-1H-pyrazolo[3,4-d]pyrimidine-4-carbonitrile **25f (100** mg, 231 µmol), (2,3-dichlorophenyl)boronic acid **1g** (176 mg, 924 µmol), methanesulfonato(2-dicyclohexylphosphino-2',6'-di-isopropoxy-1,1'-biphenyl)(2-amino-1,1'-biphenyl-2-yl)palladium (38.7 mg, 46.2 µmol), 2-dicyclohexylphosphino-2',6'-di-isopropoxy-1,1'-biphenyl (43 mg, 92 µmol) and potassium phosphate (147 mg, 693 µmol) were added to a mixed solution of 1,4-dioxane (5 mL) and water (1 mL), subjected to argon gas displacement thrice, then heated to 100°C, and reacted overnight. After the reaction was completed, the reaction solution was concentrated under reduced pressure, and added with ethyl acetate (10 mL) and water (10 mL) for extraction and separation, then aqueous phases were extracted with ethyl acetate (10 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain 6-(4-amino-4-(3-chlorophenyl)piperidin-1-yl)-3-(2,3-dichlorophenyl)-1H-pyrazolo[3,4-d]pyrimidine-4-carbonitrile **25g** (75 mg) with a yield of 65.1%.
MS m/z (ESI): 480.8 [M-16]

### Step 7

### 6-(4-amino-4-(3-chlorophenyl)piperidin-1-yl)-3-(2,3-dichlorophenyl)-1H-pyrazolo[3,4-d]pyrimidine-4-carboxylic acid

6-(4-amino-4-(3-chlorophenyl)piperidin-1-yl)-3-(2,3-dichlorophenyl)-1H-pyrazolo[3,4-d]pyrimidine-4-carbonitrile **25g** (75 mg, 150 µmol) was added to 3 mL of concentrated hydrochloric acid, and heated and refluxed for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(3-chlorophenyl)piperidin-1-yl)-3-(2,3-dichlorophenyl)-1H-pyrazolo[3,4-d]pyrimidine-4-carboxylic acid **25** (7 mg) with a yield of 4.7%.
MS m/z (ESI): 516.8 [M+1]

### Example 26

### 6-(4-amino-4-(1H-indazole-5-yl)piperidin-1-yl)-3-(2,3-dichlorophenyl)-1H-pyrazolo[3,4-d]pyrimidine-4-carboxamide

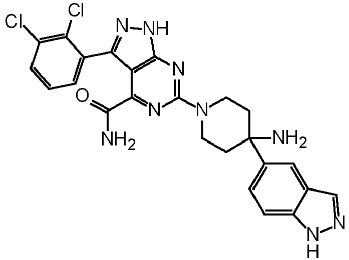

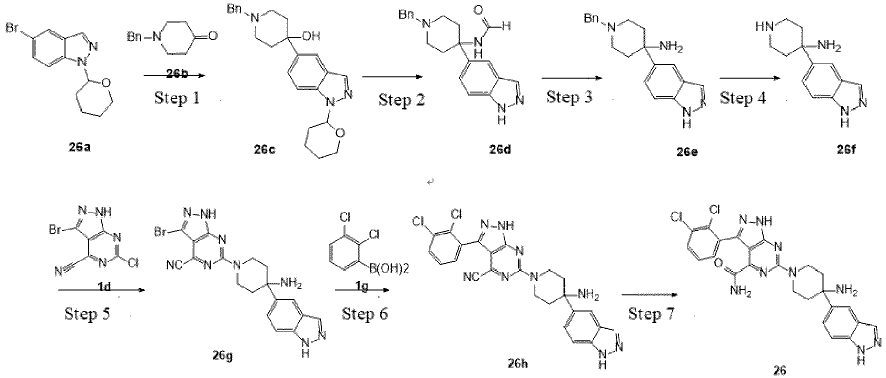

### Step 1

### 1-benzyl-4-(1-(tetrahydro-2H-pyran-2-yl)-1H-indazole-5-yl)piperidin-4-ol

5-bromo-1-(tetrahydro-2H-pyran-2-yl)-1H-indazole **26a** (321.16 mg, 1.70 mmol, prepared according to patent WO 2017060326) was added to 5 mL of tetrahydrofuran, cooled to -78°C, then dropwise added with n-butyl lithium (2.5 M, 783.22 µL), stirred at -78°C for 0.5 hour, added with 1-benzylpiperidine-4-one **26b** (321.16 mg, 1.70 mmol), stirred at -78°C for 1 hour, and then heated to -20°C. After the reaction was completed, the reaction solution was quenched with a saturated ammonium chloride solution, extracted with ethyl acetate (10 mL×2), then organic phases were combined, dried with anhydrous sodium sulfate, filtered, and concentrated under reduced pressure. The obtained residue was further purified by silica gel column chromatography (eluent: system A) to obtain 1-benzyl-4-(1-(tetrahydro-2H-pyran-2-yl)-1H-indazole-5-yl)piperidin-4-ol **26c** (350 mg) with a yield of 68.49%.
MS m/z (ESI): 392.1 [M+1]

### Step 2

### N-(1-benzyl-4-(1H-indazole-5-yl)piperidin-4-yl)carboxamide

1-benzyl-4-(1-(tetrahydro-2H-pyran-2-yl)-1H-indazole-5-yl)piperidin-4-ol **26c** (1 g, 2.55 mmol) was added to 10 mL of trifluoroacetic acid, cooled to -15°C, added with trimethylsilyl cyanide 1.01 g, 10.22 mmol, 1.28 mL) and 2 mL of concentrated sulfuric acid, slowly heated to room temperature, and stirred overnight. After the reaction was completed, the reaction solution was poured into ice water to adjust the pH to be 7-8 with a 6N sodium hydroxide solution, extracted with dichloromethane (30 mL×3), dried and filtered, and concentrated under reduced pressure. The obtained residue was purified by silica gel column chromatography (eluent: system B) to obtain N-(1-benzyl-4-(1H-indazole-5-yl)piperidin-4-yl)carboxamide **26d** (530 mg) with a yield of 62.05%.
MS m/z (ESI): 335.1 [M+1]

### Step 3

### 1-benzyl-4-(1H-indazole-5-yl)piperidine-4-amine

N-(1-benzyl-4-(1H-indazole-5-yl)piperidin-4-yl)carboxamide **26d** (530 mg, 1.58 mmol) and 1 mL of concentrated hydrochloric acid were added to 2 mL of methanol, heated to 80°C, and reacted for 2 hours. The reaction solutoin was cooled to room temperature, and then concentrated under reduced pressure to obtain 1-benzyl-4-(1H-indazole-5-yl)piperidine-4-amine **26e** (450 mg) with a yield of 92.67%, which was directly used for the next reaction without purification.
MS m/z (ESI): 307.0 [M+1]

### Step 4

### 4-(1H-indazole-5-yl)piperidine-4-amine

1-benzyl-4-(1H-indazole-5-yl)piperidine-4-amine **26e** (500 mg, 1.63 mmol) and palladium hydroxide/carbon (180 mg, 1.63 mmol) were added to 15 mL of methanol, subjected to vacuum replacement thrice, and stirred at room temperature overnight. After the reaction was completed, the reaction solution is filtered through diatomite, and then concentrated under reduced pressure to obtain 4-(1H-indazole-5-yl)piperidine-4-amine **26f** (200 mg) with a yield of 56.67%, which was directly used for the next reaction without purification.
MS m/z (ESI): 200.0 [M-16]

### Step 5

### 6-(4-amino-4-(1H-indazole-5-yl)piperidin-1-yl)-3-bromo-1H-pyrazolo[3,4-d]pyrimidine-4-carbonitrile

4-(1H-indazole-5-yl)piperidine-4-amine **26f** (80.33 mg, 371.43 µmol), 3-bromo-6-chloro-1H-pyrazolo[3,4-d]pyrimidine-4-carbonitrile **1d** (80 mg, 309.52 µmol) and N,N-diisopropylethylamine (120.01 mg, 928.57 µmol) were added to 2 mL of dimethylacetamide, heated to 95°C, and stirred for 2 hours. After the reaction was completed, the reaction solution was cooled to room temperature and concentrated under reduced pressure. The obtained residue was purified by silica gel column chromatography (eluent: system B) to obtain 6-(4-amino-4-(1H-indazole-5-yl)piperidin-1-yl)-3-bromo-1H-pyrazolo[3,4-d]pyrimidine-4-carbonitrile **26g** (120 mg) with a yield of 88.46%.
MS m/z (ESI): 421.1 [M-16]

### Step 6

### 6-(4-amino-4-(1H-indazole-5-yl)piperidin-1-yl)-3-(2,3-dichlorophenyl)-1H-pyrazolo[3,4-d]pyrimidine-4-carbonitrile

6-(4-amino-4-(1H-indazole-5-yl)piperidin-1-yl)-3-bromo-1H-pyrazolo[3,4-d]pyrimidine-4-carbonitrile **26g** (120 mg, 273.80 µmol), (2,3-dichlorophenyl)boronic acid **1g** (208.98 mg, 1.10 mmol), methanesulfonato(2-dicyclohexylphosphino-2',6'-di-isopropoxy-1,1'-biphenyl)(2-amino-1,1'-biphenyl-2-yl)palladium (45.85 mg, 54.76 µmol), 2-dicyclohexylphosphino-2',6'-di-isopropoxy-1.1'-biphenyl (51.04 mg, 109.52 µmol) and potassium phosphate (174.13 mg, 821.39 µmol) wre added to 5.5 mL of mixed solution (1,4-dioxane: water = 10: 1) in turn, subjected to argon gas displacement thrice, heated to 100°C, and stirred overnight. After the reaction was completed, the reaction solution was cooled to room temperature and concentrated under reduced pressure. The obtained residue was purified by silica gel column chromatography (eluent: system B) to obtain 6-(4-amino-4-(1H-indazole-5-yl)piperidin-1-yl)-3-(2,3-dichlorophenyl)-1H-pyrazolo[3,4-d]pyrimidine-4-carbonitrile **26h** (80 mg) with a yield of 57.93%.
MS m/z (ESI): 487.0 [M-16]

### Step 7

### 6-(4-amino-4-(1H-indazole-5-yl)piperidin-1-yl)-3-(2,3-dichlorophenyl)-1H-pyrazolo[3,4-d]pyrimidine-4-carboxamide

6-(4-amino-4-(1H-indazole-5-yl)piperidin-1-yl)-3-(2,3-dichlorophenyl)-1H-pyrazolo[3,4-d]pyrimidine-4-carbonitrile **26h** (80 mg, 158.61 µmol) and potassium hydroxide (17.80 mg, 317.23 µmol) were added to 2 mL of dimethyl sulfoxide, added with 1 mL of hydrogen peroxide in an ice water bath, heated to room temperature and stirred for 3 hours. After the reaction was completed, a small amount of trifluoroacetic acid was added in the reaction solution to adjust the pH to be 7-8, and then the reaction solution was concentrated under reduced pressure. The obtained residue was subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain the product 6-(4-amino-4-(1H-indazole-5-yl)piperidin-1-yl)-3-(2,3-dichlorophenyl)-1H-pyrazolo[3,4-d]pyrimidine-4-carboxamide **26** (19 mg) with a yield of 17.84%. MS m/z (ESI): 505.1 [M-16]

### Example 27

### 6-(4-amino-4-phenylpiperidin-1-yl)-3-(2,3-dichloropyridin-4-yl)-1H-pyrazolo[3,4-d]pyrimidine-4-carboxamide

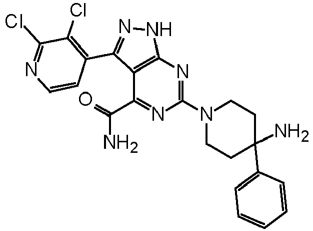

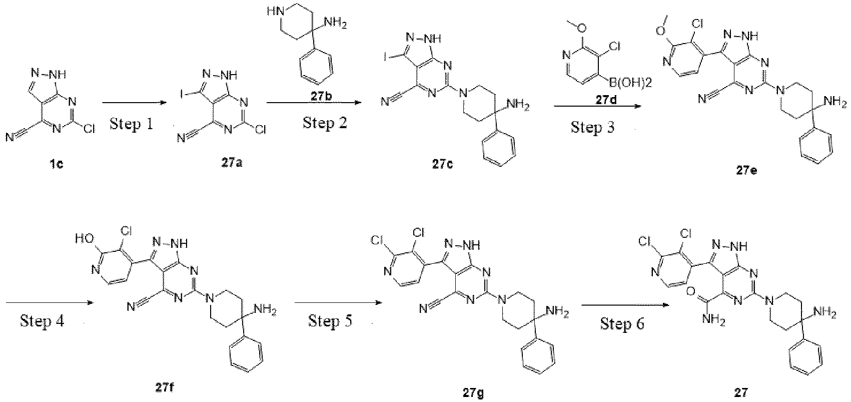

### Step 1

### 3-iodine-6-chloro-1H-pyrazolo[3,4-d]pyrimidine-4-carbonitrile

6-chloro-1H-pyrazolo[3,4-d]pyrimidine-4-carbonitrile **1c** (290 mg, 1.62 mmol) and iodosuccinimide (726.69 mg, 3.23 mmol) were added to 10 mL of dichloroethane, heated to 80°C, and reacted for 4 hours. After the reaction was completed, the reaction solution was concentrated under reduced pressure. The obtained residue was purified by silica gel column chromatography (eluent: system A) to obtain 3-iodine-6-chloro-1H-pyrazolo[3,4-d]pyrimidine-4-carbonitrile 27a (250 mg) with a yield of 50.68%.

### Step 2

### 6-(4-amino-4-phenylpiperidin-1-yl)-3-iodine-1H-pyrazolo[3,4-d]pyrimidine-4-carbonitrile

4-phenylpiperidine-4-amine **27b** (144.25 mg, 818.43 µmol), 3-iodine-6-chloro-1H-pyrazolo[3,4-d]pyrimidine-4-carbonitrile **27a** (250 mg, 818.43 µmol) and N,N-diisopropylethylamine (317.32 mg, 2.46 mmol) were added to 3 mL of N,N-dimethylacetamide, heated to 90°C, and stirred for 2 hours. After the reaction was completed, the reaction solution was cooled to room temperature, and poured into ice water to precipitate a soild, then stirred for 10 minutes to collect the soild. Then the solid was dried under vacuum to obtain 6-(4-amino-4-phenylpiperidin-1-yl)-3-iodine-1H-pyrazolo[3,4-d]pyrimidine-4-carbonitrile **27c** (300 mg) with a yield of 82.32%.
MS m/z (ESI): 428.8 [M-16]

### Step 3

### 6-(4-amino-4-phenylpiperidin-1-yl)-3-(3-chloro-2-methoxypyridin-4-yl)-1H-pyrazolo[3,4-d]pyrimidine-4-carbonitrile

6-(4-amino-4-phenylpiperidin-1-yl)-3-iodine-1H-pyrazolo[3,4-d]pyrimidine-4-carbonitrile 27c (150 mg, 336.88 µmol), (3-chloro-2-methoxypyridin-4-yl)boronic acid **27d** (157.82 mg, 842.21 µmol), methanesulfonato(2-dicyclohexylphosphino-2',6'-di-isopropoxy-1,1'-biphenyl)(2-amino-1,1'-biphenyl-2-yl)palladium (56.42 mg, 67.38 µmol), 2-dicyclohexylphosphino-2',6'-di-isopropoxy-1,1'-biphenyl (62.79 mg, 134.75 µmol) and potassium phosphate (214.26 mg, 1.01mmol) were added to 3.3 mL of mixed solution of (1,4-dioxane: water = 10: 1) in turn, subjected to argon gas displacement trice, heated to 100°C, and reacted for 2 hours. After the reaction was completed, the reaction solution was concentrated under reduced pressure. The obtained residue was purified by silica gel column chromatography (eluent: system B) to obtain 6-(4-amino-4-phenylpiperidin-1-yl)-3-(3-chloro-2-methoxypyridin-4-yl)-1H-pyrazolo[3,4-d]pyrimidine-4-carbonitrile **27e** (40 mg) with a yield of 25.76%.
MS m/z (ESI): 443.8 [M-16]

### Step 4

### 6-(4-amino-4-phenylpiperidin-1-yl)-3-(3-chloro-2-hydroxypyridin-4-yl)-1H-pyrazolo[3,4-d]pyrimidine-4-carbonitrile

6-(4-amino-4-phenylpiperidin-1-yl)-3-(3-chloro-2-methoxypyridin-4-yl)-1H-pyrazolo[3,4-d]pyrimidine-4-carbonitrile **27e** (40 mg, 86.78 µmol) and 2 mL of 4M hydrochloric acid dioxane solution were added to 1 mL of dichloromethane, heated to 50°C and stirred for 4 hours. After the reaction was completed, the reaction solution was separated on a C₁₈ reversed phase column (C₁₈ separation column 20-45 µm; mobile phase A: H₂O, mobile phase B: CH₃CN) to obtain 6-(4-amino-4-phenylpiperidin-1-yl)-3-(3-chloro-2-hydroxypyridin-4-yl)-1H-pyrazolo[3,4-d]pyrimidine-4-carbonitrile **27f (30** mg) with a yield of 77.35%.
MS m/z (ESI): 429.9 [M-16]

### Step 5

### 6-(4-amino-4-phenylpiperidin-1-yl)-3-(2,3-dichloropyridin-4-yl)-1H-pyrazolo[3,4-d]pyrimidine-4-carbonitrile

6-(4-amino-4-phenylpiperidin-1-yl)-3-(3-chloro-2-hydroxypyridin-4-yl)-1H-pyrazolo[3,4-d]pyrimidine-4-carbonitrile **27f** (30 mg, 67.13 µmol) was added to 1.5 mL of phosphorus oxychloride, heated to 110°C, and stirred overnight. After the reaction was completed, the reaction solution was cooled to room temperature, and poured to ice water, stirred for 0.5 hour, extracted with ethyl acetate (10 mL×3), then organic phases were combined and washed with a saturated sodium bicarbonate solution, dried with anhydrous sodium sulfate, filtered, and concentrated under reduced pressure to obtian 6-(4-amino-4-phenylpiperidin-1-yl)-3-(2,3-dichloropyridin-4-yl)-1H-pyrazolo[3,4-d]pyrimidine-4-carbonitrile **27g** (30 mg) with a yield of 96.04%, which was directly used for the next reaction without purification.
MS m/z (ESI): 448.1 [M-16]

### Step 6

### 6-(4-amino-4-phenylpiperidin-1-yl)-3-(2,3-dichloropyridin-4-yl)-1H-pyrazolo[3,4-d]pyrimidine-4-carboxamide

6-(4-amino-4-phenylpiperidin-1-yl)-3-(2,3-dichloropyridin-4-yl)-1H-pyrazolo[3,4-d]pyrimidine-4-carbonitrile **27g** (30 mg, 64.47 µmol), sodium hydroxide (5.16 mg, 128.94 µmol) and 0.5 mL of hydrogen peroxide were added to 1 mL of dimethyl sulfoxide, and stirred at room temperature for 1 hour. After the reaction was completed, a small amount of trifluoroacetic acid was added in the reaction solution to adjust the pH to be 7-8, and then the reaction solution was concentrated under reduced pressure. The obtained residue was subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain the product 6-(4-amino-4-phenylpiperidin-1-yl)-3-(2,3-dichloropyridin-4-yl)-1H-pyrazolo[3,4-d]pyrimidine-4-carboxamide **27** (4.1 mg) with a yield of 10.01%.
MS m/z (ESI): 482.8 [M+1]
¹H NMR (400 MHz, CD₃OD) δ 8.36 (d, *J=* 4.9 Hz, 1H), 7.72 (d, *J=* 7.7 Hz, 2H), 7.58 (t, *J=* 7.6 Hz, 2H), 7.50 (s, 1H), 7.44 (d, *J=* 4.8 Hz, 1H), 4.69 (d, *J=* 14.2 Hz, 2H), 3.47 (t, *J=* 12.2 Hz, 2H), 2.77 (d, *J* = 13.8 Hz, 2H), 2.15 (ddd, J = 14.2, 10.9, 3.8 Hz, 2H).

### Example 28

### 6-(4-amino-4-phenylpiperidin-1-yl)-3-(3-chloro-2-methoxypyridin-4-yl)-1H-pyrazolo[3,4-d]pyrimidine-4-carboxamide

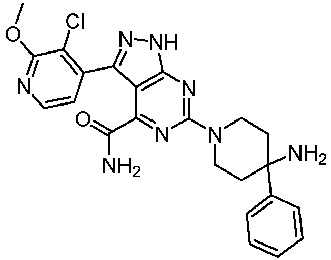

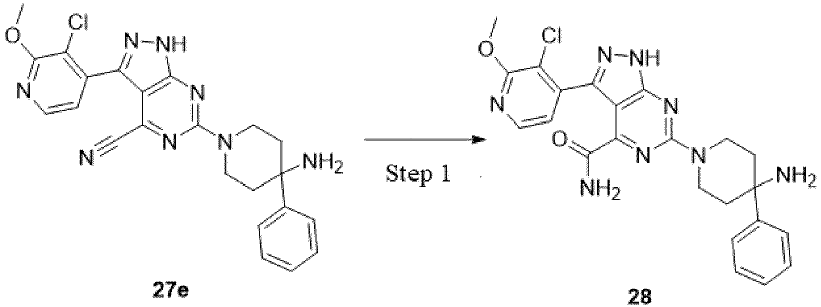

### Step 1

### 6-(4-amino-4-phenylpiperidin-1-yl)-3-(3-chloro-2-methoxypyridin-4-yl)-1H-pyrazolo[3,4-d]pyrimidine-4-carboxamide

6-(4-amino-4-phenylpiperidin-1-yl)-3-(3-chloro-2-methoxypyridin-4-yl)-1H-pyrazolo[3,4-d]pyrimidine-4-carbonitrile 27e was dissolved in methanol (10 mL), added with sodium hydroxide (99.82 mg, 2.50 mmol) and hydrogen peroxide (1 mL) in turn, and then stirred at room temperature for 2 hours. After the reaction was completed, a trifluoroacetic acid was used to adjust the pH to be 7, then the reaction solution was concentrated under reduced pressure, and subjected to separation to obtain 6-(4-amino-4-phenylpiperidin-1-yl)-3-(3-chloro-2-methoxypyridin-4-yl)-1H-pyrazolo[3,4-d]pyrimidine-4-carboxamide **28** (32 mg) with a yield of 10.38%.
MS m/z (ESI): 478.9 [M+1]
¹H NMR (400 MHz, CD₃OD) δ 8.06-8.12 (m, 1H), 7.72 (d, *J* = 7.8 Hz, 2H), 7.57 (t, *J* = 7.6 Hz, 2H), 7.51 (d, *J=* 7.2 Hz, 1H), 6.99-7.05 (m, 1H), 4.68 (d, *J=* 14.2 Hz, 2H), 4.01 (d, *J=* 1.3 Hz, 3H), 3.46 (t, *J=* 12.2 Hz, 2H), 2.77 (d, *J=* 14.0 Hz, 2H), 2.08-2.23 (m, 2H).

### Example 29

### 6-(4-amino-4-phenylpiperidin-1-yl)-3-(2-chloro-3-methylphenyl)-1H-pyrazolo[3,4-d]pyrimidine-4-carboxamide

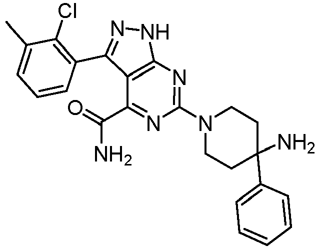

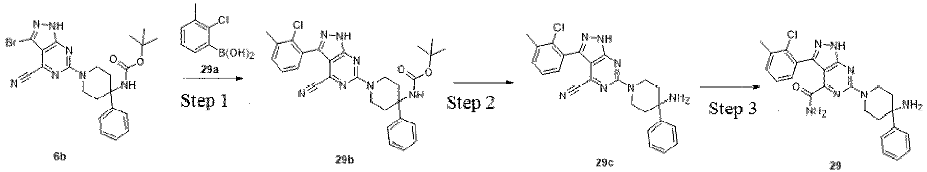

### Step 1

### Tert-butyl(1-(3-(2-chloro-3-methylphenyl)-4-cyano-1H-pyrazolo[3,4-d]pyrimidin-6-yl)-4-phenylpiperidin-4-yl)carbamate

Under the protection of argon gas, tert-butyl (1-(3-bromo-4-cyano-1H-pyrazolo[3,4-d]pyrimidin-6-yl)-4-phenylpiperidin-4-yl)carbamate **6b** (100 mg, 200.65 µmol), (2-chloro-3-methylphenyl)boronic acid **29a** (136.77 mg, 802.61 µmol), methanesulfonato(2-dicyclohexylphosphino-2',6'-di-isopropoxy-1,1'-biphenyl)(2-amino-1,1'-biphenyl-2-yl)palladium (33.60 mg, 40.13 µmol), 2-dicyclohexylphosphino-2',6'-di-isopropoxy-1,1'-biphenyl (37.40 mg, 80.26 µmol) and potassium phosphate (212.69 mg, 1.00 mmol) were added to 11 mL of mixed solution (1,4-dioxane: water = 10:1) in turn, heated to 130°C, and reacted for 3 hours. After the reaction was completed, the reaction solution was concentrated under reduced pressure. The obtained residue was further analyzed and purified by silica gel column chromatography (eluent: system A) to obtain tert-butyl (1-(3-(2-chloro-3-methylphenyl)-4-cyano-1H-pyrazolo[3,4-d]pyrimidin-6-yl)-4-phenylpiperidin-4-yl)carbamate **29b** (100 mg) with a yield of 91.61%.
MS m/z (ESI): 543.9 [M+1]

### Step 2

### 6-(4-amino-4-phenylpiperidin-1-yl)-3-(2-chloro-3-methylphenyl)-1H-pyrazolo[3,4-d]pyrimidine-4-carbonitrile

Tert-butyl (1-(3-(2-chloro-3-methylphenyl)-4-cyano-1H-pyrazolo[3,4-d]pyrimidin-6-yl)-4-phenylpiperidin-4-yl)carbamate **29b** (100 mg, 183.81 µmol) and trifluoroacetic acid (0.5 mL) were added to dichloromethane (1.5 mL) in turn, and reacted at room temperature for 1 hour. After the reaction was completed, the reaction solution was concentrated under reduced pressure to obtain 6-(4-amino-4-phenylpiperidin-1-yl)-3-(2-chloro-3-methylphenyl)-1H-pyrazolo[3,4-d]pyrimidine-4-carbonitrile 29c (81 mg) with a yield of 99.27%, which was directly used for the next reaction without purification.
MS m/z (ESI): 426.9 [M-16]

### Step 3

### 6-(4-amino-4-phenylpiperidin-1-yl)-3-(2-chloro-3-methylphenyl)-1H-pyrazolo[3,4-d]pyrimidine-4-carboxamide

6-(4-amino-4-phenylpiperidin-1-yl)-3-(2-chloro-3-methylphenyl)-1H-pyrazolo[3,4-d]pyrimidine-4-carbonitrile **29c** (81 mg, 182.46 µmol) and sodium hydroxide (43.79 mg, 1.09 mmol) were added to methanol (2 mL), added with hydrogen peroxide (0.2 mL), and stirred at room temperature for 2 hours. After the reaction was completed, a trifluoroacetic acid was used to adjust the pH to be 7, then the reaction solution was concentrated under reduced pressure, and subjected to separation to obtain 6-(4-amino-4-phenylpiperidin-1-yl)-3-(2-chloro-3-methylphenyl)-1H-pyrazolo[3,4-d]pyrimidine-4-carboxamide **29** (50 mg) with a yield of 47.37%. MS m/z (ESI): 462.2 [M+1] ¹H NMR (400 MHz, CD₃OD) δ 7.72 (d, *J=* 7.8 Hz, 2H), 7.57 (t, *J=* 7.6 Hz, 2H), 7.50 (d, *J=* 7.3 Hz, 1H), 7.34 (d, *J* = 7.1 Hz, 1H), 7.21-7.30 (m, 2H), 4.67 (d, *J* = 14.4 Hz, 2H), 3.45 (t, *J* = 12.0 Hz, 2H), 2.76 (d, *J* = 13.9 Hz, 2H), 2.40 (s, 3H), 2.16 (td, *J* = 10.2, 5.0 Hz, 2H).

### Example 30

### 1-(3-(2,3-dichlorophenyl)-4-(1H-tetrazol-5-yl)-1H-pyrazolo[3,4-d]pyrimidin-6-yl)-4-phenylpiperidin-4-amine

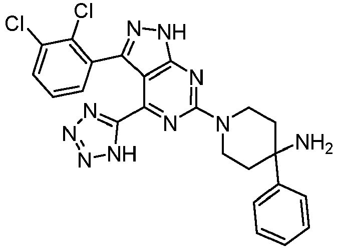

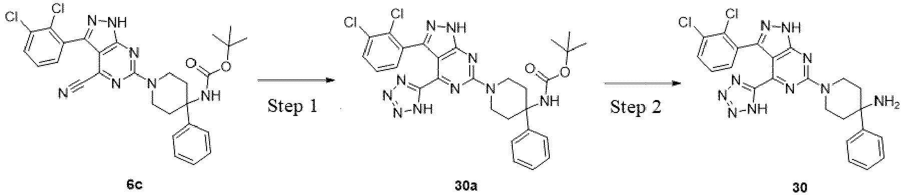

### Step 1

### Tert-butyl (1-(3-(2,3-dichlorophenyl)-4-(1H-tetrazol-5-yl)-1H-pyrazolo[3,4-d]pyrimidin-6-yl)-4-phenylpiperidin-4-yl) carbamate

Tert-butyl(1-(4-cyano-3-(2,3-dichlorophenyl)-1H-pyrazolo[3,4-d]pyrimidin-6-yl)-4-phenylpiperidin-4-yl)carbamate **6c** (200 mg, 354.32 µmol), sodium azide (230.31 mg, 3.54 mmol) and ammonium chloride (191.33 mg, 3.54 mmol)were added to N,N-dimethylformamide (3 mL), heated to 130°C, and reacted for 6 hours. After the reaction was completed, the reaction solution was cooled to room temperature, then an appropriate amount of water was added to the reaction solution to quench the reaction, a solid was precipitated, then the solid was filtered and dried to obtain tert-butyl (1-(3-(2,3-dichlorophenyl)-4-(1H-tetrazol-5-yl)-1H-pyrazolo[3,4-d]pyrimidin-6-yl)-4-phenylpiperidine-4-yl)carbamate **30a** (150 mg) with a yield of 69.69%, which was directly used for the next reaction without purification.
MS m/z (ESI): 607.2 [M+1]

### Step 2

### 1-(3-(2,3-dichlorophenyl)-4-(1H-tetrazol-5-yl)-1H-pyrazolo[3,4-d]pyrimidin-6-yl)-4-phenylpiperidin-4-amine

Tert-butyl (1-(3-(2,3-dichlorophenyl)-4-(1H-tetrazol-5-yl)-1H-pyrazolo[3,4-d]pyrimidin-6-yl)-4-phenylpiperidin-4-yl)carbamate **30a** (150 mg, 246.92 µmol) and trifluoroacetic acid (0.5 mL) were added to dichloromethane (2 mL), and stirred at room temperature for 2 hours. After the reaction was completed, the reaction solution was concentrated under reduced pressure, and subjected to separation to obtain 1-(3-(2,3-dichlorophenyl)-4-(1H-tetrazol-5-yl)-1H-pyrazolo[3,4-d]pyrimidin-6-yl)-4-phenylpiperidin-4-amine **30** (24 mg) with a yield of 15.33%.
MS m/z (ESI): 507.1 [M+1]
¹H NMR (400 MHz, CD₃OD) δ 7.73 (d, *J* = 7.9 Hz, 2H), 7.59 (q, *J* = 8.1, 7.6 Hz, 3H), 7.51 (d, *J* = 7.3 Hz, 1H), 7.43 (d, *J* = 7.6 Hz, 1H), 7.38 (d, *J* = 7.8 Hz, 1H), 4.73 (d, *J* = 14.1 Hz, 2H), 3.49 (t, *J* = 12.1 Hz, 2H), 2.78 (d, *J* = 14.0 Hz, 2H), 2.18 (td, *J* = 11.0, 10.6, 5.5 Hz, 2H).

### Example 32

### 6-(4-amino-4-(2-fluorophenyl)piperidin-1-yl)-3-(3-chloro-2-methoxypyridin-4-yl)-1H-pyrazolo[3,4-d]pyrimidine-4-carboxamide

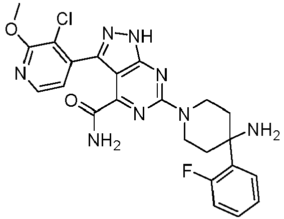

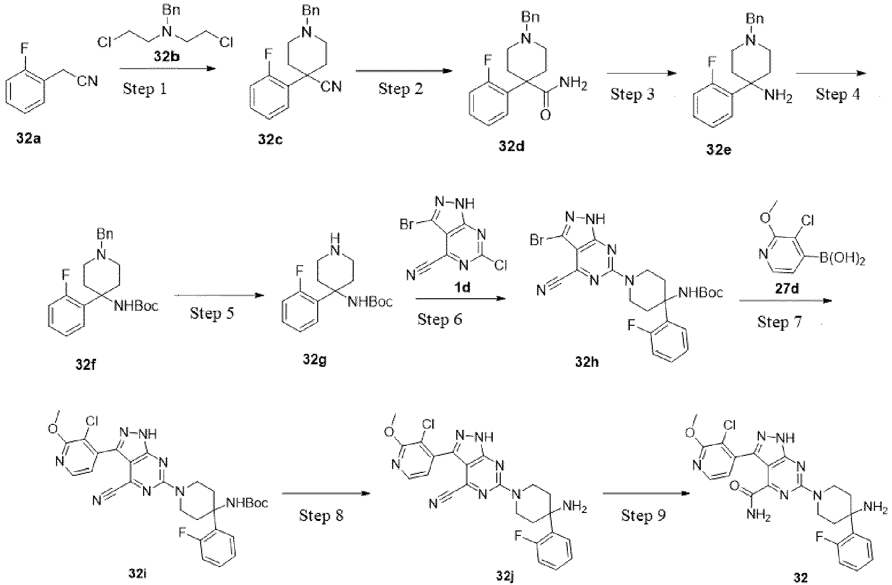

### Step 1

### 1-benzyl-4-(2-fluorophenyl) piperidine-4-carbonitrile

In an ice water bath, sodium hydride (3.55 g, 88.80 mmol) was added to N,N-dimethylformamide (10 mL) containing 2-(2-fluorophenyl)acetonitrile **32a** (2 g, 14.80 mmol) and N-benzyl-2-chloro-N-(2-chloroethyl)ethan-1-amine **32b** (3.78 g, 16.28 mmol), stirred in an ice bath for 1 hour, heated to 60°C, and then stirred for 16 hours. After the reaction was completed, the reaction solution was poured to 100 mL of ice water, and stirred for 0.5 hour to collect a solid, then beated with petroleum ether, filtered and dried to obtain a solid. The mothor liquor was extracted with ethyl acetate, dried by anhydrous sodium sulfate, and then concentrated under reduced pressure. The obtained residue was further analyzed and purified by silica gel column chromatography (eluent: system A) to obtain 1-benzyl-4-(2-fluorophenyl) piperidine-4-carbonitrile **32c** (4.1 g) with a yield of 94.11%.
MS m/z (ESI): 295.0 [M+1]

### Step 2

### 1-benzyl-4-(2-fluorophenyl)piperidine-4-carboxamide

Potassium hydroxide (1.56 g, 27.86 mmol) and 1-benzyl-4-(2-fluorophenyl) piperidine-4-carbonitrile **32c** (4.1 g, 13.93 mmol) were added to a solution of dimethyl sulfoxide (10 mL), and hydrogen peroxide (10 mL) was slowly added dropwise to the reaction solution. After the dropwise addition was completed, the reaction solution was stirred at room temperature for 5 hours. After the reaction was completed, the reaction solution was added with water (50 mL) to precipitate a yellow solid, and filtered, then the filter cake was washed with water, and dried in vacuum to obtain 1-benzyl-4-(2-fluorophenyl)piperidine-4-carboxamide **32d** (3.1 g) with a yield of 71.25%. MS m/z (ESI): 313.1 [M+1]

### Step 3

### 1-benzyl-4-(2-fluorophenyl)piperidin-4-amine

Potassium hydroxide (2.51 g, 44.66 mmol) was added to a mixed solution of acetonitrile (20 mL) and water (30 mL) containing 1-benzyl-4-(2-fluorophenyl)piperidine-4-carboxamide **32d** (3.1 g, 9.92 mmol) and then added with 1,3-dibromo-5,5-dimethylhydantoin (1.56 g, 5.46 mmol) in a water bath in batches, and stirred at room temperature for 1 hour. After the reaction was completed, the reaction solution was added with sodium sulfite (125.04 mg, 992.38 µmol) and stirred at room temperature for 15 minutes, then added with ethyl acetate (50 mL) and potassium phosphate (1.32 g, 10.92 mmol) for liquid separation, aqueous phases were extracted with ethyl acetate (50 mL×2), organic phases were combined and washed with a sodium chloride solution, and dried. The obtained residue was further analyzed and purified by silica gel column chromatography (eluent: system A) to obtain 1-benzyl-4-(2-fluorophenyl)piperidin-4-amine **32e** (2.4 g) with a yield of 85.05%.
MS m/z (ESI): 285.1 [M+1]

### Step 4

### Tert-butyl (1-benzyl-4-(2-fluorophenyl)piperidin-4-yl)carbamate

1-benzyl-4-(2-fluorophenyl)piperidin-4-amine **32e** (2.4 g, 8.44 mmol), sodium hydroxide (405.11 mg, 10.13 mmol) and di-tert-butyl dicarbonate (3.68 g, 16.88 mmol) were added to a mixed solution of water (5 mL) and 1,4-dioxane (10 mL), and stirred at room temperature for 3 hours. After the reaction was completed, the reaction solution was filtered, and the filtrate was extracted with ethyl acetate (50 mL×3), dried with anhydrous sodium sulfate, and then concentrated under reduced pressure. The obtained residue was further analyzed and purified by silica gel column chromatography (eluent: system A) to obtain tert-butyl (1-benzyl-4-(2-fluorophenyl)piperidin-4-yl)carbamate **32f** (1.9 g) with a yield of 58.55%.
MS m/z (ESI): 385.1 [M+1]

### Step 5

### Tert-butyl (4-(2-fluorophenyl)piperidin-4-yl)carbamate

Tert-butyl (1-benzyl-4-(2-fluorophenyl)piperidin-4-yl)carbamate **32f** (900 mg, 2.34 mmol) and 10% palladium carbon (450 mg) were added to methanol (30 mL), and hydrogenated at room temperature and pressure in hydrogen for 18 hours. After the reaction was completed, the reaction solution was filtered by diatomite to remove Pd/C, rinsed with methanol, and then concentrated under reduced pressure to obtain tert-butyl (4-(2-fluorophenyl)piperidin-4-yl)carbamate 32g (689 mg) with a yield of 99.99%, which was directly used for the next reaction without purification. MS m/z (ESI): 295.0 [M+1]

### Step 6

### Tert-butyl (1-(3-bromo-4-cyano-1H-pyrazolo[3,4-d]pyrimidin-6-yl)-4-(2-fluorophenyl)piperidin-4-yl)carbamate

N,N-diisopropylethylamine (737.60 mg, 5.71 mmol, 943.22 µL), tert-butyl (4-(2-fluorophenyl)piperidin-4-yl) carbamate **32g** (560 mg, 1.90 mmol) and 3-bromo-6-chloro-1H-pyrazolo[3,4-d]pyrimidine-4-carbonitrile **1d** (491.70 mg, 1.90 mmol) were added to a solution of N,N-dimethylacetamide (3 mL), heated to 100°C, and stirred for 1 hour. After the reaction was completed, the mixed solution was poured with 100 mL of water and extracted with ethyl acetate (50 mL×3), then organic phases were combined, dried with anhydrous sodium sulfate, filtered, and concentrated under reduced pressure. The obtained residue was further analyzed and purified by silica gel column chromatography (eluent: system A) to obtain tert-butyl (1-(3-bromo-4-cyano-1H-pyrazolo[3,4-d]pyrimidin-6-yl)-4-(2-fluorophenyl)piperidin-4-yl)carbamate **32h** (600 mg) with a yield of 61.08%.
MS m/z (ESI): 516.1 [M+1]

### Step 7

### Tert-butyl(1-(3-(3-chloro-2-methoxypyridin-4-yl)-4-cyano-1H-pyrazolo[3,4-d]pyrimidin-6-yl)-4-(2-fluorophenyl)piperidin-4-yl)carbamate

Tert-butyl(1-(3-bromo-4-cyano-1H-pyrazolo[3,4-d]pyrimidin-6-yl)-4-(2-fluorophenyl)piperidin-4-yl) carbamate **32h** (250 mg, 484.15 µmol), (3-chloro-2-methoxypyridin-4-yl)boronic acid **27d** (272.17 mg, 1.45 mmol), methanesulfonato(2-dicyclohexylphosphino-2',6'-di-isopropoxy-1,1'-biphenyl)(2-amino-1,1'-biphenyl-2-yl)palladium (162.17 mg, 193.66 µmol), 2-dicyclohexylphosphino-2',6'-di-isopropoxy-1,1'-biphenyl (180.49 mg, 387.32 µmol) and potassium phosphate (513.20 mg, 2.42 mmol) were added to a mixed solution of 1,4-dioxane (6 mL) and water (0.6 mL), subjected to argon gas displacement thrice, heated to 130°C, and reacted for 4.5 hours. After the reaction was completed, the reaction solution was concentrated under reduced pressure. The obtained residue was further analyzed and purified by silica gel column chromatography (eluent: system A) to obtain tert-butyl(1-(3-(3-chloro-2-methoxypyridin-4-yl)-4-cyano-1H-pyrazolo[3,4-d]pyrimidin-6-yl)-4-(2-fluorophenyl)piperidin-4-yl)carbamate **32i** (160 mg) with a yield of 57.07%.
MS m/z (ESI): 578.9 [M+1]

### Step 8

### 6-(4-amino-4-(2-fluorophenyl)piperidin-1-yl)-3-(3-chloro-2-methoxypyridin-4-yl)-1H-pyrazolo[3,4-d]pyrimidine-4-carbonitrile

Tert-butyl(1-(3-(3-chloro-2-methoxypyridin-4-yl)-4-cyano-1H-pyrazolo[3,4-d]pyrimidin-6-yl)-4-(2-fluorophenyl)piperidin-4-yl)tert-butyl carbamate **32i** (30 mg, 51.81 µmol) and trifluoroacetic acid (1 mL) were added to a solution of dichloromethane (3 mL), and stirred at room temperature for 1 hour. After the reaction was completed, the reaction solution was concentrated under reduced pressure to obtain 6-(4-amino-4-(2-fluorophenyl)piperidin-1-yl)-3-(3-chloro-2-methoxypyridin-4-yl)-1H-pyrazolo[3,4-d]pyrimidine-4-carbonitrile **32j,** which was directly used for the next reaction without purification.
MS m/z (ESI): 462.1 [M-16]

### Step 9

### 6-(4-amino-4-(2-fluorophenyl)piperidin-1-yl)-3-(3-chloro-2-methoxypyridin-4-yl)-1H-pyrazolo[3,4-d]pyrimidine-4-carboxamide

The above-mentioned crude product 6-(4-amino-4-(2-fluorophenyl)piperidin-1-yl)-3-(3-chloro-2-methoxypyridin-4-yl)-1H-pyrazolo[3,4-d]pyrimidine-4-carbonitrile **32j** was dissolved in methanol (3 mL), added with sodium hydroxide (10.36 mg, 258.92 µmol) and hydrogen peroxide (0.3 mL) in turn, and then stirred at room temperature for 2 hours. After the reaction was completed, a trifluoroacetic acid was used to adjust the pH to be 7, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2-fluorophenyl)piperidin-1-yl)-3-(3-chloro-2-methoxypyridin-4-yl)-1H-pyrazolo[3,4-d]pyrimidine-4-carboxamide **32** (7 mg) with a yield of 27.2%.
MS m/z (ESI): 497.2 [M+1]
¹H NMR (400 MHz, CD₃OD) δ 8.01 (d, *J* = 5.0 Hz, 1H), 7.61 (s, 1H), 7.47 (dd, *J* = 5.9, 2.0 Hz, 1H), 7.17-7.32 (m, 2H), 6.94 (d, *J=* 5.1 Hz, 1H), 4.35-4.45 (m, 2H), 3.92 (s, 3H), 3.64 (ddd, *J* = 13.6, 9.7, 3.1 Hz, 2H), 2.64-2.73 (m, 2H), 2.06-2.15 (m, 2H).

### Example 33

### 6-(4-amino-4-(2-(trifluoromethyl)phenyl)piperidin-1-yl)-3-(2,3-dichlorophenyl)-1H-pyrazolo[3,4-d]pyrimidine-4-carboxamide

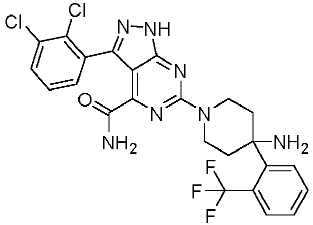

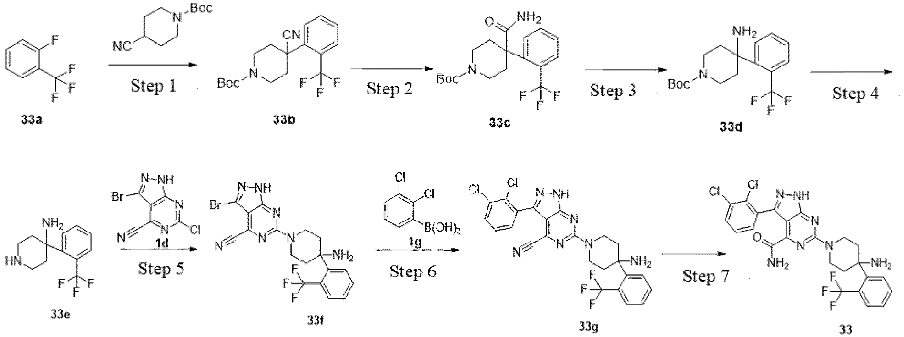

### Step 1

### Tert-butyl 4-cyano-4-(2-(trifluoromethyl)phenyl)piperidine-1-carboxylate

1-fluoro-2-(trifluoromethyl)benzene **33a** (1.56 g, 9.51 mmol), tert-butyl 4-cyanopiperidine-1-carboxylate (2 g, 9.51 mmol) and a 1 M tetrahydrofuran solution (2.85 g, 14.27 mmol) containing potassium bis(trimethylsilyl)amide were added to 20 mL of toluene, heated to 70°C, and reacted overnight. After the reaction was completed, the reaction solution was added with 20 mL of water and ethyl acetate (20 mL×3) for extraction, and then washed with a saturated sodium chloride solution (20 mL). Organic phases were dried with anhydrous sodium sulfate, filtered, concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, and mobile phase B: CH₃CN) to obtain tert-butyl 4-cyano-4-(2-(trifluoromethyl)phenyl)piperidine-1-tert-carboxylate **33b** (410 mg) with a yield of 12.16%. MS m/z (ESI): 255.1 [M-99]

### Step 2

Tert-butyl 4-carbamoyl-4-(2-(trifluoromethyl)phenyl)piperidine-1-carboxylate Potassium hydroxide (129.52 mg, 2.31 mmol) and tert-butyl 4-cyano-4-(2-(trifluoromethyl)phenyl)piperidine-1-carboxylate **33b** (409 mg, 1.15 mmol) were added to a solution of dimethyl sulfoxide (2 mL), and hydrogen peroxide (0.8 mL) was slowly added to the reaction solution in a water bath, then the reaction solution was heated to room temperature, and reacted for 30 minutes. After the reaction was completed, the reaction solution was added with 100 mL of water to precipitate a large amount of solids, and the liquid was removed by filtration to obtain tert-butyl 4-carbamoyl-4-(2-(trifluoromethyl)phenyl)piperidine-1-carboxylate **33c** (400 mg) with a yield of 93.07%.
MS m/z (ESI): 317.1 [M-55]

### Step 3

Tert-butyl 4-amino-4-(2-(trifluoromethyl)phenyl)piperidine-1-carboxylate Potassium hydroxide (13.56 mg, 241.69 µmol) and tert-butyl 4-carbamoyl-4-(2-(trifluoromethyl)phenyl)piperidine-1-carboxylate **33c** (20 mg, 53.71 µmol) were added to a mixed solution of acetonitrile (5 mL) and water (5 mL), added with 1,3-dibromo-5,5-dimethylhydantoin (8.45 mg, 29.54 µmol) in a water bath in batches, and stirred at room temperature for 1 hour. After the reaction was completed, the reaction solution was concentrated under reduced pressure, and separated on a C₁₈ reversed phase chromatographic column (C₁₈ separation column 20-45 µm; mobile phase A: H₂O, mobile phase B: CH₃CN) to obtain tert-butyl 4-amino-4-(2-(trifluoromethyl)phenyl)piperidine-1-carboxylate **33d** (18.5 mg) with a yield of 100%.
MS m/z (ESI): 289.1 [M-55]

### Step 4

### 4-(2-(trifluoromethyl)phenyl)piperidin-4-amine

Tert-butyl 4-amino-4-(2-(trifluoromethyl)phenyl)piperidine-1-carboxylate **33d** (199 mg, 577.87 µmol) and 0.5 mL of trifluoroacetic acid were added to 2 mL of dichloromethane solution, and continuously reacted for 1 hour. After the reaction was completed, the reaction solution was concentrated under reduced pressure to obtain 4-(2-(trifluoromethyl)phenyl)piperidin-4-amine **33e** (141 mg) with a yield of 99.9%, which was directly used for the next reaction without purification. MS m/z (ESI): 228.1 [M-16]

### Step 5

### 6-(4-amino-4-(2-(trifluoromethyl)phenyl)piperidin-1-yl)-3-bromo-1H-pyrazolo[3,4-d]pyrimidine-4-carbonitrile

3-bromo-6-chloro-1H-pyrazolo[3,4-d]pyrimidine-4-carbonitrile **1d** (136 mg, 526.19 µmol) was added to N-methyl pyrrolidone (2 mL), added with N,N-dimethylacetamide (203.47 mg, 1.57 mmol) and 4-(2-(trifluoromethyl)phenyl)piperidin-4-amine **33e** (141 mg, 577.26 µmol), heated to 80°C, and reacted for 1 hour. After the reaction was completed, the reaction solution was concentrated under reduced pressure. The obtained residue was purified by silica gel column chromatography (eluent: system B) to obtain 6-(4-amino-4-(2-(trifluoromethyl)phenyl)piperidin-1-yl)-3-bromo-1H-pyrazolo[3,4-d]pyrimidine-4-carbonitrile **33f** (190 mg) with a yield of 77.65%. MS m/z (ESI): 449.0 [M-16]

### Step 6

### 6-(4-amino-4-(2-(trifluoromethyl)phenyl)piperidin-1-yl)-3-(2,3-dichlorophenyl)-1H-pyrazolo[3,4-d]pyrimidine-4-carbonitrile

6-(4-amino-4-(2-(trifluoromethyl)phenyl)piperidin-1-yl)-3-bromo-1H-pyrazolo[3,4-d]pyrimidine-4-carbonitrile **33f** (190 mg, 407.50 µmol), (2,3-dichlorophenyl)boronic acid **1g** (311.04 mg, 1.63 mmol), methanesulfonato(2-dicyclohexylphosphino-2',6'-di-isopropoxy-1,1'-biphenyl)(2-amino-1,1'-biphenyl-2-yl)palladium (68.25 mg, 81.50 µmol), 2-dicyclohexylphosphino-2',6'-di-isopropoxy-1,1'-biphenyl (76.06 mg, 163.00 µmol) and potassium phosphate (259.62 mg, 1.22 mmol) were added to a mixed solution of 1,4-dioxane (8 mL) and water (0.8 mL), subjected to argon gas displacement thrice, heated to 100°C, and reacted for 16 hours. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2-(trifluoromethyl)phenyl)piperidine-1-yl)-3-(2,3-dichlorophenyl)-1H-pyrazolo[3,4-d]pyrimidine-4-carbonitrile **33g** (75 mg) with a yield of 34.57%. MS m/z (ESI): 515.0 [M-16]

### Step 7

### 6-(4-amino-4-(2-(trifluoromethyl)phenyl)piperidin-1-yl)-3-(2,3-dichlorophenyl)-1H-pyrazolo[3,4-d]pyrimidine-4-carboxamide

5 M sodium hydroxide (0.5 mL) was added to a solution of methanol (1 mL) containing 6-(4-amino-4-(2-(trifluoromethyl)phenyl)piperidin-1-yl)-3-(2,3-dichlorophenyl)-1H-pyrazolo[3,4-d]pyrimidine-4-carbonitrile **33g** (55 mg, 103.32 µmol), then added with hydrogen peroxide (0.5 mL), and stirred at room temperature for 1 hour. After the reaction was completed, a trifluoroacetic acid was added to adjust the pH to be acidic, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2-(trifluoromethyl)phenyl)piperidin-1-yl)-3-(2,3-dichlorophenyl)-1H-pyrazolo[3,4-d]pyrimidine-4-carboxamide **33** (2 mg) with a yield of 3.5%. MS m/z (ESI): 550.1 [M+1]
¹H NMR (400 MHz, DMSO-*d₆*) δ 13.58 (s, 1H), 8.48 (s, 2H), 8.14 (s, 1H), 7.97 (t, *J=* 8.1 Hz, 2H), 7.85 (t, *J=* 7.7 Hz, 1H), 7.73 (t, *J=* 7.7 Hz, 1H), 7.61-7.68 (m, 2H), 7.40 (d, *J=* 4.3 Hz, 2H), 4.24 (s, 2H), 3.75 (s, 2H), 2.67 (s,2H), 2.22 (s, 2H).

### Example 34

### 6-(4-amino-4-(2-(trifluoromethyl)phenyl)piperidin-1-yl)-3-(2,3-dichlorophenyl)-1H-pyrazolo[3,4-d]pyrimidine-4-carboxylic acid

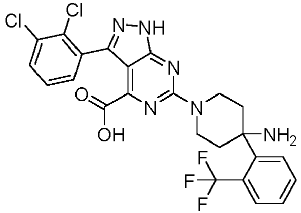

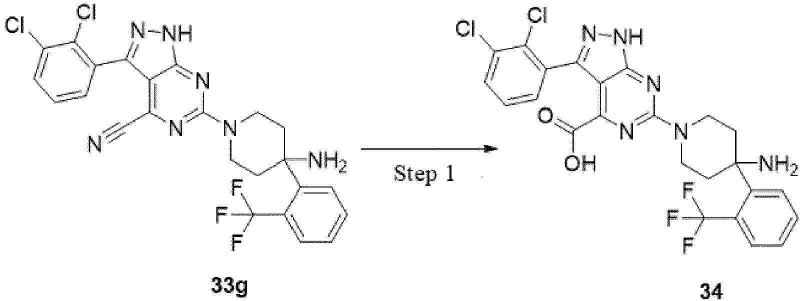

6-(4-amino-4-(2-(trifluoromethyl)phenyl)piperidin-1-yl)-3-(2,3-dichlorophenyl)-1H-pyrazolo[3,4-d]pyrimidine-4-carbonitrile 33g (20 mg, 37.57 µmol) was added to concentrated hydrochloric acid (0.7 mL), sealed, heated to 100°C, and reacted for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2-(trifluoromethyl)phenyl)piperidin-1-yl)-3-(2,3-dichlorophenyl)-1H-pyrazolo[3,4-d]pyrimidine-4-carboxylic acid **34** (3 mg) with a yield of 14.4%.
MS m/z (ESI): 551.1 [M+1]
¹H NMR (400 MHz, DMSO-*d₆*) δ 13.67 (s, 1H), 8.50 (s, 2H), 7.97 (d, *J* = 7.9 Hz, 2H), 7.82 (s, 1H), 7.71 (dd, *J* = 6.3, 3.4 Hz, 3H), 7.45 (q, *J* = 3.4, 2.6 Hz, 2H), 4.15 (s, 2H), 3.73 (s, 2H), 2.67 (s, 2H), 2.20 (s, 2H).

### Example 35

### 6-(4-amino-4-(pyridin-4-ylmethyl)piperidin-1-yl)-3-(2,3-dichlorophenyl)-1H-pyrazolo[3,4-d]pyrimidine-4-carboxamide

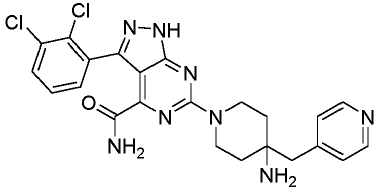

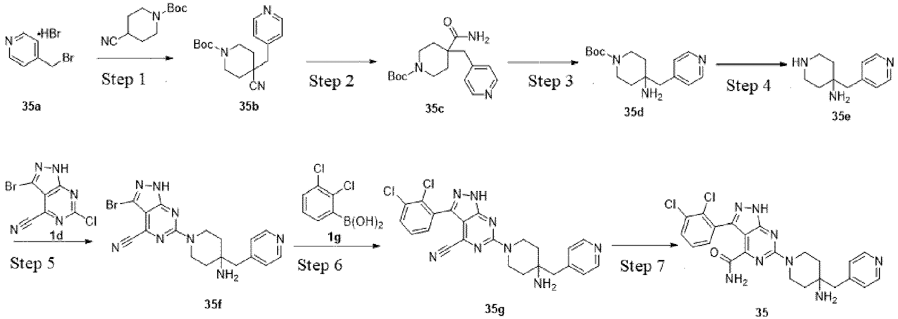

### Step 1

Tert-butyl 4-cyano-4-(pyridin-4-ylmethyl)piperidine-1-carboxylate 4-(bromomethyl)pyridine hydrobromide **35a** (1.06 g, 4.19 mmol),tert-butyl 4-cyanopiperidine-1-carboxylate (800 mg, 3.80 mmol) and N,N-diisopropylethylamine (639.22 mg, 4.95 mmol) were added to toluene (2 mL), stirred at room temperature for 15 minutes, cooled to 0°C, dropwise added with a 1 M tetrahydrofuran solution (834.83 mg, 4.19 mmol) containing potassium bis(trimethylsilyl)amide, then heated to room temperature, and reacted for 16 hours. After the reaction was completed, the reaction solution was added with 20 mL of saturated sodium chloride solution and extracted with ethyl acetate (20 mL×3), and then washed with a saturated sodium chloride solution (20 mL). Organic phases were dried with anhydrous sodium sulfate, filtered, concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, and mobile phase B: CH₃CN) to obtain tert-butyl 4-cyano-4-(pyridin-4-ylmethyl)piperidine-1-carboxylate **35b** (630 mg) with a yield of 54.94%.
MS m/z (ESI): 302.0 [M+1]

### Step 2

### Tert-butyl 4-carbamoyl-4-(pyridin-4-ylmethyl)piperidine-1-carboxylate

Potassium hydroxide (234.58 mg, 4.18 mmol) and tert-butyl 4-cyano-4-(pyridin-4-ylmethyl)piperidine-1-carboxylate **35b** (630 mg, 2.09 mmol) were added to dimethyl sulfoxide (2.3 mL), and hydrogen peroxide (1 mL) was slowly added to the reaction solution in a water bath, then the reaction solution was heated to room temperature, and reacted for 30 minutes. After the reaction was completed, the reaction solution was added with 100 mL of water, and extracted with ethyl acetate (20 mL×2), washed with a saturated sodium chloride solution (20 mL), dried with anhydrous sodium sulfate, and concentrated under reduced pressure to obtain tert-butyl 4-carbamoyl-4-(pyridin-4-ylmethyl)piperidine-1-carboxylate **35c** (667 mg) with a yield of 99.90%, which was directly used for the next reaction without purification.
MS m/z (ESI): 320.0 [M+1]

### Step 3

### Tert-butyl 4-amino-4-(pyridin-4-ylmethyl)piperidine-1-carboxylate

Potassium hydroxide (527.29 mg, 9.40 mmol) and tert-butyl 4-carbamoyl-4-(pyridin-4-ylmethyl)piperidine-1-carboxylate **35c** (667 mg, 2.09 mmol) were added to a mixed solution of acetonitrile (2 mL) and water (2 mL), added with 1,3-dibromo-5,5-dimethylhydantoin (328.40 mg, 1.15 mmol) in a water bath in batches, and stirred at room temperature for 1 hour. After the reaction was completed, the reaction solution was concentrated under reduced pressure, and separated on a C₁₈ reversed phase chromatographic column (C₁₈ separation column 20-45 µm; mobile phase A: H₂O, mobile phase B: CH₃CN) to obtain tert-butyl 4-amino-4-(pyridin-4-ylmethyl)piperidine-1-carboxylate **35d** (500 mg) with a yield of 82.17%.
MS m/z (ESI): 292.2 [M+1]

### Step 4

### 4-(pyridin-4-ylmethyl)piperidin-4-amine

Tert-butyl 4-amino-4-(pyridin-4-ylmethyl)piperidine-1-carboxylate **35d** (200 mg, 686.37 µmol) and 0.5 mL of trifluoroacetic acid were added to 2 mL of dichloromethane, and reacted for 1 hour. After the reaction was completed, the reaction solution was concentrated under reduced pressure to obtain 4-(pyridin-4-ylmethyl)piperidin-4-amine **35e** (131 mg) with a yield of 99.78%, which was directly used for the next reaction without purification.
MS m/z (ESI): 192.1 [M+1]

### Step 5

### 6-(4-amino-4-(pyridin-4-ylmethyl)piperidin-1-yl)-3-bromo-1H-pyrazolo[3,4-d]pyrimidine-4-carbonitrile

3-bromo-6-chloro-1H-pyrazolo[3,4-d]pyrimidine-4-carbonitrile **1d** (147.51 mg, 570.74 µmol) was added to N,N-dimethylacetamide (3 mL), added with N,N-diisopropylethylamine (221.29 mg, 1.71 mmol) and 4-(pyridin-4-ylmethyl)piperidin-4-amine **35e** (131 mg, 684.89 µmol), heated to 80°C, and reacted for 1 hour. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-4-(pyridin-4-ylmethyl)piperidin-1-yl)-3-bromo-1H-pyrazolo[3,4-d]pyrimidine-4-carbonitrile **35f** (120 mg) with a yield of 50.88%.
MS m/z (ESI): 413.1 [M+1]

### Step 6

### 6-(4-amino-4-(pyridin-4-ylmethyl)piperidin-1-yl)-3-(2,3-dichlorophenyl)-1H-pyrazolo[3,4-d]pyrimidine-4-carbonitrile

6-(4-amino-4-(pyridin-4-ylmethyl)piperidin-1-yl)-3-bromo-1H-pyrazolo[3,4-d]pyrimidine-4-carbonitrile **35f** (70.17 mg, 169.79 µmol), (2,3-dichlorophenyl)boronic acid **1g** (129.59 mg, 679.14 µmol), methanesulfonato(2-dicyclohexylphosphino-2',6'-di-isopropoxy-1,1'-biphenyl)(2-amino-1,1'-biphenyl-2-yl)palladium (28.43 mg, 33.96 µmol), 2-dicyclohexylphosphino-2',6'-di-isopropoxy-1,1'-biphenyl (31.69 mg, 67.91 µmol) and potassium phosphate (108.17 mg, 509.36 µmol) were added to a mixed solution of 1,4-dioxane (2 mL) and water (0.2 mL), subjected to argon gas displacement thrice, heated to 100°C, and reacted for 16 hours. After the reaction was completed, the reaction solution was concentrated under reduced pressure and separated on a C¹⁸ reversed phase 97hromatographic column (C₁₈ separation column 20-45 µm; mobile phase A: H₂O, mobile phase B: CH₃CN) to obtain 6-(4-amino-4-(pyridin-4-ylmethyl)piperidin-1-yl)-3-(2,3-dichlorophenyl)-1H-pyrazolo[3,4-d]pyrimidine-4-carbonitrile **35g** (15 mg) with a yield of 18.43%. MS m/z (ESI): 479.1 [M+1]

### Step 7

### 6-(4-amino-4-(pyridin-4-ylmethyl)piperidin-1-yl)-3-(2,3-dichlorophenyl)-1H-pyrazolo[3,4-d]pyrimidine-4-carboxamide

5 M sodium hydroxide solution (0.5 mL) was added to a solution of methanol (1 mL) containing 6-(4-amino-4-(pyridin-4-ylmethyl)piperidin-1-yl)-3-(2,3-dichlorophenyl)-1H-pyrazolo[3,4-d]pyrimidine-4-carbonitrile **35g** (17.04 mg, 35.54 µmol), then added with hydrogen peroxide (0.5 mL), and stirred at room temperature for 1 hour. After the reaction was completed, a trifluoroacetic acid was added to adjust the pH to be acidic, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-4-ylmethyl)piperidin-1-yl)-3-(2,3-dichlorophenyl)-1H-pyrazolo[3,4-d]pyrimidine-4-carboxamide **35** (1 mg) with a yield of 5.66%.
MS m/z (ESI): 497.1 [M+1]
¹H NMR (400 MHz, DMSO-*d₆*) δ 13.57 (s, 1H), 8.63 (d, *J* = 4.9 Hz, 1H), 8.12 (s, 1H), 7.99 (s, 1H), 7.66 (d, *J* = 5.2 Hz, 1H), 7.40 (d, *J* = 4.8 Hz, 2H), 7.21 (s, 1H), 6.66 (s, 1H), 5.32 (s, 2H), 1.92-2.11 (m, 4H), 1.79 (d*, J* = 30.8 Hz, 4H), 1.45 (s, 2H).

### Example 36

### 6-(4-amino-4-(pyridin-3-ylmethyl)piperidin-1-yl)-3-(2,3-dichlorophenyl)-1H-pyrazolo[3,4-d]pyrimidine-4-carboxamide

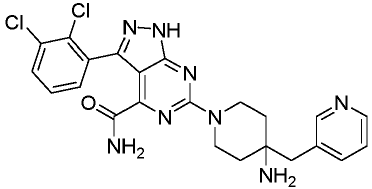

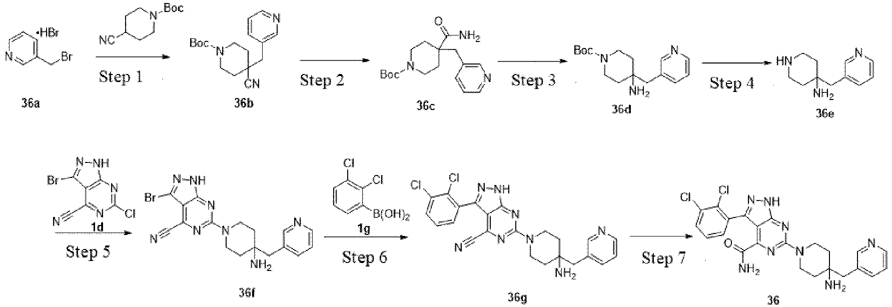

### Step 1

### Tert-butyl 4-cyano-4-(pyridin-3-ylmethyl)piperidine-1-carboxylate

3-(bromomethyl)pyridine hydrobromide **36a** (1.98 g, 7.85 mmol), tert-butyl 4-cyanopiperidine-1-carboxylate (1.5 g, 7.13 mmol) and N,N-diisopropylethylamine (1.20 g, 9.27 mmol) were added to 4 mL of toluene, stirred at room temperature for 15 minutes, cooled to 0°C, dropwise added with a 1 M tetrahydrofuran solution (1.57 g, 7.85 mmol) containing potassium bis(trimethylsilyl)amide, then heated to room temperature, and reacted for 16 hours. After reaction was completed, the reaction solution was added with 20 mL of saturated sodium chloride solution and extracted with ethyl acetate (20 mL×3), and then washed with a saturated sodium chloride solution (20 mL). Organic phases were combined, dried with anhydrous sodium sulfate, filtered, concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, and mobile phase B: CH₃CN) to obtain tert-butyl 4-cyano-4-(pyridin-3-ylmethyl)piperidine-1-carboxylate **36b** (1.18 g) with a yield of 54.89%.
MS m/z (ESI): 302.0 [M+1]

### Step 2

### Tert-butyl 4-carbamoyl-4-(pyridin-3-ylmethyl)piperidine-1-carboxylate

Potassium hydroxide (439.37 mg, 7.83 mmol) and tert-butyl 4-cyano-4-(pyridin-3-ylmethyl)piperidine-1-carboxylate **36b** (1.18 g, 3.92 mmol) were added to dimethyl sulfoxide (6.81 mL), and hydrogen peroxide (2.7 mL) was slowly added to the reaction solution in a water bath, then the reaction solution was heated to room temperature, and reacted for 30 minutes. After reaction was completed, the reaction solution was added with 100 mL of water, and extracted with ethyl acetate (20 mL×2), washed with a saturated sodium chloride solution (20 mL), dried with anhydrous sodium sulfate, and concentrated under reduced pressure to obtain tert-butyl 4-carbamoyl-4-(pyridin-3-ylmethyl)piperidine-1-carboxylate **36c** (1.25 g) with a yield of 100%, which was directly used for the next reaction without purification.
MS m/z (ESI): 320.0 [M+1]

### Step 3

### Tert-butyl 4-amino-4-(pyridin-3-ylmethyl)piperidine-1-carboxylate

Potassium hydroxide (988.17 mg, 17.61 mmol) was added to a mixed solution of acetonitrile (3 mL) and water (3 mL) containing tert-butyl 4-carbamoyl-4-(pyridin-3-ylmethyl)piperidine-1-carboxylate **36c** (1.25 g, 3.91 mmol), added with 1,3-dibromo-5,5-dimethylhydantoin (615.44 mg, 2.15 mmol) in a water bath in batches, and stirred at room temperature for 1 hour. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain tert-butyl 4-amino-4-(pyridin-3-ylmethyl)piperidine-1-carboxylate **36d** (700 mg) with a yield of 61.38%.
MS m/z (ESI): 292.2 [M+1]

### Step 4

### 4-(pyridin-3-ylmethyl)piperidin-4-amine

Tert-butyl 4-amino-4-(pyridin-3-ylmethyl)piperidine-1-carboxylate **36d** (200 mg, 686.37 µmol) and 0.5 mL of trifluoroacetic acid were added to 2 mL of dichloromethane, and reacted for 1 hour. After the reaction was completed, the reaction solution was directly concentrated under reduced pressure to obtain 4-(pyridin-3-ylmethyl)piperidin-4-amine **36e** (131 mg) with a yield of 99.78%, which was directly used for the next reaction without purification.
MS m/z (ESI): 192.1 [M+1]

### Step 5

### 6-(4-amino-4-(pyridine-3-ylmethyl)piperidine-1-yl)-3-bromo-1H-pyrazolo[3,4-d]pyrimidine-4-nitrile

3-bromo-6-chloro-1H-pyrazolo[3,4-d]pyrimidine-4-carbonitrile 1d (147.51 mg, 570.74 µmol) was added to N,N-dimethylacetamide (3 mL), added with N,N-diisopropylethylamine (221.29 mg, 1.71 mmol) and 4-(pyridin-3-ylmethyl)piperidin-4-amine **36e** (131.00 mg, 684.89 µmol),heated to 80°C, and reacted for 1 hour. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-4-(pyridin-3-ylmethyl)piperidin-1-yl)-3-bromo-1H-pyrazolo[3,4-d]pyrimidine-4-carbonitrile **36f** (80 mg) with a yield of 33.92%.
MS m/z (ESI): 413.1 [M+1]

### Step 6

### 6-(4-amino-4-(pyridin-3-ylmethyl)piperidin-1-yl)-3-(2,3-dichlorophenyl)-1H-pyrazolo[3,4-d]pyrimidine-4-carbonitrile

6-(4-amino-4-(pyridin-3-ylmethyl)piperidin-1-yl)-3-bromo-1H-pyrazolo[3,4-d]pyrimidine-4-carbonitrile **36f** (80.19 mg, 194.04 µmol), (2,3-dichlorophenyl)boronic acid **1g** (148.11 mg, 776.16 µmol), methanesulfonato(2-dicyclohexylphosphino-2',6'-di-isopropoxy-1,1'-biphenyl)(2-amino-1,1'-biphenyl-2-yl)palladium (32.50 mg, 38.81 µmol), 2-dicyclohexylphosphino-2',6'-di-isopropoxy-1,1'-biphenyl (36.22 mg, 77.62 µmol) and potassium phosphate (123.62 mg, 582.12 µmol) were added to a mixed solution of 1,4-dioxane (10 mL) and water (1 mL), subjected to argon gas displacement thrice, heated to 100°C, and reacted for 16 hours. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-4-(pyridin-3-ylmethyl)piperidin-1-yl)-3-(2,3-dichlorophenyl)-1H-pyrazolo[3,4-d]pyrimidine-4-carbonitrile **36g** (12 mg) with a yield of 12.90%. MS m/z (ESI): 479.1 [M+1]

### Step 7

### 6-(4-amino-4-(pyridin-3-ylmethyl)piperidin-1-yl)-3-(2,3-dichlorophenyl)-1H-pyrazolo[3,4-d]pyrimidine-4-carboxamide

5 M sodium hydroxide solution (0.5 mL) was added to a solution of methanol (1 mL) containing 6-(4-amino-4-(pyridin-3-ylmethyl)piperidin-1-yl)-3-(2,3-dichlorophenyl)-1H-pyrazolo[3,4-d]pyrimidine-4-carbonitrile **36g** (11.02 mg, 22.99 µmol), then added with hydrogen peroxide (0.5 mL), and stirred at room temperature for 1 hour. After the reaction was completed, a trifluoroacetic acid was added to adjust the pH to be acidic, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-3-ylmethyl)piperidin-1-yl)-3-(2,3-dichlorophenyl)-1H-pyrazolo[3,4-d]pyrimidine-4-carboxamide **36** (1 mg) with a yield of 8.74%. MS m/z (ESI): 497.1 [M+1]

### Example 37

### 6-(4-amino-4-(pyridin-2-ylmethyl)piperidin-1-yl)-3-(2,3-dichlorophenyl)-1H-pyrazolo[3,4-d]pyrimidine-4-carboxamide

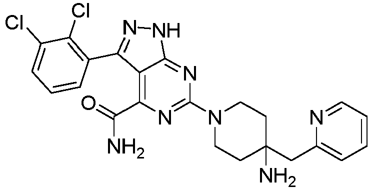

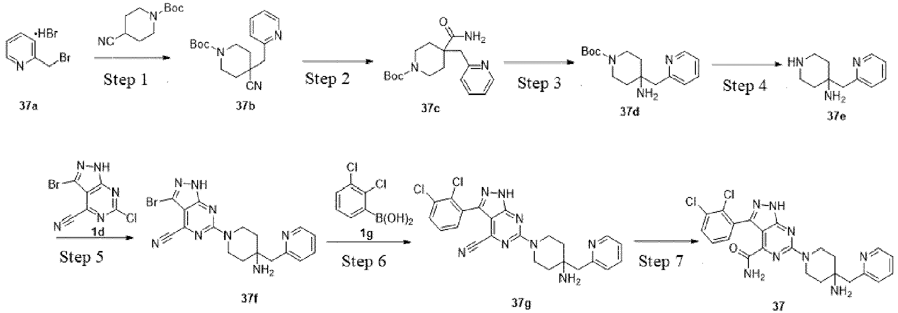

### Step 1

### Tert-butyl 4-cyano-4-(pyridin-2-ylmethyl)piperidine-1-carboxylate

2-(bromomethyl)pyridine hydrobromide **37a** (2.65 g, 10.46 mmol), tert-butyl 4-cyanopiperidine-1-carboxylate (2 g, 9.51 mmol) and N,N-diisopropylethylamine (1.60 g, 12.36 mmol) were added to 2 mL of toluene, stirred at room temperature for 15 minutes, cooled to 0°C, dropwise added with a 1 M tetrahydrofuran solution (2.09 g, 10.46 mmol) containing potassium bis(trimethylsilyl)amide, then heated to room temperature, and reacted for 1 hour. After the reaction was completed, the reaction solution was added with 20 mL of saturated sodium chloride solution and extracted with ethyl acetate (20 mL×3), and then washed with a saturated sodium chloride solution (20 mL). Organic phases were dried with anhydrous sodium sulfate, filtered, concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, and mobile phase B: CH₃CN) to obtain tert-butyl 4-cyano-4-(pyridin-2-ylmethyl)piperidine-1-carboxylate 37b (1.4 g) with a yield of 48.84%.
MS m/z (ESI): 302.0 [M+1]

### Step 2

### Tert-butyl 4-carbamoyl-4-(pyridin-2-ylmethyl)piperidine-1-carboxylate

Potassium hydroxide (703.74 mg, 12.54 mmol) and tert-butyl 4-cyano-4-(pyridin-2-ylmethyl)piperidine-1-carboxylate **37b** (1.89 g, 6.27 mmol) were added to dimethyl sulfoxide (10.9 mL), and hydrogen peroxide (4.3 mL) was slowly added to the reaction solution in a water bath, then the reaction solution was heated to room temperature, and reacted for 30 minutes. After the reaction was completed, the reaction solution was added with 100 mL of water, and extracted with ethyl acetate (20 mL×2), washed with a saturated sodium chloride solution (20 mL), dried with anhydrous sodium sulfate, and concentrated under reduced pressure to obtain tert-butyl 4-carbamoyl-4-(pyridin-2-ylmethyl)piperidine-1-carboxylate **37c** (2 g) with a yield of 99.85%, which was directly used for the next reaction without purification. MS m/z (ESI): 320.0 [M+1]

### Step 3

### Tert-butyl 4-amino-4-(pyridin-2-ylmethyl)piperidine-1-carboxylate

Potassium hydroxide (1.58 g, 28.18 mmol) was added to a mixed solution of acetonitrile (6 mL) and water (6 mL) containing tert-butyl 4-carbamoyl-4-(pyridin-2-ylmethyl)piperidine-1-carboxylate 37c (2 g, 6.26 mmol), added with 1,3-dibromo-5,5-dimethylhydantoin (984.70 mg, 3.44 mmol) in a water bath in batches, and stirred at room temperature for 1 hour. The reaction solution was concentrated under reduced pressure and separated on a C₁₈ reversed phase chromatographic column (C₁₈ separation column 20-45 µm; mobile phase A: H₂O, mobile phase B: CH₃CN) to obtain tert-butyl 4-amino-4-(pyridin-2-ylmethyl)piperidine-1-carboxylate **37d** (1.3 g) with a yield of 71.25%.
MS m/z (ESI): 292.2 [M+1]

### Step 4

### 4-(pyridin-2-ylmethyl)piperidin-4-amine

Tert-butyl 4-amino-4-(pyridin-2-ylmethyl)piperidine-1-carboylate **37d** (219 mg, 751.58 µmol) and 0.5 mL of trifluoroacetic acid were added to 2 mL of dichloromethane, and reacted for 1 hour. After the reaction was completed, the reaction solution was directly concentrated under reduced pressure to obtain 4-(pyridin-2-ylmethyl)piperidin-4-amine **37e** (143.76 mg) with a yield of 100.00%, which was directly used for the next reaction without purification.
MS m/z (ESI): 192.1 [M+1]

### Step 5

### 6-(4-amino-4-(pyridin-2-ylmethyl)piperidin-1-yl)-3-bromo-1H-pyrazolo[3,4-d]pyrimidine-4-carbonitrile

3-bromo-6-chloro-1H-pyrazolo[3,4-d]pyrimidine-4-carbonitrile **1d** (161.03 mg, 623.02 µmol) was added to N,N-dimethylacetamide (3 mL), added with N,N-diisopropylethylamine (241.56 mg, 1.87 mmol) and 4-(pyridin-2-ylmethyl)piperidin-4-amine **37e** (143.0 mg, 747.63 µmol), heated to 80°C, and reacted for 1 hour. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-4-(pyridin-2-ylmethyl)piperidin-1-yl)-3-bromo-1H-pyrazolo[3,4-d]pyrimidine-4-carbonitrile **37f** (200 mg) with a yield of 77.68%.
MS m/z (ESI): 413.1 [M+1]

### Step 6

### 6-(4-amino-4-(pyridin-2-ylmethyl)piperidin-1-yl)-3-(2,3-dichlorophenyl)-1H-pyrazolo[3,4-d]pyrimidine-4-carbonitrile

6-(4-amino-4-(pyridin-2-ylmethyl)piperidin-1-yl)-3-bromo-1H-pyrazolo[3,4-d]pyrimidine-4-carbonitrile **37f** (200.48 mg, 485.10 µmol), (2,3-dichlorophenyl)boronic acid **1g** (370.27 mg, 1.94 mmol), methanesulfonato(2-dicyclohexylphosphino-2',6'-di-isopropoxy-1,1'-biphenyl)(2-amino-1,1'-biphenyl-2-yl)palladium (81.24 mg, 97.02 µmol), 2-dicyclohexylphosphino-2',6'-di-isopropoxy-1,1'-biphenyl (90.54 mg, 194.04 µmol) and potassium phosphate (309.06 mg, 1.46 mmol) were added to a mixed solution of 1,4-dioxane (10 mL) and water (1 mL), subjected to argon gas displacement thrice, heated to 100°C, and reacted for 16 hours. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-4-(pyridin-2-ylmethyl)piperidin-1-yl)-3-(2,3-dichlorophenyl)-1H-pyrazolo[3,4-d]pyrimidine-4-carbonitrile **37g** (30 mg) with a yield of 12.90%. MS m/z (ESI): 479.1 [M+1]

### Step 7

### 6-(4-amino-4-(pyridin-2-ylmethyl)piperidin-1-yl)-3-(2,3-dichlorophenyl)-1H-pyrazolo[3,4-d]pyrimidine-4-carboxamide

5 M sodium hydroxide solution (0.5 mL) was added to a solution of methanol (1 mL) containing 6-(4-amino-4-(pyridin-2-ylmethyl)piperidin-1-yl)-3-(2,3-dichlorophenyl)-1H-pyrazolo[3,4-d]pyrimidine-4-carbonitrile **37g** (30 mg, 62.58 µmol), then added with hydrogen peroxide (0.5 mL), and stirred at room temperature for 1 hour. After the reaction was completed, a trifluoroacetic acid was added to adjust the pH to be acidic, and then the reaction solution was concentrated under reduced pressure, and subjected to liquid phase separation (separation column AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-2-ylmethyl)piperidin-1-yl)-3-(2,3-dichlorophenyl)-1H-pyrazolo[3,4-d]pyrimidine-4-carboxamide 37 (1 mg) with a yield of 3.21%. MS m/z (ESI): 497.1 [M+1]

### Example 38

### 6-(4-amino-4-(pyridin-2-yl)piperidin-1-yl)-3-(2,3-dichlorophenyl)-1H-pyrazolo[3,4-d]pyrimidine-4-carboxamide

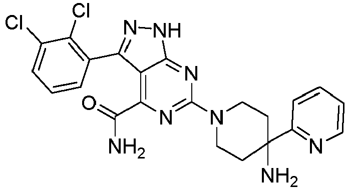

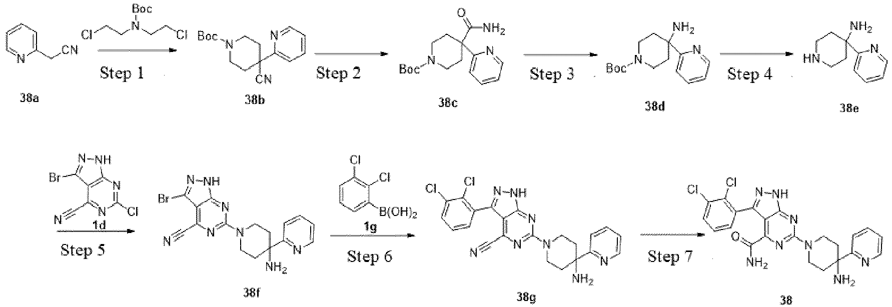

### Step 1

### Tert-butyl 4-cyano-4-(pyridine-2-yl)piperidine-1-carboxylate

Under argon gas, sodium hydride (1.39 g, 53.45 mmol) was added to a solution of N,N-dimethylformamide (30 mL) containing 2-(pyridine-2-yl)acetonitrile **38a** (1.80 g, 15.27 mmol), cooled to 0°C, added with tert-butyl bis(2-chloroethyl)carbamate (4.07 g, 16.80 mmol) in batches, continuously stirred for 1 hour, heated to 60°C, and reacted for 16 hours. After the reaction was completed, the reaction solution was cooled to room temperature, added with 40 mL of saturated aqueous ammonium chloride solution, extracted with ethyl acetate (50 mL×3), and washed with a saturated sodium chloride solution (30 mL). Organic phases were dried with anhydrous sodium sulfate, filtered, and concentrated under reduced pressure. The obtained residue was further analyzed and purified by silicagel column chromatography (eluent: system A) to obtain tert-butyl 4-cyano-4-(pyridin-2-yl)piperidine-1-carboxylate **38b** (3.27 g) with a yield of 74.51%.
MS m/z (ESI): 231.9 [M-55]

### Step 2

### Tert-butyl 4-carbamoyl-4-(pyridin-2-yl)piperidine-1-carboxylate

Tert-butyl 4-cyano-4-(pyridin-2-yl)piperidine-1-carboxylate **38b** (3.27 g, 11.38 mmol) and potassium hydroxide (1.28 g, 22.76 mmol) were added to dimethyl sulfoxide (8.12 mL), and hydrogen peroxide (8.12 mL) was slowly added to the reaction solution, then the reaction solution was heated to room temperature, and reacted for 30 minutes. After the reaction was completed, the reaction solution was added with 100 mL of water to precipitate a large amount of solids and filterd to obtain tert-butyl 4-carbamoyl-4-(pyridine-2-yl)piperidine-1-carboxylate **38c** (1.3 g) with a yield of 37.41%.
MS m/z (ESI): 306.2 [M+1]

### Step 3

### Tert-butyl 4-amino-4-(pyridin-2-yl)piperidine-1-carboxylate

Potassium hydroxide (1.07 g, 19.16 mmol) was added to a mixed solution of acetonitrile (3.4 mL) and water (13.7 mL) containing tert-butyl 4-carbamoyl-4-(pyridin-2-yl)piperidine-1-carboxylate **38c** (1.3 g, 4.26 mmol), added with 1,3-dibromo-5,5-dimethylhydantoin (669.46 mg, 2.34 mmol) in a water bath in batches, and stirred at room temperature for 16 hours. After the reaction was completed, the reaction solution was added with sodium sulphite and stirred at room temperature for 15 minutes, then added with 50 mL of ethyl acetate and potassium phosphate, and then concentrated under reduced pressure. The obtained residue was further analyzed and purified by silica gel column chromatography (eluent: system B) to obtain tert-butyl 4-amino-4-(pyridin-2-yl)piperidine-1-carboxylate **38d** (1.18 g) with a yield of 100%, which was directly used for the next reaction without purification.
MS m/z (ESI): 278.1 [M+1]

### Step 4

### 4-(pyridin-2-yl)piperidine-4-amine

Tert-butyl 4-amino-4-(pyridin-2-yl)piperidine-1-carboxylate **38d** (180 mg, 648.97 µmol) was added to dichloromethane (4 mL), and then added with trifluoroacetic acid (1 mL) and reacted for 1 hour. After the reaction was completed, the reaction solution was concentrated under reduced pressure to obtain 4-(pyridin-2-yl)piperidin-4-amine **38e** (115.03 mg) with a yield of 100%, which was directly used for the next reaction without purification.
MS m/z (ESI): 178.1 [M+1]

### Step 5

### 6-(4-amino-4-(pyridin-2-yl)piperidin-1-yl)-3-bromo-1H-pyrazolo[3,4-d]pyrimidine-4-carbonitrile

3-bromo-6-chloro-1H-pyrazolo[3,4-d]pyrimidine-4-carbonitrile **1d** (130 mg, 502.97 µmol) was added to N-methyl pyrrolidone (5 mL), then added with N,N-diisopropylethylamine (195.01 mg, 1.51 mmol) and 4-(pyridin-2-yl)piperidin-4-amine **38e** (89.15 mg, 502.97 µmol), heated to 110°C, and reacted for 1 hour. After the reaction was completed, the reaction solution was concentrated under reduced pressure. The obtained residue was further analyzed and purified by silica gel column chromatography (eluent: system B) to obtain 6-(4-amino-4-(pyridin-2-yl)piperidin-1-yl)-3-bromo-1H-pyrazolo[3,4-d]pyrimidine-4-carbonitrile **38f** (150 mg) with a yield of 74.70%.
MS m/z (ESI): 398.9 [M+1]

### Step 6

### 6-(4-amino-4-(pyridin-2-yl)piperidin-1-yl)-3-(2,3-dichlorophenyl)-1H-pyrazolo[3,4-d]pyrimidine-4-carbonitrile

6-(4-amino-4-(pyridin-2-yl)piperidin-1-yl)-3-bromo-1H-pyrazolo[3,4-d]pyrimidine-4-carbonitrile **38f** (150 mg, 375.71 µmol), (2,3-dichlorophenyl)boronic acid **1g** (286.77 mg, 1.50 mmol), methanesulfonato(2-dicyclohexylphosphino-2',6'-di-isopropoxy-1,1'-biphenyl)(2-amino-1,1'-biphenyl-2-yl)palladium (62.92 mg, 75.14 µmol), 2-dicyclohexylphosphino-2',6'-di-isopropoxy-1,1'-biphenyl (70.13 mg, 150.28 µmol) and potassium phosphate (239.37 mg, 1.13 mmol) were added to a mixed solution of 1,4-dioxane (10 mL) and water (1 mL), subjected to argon gas displacement thrice, heated to 100°C, and reacted for 16 hours. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-2-yl)piperidin-1-yl)-3-(2,3-dichlorophenyl)-1H-pyrazolo[3,4-d]pyrimidine-4-carbonitrile **38g** (26 mg) with a yield of 14.87%.
MS m/z (ESI): 464.9 [M+1]

### Step 7

### 6-(4-amino-4-(pyridin-2-yl)piperidin-1-yl)-3-(2,3-dichlorophenyl)-1H-pyrazolo[3,4-d]pyrimidine-4-carboxamide

6-(4-amino-4-(pyridin-2-yl)piperidin-1-yl)-3-(2,3-dichlorophenyl)-1H-pyrazolo[3,4-d]pyrimidine-4-carbonitrile **38g** (26.27 mg, 55.87 µmol), sodium hydroxide (0.5 mL) and hydrogen peroxide (0.5 mL) were added to a mixed solution of methanol (1 mL) in turn, and stirred at room temperature for 1 hour. After the reaction was completed, a trifluoroacetic acid was added to adjust the pH to be acidic, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-2-yl)piperidin-1-yl)-3-(2,3-dichlorophenyl)-1H-pyrazolo[3,4-d]pyrimidine-4-carboxamide **38** (5 mg) with a yield of 18.51%.
MS m/z (ESI): 482.9 [M+1]
¹H NMR (400 MHz, CD₃OD) δ 8.75 (d, *J* = 4.8 Hz, 1H), 7.97 (t, *J=* 7.8 Hz, 1H), 7.75 (d, *J* = 8.1 Hz, 1H), 7.61 (dd, *J* = 7.4, 1.9 Hz, 1H), 7.48 (dd, *J* = 7.6, 4.9 Hz, 1H), 7.34-7.44 (m, 2H), 4.21 (d, *J* = 25.2 Hz, 4H), 2.58-2.69 (m, 2H), 2.17 (dd, *J* = 13.9, 7.4 Hz, 2H).

### Example 39

### (S)-6-(5-amino-5,7-dihydrospiro[cyclopenta[b]pyridine-6,4'-piperidin]-1'-yl)-3-((2,3-dichlorophenyl)thio)-1H-pyrazolo[3,4-d]pyrimidine-4-carboxamide

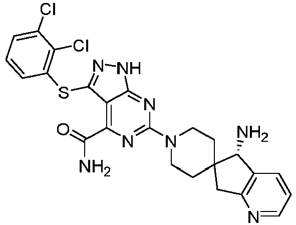

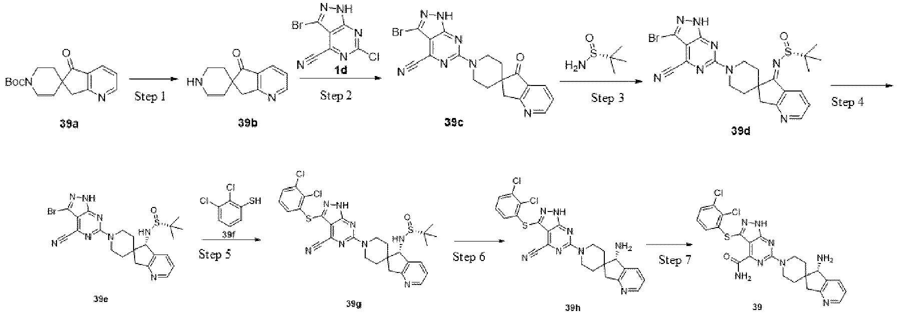

### Step 1

### spiro[cyclopenta[b]pyridine-6,4'-piperidin]-5(7H)-one

tert-butyl 5-oxo-5,7-dihydrospiro[cyclopenta[b]pyridine 6,4'-piperidine]-1'-carboxylate **39a** (600 mg, 1.98 mmol) was added to dichloromethane (4 mL), and continuously reacted for 1 hour. After the reaction was completed, the reaction solution was concentrated under reduced pressure to obtain spiro[cyclopenta[b]pyridine-6,4'-piperidin]-5(7H)-one **39b** (401 mg) with a yield of 99.92%, which was directly used for the next reaction without purification.
MS m/z (ESI): 202.9 [M+1]

### Step 2

### 3-bromo-6-(5-oxo-5,7-dihydrospiro[cyclopenta[b]pyridine-6,4'-piperidin]-1'-yl)-1H-pyrazolo[3,4-d]pyrimidine-4-carbonitrile

3-bromo-6-chloro-1H-pyrazolo[3,4-d]pyrimidine-4-carbonitrile **1d** (300mg, 1.16 mmol), N,N-diisopropylethylamine (750.05 mg, 5.80 mmol) and spiro[cyclopenta[b]pyridine-6,4'-piperidin]-5(7H)-one **39b** (399.08 mg, 1.97 mmol) were added to N,N-dimethylacetamide (2 mL), heated to 90°C, and reacted for 1 hour. After the reaction was completed, the reaction solution was concentrated under reduced pressure. The obtained residue was further analyzed and purified by silica gel column chromatography (eluent: system A) to obtain 3-bromo-6-(5-oxo-5,7-dihydrospiro[cyclopenta[b]pyridine-6,4'-piperidin]-1'-yl)-1H-pyrazolo[3,4-d]pyrimidine-4-carbonitrile **39c** (350 mg) with a yield of 71.08%.
MS m/z (ESI): 424.0 [M+1]

### Step 3

### (R,Z)-N-(1'-(3-bromo-4-cyano-1H-pyrazolo[3,4-d]pyrimidin-6-yl)spiro[cyclopenta[b]pyridine-6,4'-piperidin]-5(7H)-ylidene)-2-methylpropane-2-sulfinamide

3-bromo-6-(5-oxo-5,7-dihydrospiro[cyclopenta[b]pyridine-6,4'-piperidin]-1'-yl)-1H-pyrazolo[3,4-d]pyrimidine-4-carbonitrile **39c** (369 mg, 869.76 µmol) and (R)-2-methylpropane-2-sulfinamide (316.25 mg, 2.61 mmol) were added to tetrahydrofuran (2 mL), and reacted at 100°C for 16 hours. After the reaction was completed, the reaction solution was added with water and extracted with ethyl acetate (30 mL×2) to separate an aqueous layer. Combined organic phases were washed with a saturated sodium chloride solution (30 mL×2) in turn, dried with anhydrous sodium sulfate, filtered, and concentrated under reduced pressure to obtain (R,Z)-N-(1'-(3-bromo-4-cyano-1H-pyrazolo[3,4-d]pyrimidin-6-yl)spiro[cyclopenta[b]pyridine-6,4'-piperidin]-5(7H)-ylidene)-2-methylpropane-2-sulfinamide **39d** (458 mg) with a yield of 99.84%, which was directly used for the next reaction without purification.
MS m/z (ESI): 527.1 [M+1]

### Step 4

(R)-N-((S)-1-(3-bromo-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide 9-borabicyclo[3.3.1]nonane (0.5 M, 5.21 mL) was added to a solution of tetrahydrofuran (5 mL) containing (R,Z)-N-(1'-(3-bromo-4-cyano-1H-pyrazolo[3,4-d]pyrimidin-6-yl)spiro[cyclopenta[b]pyridine-6,4'-piperidin]-5(7H)-ylidene)-2-methylpropane-2-sulfinamide **39d** (458 mg, 868.35 µmol), and stirred at room temperature for 2 hours. After the reaction was completed, the reaction solution was added with 50 mL of water, extracted with ethyl acetate (50 mL×3), and washed with a saturated sodium chloride solution (50 mL). Organic phases were dried with anhydrous sodium sulfate, filtered, and concentrated under reduced pressure. The obtained residue was further analyzed and purified by silica gel column chromatography (eluent: system A) to obtain (R)-N-((S)-1'-(3-bromo-4-cyano-1H-pyrazolo[3,4-d]pyrimidi-6-yl)-5,7-dihydrospiro[cyclopenta[b]pyridine-6,4'-piperidin]-5-yl)-2-methylpropane-2-sulfinamide **39e** (300 mg) with a yield of 65.25%.
MS m/z (ESI): 529.2 [M+1]

### Step 5

(R)-N-((S)-1'-(3-((2,3-dichlorophenyl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide (R)-N-((S)-1-(3-bromo-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **39e** (150 mg, 283.31 µmol), 2,3-dichlorobenzenethiol **39f** (76.10 mg, 424.97 µmol), tris(dibenzylideneacetone)dipalladium (15.57 mg, 17.00 µmol), 4,5-bis(diphenylphosphino)-9,9-dimethylxanthene (16.39 mg, 28.33 µmol) and N,N-diisopropylethylamine (73.23 mg, 566.62 µmol) were added to 1,4-dioxane (4 mL), subjected to nitrogen gas displacement, heated to 100°C, and reacted for 3 hours. After the reaction was completed, the reaction solution was cooled to room temperature, and concentrated under reduced pressure. The obtained residue was further analyzed and purified by silica gel column chromatography (eluent: system B) to obtain (R)-N-((S)-1'-(3-((2,3-dichlorophenyl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **39g** (125 mg) with a yield of 70.30%.
MS m/z (ESI): 627.1 [M+1]

### Step 6

### (S)-6-(5-amino-5,7-dihydrospiro[cyclopenta[b]pyridine-6,4'-piperidin]-1'-yl)-3-((2,3-dichlorophenyl)thio)-1H-pyrazolo[3,4-d]pyrimidine-4-carbonitrile

A solution of dichloromethane (4 mL) containing (R)-N-((S)-1'-(3-((2,3-dichlorophenyl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **39g** (125 mg, 199.17 µmol) was slowly dropwise added to hydrochloric acid in methanol (4 M, 199.17 µL), and reacted for 1 hour. After the reaction was completed, the reaction solution was concentrated under reduced pressure to obtain (S)-6-(5-amino-5,7-dihydrospiro[cyclopenta[b]pyridine-6,4'-piperidin]-1'-yl)-3-((2,3-dichlorophenyl)thio)-1H-pyrazolo[3,4-d]pyrimidine-4-carbonitrile **39h** (104 mg) with a yield of 99.76%, which was directly used for the next reaction without purification. MS m/z (ESI): 522.8 [M+1]

### Step 7

### (S)-6-(5-amino-5,7-dihydrospiro[cyclopenta[b]pyridine-6,4'-piperidin]-1'-yl)-3-((2,3-dichlorophenyl)thio)-1H-pyrazolo[3,4-d]pyrimidine-4-carboxamide

5 M sodium hydroxide solution (0.25 mL) was added to a solution of methanol (2 mL) containing (S)-6-(5-amino-5,7-dihydrospiro[cyclopenta[b]pyridine-6,4'-piperidin]-1'-yl)-3-((2,3-dichlorophenyl)thio)-1H-pyrazolo[3,4-d]pyrimidine-4-carbonitrile **39h** (104 mg, 198.69 µmol), then added with hydrogen peroxide (0.5 mL), and stirred at room temperature for 30 minutes. After the reaction was completed, a trifluoroacetic acid was added to adjust the pH to be acidic, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain (S)-6-(5-amino-5,7-dihydrospiro[cyclopenta[b]pyridine-6,4'-piperidin]-1'-yl)-3-((2,3-dichlorophenyl)thio)-1H-pyrazolo[3,4-d]pyrimidine-4-carboxamide **39** (3 mg) with a yield of 2.13%.
MS m/z (ESI): 541.1 [M+1]
¹H NMR (400 MHz, DMSO-*d*₆) δ 13.34 (s, 1H), 8.54 (d, *J* = 5.0 Hz, 1H), 8.30 (s, 2H), 8.24 (s, 1H), 7.91 (d, *J* = 5.0 Hz, 1H), 7.88 (s, 1H), 7.63 (dd, *J* = 8.0, 1.5 Hz, 1H), 7.44 (d, *J* = 7.6 Hz, 1H), 7.35 (dd, *J* = 7.7, 4.9 Hz, 2H), 4.46 (d, *J* = 5.5 Hz, 1H), 3.27 (d, *J* = 16.6 Hz, 2H), 3.07-3.21 (m, 2H), 1.73 (d, *J* = 14.2 Hz, 2H), 1.54 (d, *J* = 13.2 Hz, 2H).

### Example 40

### (S)-6-(5-amino-5,7-dihydrospiro[cyclopenta[b]pyridine-6,4'-piperidin]-1'-yl)-3-(2-(trifluoromethyl)pyridin-3-yl)-1H-pyrazolo[3,4-d]pyrimidine-4-carboxamide

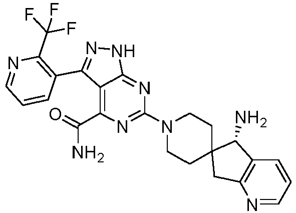

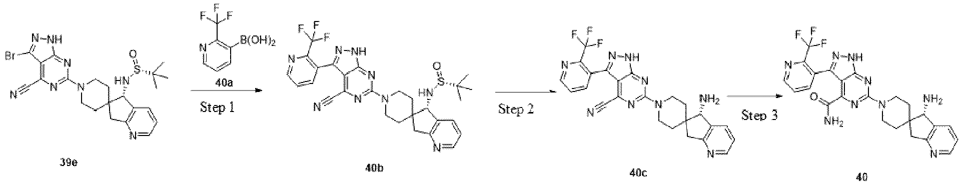

### Step 1

(R)-N-((S)-1'-(3-(2-(trifluoromethyl)pyridin-3-yl)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide (R)-N-((S)-1-(3-bromo-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **39e** (150 mg, 283.31 µmol), (2-(trifluoromethyl)pyridin-3-yl)boronic acid **40a** (216.35 mg, 1.13 mmol), methanesulfonato(2-dicyclohexylphosphino-2',6'-di-isopropoxy-1,1'-biphenyl)(2-amino-1,1'-biphenyl-2-yl)palladium (47.45 mg, 56.66 µmol), 2-dicyclohexylphosphino-2',6'-di-isopropoxy-1,1'-biphenyl (52.88 mg, 113.32 µmol) and potassium phosphate (180.41 mg, 849.93 µmol) were added to 1,4-dioxane (3 mL), subjected to nitrogen gas displacement, heated to 100°C, and reacted for 7 hours. After the reaction was completed, the reaction solution was cooled to room temperature. The obtained residue was further analyzed and purified by silicagel column chromatography (eluent: system B) to obtain (R)-N-((S)-1'-(3-(2-(trifluoromethyl)pyridin-3-yl)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **40b** (80 mg) with a yield of 47.41%.
MS m/z (ESI): 596.2 [M+1]

### Step 2

### (S)-6-(5-amino-5,7-dihydrospiro[cyclopenta[b]pyridine-6,4'-piperidin]-1'-yl)-3-(2-(trifluoromethyl)pyridin-3-yl)-1H-pyrazolo[3,4-d]pyrimidine-4-carbonitrile

A solution of dichloromethane (4 mL) containing (R)-N-((S)-1'-(3-(2-(trifluoromethyl)pyridin-3-yl)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **40b** (80 mg, 134.31 µmol) was slowly dropwise added to hydrochloric acid in methanol (4 M, 134.31 µL), and reacted for 1 hour. After the reaction was completed, the reaction solution was concentrated under reduced pressure to obtain (S)-6-(5-amino-5,7-dihydrospiro[cyclopenta[b]pyridine-6,4'-piperidin]-1'-yl)-3-(2-(trifluoromethyl)pyridin-3-yl)-1H-pyrazolo[3,4-d]pyrimidine-4-carbonitrile **40c** (66 mg) with a yield of 99.99%, which was directly used for the next reaction without purification.
MS m/z (ESI): 492.2 [M+1]

### Step 3

### (S)-6-(5-amino-5,7-dihydrospiro[cyclopenta[b]pyridine-6,4'-piperidin]-1'-yl)-3-(2-(trifluoromethyl)pyridin-3-yl)-1H-pyrazolo[3,4-d]pyrimidine-4-carboxamide

5 M sodium hydroxide solution (1.31 mL) was added to a solution of methanol (952.38 µL) containing (S)-6-(5-amino-5,7-dihydrospiro[cyclopenta[b]pyridine-6,4'-piperidin]-1'-yl)-3-(2-(trifluoromethyl)pyridine-3-yl)-1H-pyrazolo[3,4-d]pyrimidine-4-carbonitrile **40c** (66 mg, 134.29 µmol), then added with hydrogen peroxide (238.10 µL), and stirred at room temperature for 30 minutes. After the reaction was completed, a trifluoroacetic acid was added to adjust the pH to be acidic, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain (S)-6-(5-amino-5,7-dihydrospiro[cyclopenta[b]pyridine-6,4'-piperidin]-1'-yl)-3-(2-(trifluoromethyl)pyridin-3-yl)-1H-pyrazolo[3,4-d]pyrimidine-4-carboxamide **40** (10 mg) with a yield of 14.6%.
MS m/z (ESI): 510.2 [M+1]
¹H NMR (400 MHz, DMSO-*d*₆) δ 8.76 (d, *J* = 4.6 Hz, 1H), 8.55 (d, *J* = 4.9 Hz, 1H), 8.28-8.43 (m, 3H), 8.13 (s, 1H), 7.91 (t, *J* = 7.9 Hz, 2H), 7.73 (dd, *J=* 7.8, 4.8 Hz, 1H), 7.58 (s, 1H), 7.36 (dd, *J* = 7.6, 5.1 Hz, 1H), 4.74 (s, 2H), 4.47 (d, *J* = 5.4 Hz, 1H), 3.26-3.34 (m, 3H), 3.16 (d, *J* = 16.9 Hz, 1H), 1.76 (d, *J* = 13.8 Hz, 2H), 1.57 (d, *J* = 13.3 Hz, 2H).

### Example 41

### (S)-6-(5-amino-5,7-dihydrospiro[cyclopenta[b]pyridine-6,4'-piperidin]-1'-yl)-3-((3-chloro-2-cyclopropoxypyridin-4-yl)thio)-1H-pyrazolo[3,4-d]pyrimidine-4-carboxamide

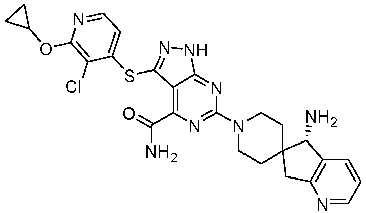

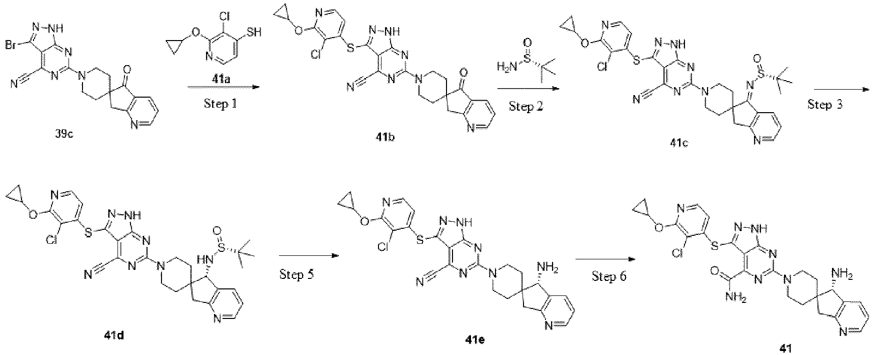

### Step 1

3-((3-chloro-2-cyclopropoxypyridin-4-yl)thio)-6-(5-oxo-5,7-dihydrospiro[cyclopenta[b]pyridine-6,4'-piperidin]-1'-yl)-1H-pyrazolo[3,4-d]pyrimidine-4-carbonitrile 3-bromo-6-(5-oxo-5,7-dihydrospiro[cyclopenta[b]pyridine-6,4'-piperidin]-1'-yl)-1H-pyrazolo[3,4-d]pyrimidine-4-carbonitrile **39c** (150 mg, 353.56 µmol), 3-chloro-2-(cyclopropoxy)pyridine-4-thiol **41a** (142.61 mg, 707.12 µmol, self-prepared according to patent WO2018013597), cuprous iodide (33.67 mg, 176.78 µmol), copper (22.47 mg, 353.56 µmol) and potassium carbonate (146.60 mg, 1.06 mmol) were added to N,N-dimethylformamide (3 mL), subjected to nitrogen gas displacement, heated to 130°C, and reacted for 17 hours. After the reaction was completed, the reaction solution was cooled to room temperature, and concentrated under reduced pressure. The obtained residue was further analyzed and purified by silica gel column chromatography (eluent: system A) to obtain 3-((3-chloro-2-cyclopropoxypyridin-4-yl)thio)-6-(5-oxo-5,7-dihydrospiro[cyclopenta[b]pyridine-6,4'-piperidin]-1'-yl)-1H-pyrazolo[3,4-d]pyrimidine-4-carbonitrile **41b** (60 mg) with a yield of 31.14%.
MS m/z (ESI): 545.2 [M+1]

### Step 2

### (R,Z)-N-(1'-(3-((3-chloro-2-cyclopropoxypyridin-4-yl)thio)-4-cyano-1H-pyrazolo[3,4-d]pyrimidin-6-yl)spiro[cyclopenta[b]pyridine-6,4'-piperidin]-5(7H)-ylidene)-2-methylpropane-2-sulfinamide

3-((3-chloro-2-cyclopropoxypyridin-4-yl)thio)-6-(5-oxo-5,7-dihydrospiro[cyclopenta[b]pyridine-6,4'-piperidin]-1'-yl)-1H-pyrazolo[3,4-d]pyrimidine-4-carbonitrile **41b** (60 mg, 110.09 µmol) and (R)-2-methylpropane-2-sulfinamide (40.03 mg, 330.27 µmol) were added to tetraethyl titanate (2.00 mL), heated to 100°C, and reacted for 3 hours. After the reaction was completed, the reaction solution was added with 20 mL of water and extracted with ethyl acetate (30 mL×2). The aqueous layer was separated. Combined organic phases were washed with a saturated sodium chloride solution (30 mL×2) in turn, dried with anhydrous sodium sulfate, filtered, and concentrated under reduced pressure to obtain (R,Z)-N-(1'-(3-((3-chloro-2-cyclopropoxypyridin-4-yl)thio)-4-cyano-1H-pyrazolo[3,4-d]pyrimidin-6-yl)spiro[cyclopenta[b]pyridine-6,4'-piperidin]-5(7H)-ylidene)-2-methylpropane-2-sulfinamide **41c** (71 mg) with a yield of 99.50%, which was directly used for the next reaction without purification.
MS m/z (ESI): 648.2 [M+1]

### Step 3

### (R)-N-((S)-1'-(3-((3-chloro-2-cyclopropoxypyridin-4-yl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide

9-borabicyclo[3.3.1]nonane (0.5 M, 657.20 µL) was added to a solution of tetrahydrofuran (1 mL) containing (R,Z)-N-(1'-(3-((3-chloro-2-cyclopropoxypyridin-4-yl)thio)-4-cyano-1H-pyrazolo[3,4-d]pyrimidin-6-yl)spiro[cyclopenta[b]pyridine-6,4'-piperidine]-5(7H)-ylidene)-2-methylpropane-2-sulfinamide **41c** (71 mg, 109.53 µmol), and stirred at room temperature for 2 hours. After the reaction was completed, the reaction solution was added with 50 mL of water, extracted with ethyl acetate (50 mL×3), and washed with a saturated sodium chloride solution (50 mL). Organic phases were dried with anhydrous sodium sulfate, filtered, and concentrated under reduced pressure. The obtained residue was further analyzed and purified by silica gel column chromatography (eluent: system A) to obtain (R)-N-((S)-1'-(3-((3-chloro-2-cyclopropoxypyridin-4-yl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **41d** (40 mg) with a yield of 56.16%.
MS m/z (ESI): 650.3 [M+1]

### Step 4

### (S)-6-(5-amino-5,7-dihydrospiro[cyclopenta[b]pyridine-6,4'-piperidin]-1'-yl)-3-((3-chloro-2-cyclopropoxypyridin-4-yl)thio)-1H-pyrazolo[3,4-d]pyrimidine-4-carbonitrile

A solution of dichloromethane (3.94 mL) containing (R)-N-((S)-1'-(3-((3-chloro-2-cyclopropoxypyridin-4-yl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **41d** (40 mg, 61.52 µmol)) was slowly dropwise added to hydrochloric acid in methanol (4 M, 61.52 µL), and reacted for 1 hour. After the reaction was completed, the reaction solution was concentrated under reduced pressure to obtain (S)-6-(5-amino-5,7-dihydrospiro[cyclopenta[b]pyridine-6,4'-piperidin]-1'-yl)-3-((3-chloro-2-cyclopropoxypyridin-4-yl)thio)-1H-pyrazolo[3,4-d]pyrimidine-4-carbonitrile **41e** (33 mg) with a yield of 98.24%, which was directly used for the next reaction without purification.
MS m/z (ESI): 546.2 [M+1]

### Step 5

(S)-6-(5-amino-5,7-dihydrospiro[cyclopenta[b]pyridine-6,4'-piperidin]-1'-yl)-3-((3-chloro-2-cyclopropoxypyridin-4-yl)thio)-1H-pyrazolo[3,4-d]pyrimidine-4-carboxamide Sodium hydroxide (0.15 mL) was added to a solution of methanol (1.5 mL) containing (S)-6-(5-amino-5,7-dihydrospiro[cyclopenta[b]pyridine-6,4'-piperidin]-1'-yl)-3-((3-chloro-2-cyclopropoxypyridin-4-yl)thio)-1H-pyrazolo[3,4-d]pyrimidine-4-carbonitrile **41e** (33 mg, 60.43 µmol), then added with hydrogen peroxide (0.3 mL), and stirred at room temperature for 30 minutes. After the reaction was completed, a trifluoroacetic acid was added to adjust the pH to be acidic, and then the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH3CN) to obtain (S)-6-(5-amino-5,7-dihydrospiro[cyclopenta[b]pyridine-6,4'-piperidin]-1'-yl)-3-((3-chloro-2-cyclopropoxypyridin-4-yl)thio)-1H-pyrazolo[3,4-d]pyrimidine-4-carboxamide **41** (11 mg) with a yield of 32.2%.
MS m/z (ESI): 563.9 [M+1]
¹H NMR (400 MHz, DMSO-*d*₆) δ 8.54 (d, *J* = 5.0 Hz, 1H), 8.29 (s, 2H), 8.12 (s, 1H), 7.86-7.97 (m, 2H), 7.81 (s, 1H), 7.35 (t, *J* = 6.3 Hz, 1H), 6.55 (d, *J* = 5.4 Hz, 1H), 4.46 (s, 1H), 4.32 (s, 1H), 3.31 (d, *J* = 13.4 Hz, 2H), 3.25 (s, 1H), 3.14 (d, *J* = 16.9 Hz, 2H), 2.67 (s, 1H), 2.33 (s, 1H), 1.73 (d, *J* = 14.2 Hz, 2H), 1.55 (d, *J* = 12.9 Hz, 2H), 0.67-0.84 (m, 4H).

### Example 42

### Ethyl (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxylate

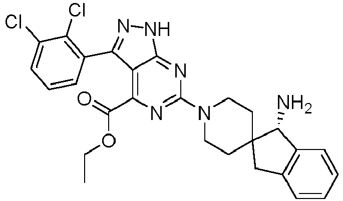

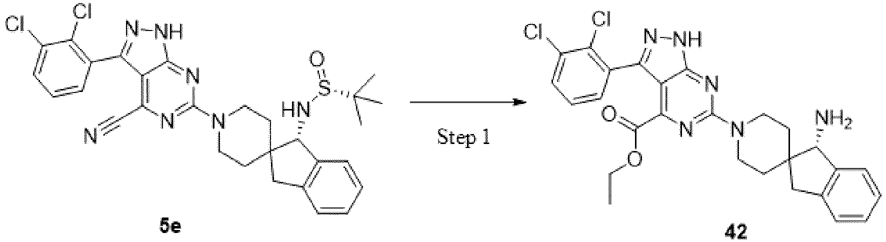

### Step 1

### Ethyl (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxylate

(R)-N-((S)-1'-(4-cyano-3-(2,3-dichlorophenyl)-1H-pyrazolo[3,4-d]pyrimidin-6-yl)-1,3-dihydrospiro[indene-2,4'-piperidin]-1-yl)-2-methylpropane-2-sulfinamide **5e** (130 mg, 218.65 µmol) was added to a concentrated hydrochloric acid (3 mL) and ethanol (3 mL), heated to 100°C, and reacted for 1 hour. After the reaction was completed, the reaction solution was concentrated under reduced pressure, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain ethyl (S)-6-(1-amino-1,3-dihydrospiro[indene-2,4'-piperidin]-1'-yl)-3-(2,3-dichlorophenyl)-1H-pyrazolo[3,4-d]pyrimidine-4-carboxylate **42** (20 mg) with a yield of 17%.
MS m/z (ESI): 537.2 [M+1]
¹H NMR (400 MHz, DMSO-*d*₆) δ 8.29 (s, 3H), 7.72-7.85 (m, 1H), 7.51 (d, *J* = 5.0 Hz, 3H), 7.37 (d, *J* = 4.8 Hz, 2H), 7.33 (d, *J* = 6.6 Hz, 1H), 4.60 (d, *J* = 25.5 Hz, 2H), 4.39 (d, *J* = 6.1 Hz, 1H), 3.91 (d, *J* = 7.3 Hz, 2H), 3.22 (d, *J* = 16.4 Hz, 1H), 3.07 (s, 1H), 1.63-1.83 (m, 2H), 1.56 (s, 2H), 1.24 (s, 2H), 0.88 (t, *J* = 7.2 Hz, 3H).

### Example 43

### 6-(4-amino-4-((methylsulfonyl)methyl)piperidin-1-yl)-3-(2,3-dichlorophenyl)-1H-pyrazolo[3,4-d]pyrimidine-4-carboxamide

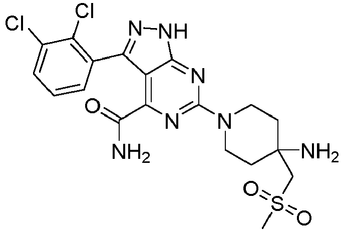

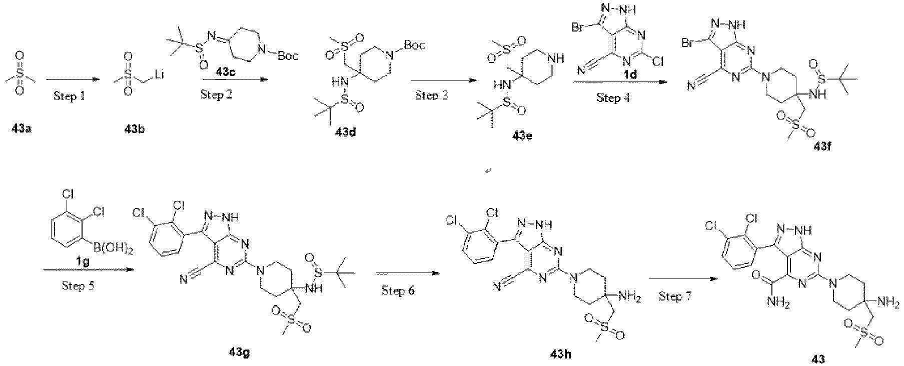

### Step 1

### ((methylsulfonyl)methyl)lithium

(Methylsulfonyl)methane **43a** (300 mg, 3.19 mmol) was added to tetrahydrofuran (4 mL), subjected to nitrogen gas displacement, dropwise added with a normal hexane solution (2.5 M, 1.66 mL) containing n-butyl lithium at -20°C, and continuously at -20°C for 1 hour. After the reaction was completed, ((methylsulfonyl)methyl)lithium **43b** was obtained, which was directly used for the next step without treatment.

### Step 2

### Tert-butyl 4-((tert-butylsulfinyl)amino)-4-((methylsulfonyl)methyl)piperidine-1-carboxylate

Tert-butyl 4-((tert-butylsulfinyl)imino)piperidine-1-carboxylate **43c** (384 mg, 1.27 mmol, self-prepared according to patent WO 2007125321) was added to tetrahydrofuran (3 mL), then the reaction solution was dropwise added to the above-mentioned reaction system at -20°C, and continuously reacted for 1 hour. After the reaction was completed, the reaction solution was added with a saturated ammonium chloride solution and the reaction solution was extracted with ethyl acetate (30 mL×2) to separate an aqueous layer. Combined organic phases were washed with a saturated sodium chloride solution (30 mL×2) in turn, dried with anhydrous sodium sulfate, filtered, and concentrated under reduced pressure. The obtained residue was further analyzed and purified by silica gel column chromatography (eluent: system A) to obtain tert-butyl 4-((tert-butylsulfinyl)amino)-4-((methylsulfonyl)methyl)piperidine-1-carboxylate **43d** (120 mg) with a yield of 23.83%.
MS m/z (ESI): 397.3 [M+1]

### Step 3

### 2-methyl-N-(4-((methylsulfonyl)methyl)piperidin-4-yl)propane-2-sulfinamide

Tert-butyl 4-((tert-butylsulfinyl)amino)-4-((methylsulfonyl)methyl)piperidine-1-carboxylate **43d** (120 mg, 302.60 µmol) was added to dichloromethane (2.5 mL), then added with trifluoroacetic acid (0.5 mL) at room temperature, and reacted for 2 hours. After the reaction was completed, the reaction solution was concentrated under reduced pressure to obtain (2-methyl-N-(4-((methylsulfonyl)methyl)piperidin-4-yl)propane-2-sulfinamide **43e** (89.71 mg) with a yield of 100.00%, which was directly used for the next reaction without purification.
MS m/z (ESI): 297.2 [M+1]

### Step 4

### N-(1-(3-bromo-4-cyano-1H-pyrazolo[3,4-d]pyrimidin-6-yl)-4-((methylsulfonyl)methyl)piperidin-4-yl)-2-methylpropane-2-sulfinamide

3-bromo-6-chloro-1H-pyrazolo[3,4-d]pyrimidine-4-carbonitrile **1d** (76.67 mg, 296.63 µmol), 2-methyl-N-(4-((methylsulfonyl)methyl)piperidin-4-yl)propane-2-sulfinamide **43e** (87.94 mg, 296.63 µmol) and N,N-diisopropylethylamine(191.68 mg, 1.48 mmol) were added to N,N-dimethylacetamide (2 mL), heated to 80°C, and reacted for 1 hour. After the reaction was completed, the reaction solution was concentrated under reduced pressure. The obtained residue was purified by silica gel column chromatography (eluent: system A) to obtain N-(1-(3-bromo-4-cyano-1H-pyrazolo[3,4-d]pyrimidin-6-yl)-4-((methylsulfonyl)methyl)piperidin-4-yl)-2-methylpropane-2-sulfinamide **43f** (70 mg) with a yield of 45.52%.
MS m/z (ESI): 518.0 [M+1]

### Step 5

### N-(1-(3-(2,3-dichlorophenyl)-4-cyano-1H-pyrazolo[3,4-d]pyrimidin-6-yl)-4-((methylsulfonyl)methyl)piperidin-4-yl)-2-methylpropane-2-sulfinamide

N-(1-(3-bromo-4-cyano-1H-pyrazolo[3,4-d]pyrimidin-6-yl)-4-((methylsulfonyl)methyl)piperidin-4-yl)-2-methylpropane-2-sulfinamide **43f** (70 mg, 135.02 µmol), (2,3-dichlorophenyl)boronic acid **1g** (103.06 mg, 540.07 µmol), methanesulfonato(2-dicyclohexylphosphino-2',6'-di-isopropoxy-1,1'-biphenyl)(2-amino-1,1'-biphenyl-2-yl)palladium (22.61 mg, 27.00 µmol), 2-dicyclohexylphosphino-2',6'-di-isopropoxy-1,1'-biphenyl (25.20 mg, 54.01 µmol) and potassium phosphate (86.02 mg, 405.05 µmol) were added to a mixed solution of 1,4-dioxane (2 mL) and water (0.2 mL), subjected to argon gas displacement thrice, heated to 100°C, and reacted for 16 hours. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain N-(1-(3-(2,3-dichlorophenyl)-4-cyano-1H-pyrazolo[3,4-d]pyrimidin-6-yl)-4-((methylsulfonyl)methyl)piperidin-4-yl)-2-methylpropane-2-sulfinamide **43g** (60 mg) with a yield of 76%.
MS m/z (ESI): 584.1 [M+1]

### Step 6

### 6-(4-amino-4-((methylsulfonyl)methyl)piperidin-1-yl)-3-(2,3-dichlorophenyl)-1H-pyrazolo[3,4-d]pyrimidine-4-carbonitrile

N-(1-(3-(2,3-dichlorophenyl)-4-cyano-1H-pyrazolo[3,4-d]pyrimidin-6-yl)-4-((methylsulfonyl)methyl)piperidin-4-yl)-2-methylpropane-2-sulfinamide **43g** (10 mg, 17.11 µmol) was added to dichloromethane (2 mL), and dropwise added with hydrochloric acid in methanol (4 M, 17.11 µL), and reacted for 1 hour. After the reaction was completed, the reaction solution was concentrated under reduced pressure to obtain 6-(4-amino-4-((methylsulfonyl)methyl)piperidin-1-yl)-3-(2,3-dichlorophenyl)-1H-pyrazolo[3,4-d]pyrimidine-4-carbonitrile **43h** (8.22 mg) with a yield of 100.00%, which was directly used for the next reaction without purification.
MS m/z (ESI): 479.8 [M+1]

### Step 7

### 6-(4-amino-4-((methylsulfonyl)methyl)piperidin-1-yl)-3-(2,3-dichlorophenyl)-1H-pyrazolo[3,4-d]pyrimidine-4-carboxamide

Sodium hydroxide solution (0.15 mL) was added to a solution of methanol (1 mL) containing 6-(4-amino-4-((methylsulfonyl)methyl)piperidin-1-yl)-3-(2,3-dichlorophenyl)-1H-pyrazolo[3,4-d]pyrimidine-4-carbonitrile **43h** (8.22 mg, 17.11 µmol), then added with hydrogen peroxide (0.3 mL), and stirred at room temperature for 1 hour. After the reaction was completed, a trifluoroacetic acid was added to adjust the pH to be acidic, and then the reaction solution was subjected to liquid chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-((methylsulfonyl)methyl)piperidin-1-yl)-3-(2,3-dichlorophenyl)-1H-pyrazolo[3,4-d]pyrimidine-4-carboxamide **43** (7 mg) with a yield of 82.15%. MS m/z (ESI): 498.1 [M+1]
¹H NMR (400 MHz, DMSO-*d*₆) δ 13.60 (s, 1H), 8.27 (s, 2H), 8.16 (s, 1H), 7.61-7.73 (m, 2H), 7.40 (d, *J* = 4.9 Hz, 2H), 4.58 (s, 1H), 4.31 (s, 1H), 3.90 (s, 2H), 3.64 (s, 2H), 3.19 (s, 3H), 2.13 (d, *J* = 13.6 Hz, 2H), 1.85 (s, 2H).

### Example 44

### 6-(4-amino-4-(6-methoxypyridin-3-yl)piperidin-1-yl)-3-(2,3-dichlorophenyl)-1H-pyrazolo[3,4-d]pyrimidine-4-carboxamide

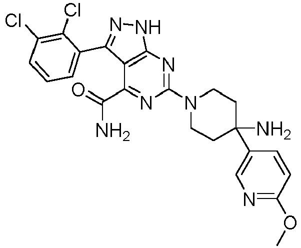

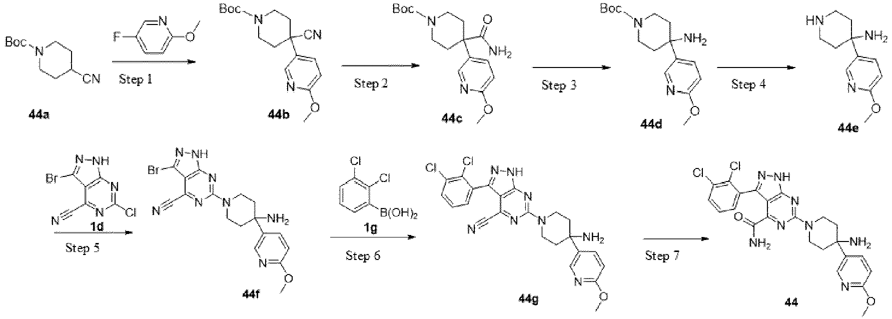

### Step 1

### Tert-butyl 4-(6-methoxypyridin-3-yl)-4-cyanopiperidine-1-carboxylate

At room temperature, tert-butyl 4-cyanopiperidine-1-carboxylate **44a** (19.85 g, 94.4 mmol), 5-fluoro-2-methoxypyridine (3.0 g, 23.6 mmol) and 1 M tetrahydrofuran solution (35.4 mL) containing potassium bis(trimethylsilyl)amide were added to a solution of tetrahydrofuran (40 mL) in turn, and stirred for 1 hour under the protection of argon gas. After the reaction was completed, the reaction solution was cooled to room temperature, added with a saturated aqueous ammonium chloride solution (30 mL), concentrated under reduced pressure, then added with ethyl acetate (30 mL) for liquid separation, then aqueous phases were washed with ethyl acetate (30 mL×2), and organic phases were combined, and washed with saturated salt water, dried by anhydrous sodium sulfate, and concentrated under reduced pressure. The obtained residue was further separated and purified by silica gel column chromatography (eluent: system A) to obtain the product tert-butyl 4-(6-methoxypyridin-3-yl)-4-cyanopiperidine-1-carboxylate **44b** (680 mg) with a yield of 9.1%. MS m/z (ESI): 318.0 [M+1]

### Step 2

### Tert-butyl 4-carbamoyl-4-(6-methoxypyridin-3-yl)piperidine-1-carboxylate

Potassium hydroxide (240 mg, 4.3 mmol) and tert-butyl 4-cyano-4-(6-methoxypyridin-3-yl)piperidine-1-carboxylate **44b** (680 mg, 2.14 mmol) were added to a solution of dimethyl sulfoxide (2 mL), and hydrogen peroxide (30%, 1 mL) was slowly added dropwise to the reaction solution. After the dropwise addition was completed, the reaction solution was stirred for 1 hour. After the reaction was completed, the reaction solution was added with 50 mL of water to precipitate a yellow solid, and filtered, then the filter cake was washed with water, and dried in vacuum to obtain the product tert-butyl 4-carbamoyl-4-(6-methoxypyridin-3-yl)piperidine-1-carboxylate **44c** (370 mg) with a yield of 51.5%.
MS m/z (ESI): 335.9 [M+1]

### Step 3

### Tert butyl 4-amino-4-(6-methoxypyridin-3-yl)piperidine-1-carboxylate

[Bis(trifluoroacetoxy)iodo]benzene (522 mg, 1.21 mmol) was added to a solution of acetonitrile (2 mL) containing tert-butyl 4-carbamoyl-4-(6-methoxypyridin-3-yl)piperidine-1-carboxylate **44c** (370 mg, 1.1 mmol), and stirred at room temperature for 2 hours. After the reaction was completed, the reaction solution was added with a saturated sodium bicarbonate solution (10 mL), and extracted with ethyl acetate (10 mL×3), then organic phases were combined, washed with a saturated sodium chloride solution (10 mL). The organic phases were dried with anhydrous sodium sulfate, filtered, and concentrated under reduced pressure. The obtained residue was purified by silica gel column chromatography (eluent: system A) to obtain the product tert-butyl 4-amino-4-(6-methoxypyridin-3-yl)piperidine-1-carboxylate **44d** (170 mg) with a yield of 50.1%. MS m/z (ESI): 307.8 [M+1]

### Step 4

### 4-(6-methoxypyridin-3-yl)piperidin-4-amine

A trifluoroacetic acid (1 mL) was dropwise added to 3 mL of dichloromethane solution containing tert-butyl 4-amino-4-(6-methoxypyridin-3-yl)piperidine-1-carboxylate **44d** (170 mg, 553 µmol), and reacted at room temperature for 1 hour. The reaction solution was concentrated under reduced pressure to obtain 4-(6-methoxypyridin-3-yl)piperidin-4-amine **44e,** which was directly used for the next reaction without purification.
MS m/z (ESI): 191.2 [M-16]

### Step 5

### 6-(4-amino-4-(6-methoxypyridin-3-yl)piperidin-1-yl)-3-bromo-1H-pyrazolo[3,4-d]pyrimidine-4-carbonitrile

N,N-diisopropylethylamine (225 mg, 1.74 mmol) and the above-mentioned crude product 4-(6-methoxypyridin-3-yl)piperidin-4-amine **44e** were added to a solution of N-methyl pyrrolidone (5 mL) containing 3-bromo-6-chloro-1H-pyrazolo[3,4-d]pyrimidine-4-carbonitrile **1d** (150 mg, 580 µmol), heated to 100°C, and stirred for 1 hour. After the reaction was completed, the reaction solution was subjected to reverse chromatographic purification (C₁₈ separation column 20-45 µm; mobile phase A: H₂O, mobile phase B: CH₃CN) to obtain the product 6-(4-amino-4-(6-methoxypyridin-3-yl)piperidin-1-yl)-3-bromo-1H-pyrazolo[3,4-d]pyrimidine-4-carbonitrile **44f** (150 mg) with a yield of 63.2%.
MS m/z (ESI): 411.8 [M-16]

### Step 6

### 6-(4-amino-4-(6-methoxypyridin-3-yl)piperidin-1-yl)-3-(2,3-dichlorophenyl)-1H-pyrazolo[3,4-d]pyrimidine-4-carbonitrile

6-(4-amino-4-(6-methoxypyridin-3-yl)piperidin-1-yl)-3-bromo-1H-pyrazolo[3,4-d]pyrimidine-4-carbonitrile **44f** (150 mg, 349 µmol), (2,3-dichlorophenyl)boronic acid **1g** (266.7 mg, 1.4 mmol), methanesulfonato(2-dicyclohexylphosphino-2',6'-di-isopropoxy-1,1'-biphenyl)(2-amino-1,1'-biphenyl-2-yl)palladium (58.5 mg, 69.9 µmol), 2-dicyclohexylphosphino-2',6'-di-isopropoxy-1,1'-biphenyl (65.2 mg, 139.8 µmol) and potassium phosphate (222.5 mg, 1.05 mmol) were added to a mixed solution of 1,4-dioxane (5 mL) and water (1 mL), subjected to argon gas displacement thrice, heated to 100°C, and reacted overnight. After the reaction was completed, the reaction solution was concentrated under reduced pressure, and added with ethyl acetate (10 mL) and water (10 mL) for liquid separation, then aqueous phases were extracted with ethyl acetate (10 mL×2), and organic phases were combined, and washed with saturated salt water, dried by anhydrous sodium sulfate, and concentrated under reduced pressure. The obtained residue was purified by silica gel column chromatography (eluent: system A) to obtain 6-(4-amino-4-(6-methoxypyridin-3-yl)piperidin-1-yl)-3 -(2,3 -dichlorophenyl)-1H-pyrazolo[3,4-d]pyrimidine-4-carbonitrile **44g** (30 mg) with a yield of 17.3%.
MS m/z (ESI): 477.8 [M-16]

### Step 7

### 6-(4-amino-4-(6-methoxypyridin-3-yl)piperidin-1-yl)-3-(2,3-dichlorophenyl)-1H-pyrazolo[3,4-d]pyrimidine-4-carboxamide

Potassium hydroxide (6.8 mg, 121 µmol) and 6-(4-amino-4-(6-methoxypyridin-3-yl)piperidin-1-yl)-3-(2,3-dichlorophenyl)-1H-pyrazolo[3,4-d]pyrimidine-4-carbonitrile **44g** (30 mg, 61 µmol) were added to a solution of dimethyl sulfoxide (1 mL), and then hydrogen peroxide (30%, 0.5 mL) was slowly added dropwise to the reaction solution. After the dropwise addition was completed, the reaction solution was stirred for 1 hour. After the reaction was completed, a trifluoroacetic acid was dropwise added to adjust the pH to be 3-4, and then the reaction solution was subjected to liquid chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(6-methoxypyridin-3-yl)piperidin-1-yl)-3-(2,3-dichlorophenyl)-1H-pyrazolo[3,4-d]pyrimidine-4-carboxamide **44** (5 mg) with a yield of 16%.
MS m/z (ESI): 512.8 [M+1]

### Example 45

### 6-(4-amino-4-(6-chloropyridin-3-yl)piperidin-1-yl)-3-(2,3-dichlorophenyl)-1H-pyrazolo[3,4-d]pyrimidine-4-carboxamide

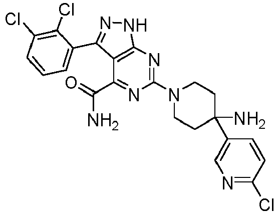

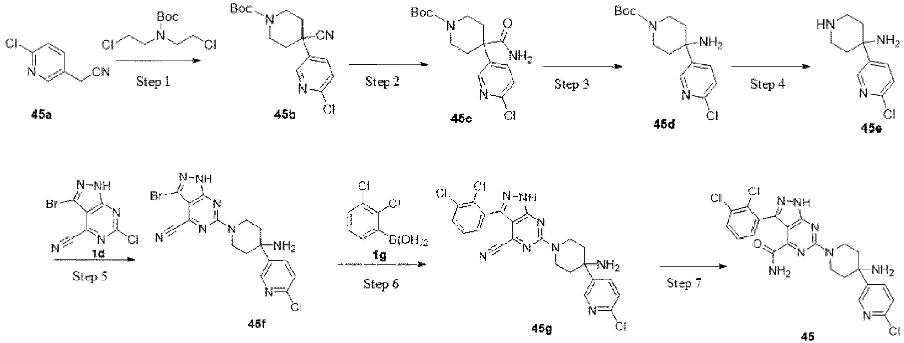

### Step 1

### Tert-butyl 4-(6-chloropyridin-3-yl)-4-cyanopiperidine-1-carboxylate

In an ice water bath, sodium hydride (3.6 g, 90.0 mmol, 60%) was added to a solution of N,N-dimethylformamide (20 mL) containing 2-(6-chloropyridin-3-yl)acetonitrile **45a** (2.29 g, 15 mmol) and tert-butyl bis(2-chloroethyl)carbamate (3.99 g, 16.5 mmol), stirred for 1 hour, heated to 60°C, and then stirred overnight. After the reaction was completed, the reaction solution was cooled to room temperature, quenched with a saturated aqueous ammonium chloride solution (30 mL), and added with ethyl acetate (30 mL) for liquid separation, then aqueous phases were washed with ethyl acetate (30 mL×2), and organic phases were combined, and washed with saturated salt water, dried with anhydrous sodium sulfate, and concentrated under reduced pressure. The obtained residue was purified by silica gel column chromatography (eluent: system A) to obtain the product tert-butyl 4-(6-chloropyridin-3-yl)-4-cyanopiperidine-1-carboxylate **45b** (0.9 g) with a yield of 18.7%.
MS m/z (ESI): 322.0 [M+1]

### Step 2

### Tert-butyl 4-carbamoyl-4-(6-chloropyridin-3-yl)piperidine-1-carboxylate

Potassium hydroxide (314 mg, 5.6 mmol) and tert-butyl 4-(6-chloropyridin-3-yl)-4-cyanopiperidine-1-carboxylate **45b** (0.9 g, 2.8 mmol) were added to a solution of dimethyl sulfoxide (4 mL), and hydrogen peroxide (30%, 2 mL) was slowly added dropwise to the reaction solution. After the dropwise addition was completed, the reaction solution was stirred for 1 hour. After the reaction was completed, the reaction solution was added with 50 mL of water to precipitate a yellow solid, and filtered, then the filter cake was washed with water, and dried in vacuum to obtain the product tert-butyl 4-carbamoyl-4-(6-chloropyridin-3-yl)piperidine-1-carboxylate **45c** (0.9 g) with a yield of 94.7%.
MS m/z (ESI): 340.0 [M+1]

### Step 3

### Tert-butyl 4-amino-4-(6-chloropyridine-3-yl)piperidine-1-carboxylate

[Bis(trifluoroacetoxy)iodo]benzene (1.25 g, 2.91 mmol) was added to a solution of acetonitrile (10 mL) containing tert-butyl 4-carbamoyl-4-(6-chloropyridin-3-yl)piperidine-1-carboxylate **45c** (0.9 g, 2.65 mmol), and stirred at room temperature for 2 hours. After the reaction was completed, the reaction solution was added with a saturated sodium bicarbonate solution (40 mL), and extracted with ethyl acetate (30 mL×3), then organic phases were combined, washed with a saturated sodium chloride solution (30 mL). The organic phases were dried with anhydrous sodium sulfate, filtered, and concentrated under reduced pressure. The obtained residue was purified by silica gel column chromatography (eluent: system A) to obtain the product tert-butyl 4-amino-4-(6-chloropyridin-3-yl)piperidine-1-carboxylate **45d** (0.4 g) with a yield of 48.4%. MS m/z (ESI): 311.9 [M+1]

### Step 4

### 4-(6-chloropyridin-3-yl)piperidin-4-amine

A trifluoroacetic acid (1 mL) was dropwise added to a solution of dichloromethane (3 mL) containing tert-butyl 4-amino-4-(6-chloropyridin-3-yl)piperidine-1-carboxylate **45d** (300 mg, 962 µmol), and stirred at room temperature for 1 hour. The reaction solution was concentrated under reduced pressure to obtain 4-(6-chloropyridin-3-yl)piperidin-4-amine **45e,** which was directly used for the next reaction without purification.
MS m/z (ESI): 212.0 [M+1]

### Step 5

### 6-(4-amino-4-(6-chloropyridin-3-yl)piperidin-1-yl)-3-bromo-1H-pyrazolo[3,4-d]pyrimidine-4-carbonitrile

N,N-diisopropylethylamine (300 mg, 2.32 mmol) and the above-mentioned crude product 4-(6-chloropyridin-3-yl)piperidin-4-amine **45e** were added to a solution of N-methyl pyrrolidone (5 mL) containing 3-bromo-6-chloro-1H-pyrazolo[3,4-d]pyrimidine-4-carbonitrile **1d** (200 mg, 774 µmol), heated to 100°C, and stirred for 1 hour. After the reaction was completed, the reaction solution was purified on a reversed phase chromatographic column (C₁₈ separation column 20-45 µm; mobile phase A: H₂O, mobile phase B: CH₃CN) to obtain 6-(4-amino-4-(6-chloropyridin-3-yl)piperidin-1-yl)-3-bromo-1H-pyrazolo[3,4-d]pyrimidine-4-carbonitrile **45f** (280 mg) with a yield of 83.4%.
MS m/z (ESI): 415.8 [M-16]

### Step 6

### 6-(4-amino-4-(6-chloropyridin-3-yl)piperidin-1-yl)-3-(2,3-dichlorophenyl)-1H-pyrazolo[3,4-d]pyrimidine-4-carbonitrile

6-(4-amino-4-(6-chloropyridin-3-yl)piperidin-1-yl)-3-bromo-1H-pyrazolo[3,4-d]pyrimidine-4-carbonitrile **45f** (280 mg, 646 µmol), (2,3-dichlorophenyl)boronic acid **1g** ( 493 mg, 2.58 mmol), methanesulfonato(2-dicyclohexylphosphino-2',6'-di-isopropoxy-1,1'-biphenyl)(2-amino-1,1'-biphenyl-2-yl)palladium (108 mg, 129 µmol), 2-dicyclohexylphosphino-2',6'-di-isopropoxy-1,1'-biphenyl (121 mg, 258 µmol) and potassium phosphate (411 mg, 1.94 mmol) were added to a mixed solution of 1,4-dioxane (5 mL) and water (1 mL), subjected to argon gas displacement thrice, heated to 100°C, and reacted overnight. After the reaction was completed, the reaction solution was concentrated under reduced pressure, and added with ethyl acetate (10 mL) and water (10 mL) for liquid separation, then aqueous phases were extracted with ethyl acetate (10 mL×2), and organic phases were combined, and washed with saturated salt water, dried by anhydrous sodium sulfate, and concentrated under reduced pressure. The obtained residue was purified by silica gel column chromatography (eluent: system A) to obtain 6-(4-amino-4-(6-chloropyridin-3-yl)piperidin-1-yl)-3-(2,3-dichlorophenyl)-1H-pyrazolo[3,4-d]pyrimidine-4-carbonitrile **45g** (120 mg) with a yield of 37.2%.
MS m/z (ESI): 481.8 [M-16]

### Step 7

### 6-(4-amino-4-(6-chloropyridin-3-yl)piperidin-1-yl)-3-(2,3-dichlorophenyl)-1H-pyrazolo[3,4-d]pyrimidine-4-carboxamide

Potassium hydroxide (8.1 mg, 144 µmol)) and 6-(4-amino-4-(6-chloropyridin-3-yl)piperidin-1-yl)-3-(2,3-dichlorophenyl)-1H-pyrazolo[3,4-d]pyrimidine-4-carbonitrile **45g** (36 mg, 72 µmol) were added to a solution of dimethyl sulfoxide (1 mL), and then hydrogen peroxide (30%, 0.5 mL) was slowly added dropwise to the reaction solution. After the dropwise addition was completed, the reaction solution was stirred for 1 hour. After the reaction was completed, a trifluoroacetic acid was dropwise added to adjust the pH to be 3-4, and then the reaction solution was subjected to liquid chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(6-chloropyridin-3-yl)piperidin-1-yl)-3-(2,3-dichlorophenyl)-1H-pyrazolo[3,4-d]pyrimidine-4-carboxamide **45** (12 mg) with a yield of 32%.
MS m/z (ESI): 516.8 [M+1]
¹H NMR (400 MHz, DMSO-*d*₆) δ 8.76 (d, *J* = 4.0 Hz, 1H), 8.54 (br, 3H), 8.09-8.20 (m, 2H), 7.62-7.76 (m, 3H), 7.35-7.44 (m, 2H), 4.11-4.67 (m, 2H), 3.40-3.70 (m, 2H), 2.55-2.72 (m, 2H), 2.02-2.20 (m, 2H).

### Example 46

### 6-(4-amino-4-(4-methoxyphenyl)piperidin-1-yl)-3-(2,3-dichlorophenyl)-1H-pyrazolo[3,4-d]pyrimidine-4-carboxamide

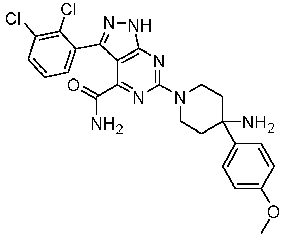

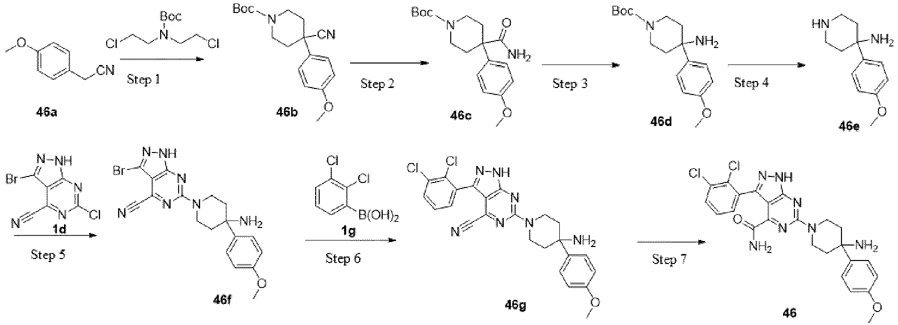

### Step 1

### Tert-butyl 4-cyano-4-(4-methoxyphenyl)piperidine-1-carboxylate

In an ice water bath, tert-butyl bis(2-chloroethyl)carbamate (4 g, 16.52 mmol) and 2-(4-methoxyphenyl)acetonitrile **46a** (2.21 g, 15.02 mmol) were added to N,N-dimethylformamide (35 mL) in turn, added with 60% sodium hydride (3 g, 75.09 mmol) in batches into the above-mentioned mixed solution, and then heated to 60°C, and reacted for 5 hours. The reaction solution was cooled to room temperature, quenched with water (100 mL), and extracted with ethyl acetate (100 mL×2). Organic phases were combined, washed with a saturated sodium chloride solution (100 mL), dried with anhydrous sodium sulfate and concentrated under reduced pressure. The obtained residue was purified by silica gel column chromatography (eluent: system A) to obtain tert-butyl 4-cyano-4-(4-methoxyphenyl)piperidine-1-carboxylate **46b** (1.78 g) with a yield of 37.5%.
MS m/z (ESI): 217.1 [M-99]

### Step 2

### Tert-butyl 4-carbamoyl-4-(4-methoxyphenyl)piperidine-1-carboxylate

Tert-butyl 4-cyano-4-(4-methoxyphenyl)piperidine-1-carboxylate **46b** (1.78 g, 5.63 mmol) and potassium hydroxide (631.34 mg, 11.25 mmol) were added to dimethyl sulfoxide (8 mL) in turn, dropwise added with hydrogen peroxide (4 mL), and continuously stirred for 1 hour. The reaction solution was added with a large amount of water (50 mL) to precipitate a white solid, which was filtered and dried to obtain tert-butyl 4-carbamoyl-4-(4-methoxyphenyl)piperidine-1-carboxylate **46c** (1.45 g) with a yield of 77.1%.
MS m/z (ESI): 279.0 [M-55]

### Step 3

Tert-butyl 4-amino-4-(4-methoxyphenyl)piperidine-1-carboxylate 1,3-dibromo-5,5-dimethylhydantoin (619.88 mg, 2.17 mmol), potassium hydroxide (1.09 g, 19.51 mmol) and tert-butyl 4-carbamoyl-4-(4-methoxyphenyl)piperidine-1-carboxylate **46c** (1.45 g, 4.34 mmol) were added to a mixed solution of acetonitrile (10 mL) and water (10 mL) in turn, and stirred at room temperature for 2 hours. The reaction solution was concentrated under reduced pressure and purified on a C₁₈ reversed phase chromatographic column (C₁₈ separation column 20-45 µm; mobile phase A: H₂O, mobile phase B: CH₃CN) to obtain tert-butyl 4-amino-4-(4-methoxyphenyl)piperidine-1-carboxylate **46d** (1.18 g) with a yield of 88.8%.
MS m/z (ESI): 234.1 [M-72]

### Step 4

### 4-(4-methoxyphenyl)piperidin-4-amine

Tert-butyl 4-amino-4-(4-methoxyphenyl)piperidine-1-carboxylate **46d** (818 mg, 2.67 mmol) was dissolved in dichloromethane (10 mL), slowly added with a trifluoroacetic acid (6.56 g, 57.51 mmol), and stirred overnight at room temperature. After the reaction was completed, the reaction solution was concentrated under reduced pressure to obtain 4-(4-methoxyphenyl)piperidin-4-amine **46e,** which was directly used for the next reaction without purification.
MS m/z (ESI): 190.1 [M-16]

### Step 5

### 6-(4-amino-4-(4-methoxyphenyl)piperidin-1-yl)-3-bromo-1H-pyrazolo[3,4-d]pyrimidine-4-carbonitrile

The above-mentioned crude product 4-(4-methoxyphenyl)piperidin-4-amine **46e,** 3-bromo-6-chloro-1H-pyrazolo[3,4-d]pyrimidine-4-carbonitrile **1d** (689.12 mg, 2.67 mmol) and N,N-diisopropylethylamine (1.38 g, 10.66 mmol) were added to N-methyl pyrrolidone (8 mL) in turn, subjected to argon gas replacement thrice, and continuously stirred at 100°C for 4 hours. The reaction solution was quenched with water (30 mL), and extracted with ethyl acetate (30 mL×3). Organic phases were combined, dried with anhydrous sodium sulfate and concentrated under reduced pressure. The obtained residue was purified by silica gel column chromatography (eluent: system B) to obtain 6-(4-amino-4-(4-methoxyphenyl)piperidin-1-yl)-3-bromo-1H-pyrazolo[3,4-d]pyrimidine-4-carbonitrile **46f** (1.1 g) with a yield of 96.3%.
MS m/z (ESI): 411.0 [M-16]

### Step 6

### 6-(4-amino-4-(4-methoxyphenyl)piperidin-1-yl)-3-(2,3-dichlorophenyl)-1H-pyrazolo[3,4-d]pyrimidine-4-carbonitrile

6-(4-amino-4-(4-methoxyphenyl)piperidin-1-yl)-3-bromo-1H-pyrazolo[3,4-d]pyrimidine-4-carbonitrile **46f** (1.1 g, 2.57 mmol), (2,3-dichlorophenyl)boronic acid **1g** (1.96 g, 10.27 mmol), methanesulfonato(2-dicyclohexylphosphino-2',6'-di-isopropoxy-1,1'-biphenyl)(2-amino-1,1'-biphenyl-2-yl)palladium (430.14 mg, 513.68 µmol), 2-dicyclohexylphosphino-2',6'-di-isopropoxy-1,1'-biphenyl (479.39 mg, 1.03 mmol) and potassium phosphate (2.18 g, 10.27 mmol) were added to a mixed solution of 1,4-dioxane (12 mL) and water (1.2 mL) in turn, subjected to argon gas displacement thrice, heated to 100°C, and reacted overnight. After the reaction was completed, the reaction solution was concentrated under reduced pressure, added with water (10 mL) and extracted with ethyl acetate thrice (100 mL×3), then organic phases were combined, and washed with saturated salt water, dried by anhydrous sodium sulfate, and concentrated under reduced pressure. The obtained residue was purified by silica gel column chromatography (eluent: system B) to obtain 6-(4-amino-4-(4-methoxyphenyl)piperidin-1-yl)-3-(2,3-dichlorophenyl)-1H-pyrazolo[3,4-d]pyrimidine-4-carbonitrile **46g** (400 mg) with a yield of 31.5%.
MS m/z (ESI): 476.9 [M-16]

### Step 7

### 6-(4-amino-4-(4-methoxyphenyl)piperidin-1-yl)-3-(2,3-dichlorophenyl)-1H-pyrazolo[3,4-d]pyrimidine-4-carboxamide

6-(4-amino-4-(4-methoxyphenyl)piperidin-1-yl)-3-(2,3-dichlorophenyl)-1H-pyrazolo[3,4-d]pyrimidine-4-carbonitrile **46g** (300 mg, 606.83 µmol) and potassium hydroxide (68.10 mg, 1.21 mmol) were added to dimethyl sulfoxide (4 mL) in turn, and then dropwise added with hydrogen peroxide (1 mL). After the reaction solution was continuously stirred at room temperature for 1 hour, the reaction solution was added with water (20 mL) to precipitate a faint yellow solid, neutralized with a dilute hydrochloric acid, and extracted with ethyl acetate (20 mL×3), then organic phases were combined, and washed with saturated salt water, dried by anhydrous sodium sulfate, and concentrated under reduced pressure. The obtained residue was purified by silica gel column chromatography (eluent: system B) to obtain 6-(4-amino-4-(4-methoxyphenyl)piperidin-1-yl)-3-(2,3-dichlorophenyl)-1H-pyrazolo[3,4-d]pyrimidine-4-carboxamide **46** (200 mg) with a yield of 64.6%.
MS m/z (ESI): 495.1 [M-16]
¹H NMR (400MHz, CD₃OD) δ 7.62-7.66 (m, 2H), 7.58 (dd, *J* = 7.6, 2.0 Hz, 1H), 7.33-7.40 (m, 2H), 7.08-7.12 (m, 2H), 4.72-4.75 (m, 2H), 3.85 (s, 3H), 3.33-3.40 (m, 2H), 2.72-2.76 (m, 2H), 2.08-2.16 (m, 2H).

### Example 47

### 6-(4-amino-4-(4-hydroxyphenyl)piperidin-1-yl)-3-(2,3-dichlorophenyl)-1H-pyrazolo[3,4-d]pyrimidine-4-carboxamide

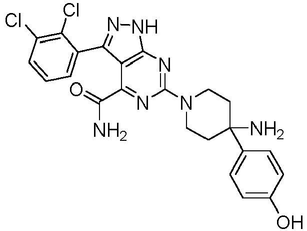

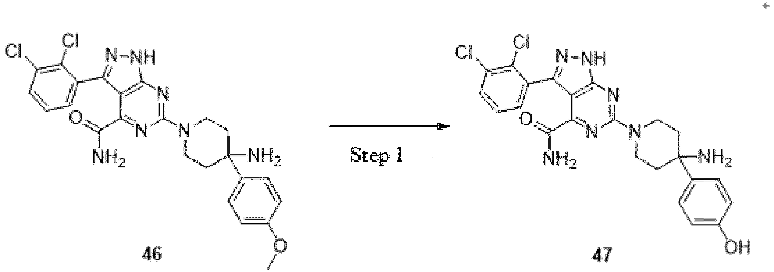

### Step 1

### 6-(4-amino-4-(4-hydroxyphenyl)piperidin-1-yl)-3-(2,3-dichlorophenyl)-1H-pyrazolo[3,4-d]pyrimidine-4-carboxamide

6-(4-amino-4-(4-methoxyphenyl)piperidin-1-yl)-3-(2,3-dichlorophenyl)-1H-pyrazolo[3,4-d]pyrimidine-4-carboxamide **46** (50 mg, 97.58 µmol) was dissolved in dichloromethane (3 mL), slowly added dropwise with boron tribromide (5 mL, 1.0 M dichloromethane solution), and reacted at room temperature for 2 hours. After the reaction was completed, the reaction solution was added with ice water (100 mL), and extracted with dichloromethane (100 mL×3). Organic phases were combined, concentrated under reduced pressure, and subjected to liquid chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(4-hydroxyphenyl)piperidin-1-yl)-3-(2,3-dichlorophenyl)-1H-pyrazolo[3,4-d]pyrimidine-4-carboxamide **47** (15 mg) with a yield of 30.9%.
MS m/z (ESI): 481.1 [M-16]
¹H NMR (400MHz, CD₃OD) δ 7.58 (dd, *J* = 7.6, 2.0 Hz, 1H), 7.52-7.55 (m, 2H), 7.33-7.40 (m, 2H), 6.94-6.96 (m, 2H), 4.72-4.76 (m, 2H), 3.32-3.38 (m, 2H), 2.71-2.75 (m, 2H), 2.06-2.13 (m, 2H).

### Example 48

### 6-(4-amino-4-(2-methoxyphenyl)piperidin-1-yl)-3-(2,3-dichlorophenyl)-1H-pyrazolo[3,4-d]pyrimidine-4-carboxamide

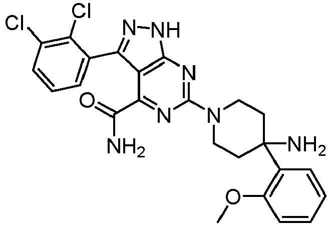

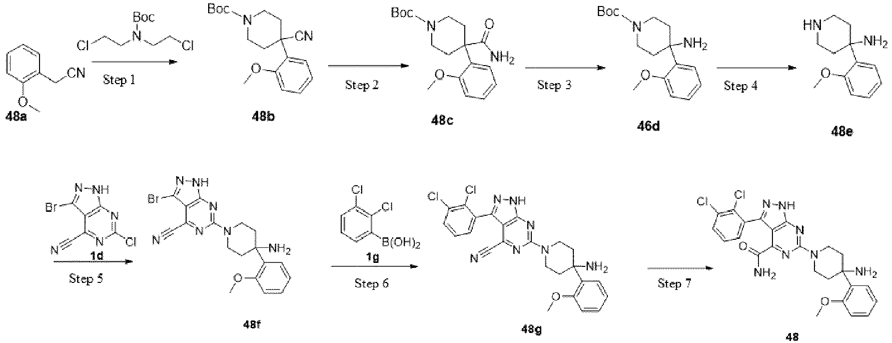

### Step 1

Tert-buttyl 4-cyano-4-(2-methoxyphenyl)piperidine-1-carboxylate 2-(2-methoxyphenyl)acetonitrile **48a** (1 g, 6.79 mmol) and tert-butyl bis(2-chloroethyl)carbamate (1.81 g, 7.47 mmol) were dissolved in N,N-dimethylformamide (6 mL), added with 60% sodium hydride (1.06 g, 26.51 mmol) in batches, stirred for 40 minutes, heated to 70°C, and reacted overnight. The reaction solution was cooled to room temperature, quenched with water (100mL), extracted with ethyl acetate (100 mL×3). Organic phases were combined, washed with a saturated sodium chloride solution (100 mL), dried with anhydrous sodium sulfate and concentrated under reduced pressure. The obtained residue was purified by silica gel column chromatography (eluent: system A) to obtain tert-butyl 4-cyano-4-(2-methoxyphenyl)piperidine-1-carboxylate **48b** (1.7 g) with a yield of 79.1%.
MS m/z (ESI): 217.0 [M-99]

### Step 2

### Tert-butyl 4-carbamoyl-4-(2-methoxyphenyl)piperidine-1-carboxylate

Potassium hydroxide (283.75 mg, 5.06 mmol) and tert-butyl 4-cyano-4-(2-methoxyphenyl)piperidine-1-carboxylate **48b** (0.8 g, 2.53 mmol) were dissolved in dimethyl sulfoxide (5 mL), slowly added dropwise with hydrogen peroxide (5 mL), and reacted at room temperature overnight. The reaction solution was added with a large amount of water to precipitate a yellow solid, which was filtered, then the filter cake was washed with water and dried to obtain tert-butyl 4-carbamoyl-4-(2-methoxyphenyl)piperidine-1-carboxylate **48c** (400 mg) with a yield of 47.3%.
MS m/z (ESI): 279.0 [M-55]

### Step 3

### Tert-butyl 4-amino-4-(2-methoxyphenyl)piperidine-1-carboxylate

Potassium hydroxide (302.02 mg, 5.38 mmol) was added to a mixed solution of acetonitrile (2 mL) and water (3 mL) containing tert-butyl 4-carbamoyl-4-(2-methoxyphenyl)piperidine-1-carboxylate **48c** (400 mg, 1.20 mmol), added with 1,3-dibromo-5,5-dimethylhydantoin (188.10 mg, 657.88 µmol) in batches, and stirred at room temperature for 1 hour. The reaction solution was added with water (100 mL) and potassium phosphate (279.30 mg, 1.32 mmol) and stirred for 15 minutes, then added with ethyl acetate (20 mL) and sodium sulphite (15.07 mg, 119.61 µmol) for liquid separation, aqueous phases were extracted with ethyl acetate (20 mL×2), organic phases were combined and washed with a sodium chloride solution (20 mL), dried, and concentrated under reduced pressure to obtain tert-butyl 4-amino-4-(2-methoxyphenyl)piperidine-1-carboxylate **48d** (366 m) with a yield of 99.6%, which was directly used for the next reaction without purification.
MS m/z (ESI): 234.1 [M-72]

### Step 4

### 4-(2-methoxyphenyl)piperidin-4-amine

Tert-butyl 4-amino-4-(2-methoxyphenyl)piperidine-1-carboxylate **48d** (366 mg, 1.19 mmol) was dissolved in dichloromethane (15 mL), slowly added with a trifluoroacetic acid (3 g, 26.31 mmol), and stirred overnight at room temperature. After the reaction was completed, the reaction solution was concentrated under reduced pressure to obtain 4-(2-methoxyphenyl)piperidin-4-amine **48e,** which was directly used for the next reaction without purification.
MS m/z (ESI): 190.1 [M-16]

### Step 5

### 6-(4-amino-4-(2-methoxyphenyl)piperidin-1-yl)-3-bromo-1H-pyrazolo[3,4-d]pyrimidine-4-carbonitrile

3-bromo-6-chloro-1H-pyrazolo[3,4-d]pyrimidine-4-carbonitrile **1d** (283.17 mg, 1.10 mmol), the above-mentioned crude product 4-(2-methoxyphenyl)piperidin-4-amine **48e** and N,N-diisopropylethylamine (566.37 mg, 4.38 mmol) were added to N-methyl pyrrolidone (3 mL) in turn, subjected to argon gas replacement, and continuously stirred at 100°C for 2 hours. The reaction solution was quenched with water (30 mL), and extracted with ethyl acetate (30 mL×3). Organic phases were combined, dried with anhydrous sodium sulfate and concentrated under reduced pressure. The obtained residue was purified by silica gel column chromatography (eluent: system B) to obtain 6-(4-amino-4-(2-methoxyphenyl)piperidin-1-yl)-3-bromo-1H-pyrazolo[3,4-d]pyrimidine-4-carbonitrile **48f** (360 mg) with a yield of 70.8%.
MS m/z (ESI): 411.0 [M-16]

### Step 6

### 6-(4-amino-4-(2-methoxyphenyl)piperidin-1-yl)-3-(2,3-dichlorophenyl)-1H-pyrazolo[3,4-d]pyrimidine-4-carbonitrile

6-(4-amino-4-(2-methoxyphenyl)piperidin-1-yl)-3-bromo-1H-pyrazolo[3,4-d]pyrimidine-4-carbonitrile **48f** (360 mg, 840.56 µmol), (2,3-dichlorophenyl)boronic acid **1g** (641.58 mg, 3.36 mmol), methanesulfonato(2-dicyclohexylphosphino-2',6'-di-isopropoxy-1,1'-biphenyl)(2-amino-1,1'-biphenyl-2-yl)palladium (140.77 mg, 168.11 µmol), 2-dicyclohexylphosphino-2',6'-di-isopropoxy-1,1'-biphenyl (156.89 mg, 336.22 µmol) and potassium phosphate (713.70 mg, 3.36 mmol) were added to a mixed solution of 1,4-dioxane (4 mL) and water ( (0.4 mL) in turn, subjected to argon gas displacement thrice, heated to 100°C, and reacted overnight. After the reaction was completed, the reaction solution was cooled to room temperature, added with water (20 mL) and extracted with ethyl acetate (50 mL×3), then organic phases were combined, and washed with saturated salt water, dried by anhydrous sodium sulfate, and concentrated under reduced pressure. The obtained residue was purified by silica gel column chromatography (eluent: system B) to obtain 6-(4-amino-4-(2-methoxyphenyl)piperidin-1-yl)-3-(2,3-dichlorophenyl)-1H-pyrazolo[3,4-d]pyrimidine-4-carbonitrile **48g** (160 mg) with a yield of 38.5%.
MS m/z (ESI): 477.1 [M-16]

### Step 7

### 6-(4-amino-4-(2-methoxyphenyl)piperidin-1-yl)-3-(2,3-dichlorophenyl)-1H-pyrazolo[3,4-d]pyrimidine-4-carboxamide

6-(4-amino-4-(2-methoxyphenyl)piperidin-1-yl)-3-(2,3-dichlorophenyl)-1H-pyrazolo[3,4-d]pyrimidine-4-carbonitrile **48g** (160 mg, 323.64 µmol) and potassium hydroxide (36.32 mg, 647.28 µmol) were added to dimethyl sulfoxide (5 mL) in turn, and then dropwise added with hydrogen peroxide (0.5 mL). After the reaction solution was continuously stirred at room temperature for 1 hour, the reaction solution was added with water (20 mL) to precipitate a faint yellow solid, neutralized with a dilute hydrochloric acid, and extracted with ethyl acetate (20 mL×3), then organic phases were combined, and washed with saturated salt water, dried by anhydrous sodium sulfate, and concentrated under reduced pressure. The obtained residue was purified by silica gel column chromatography (eluent: system B) to obtain 6-(4-amino-4-(2-methoxyphenyl)piperidin-1-yl)-3-(2,3 -dichlorophenyl)-1H-pyrazolo[3,4-d]pyrimidine-4-carboxamide **48** (90 mg) with a yield of 54.4%.
MS m/z (ESI): 495.1 [M-16]

### Example 49

### 6-(4-amino-4-(2-hydroxyphenyl)piperidin-1-yl)-3-(2,3-dichlorophenyl)-1H-pyrazolo[3,4-d]pyrimidine-4-carboxamide

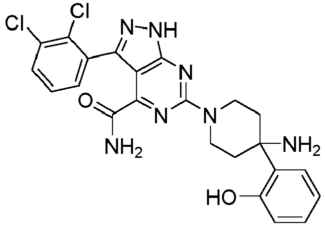

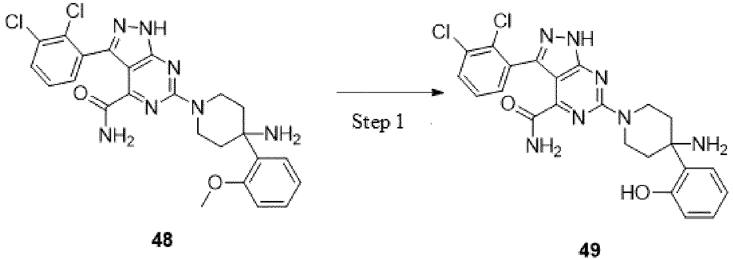

### Step 1

### 6-(4-amino-4-(2-hydroxyphenyl)piperidin-1-yl)-3-(2,3-dichlorophenyl)-1H-pyrazolo[3,4-d]pyrimidine-4-carboxamide

6-(4-amino-4-(2-methoxyphenyl)piperidin-1-yl)-3-(2,3-dichlorophenyl)-1H-pyrazolo[3,4-d]pyrimidine-4-carboxamide **48** (20 mg, 39 µmol) was dissolved in dichloromethane (6 mL), and dropwise added with boron tribromide (488.92 mg, 1.95 mmol). After the reaction was completed, the reaction solution was added with dichloromethane (40 mL) and water (50 mL) for extraction. Aqueous phases were extracted with dichloromethane (40 mL×3). Organic phases were combined, concentrated under reduced pressure, and subjected to liquid chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(2-hydroxyphenyl)piperidin-1-yl)-3-(2,3-dichlorophenyl)-1H-pyrazolo[3,4-d]pyrimidine-4-carboxamide **49** (3 mg) with a yield of 15.4%.
MS m/z (ESI): 481.0 [M-16]

### Example 50

### 6-(4-amino-4-(3-methoxyphenyl)piperidin-1-yl)-3-(2,3-dichlorophenyl)-1H-pyrazolo[3,4-d]pyrimidine-4-carboxamide

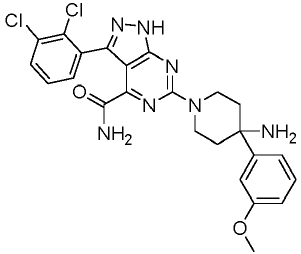

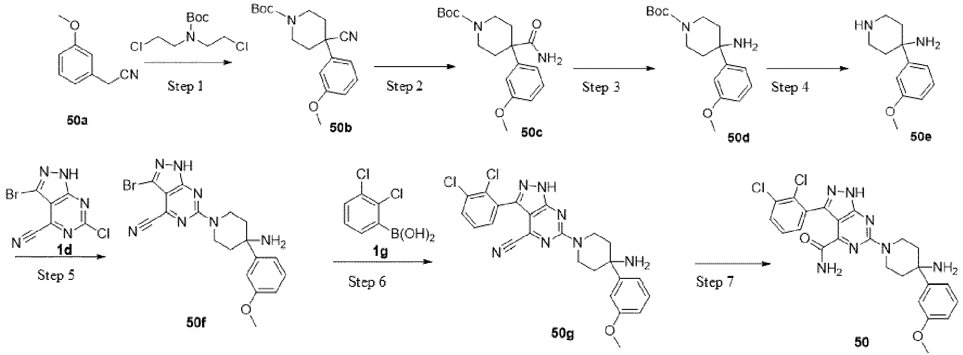

### Step 1

Tert-butyl 4-cyano-4-(3-methoxyphenyl)piperidine-1-carboxylate 2-(3-methoxyphenyl)acetonitrile **50a** (2 g, 13.59 mmol) and tert-butyl bis(2-chloroethyl)carbamate (3.62 g, 14.95 mmol) were dissolved in N,N-dimethylformamide (12 mL), added with 60% sodium hydride (2.17 g, 54.36 mmol) in batches, stirred for 40 minutes, heated to 70°C, and reacted overnight. The reaction solution was cooled to room temperature, quenched with water (100 mL), and extracted with ethyl acetate (100 mL×3). Organic phases were combined, washed with a saturated sodium chloride solution (100 mL), dried with anhydrous sodium sulfate and concentrated under reduced pressure. The obtained residue was purified by silica gel column chromatography (eluent: system A) to obtain tert-butyl 4-cyano-4-(3-methoxyphenyl)piperidine-1-carboxylate **50b** (3.8 g) with a yield of 88.3%.
MS m/z (ESI): 217.0 [M-99]

### Step 2

### Tert-butyl 4-carbamoyl-4-(3-methoxyphenyl)piperidine-1-carboxylate

Potassium hydroxide (709.37 mg, 12.64 mmol) and tert-butyl 4-cyano-4-(3-methoxyphenyl)piperidine-1-carboxylate **50b** (2.00 g, 6.32 mmol) were dissolved in dimethyl sulfoxide (10 mL), slowly added dropwise with hydrogen peroxide (2 mL), and reacted at room temperature overnight. After the reaction was completed, the reaction solution was added with water (50 mL) to precipitate a yellow solid, and filtered, then the filter cake was washed with water, and dried to obtain tert-butyl 4-carbamoyl-4-(3-methoxyphenyl)piperidine-1-carboxylate **50c** (1.7 g) with a yield of 80.4%.
MS m/z (ESI): 279.0 [M-55]

### Step 3

### Tert-butyl 4-amino-4-(3-methoxyphenyl)piperidine-1-carboxylate

Potassium hydroxide (1.28 g, 22.88 mmol) was added to a mixed solution of acetonitrile (6 mL) and water (9 mL) containing tert-butyl 4-carbamoyl-4-(3-methoxyphenyl)piperidine-1-carboxylate 50c (1.7 g, 5.08 mmol), added with 1,3-dibromo-5,5-dimethylhydantoin (799.43 mg, 2.80 mmol) in batches, and stirred at room temperature for 1 hour. The reaction solution was added with water (20 mL) and potassium phsophate (1.19 g, 5.59 mmol) and stirred for 15 minutes, then added with ethyl acetate (50 mL) and sodium sulphite (64.05 mg, 508.36 µmol) for liquid separation, aqueous phases were extracted with ethyl acetate (50 mL×2), organic phases were combined and washed with a sodium chloride solution (20 mL), dried and concentrated under reduced pressure. The obtained residue was further analyzed and purified by silica gel column chromatography (eluent: system A) to obtain tert-butyl 4-amino-4-(3-methoxyphenyl)piperidine-1-carboxylate **50d** (1.0 g) with a yield of 64.2%.
MS m/z (ESI): 234.1 [M-72]

### Step 4

### 4-(3-methoxyphenyl)piperidin-4-amine

Tert-butyl 4-amino-4-(3-methoxyphenyl)piperidine-1-carboxylate **50d** (1 g, 3.26 mmol) was dissolved in dichloromethane (10 mL), slowly added with a trifluoroacetic acid (5 g, 43.85 mmol), and stirred overnight at room temperature. After the reaction was completed, the reaction solution was concentrated under reduced pressure to obtain 4-(3-methoxyphenyl)piperidin-4-amine **50e,** which was directly used for the next reaction without purification.
MS m/z (ESI): 190.1 [M-16]

### Step 5

### 6-(4-amino-4-(3-methoxyphenyl)piperidin-1-yl)-3-bromo-1H-pyrazolo[3,4-d]pyrimidine-4-carbonitrile

4-(3-methoxyphenyl)piperidin-4-amine **50e** (300 mg, 1.45 mmol), 3-bromo-6-chloro-1H-pyrazolo[3,4-d]pyrimidine-4-carbonitrile **1d** (375.88 mg, 1.45 mmol) and N,N-diisopropylethylamine (751.82 mg, 5.82 mmol) were added to N-methyl pyrrolidone (5 mL) in turn, subjected to argon gas replacement, and continuously stirred at 100°C for 4 hours. After the reaction was completed, the reaction solution was quenched with water (30 mL), and extracted with ethyl acetate (30 mL×3). Organic phases were combined, dried with anhydrous sodium sulfate and concentrated under reduced pressure. The obtained residue was purified by silica gel column chromatography (eluent: system B) to obtain 6-(4-amino-4-(3-methoxyphenyl)piperidin-1-yl)-3-bromo-1H-pyrazolo[3,4-d]pyrimidine-4-carbonitrile **50f** (550 mg) with a yield of 88.3%.
MS m/z (ESI): 411.0 [M-16]

### Step 6

### 6-(4-amino-4-(3-methoxyphenyl)piperidin-1-yl)-3-(2,3-dichlorophenyl)-1H-pyrazolo[3,4-d]pyrimidine-4-carbonitrile

6-(4-amino-4-(3-methoxyphenyl)piperidin-1-yl)-3-bromo-1H-pyrazolo[3,4-d]pyrimidine-4-carbonitrile **50f** (0.55 g, 1.28 mmol), (2,3-dichlorophenyl)boronic acid **1g** (980.19 mg, 5.14 mmol), methanesulfonato(2-dicyclohexylphosphino-2',6'-di-isopropoxy-1,1'-biphenyl)(2-amino-1,1'-biphenyl-2-yl)palladium (215.07 mg, 256.84 µmol), 2-dicyclohexylphosphino-2',6'-di-isopropoxy-1,1'-biphenyl (239.70 mg, 513.68 µmol) and potassium phosphate (1.09 g, 5.14 mmol) were added to a mixed solution of 1,4-dioxane (7 mL) and water ( (0.7 mL) in turn, subjected to argon gas displacement thrice, heated to 100°C, and reacted overnight. After the reaction was completed, the reaction solution was cooled to room temperature, added with water (20 mL) and extracted with ethyl acetate (20 mL×3), then organic phases were combined, and washed with saturated brine, dried by anhydrous sodium sulfate, and concentrated under reduced pressure. The obtained residue was purified by silica gel column chromatography (eluent: system B) to obtain 6-(4-amino-4-(3-methoxyphenyl)piperidin-1-yl)-3-(2,3-dichlorophenyl)-1H-pyrazolo[3,4-d]pyrimidine-4-carbonitrile **50g** (290 mg) with a yield of 46.0%.
MS m/z (ESI): 477.1 [M-16]

### Step 7

### 6-(4-amino-4-(3-methoxyphenyl)piperidin-1-yl)-3-(2,3-dichlorophenyl)-1H-pyrazolo[3,4-d]pyrimidine-4-carboxamide

6-(4-amino-4-(3-methoxyphenyl)piperidin-1-yl)-3-(2,3-dichlorophenyl)-1H-pyrazolo[3,4-d]pyrimidine-4-carbonitrile **50g** (290.00 mg, 586.60 µmol) and potassium hydroxide (65.83 mg, 1.17 mmol) were added to dimethyl sulfoxide (4 mL) in turn, and then dropwise added with hydrogen peroxide (1 mL). After the reaction solution was continuously stirred at room temperature for 1 hour, the reaction solution was added with water (20 mL) to precipitate a faint yellow solid, neutralized with a dilute hydrochloric acid, and extracted with ethyl acetate (20 mL×3), then organic phases were combined, and washed with saturated salt water, dried by anhydrous sodium sulfate, and concentrated under reduced pressure. The obtained residue was purified by silica gel column chromatography (eluent: system B) to obtain 6-(4-amino-4-(3-methoxyphenyl)piperidin-1-yl)-3-(2,3 -dichlorophenyl)-1H-pyrazolo[3,4-d]pyrimidine-4-carboxamide **50** (210 mg) with a yield of 70%.
MS m/z (ESI): 495.1 [M-16]
¹H NMR (400MHz, CD₃OD) δ 7.58 (dd, *J* = 7.6, 2.0 Hz, 1H), 7.50 (t, *J* = 8.0 Hz, 1H), 7.33-7.40 (m, 2H), 7.26-7.29 (m, 1H), 7.24 (t, *J* = 2.0 Hz, 1H), 7.08 (dd, *J* = 8.0, 2.0 Hz, 1H), 4.66-4.69 (m, 2H), 3.87 (s, 3H), 3.46-3.51 (m, 2H), 2.72-2.75 (m, 2H), 2.10-2.19 (m, 2H).

### Example 51

### 6-(4-amino-4-(3-hydroxyphenyl)piperidin-1-yl)-3-(2,3-dichlorophenyl)-1H-pyrazolo[3,4-d]pyrimidine-4-carboxamide

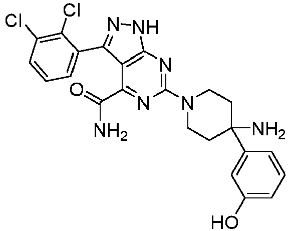

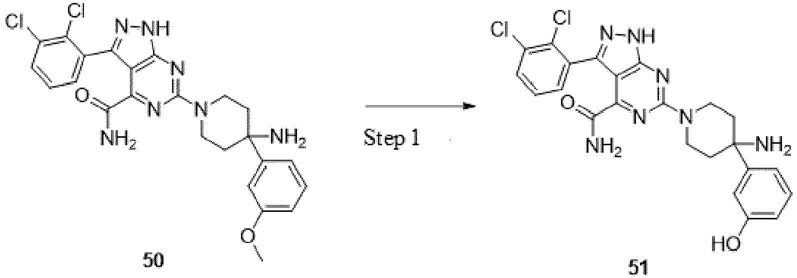

### Step 1

### 6-(4-amino-4-(3-hydroxyphenyl)piperidin-1-yl)-3-(2,3-dichlorophenyl)-1H-pyrazolo[3,4-d]pyrimidine-4-carboxamide

6-(4-amino-4-(3-methoxyphenyl)piperidin-1-yl)-3-(2,3-dichlorophenyl)-1H-pyrazolo[3,4-d]pyrimidine-4-carbonitrile **50** (80 mg, 156.13 µmol)) was dissolved in dichloromethane (4 mL), slowly added dropwise with boron tribromide (5 mL, 1.0 M dichloromethane solution) in an ice bath, and reacted at room temperature for 2 hours. The reaction solution was added with ice water (20 mL), and extracted with dichloromethane (20 mL×3). Organic phases were combined, concentrated under reduced pressure, and subjected to liquid chromatogramatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(3-hydroxyphenyl)piperidin-1-yl)-3-(2,3-dichlorophenyl)-1H-pyrazolo[3,4-d]pyrimidine-4-carboxamide **51** (15 mg) with a yield of 19.3%.
MS m/z (ESI): 481.1 [M-16]
¹H NMR (400MHz, CD₃OD) δ 7.57-7.59 (m, 1H), 7.33-7.40 (m, 3H), 7.12-7.17 (m, 2H), 6.90 (d *J* = 8.0 Hz, 1H), 4.67-4.71 (m, 2H), 3.47 (t, *J* = 12.0 Hz, 2H), 2.69-2.73 (m, 2H), 2.09-2.16 (m, 2H).

### Example 52

### 6-(4-amino-4-cyclopropylpiperidin-1-yl)-3-(2,3-dichlorophenyl)-1H-pyrazolo[3,4-d]pyrimidine-4-carboxamide

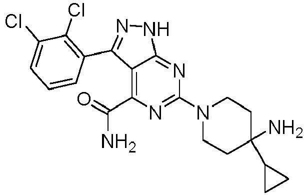

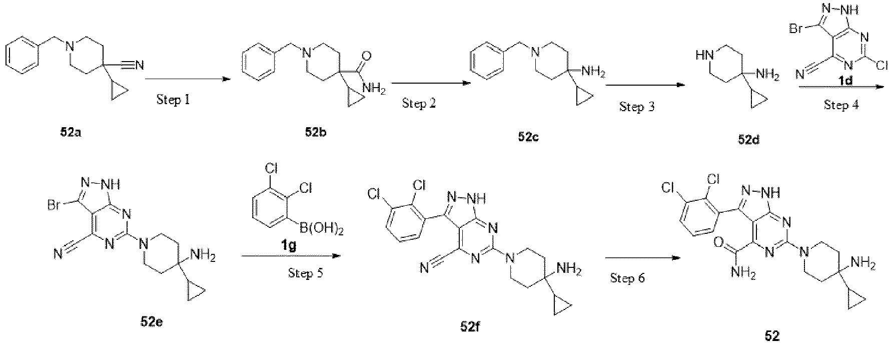

### Step 1

### 1-benzyl-4-cyclopropylpiperidine-4-carbonitrile

At room temperature, potassium hydroxide (1.31 g, 23.30 mmol) was added to a solution of tertiary butanol (20 mL) containing 1-benzyl-4-cyclopropylpiperidine-4-carbonitrile 52a (280 mg, 1.17 mmol, self-prepared according to patent WO 2003042174), heated to 110°C, and reacted overnight. The reaction solution was concentrated under reduced pressure, added with 20 mL of water, extracted with dichloromethane (30 mL×3), and washed with a saturated sodium chloride solution (20 mL). Organic phases were dried with anhydrous sodium sulfate, filtered, and concentrated under reduced pressure to obtain 1-benzyl-4-cyclopropylpiperidine-4-carboxamide **52b** (300 mg) with a yield of 99.1%, which was directly used for the next reaction without purification.
MS m/z (ESI): 259.2 [M+1]

### Step 2

### 1-benzyl-4-cyclopropylpiperidin-4-amine

Potassium hydroxide (293.19 mg, 5.23 mmol) was added to a mixed solution of acetonitrile (2 mL) and water (6 mL) containing 1-benzyl-4-cyclopropylpiperidine-4-carboxamide **52b** (300 mg, 1.16 mmol), added with 1,3-dibromo-5,5-dimethylhydantoin (249.00 mg, 870.89 µmol), and stirred at room temperature for 1.5 hours. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 1-benzyl-4-cyclopropylpiperidin-4-amine **52c** (200 mg) with a yield of 74.77%.
MS m/z (ESI): 231.2 [M+1]

### Step 3

### 4-cyclopropylpiperidin-4-amine

Palladium on carbon (72.66 mg, 520.95 µmol) was added to a solution of methanol (20 mL) containing 1-benzyl-4-cyclopropylpiperidin-4-amine **52c** (200 mg, 868.25 µmol), subjected to hydrogen gas replacement, and reacted at room temperature for 3 hours. After the reaction was completed, the reaction solution was filtered with diatomite to obtain 4-cyclopropylpiperidin-4-amine **52d** (121.7 mg) with a yield of 99.96%, which was directly used for the next reaction without purification.
MS m/z (ESI): 141.1 [M+1]

### Step 4

6-(4-amino-4-cyclopropylpiperidin-1-yl)-3-bromo-1H-pyrazolo[3,4-d]pyrimidine-4-carbonitrile 3-bromo-6-chloro-1H-pyrazolo[3,4-d]pyrimidine-4-carbonitrile **1d** (224.32 mg, 867.89 µmol), 4-cyclopropylpiperidin-4-amine **52d** (121.7 mg, 867.89 µmol) and N,N-diisopropylethylamine (336.50 mg, 2.60 mmol) were added to N,N-dimethylacetamide (2.5 mL) in turn, heated to 100°C, and reacted for 1 hour. After the reaction was completed, the reaction solution was separated on a C₁₈ reversed phase chromatographic column (C₁₈ separation column 20-45 µm; mobile phase A: H₂O, mobile phase B: CH₃CN) to 6-(4-amino-4-cyclopropylpiperidin-1-yl)-3-bromo-1H-pyrazolo[3,4-d]pyrimidine-4-carbonitrile **52e** (180 mg) with a yield of 57.26%. MS m/z (ESI): 345.0 [M-16]

### Step 5

### 6-(4-amino-4-cyclopropylpiperidin-1-yl)-3-(2,3-dichlorophenyl)-1H-pyrazolo[3,4-d]pyrimidine-4-carbonitrile

6-(4-amino-4-cyclopropylpiperidin-1-yl)-3-bromo-1H-pyrazolo[3,4-d]pyrimidine-4-carbonitrile **52e** (90 mg, 248.46 µmol), (2,3-dichlorophenyl)boronic acid **1g** (165.94 mg, 869.62 µmol), methanesulfonato(2-dicyclohexylphosphino-2',6'-di-isopropoxy-1,1'-biphenyl)(2-amino-1,1'-biphenyl-2-yl)palladium (20.81 mg, 24.85 µmol), 2-dicyclohexylphosphino-2',6'-di-isopropoxy-1,1'-biphenyl (23.19 mg, 49.69 µmol) and potassium phosphate (158.22 mg, 745.39 µmol) were added to a mixed solution of 1,4-dioxane (6 mL) and water (0.6 mL), subjected to argon gas displacement thrice, heated to 110°C, and reacted overnight. After the reaction was completed, the reaction solution was added with 20 mL of water, extracted with ethyl acetate (20 mL×3), and washed with a saturated sodium chloride solution (20 mL), then organic phases were dried with anhydrous sodium sulfate, filtered, and concentrated under reduced pressure. The obtained residue was purified by silica gel column chromatography (eluent: system A) to obtain 6-(4-amino-4-cyclopropylpiperidin-l-yl)-3-(2,3-dichlorophenyl)-1H-pyrazolo[3,4-d]pyrimidine-4-carbonitrile **52f** (15 mg) with a yield of 14.09%.
MS m/z (ESI): 411.1 [M-16]

### Step 6

### 6-(4-amino-4-cyclopropylpiperidin-1-yl)-3-(2,3-dichlorophenyl)-1H-pyrazolo[3,4-d]pyrimidine-4-carboxamide

Sodium hydroxide (7.00 mg, 175.10 µmol) was added to a solution of methanol (2 mL) containing 6-(4-amino-4-cyclopropylpiperidin-1-yl)-3-(2,3-dichlorophenyl)-1H-pyrazolo[3,4-d]pyrimidine-4-carbonitrile **52f** (15 mg, 35.02 µmol), then added with hydrogen peroxide (0.2 mL), and stirred at room temperature for 1 hour. After the reaction was completed, the reaction solution was concentrated under reduced pressure, and subjected to liquid phase chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-cyclopropylpiperidin-1-yl)-3-(2,3-dichlorophenyl)-1H-pyrazolo[3,4-d]pyrimidine-4-carboxamide **52** (2.04 mg) with a yield of 13%.
MS m/z (ESI): 446.1 [M+1]
¹H NMR (400 MHz, DMSO-*d*₆) δ 8.10 (s, 1H), 7.96 (s, 3H), 7.57-7.73 (m, 2H), 7.39 (d, *J* = 4.9 Hz, 2H), 4.20 (s, 2H), 3.80 (s, 2H), 1.68 (s, 4H), 1.23 (s, 1H), 0.43-0.65 (m, 4H).

### Example 53

### 2-(4-amino-4-phenylpiperidin-1-yl)-5-(2,3-dichlorophenyl)-7H-pyrrolo[2,3-d]pyrimidine-4-carboxamide

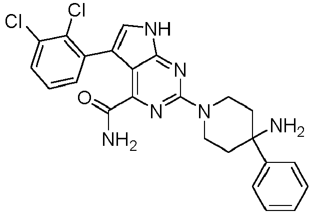

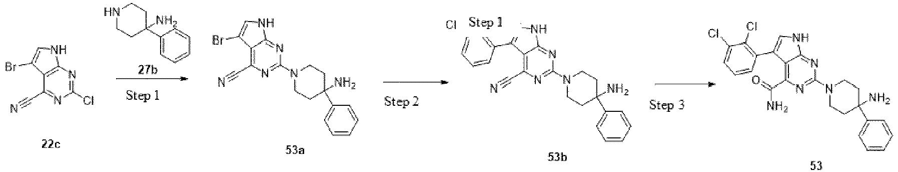

### Step 1

### 2-(4-amino-4- phenylpiperidin-1-yl)-5-bromo-7H-pyrrolo[2,3-d]pyrimidine-4-carbonitrile

N,N-diisopropylethylamine (1.40 g, 10.83 mmol) was added to a solution of N,N-dimethylacetamide (5 mL) containing 4-phenylpiperidin-4-amine **27b** (318 mg, 1.80 mmol), stirred for 30 seconds, added with 5-bromo-2-chloro-7H-pyrrolo[2,3-d]pyrimidine-4-carbonitrile 22c (464.53 mg, 1.80 mmol), heated to 120°C, and reacted overnight. The reaction solution was concentrated under reduced pressure. The obtained residue was purified by silicagel column chromatography (eluent: system B) to obtain 2-(4-amino-4- phenylpiperidin-1-yl)-5-bromo-7H-pyrrolo[2,3-d]pyrimidine-4-carbonitrile **53a** (50 mg) with a yield of 6.98%.
MS m/z (ESI): 380.2 [M-16]

### Step 2

### 2-(4-amino-4- phenylpiperidin-1-yl)-5-(2,3-dichlorophenyl)-7H-pyrrolo[2,3-d]pyrimidine-4-carbonitrile

2-(4-amino-4-phenylpiperidin-1-yl)-5-bromo-7H-pyrrolo[2,3-d]pyrimidine-4-carbonitrile **53a** (50 mg, 125.86 µmol), (2,3-dichlorophenyl)boronic acid **1g** (84.06 mg, 440.51 µmol), methanesulfonato(2-dicyclohexylphosphino-2',6'-di-isopropoxy-1,1'-biphenyl)(2-amino-1,1'-biphenyl-2-yl)palladium (10.54 mg, 12.59 µmol), 2-dicyclohexylphosphino-2',6'-di-isopropoxy-1,1'-biphenyl (11.75 mg, 25.17 µmol) and potassium phosphate (80.15 mg, 377.58 µmol) were added to a mixed solution of 1,4-dioxane (3 mL) and water (0.3 mL) in turn, subjected to argon gas displacement thrice, heated to 110°C, and reacted overnight. After the reaction was completed, the reaction solution was concentrated under reduced pressure. The obtained residue was purified by silica gel column chromatography (eluent: system B) to obtain 2-(4-amino-4- phenylpiperidin-1-yl)-5-(2,3-dichlorophenyl)-7H-pyrrolo[2,3-d]pyrimidine-4-carbonitrile **53b** (50 mg) with a yield of 85.74%.
MS m/z (ESI): 446.1 [M-16]

### Step 3

### 2-(4-amino-4-phenylpiperidin-1-yl)-5-(2,3-dichlorophenyl)-7H-pyrrolo[2,3-d]pyrimidine-4-carboxamide

Sodium hydroxide (21.58 mg, 539.54 µmol) was added to a solution of methanol (3 mL) containing 2-(4-amino-4-phenylpiperidin-1-yl)-5-(2,3-dichlorophenyl)-7H-pyrrolo[2,3-d]pyrimidine-4-carbonitrile **53b** (50 mg, 107.91 µmol), then added with hydrogen peroxide (0.3 mL), and stirred at room temperature for 1 hour. After the reaction was completed, the reaction solution was concentrated under reduced pressure, and subjected to liquid phase chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 2-(4-amino-4-phenylpiperidin-1-yl)-5-(2,3-dichlorophenyl)-7H-pyrrolo[2,3-d]pyrimidine-4-carboxamide **53** (13.14 mg) with a yield of 25%.
MS m/z (ESI): 481.1 [M+1]
¹H NMR (400 MHz, DMSO-*d*₆) δ 11.85 (s, 1H), 8.33 (s, 3H), 7.93 (s, 1H), 7.70 (d, *J* = 7.7 Hz, 2H), 7.39-7.62 (m, 5H), 7.30 (d, *J* = 5.5 Hz, 3H), 4.33 (d, *J* = 13.7 Hz, 2H), 3.90 (s, 2H), 3.44 (t, *J* = 11.5 Hz, 2H), 2.08 (t, *J* = 11.3 Hz, 2H).

### Example 54

### (S)-6-(5-amino-5,7-dihydrospiro[cyclopenta[b]pyridine-6,4'-piperidin]-1'-yl)-3-((2-aminopyrimidine-4-yl)thio)-1H-pyrazolo[3,4-d]pyrimidine-4-carboxamide

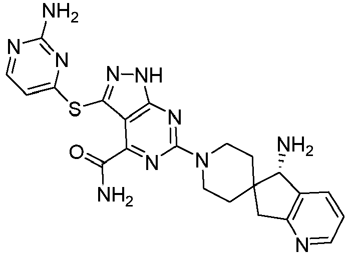

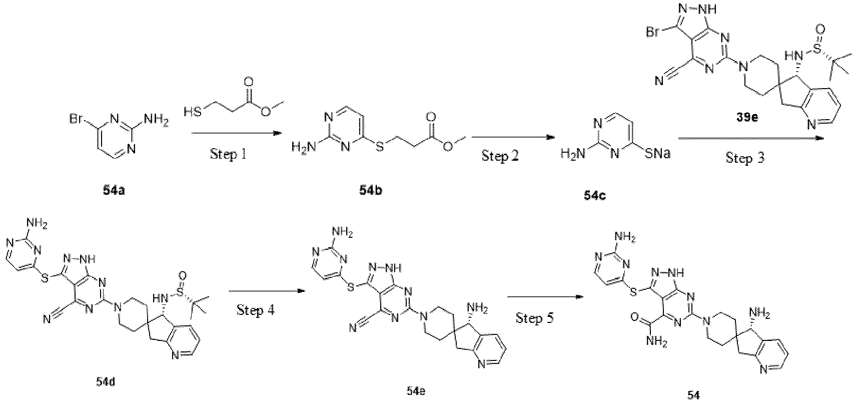

### Step 1

### Methyl 3-((2-aminopyrimidin-4-yl)thio)propanoate

Tris(dibenzylideneacetone)dipalladium (473.65 mg, 517.25 µmol), 4,5-bis(diphenylphosphino)-9,9-dimethylxanthene (598.58 mg, 1.03 mmol), N,N-diisopropylethylamine (4.01 g, 31.03 mmol) and 4-bromopyrimidin-2-amine **54a** (1.8 g, 10.34 mmol) were added to a solution of 1,4-dioxane (30 mL), subjected to argon gas displacement, then added with methyl 3-mercaptopropanoate, subjected to argon gas displacement once, heated to 85°C, and reacted for 3 hours. After the reaction was completed, the reaction solution was concentrated under reduced pressure. The obtained residue was purified by silica gel column chromatography (eluent: system A) to obtain methyl 3-((2-aminopyrimidin-4-yl)thio)propanoate **54b** (2.1 g) with a yield of 95.19%.
MS m/z (ESI): 214.1 [M+1]

### Step 2

### Sodium 2-aminopyrimidine-4-thiolate

At room temperature, sodium methylate (506.62 mg, 9.38 mmol) was added to a solution of methanol (6 mL) containing methyl 3-((2-aminopyrimidin-4-yl)thio)propanoate **54b** (1 g, 4.69 mmol), and stirred at room temperature for 1 hour. After the reaction was completed, the reaction solution was concentrated under reduced pressure to obtain sodium 2-aminopyrimidine-4-thiolate **54c** (670 mg) with a yield of 96.08%.
MS m/z (ESI): 128.1 [M+1]

### Step 3

### (R)-N-((S)-1'-(3-((2-aminopyrimidin-4-yl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide

Sodium 2-aminopyrimidine-4-thiolate **54c** (120 mg, 804.56 µmol), (R)-N-((S)-1'-(3-bromo-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **39e** (340.78 mg, 643.65 µmol), tris(dibenzylideneacetone)dipalladium (73.68 mg, 80.46 µmol), 4,5-bis(diphenylphosphino)-9,9-dimethylxanthene (93.11 mg, 160.91 µmol) and N,N-diisopropylethylamine (311.95 mg, 2.41 mmol) were added to a solution of 1,4-dioxane (3 mL), subjected to argon gas displacement, heated to 100°C, and reacted overnight. After the reaction was completed, the reaction solution was added with 20 mL of water, extracted with dichloromethane (30 mL×3), and washed with a saturated sodium chloride solution (20 mL). Organic phases were dried with anhydrous sodium sulfate, filtered, and concentrated under reduced pressure. The obtained residue was further analyzed and purified by silica gel column chromatography (eluent: system B) to obtain (R)-N-((S)-1'-(3-((2-aminopyrimidin-4-yl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **54d** (140 mg) with a yield of 37%.
MS m/z (ESI): 576.2 [M+1]

### Step 4

### (S)-6-(5-amino-5,7-dihydrospiro[cyclopenta[b]pyridine-6,4'-piperidin]-1'-yl)-3-((2-aminopyrimidin-4-yl)thio)-1H-pyrazolo[3,4-d]pyrimidine-4-carbonitrile

A concentrated hydrochloric acid (17.73 mg, 486.36 µmol) was added to a solution of methanol (3 mL) containing (R)-N-((S)-1'-(3-((2-aminopyrimidin-4-yl)thio)-4-cyano-1H-pyrazolo[3,4-d]pyrimidin-6-yl)-5,7-dihydrospiro[cyclopenta[b]pyridine-6,4'-piperidin]-5-yl)-2-methylpropane-2-sulfinamide **54d** (70 mg, 121.59 µmol),and stirred at room temperature for 2 hours. The reaction solution was concentrated under reduced pressure to obtain (S)-6-(5-amino-5,7-dihydrospiro[cyclopenta[b]pyridine-6,4'-piperidin]-1'-yl)-3-((2-aminopyrimidin-4-yl)thio)-1H-pyrazolo[3,4-d]pyrimidine-4-carbonitrile **54e** (57 mg) with a yield of 99.42%, which was directly used for the next reaction without purification.
MS m/z (ESI): 472.2 [M+1]

### Step 5

### (S)-6-(5-amino-5,7-dihydrospiro[cyclopenta[b]pyridine-6,4'-piperidin]-1'-yl)-3-((2-aminopyrimidin-4-yl)thio)-1H-pyrazolo[3,4-d]pyrimidine-4-carboxamide

Sodium hydroxide (24.18 mg, 604.40 µmol) was added to a solution of methanol (3 mL) containing (S)-6-(5-amino-5,7-dihydrospiro[cyclopenta[b]pyridine-6,4'-piperidin]-1'-yl)-3-((2-aminopyrimidin-4-yl)thio)-1H-pyrazolo[3,4-d]pyrimidine-4-carbonitrile **54e** (57 mg, 120.88 µmol), then added with hydrogen peroxide (0.4 mL), and stirred at room temperature for 1 hour. After the reaction was completed, the reaction solution was concentrated under reduced pressure, and subjected to liquid phase chromatography purification (separation column: AKZONOBEL Kromasil; 250×21.2 mm I.D.; 5 µm, 20 mL/min; mobile phase A: 0.05% TFA+ H₂O, mobile phase B: CH₃CN) to obtain (S)-6-(5-amino-5,7-dihydrospiro[cyclopenta[b]pyridine-6,4'-piperidin]-1'-yl)-3-((2-aminopyrimidin -4-yl)thio)-1H-pyrazolo[3,4-d]pyrimidine-4-carboxamide **54** (7.2 mg) with a yield of 12%.
MS m/z (ESI): 490.2 [M+1] ¹H NMR (400 MHz, CD₃OD) δ 8.48 (d, *J=* 5.2 Hz, 1H), 7.91 (d, *J=* 7.8 Hz, 1H), 7.78 (d, *J=* 6.8 Hz, 1H), 7.25-7.41 (m, 1H), 6.59 (d, *J=* 6.8 Hz, 1H), 4.81-4.85 (m, 2H), 4.45 (s, 1H), 3.33 (q, *J=* 14.9, 14.3 Hz, 2H), 3.16 (s, 2H), 1.65-1.81 (m, 2H), 1.57 (d, *J=* 13.3 Hz, 1H), 1.34-1.48 (m, 1H).

### Example 55

### 6-(4-amino-4-(pyridin-3-yl)piperidin-1-yl)-3-(2,3-dichlorophenyl)-1H-pyrazolo[3,4-d]pyrimidine-4-carboxamide

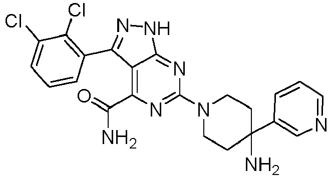

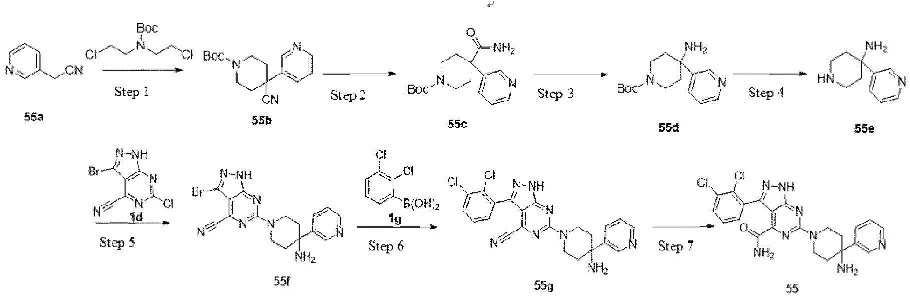

### Step 1

### Tert-butyl 4-cyano-4-(pyridin-3-yl)piperidine-1-carboxylate

Sodium hydride (3.56 g, 88.88 mmol) was added to a solution of N,N-dimethylformamide (35 mL) containing 2-(pyridin-3-yl)acetonitrile **55a** (3 g, 25.39 mmol) and tert-butyl bis(2-chloroethyl)carbamate (6.76 g, 27.93 mmol), heated to 60 °C, and reacted overnight. After the reaction was completed, the reaction solution was quenched with 40 mL of saturated ammonium chloride, extracted with ethyl acetate (40 mL×3), and washed with a saturated sodium chloride solution (30 mL). Organic phases were dried with anhydrous sodium sulfate, filtered, and concentrated under reduced pressure. The obtained residue was further analyzed and purified by silica gel column chromatography (eluent: system A) to obtain tert-butyl 4-cyano-4-(pyridin-3-yl)piperidine-1-carboxylate **55b** (6.6 g) with a yield of 90.44%.
MS m/z (ESI): 288.2 [M+1]

### Step 2

### Tert-butyl 4-carbamoyl-4-(pyridin-3-yl)piperidine-1-carboxylate

Potassium hydroxide (781.05 mg, 13.92 mmol) was added to a solution of dimethyl sulfoxide (12 mL) containing tert-butyl 4-cyano-4-(pyridin-3-yl)piperidine-1-carboxylate **55b** (2 g, 6.96 mmol), and slowly added dropwise with hydrogen peroxide (5 mL). After the reaction was completed, the reaction solution was added with 10 mL of water, continuously stirred for 1 hour, and then filterd to obtain tert-butyl 4-carbamoyl-4-(pyridin-3-yl)piperidine-1-carboxylate **55c** (1.8 g) with a yield of 84.69%.
MS m/z (ESI): 306.2 [M+1]

### Step 3

Tert-butyl 4-amino-4-(pyridin-3-yl)piperidine-1-carboxylate [Bis(trifluoroacetoxy)iodo]benzene (1.24 g, 2.88 mmol) was added to a solution of acetonitrile (12 mL) and water (12 mL) containing tert-butyl 4-carbamoyl-4-(pyridin-3-yl)piperidine-1-carboxylate **55c** (800.00 mg, 2.62 mmol), and reacted at room temperature overnight. After the reaction was completed, the reaction solution was added with 40 mL of sodium bicarbonate solution, extracted with ethyl acetate (30 mL×3), and washed with a saturated sodium chloride solution (30 mL). Organic phases were dried with anhydrous sodium sulfate, filtered, and concentrated under reduced pressure to obtain tert-butyl 4-amino-4-(pyridin-3-yl)piperidine-1-carboxylate **55d** (726 mg) with a yield of 99.91%.
MS m/z (ESI): 278.2 [M+1]

### Step 4

### 4-(pyridin-3-yl)piperidine-4-amine

A trifluoroacetic acid (1 mL) was added to a solution of dichloromethane (4 mL) containing tert-butyl 4-amino-4-(pyridin-3-yl)piperidine-1-carboxylate **55d** (150 mg, 540.81 µmol), and stirred at room temperature for 40 minutes. The reaction solution was concentrated under reduced pressure to obtain 4-(pyridin-3-yl)piperidin-4-amine **55e** (95.86 mg) with a yield of 100.00%, which was directly used for the next reaction without purification.
MS m/z (ESI): 178.1 [M+1]

### Step 5

### 6-(4-amino-4-(pyridin-3-yl)piperidin-1-yl)-3-bromo-1H-pyrazolo[3,4-d]pyrimidine-4-carbonitrile

N,N-diisopropylethylamine (314.86 mg, 2.44 mmol) and 3-bromo-6-chloro-1H-pyrazolo[3,4-d]pyrimidine-4-carbonitrile **1d** (125.94mg, 487.25 µmol) were added to a solution of N-methyl pyrrolidone (5 mL) containing 4-(pyridin-3-yl)piperidin-4-amine **55e** (95 mg, 535.98 µmοl), heated to 110°C, and reacted for 1 hour. After the reaction was completed, the reaction solution was concentrated under reduced pressure and separated on a C₁₈ reversed phase chroatographic column (C₁₈ separation column 20-45 µm; mobile phase A: H₂O, mobile phase B: CH₃CN) to obtain 6-(4-amino-4-(pyridin-3-yl)piperidin-1-yl)-3-bromo-1H-pyrazolo[3,4-d]pyrimidine-4-carbonitrile 55f (130 mg) with a yield of 66.83%.
MS m/z (ESI): 399.0 [M+1]

### Step 6

### 6-(4-amino-4-(pyridin-3-yl)piperidin-1-yl)-3-(2,3-dichlorophenyl)-1H-pyrazolo[3,4-d]pyrimidine-4-carbonitrile

6-(4-amino-4-(pyridin-3-yl)piperidin-1-yl)-3-bromo-1H-pyrazolo[3,4-d]pyrimidine-4-carbonitrile 55f (130 mg, 325.61 µmol), (2,3-dichlorophenyl)boronic acid **1g** (248.53 mg, 1.30 mmol), methanesulfonato(2-dicyclohexylphosphino-2',6'-di-isopropoxy-1,1'-biphenyl)(2-amino-1,1'-biphenyl-2-yl)palladium (54.53 mg, 65.12 µmol), 2-dicyclohexylphosphino-2',6'-di-isopropoxy-1,1'-biphenyl (60.78 mg, 130.25 µmol) and potassium phosphate (207.45 mg, 976.84 µmol) were added to a mixed solution of 1,4-dioxane (8 mL) and water (0.8 mL) in turn, subjected to argon gas displacement thrice, heated to 110°C, and reacted overnight. After the reaction was completed, the reaction solution was concentrated under reduced pressure, and subjected to liquid phase chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-3-yl)piperidin-1-yl)-3-(2,3-dichlorophenyl)-1H-pyrazolo[3,4-d]pyrimidine-4-carbonitrile 55g (10 mg) with a yield of 6.60%.
MS m/z (ESI): 465.1 [M+1]

### Step 7

### 6-(4-amino-4-(pyridin-3-yl)piperidin-1-yl)-3-(2,3-dichlorophenyl)-1H-pyrazolo[3,4-d]pyrimidine-4-carboxamide

Sodium hydroxide solution (0.5 mL) and hydrogen peroxide (0.5 mL) were added to a solution of methanol (1 mL) containing 6-(4-amino-4-(pyridin-3-yl)piperidin-1-yl)-3-(2,3-dichlorophenyl)-1H-pyrazolo[3,4-d]pyrimidine-4-carbonitrile 55g (10 mg, 21.49 µmol) in turn, and stirred at room temperature for 1 hour. After the reaction was completed, the reaction solution was concentrated under reduced pressure, and subjected to liquid phase chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-3-yl)piperidin-1-yl)-3-(2,3-dichlorophenyl)-1H-pyrazolo[3,4-d]pyrimidine-4-carboxamide 55 (2.19 mg) with a yield of 21%.
MS m/z (ESI): 483.1 [M+1]
¹H NMR (400 MHz, CD₃OD) δ 8.99 (s, 1H), 8.74 (s, 1H), 8.28 *(d, J* = 8.3 Hz, 1H), 7.71 (s, 1H), 7.61 (dd, *J=* 7.5, 2.0 Hz, 1H), 7.32-7.50 (m, 2H), 4.67 (d, *J=* 14.2 Hz, 2H), 3.57 (t, *J=* 12.0 Hz, 2H), 2.82 (d, *J=* 13.8 Hz, 2H), 2.15-2.37 (m, 2H).

### Example 56

### 6-(4-amino-4-(pyridin-4-yl)piperidin-1-yl)-3-(2,3-dichlorophenyl)-1H-pyrazolo[3,4-d]pyrimidine-4-carboxamide

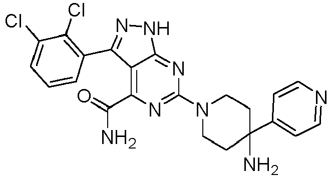

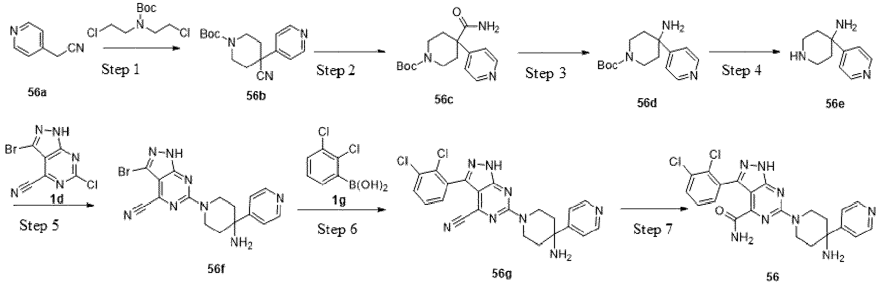

### Step 1

### Tert-butyl 4-cyano-4-(pyridin-4-yl)piperidine-1-carboxylate

Sodium hydride (1.36 g, 33.96 mmol) was added to a solution of N,N-dimethylformamide (25 mL) containing 2-(pyridin-4-yl)acetonitrile 56a (1.5 g, 9.70 mmol) in batches, heated to 60°C, and reacted overnight. After the reaction was completed, the reaction solution was quenched with 40 mL of saturated ammonium chloride, extracted with ethyl acetate (40 mL×3), and washed with a saturated sodium chloride solution (30 mL). Organic phases were dried with anhydrous sodium sulfate, filtered, and concentrated under reduced pressure. The obtained residue was further analyzed and purified by silica gel column chromatography (eluent: system A) to obtain tert-butyl 4-cyano-4-(pyridin-4-yl)piperidine-1-carboxylate 56b (1.65 g) with a yield of 59.18%.
MS m/z (ESI): 288.2 [M+1]

### Step 2

### Tert-butyl 4-carbamoyl-4-(pyridin-4-yl)piperidine-1-carboxylate

Potassium hydroxide (644.37 mg, 11.48 mmol) was added to a solution of dimethyl sulfoxide (15 mL) containing tert-butyl 4-cyano-4-(pyridin-4-yl)piperidine-1-carboxylate 56b (1.65 g, 5.74 mmol), slowly dropwise added with hydrogen peroxide (5 mL) to release heat violently. The reaction solution may be partially cooled with ice water, and reacted at room temperature for 1 hour. After the reaction was completed, a large amount of solids were percipitated and filterd to obtain the product tert-butyl 4-carbamoyl-4-(pyridin-4-yl)piperidine-1-carboxylate 56c (1.08 g) with a yield of 61.59%.
MS m/z (ESI): 306.2 [M+1]

### Step 3

### Tert-butyl 4-amino-4-(pyridin-4-yl)piperidine-1-carboxylate

Potassium hydroxide (892.99 mg, 15.92 mmol) was added to a mixed solution of acetonitrile (2.5 mL) and water (10 mL) containing tert-butyl 4-carbamoyl-4-(pyridin-4-yl)piperidine-1-carboxylate 56c (1.08 g, 3.54 mmol), added with 1,3-dibromo-5,5-dimethylhydantoin (758.41 mg, 2.65 mmol) in a water bath in batches, and stirred at room temperature for 1 hour. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain tert-butyl 4-amino-4-(pyridin-4-yl)piperidine-1-carboxylate 56d (980 mg) with a yield of 99%.
MS m/z (ESI): 278.2 [M+1]

### Step 4

### 4-(pyridin-4-yl)piperidin-4-amine

A trifluoroacetic acid (1 mL) was added to a solution of dichloromethane (4 mL) containing tert-butyl 4-amino-4-(pyridin-4-yl)piperidine-1-carboxylate 56d (250 mg, 901.35 µmol),and stirred at room temperature for 40 minutes. After the reaction was completed, the reaction solution was concentrated under reduced pressure to obtain 4-(pyridin-4-yl)piperidin-4-amine 56e (159.76 mg) with a yield of 100.00%, which was directly used for the next reaction without purification. MS m/z (ESI): 178.1 [M+1]

### Step 5

### 6-(4-amino-4-(pyridin-4-yl)piperidin-1-yl)-3-bromo-1H-pyrazolo[3,4-d]pyrimidine-4-carbonitrile

N,N-diisopropylethylamine (486.11 mg, 3.76 mmol) was added to a solution of N,N-dimethylacetamide (3 mL) containing 4-(pyridin-4-yl)piperidin-4-amine 56e (160 mg, 902.70 µmοl), added with 3-bromo-6-chloro-1H-pyrazolo[3,4-d]pyrimidine-4-carbonitrile Id (194.43 mg, 752.25 µmol), heated to 80°C, and reacted for 1.5 hours. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-4-(pyridin-4-yl)piperidin-1-yl)-3-bromo-1H-pyrazolo[3,4-d]pyrimidine-4-carbonitrile **56f** (274 mg) with a yield of 91.23%.
MS m/z (ESI): 399.1 [M+1]

### Step 6

### 6-(4-amino-4-(pyridin-4-yl)piperidin-1-yl)-3-(2,3-dichlorophenyl)-1H-pyrazolo[3,4-d]pyrimidine-4-carbonitrile

6-(4-amino-4-(pyridin-4-yl)piperidin-1-yl)-3-bromo-1H-pyrazolo[3,4-d]pyrimidine-4-carbonitrile 56f (122 mg, 305.58 µmol), (2,3-dichlorophenyl)boronic acid **1g** (291.55 mg, 1.53 mmol), methanesulfonato(2-dicyclohexylphosphino-2',6'-di-isopropoxy-1,1'-biphenyl)(2-amino-1,1'-biphenyl-2-yl)palladium (51.18 mg, 61.12 µmol), 2-dicyclohexylphosphino-2',6'-di-isopropoxy-1,1'-biphenyl (57.04 mg, 122.23 µmol) and potassium phosphate (194.68 mg, 916.73 µmol) were added to a mixed solution of 1,4-dioxane (8 mL) and water (0.8 mL) in turn, subjected to argon gas displacement thrice, heated to 100°C, and reacted overnight. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-4-(pyridin-4-yl)piperidin-1-yl)-3-(2,3-dichlorophenyl)-1H-pyrazolo[3,4-d]pyrimidine-4-carbonitrile 56g (60 mg) with a yield of 42.2%. MS m/z (ESI): 465.1 [M+1]

### Step 7

### 6-(4-amino-4-(pyridin-4-yl)piperidin-1-yl)-3-(2,3-dichlorophenyl)-1H-pyrazolo[3,4-d]pyrimidine-4-carboxamide

Sodium hydroxide solution (0.5 mL) and hydrogen peroxide (0.5 mL) were added to a solution of methanol (1 mL) containing 6-(4-amino-4-(pyridin-4-yl)piperidin-1-yl)-3-(2,3-dichlorophenyl)-1H-pyrazolo[3,4-d]pyrimidine-4-carbonitrile 56g (60 mg, 128.94 µmol) in turn, and stirred at room temperature for 1.5 hours. After the reaction was completed, the reaction solution was concentrated under reduced pressure, and subjected to liquid phase chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-(pyridin-4-yl)piperidin-1-yl)-3-(2,3-dichlorophenyl)-1H-pyrazolo[3,4-d]pyrimidine-4-carboxamide 56 (16.22 mg) with a yield of 26%.
MS m/z (ESI): 483.1 [M+1]
1H NMR (400 MHz, DMSO-*d*₆) δ 8.74 (d, *J=* 5.2 Hz, 2H), 8.58 (s, 3H), 8.14 (s, 1H), 7.61-7.78 (m, 4H), 7.40 (d, *J* = 4.8 Hz, 2H), 4.27 (s, 2H), 3.81 (s, 2H), 2.47 (s, 2H), 2.09 (s, 2H).

### Example 57

### 6-(4-amino-3-phenylpiperidin-1-yl)-3-(2,3-dichlorophenyl)-1H-pyrazolo[3,4-d]pyrimidine-4-carboxamide

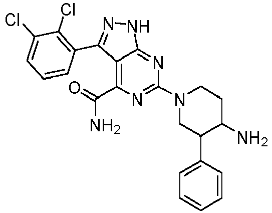

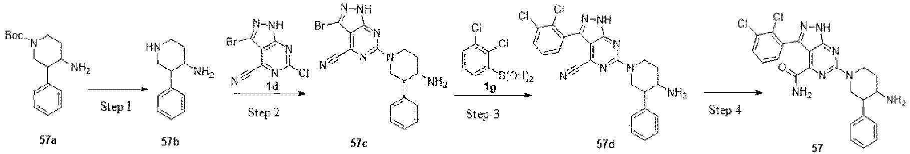

### Step 1

### 3 -phenylpiperidin-4-amine

A trifluoroacetic acid (1.5 mL) and tert-butyl 4-amino-3-phenylpiperidine-1-carboxylate 57a (350 mg, 1.27 mmol, self-prepared according to patent WO2019169153) were added to a solution of dichloromethane (6 mL), and stirred at room temperature for 40 minutes. The reaction solutoin was concentrated under reduced pressure to obtain 3-phenylpiperidin-4-amine 57b (223.21 mg) with a yield of 99%, which was directly used for the next reaction without purification.
MS m/z (ESI): 177.1 [M+1]

### Step 2

6-(4-amino-3-phenylpiperidin-1-yl)-3-bromo-1H-pyrazolo[3,4-d]pyrimidine-4-carbonitrile N,N-diisopropylethylamine (400.03 mg, 3.10 mmol) was added to a solution of N,N-dimethylacetamide (3 mL) containing 3-phenylpiperidin-4-amine 57b (223 mg, 1.26 mmol), added with 3-bromo-6-chloro-1H-pyrazolo[3,4-d]pyrimidine-4-carbonitrile Id (200 mg, 773.81 µmοl), heated to 70°C, and reacted for 1.5 hours. After the reaction was completed, the reaction solution was concentrated under reduced pressure and separated on a C₁₈ reversed phase chromatographic column (C₁₈ separation column 20-45 µm; mobile phase A: H₂O, mobile phase B: CH₃CN) to obtain 6-(4-amino-3-phenylpiperidin-1-yl)-3-bromo-1H-pyrazolo[3,4-d]pyrimidine-4-carbonitrile 57c (180 mg) with a yield of 58.41%.
MS m/z (ESI): 398.1 [M+1]

### Step 3

### 6-(4-amino-3-phenylpiperidin-1-yl)-3-(2,3-dichlorophenyl)-1H-pyrazolo[3,4-d]pyrimidine-4-carbonitrile

6-(4-amino-3-phenylpiperidin-1-yl)-3-bromo-1H-pyrazolo[3,4-d]pyrimidine-4-carbonitrile 57c (180 mg, 451.97 µmol), (2,3-dichlorophenyl)boronic acid **1g** (344.98 mg, 1.81 mmol), methanesulfonato(2-dicyclohexylphosphino-2',6'-di-isopropoxy-1,1'-biphenyl)(2-amino-1,1'-biphenyl-2-yl)palladium (75.69 mg, 90.39 µmοl), 2-dicyclohexylphosphino-2',6'-di-isopropoxy-1,1'-biphenyl (84.36 mg, 180.79 µmol) and potassium phosphate (287.95 mg, 1.36 mmol) were added to a mixed solution of 1,4-dioxane (10 mL) and water (1 mL) in turn, subjected to argon gas displacement thrice, heated to 120°C, and reacted overnight. After the reaction was completed, the reaction solution was concentrated under reduced pressure and separated on a C18 reversed phase chromatographic column (C₁₈ separation column 20-45 µm; mobile phase A: H₂O, mobile phase B: CH₃CN) to obtain 6-(4-amino-3-phenylpiperidin-1-yl)-3-(2,3-dichlorophenyl)-1H-pyrazolo[3,4-d]pyrimidine-4-carbonitrile 57d (21 mg) with a yield of 10.01%.
MS m/z (ESI): 464.1 [M+1]

### Step 4

### 6-(4-amino-3-phenylpiperidin-1-yl)-3-(2,3-dichlorophenyl)-1H-pyrazolo[3,4-d]pyrimidine-4-carboxamide

A sodium hydroxide solution (0.5 mL) was added to a solution of methanol (2 mL) containing 6-(4-amino-3-phenylpiperidin-1-yl)-3-(2,3-dichlorophenyl)-1H-pyrazolo[3,4-d]pyrimidine-4-carbonitrile 57d (21 mg, 45.22 µmol), then added with hydrogen peroxide (0.5 mL), and stirred at room temperature for 1.5 hours. After the reaction was completed, the reaction solution was concentrated under reduced pressure, and subjected to liquid phase chromatography purification (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-3-phenylpiperidin-1-yl)-3-(2,3-dichlorophenyl)-1H-pyrazolo[3,4-d]pyrimidine-4-carboxamide 57 (3.6 mg) with a yield of 16.5%.
MS m/z (ESI): 482.1 [M+1]
¹H NMR (400 MHz, DMSO-*d*₆) δ 8.11 (s, 1H), 7.86 (s, 1H), 7.58-7.77 (m, 2H), 7.41 *(dt, J=* 20.8, 8.0 Hz, 4H), 4.26 (s, 1H), 3.77 (s, 1H), 3.20 (q, *J=* 13.4, 12.6 Hz, 2H), 2.80 (s, 1H), 2.17 (d, *J* = 12.4 Hz, 1H), 1.98 (s, 1H), 1.59 (s, 1H).

### Example 58

### 6-(4-amino-4-ethylpiperidin-1-yl)-3-(2,3-dichlorophenyl)-1H-pyrazolo[3,4-d]pyrimidine-4-carboxamide

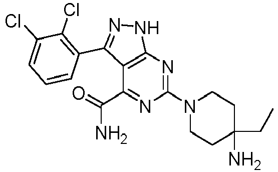

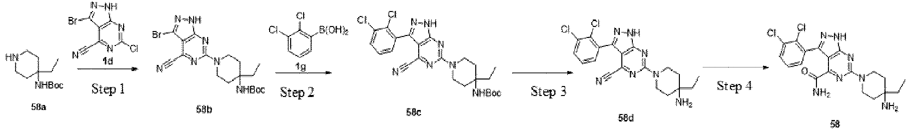

### Step 1

### Tert-butyl (1-(3-bromo-4-cyano-1H-pyrazolo[3,4-d]pyrimidin-6-yl)-4-ethylpiperidin-4-yl)carbamate

3-bromo-6-chloro-1H-pyrazolo[3,4-d]pyrimidine-4-carbonitrile Id (150 mg, 580 µmol), tert-butyl (4-ethylpiperidin-4-yl) carbamate 58a (133 mg, 580 µmol) and N,N-diisopropylethylamine (300 mg, 2.32 mmol, 385 µL) were added to N-methyl pyrrolidone (3 mL) in turn, subjected to argon gas displacement, heated to 100°C, and reacted for 4 hours. After the reaction was completed, the reaction solution was cooled to room temperature, quenched with a saturated aqueous ammonium chloride solution (30 mL), then added with ethyl acetate (30 mL), and aqueous phases were washed with ethyl acetate (30 mL×2). Oorganic phases were combined, and washed with saturated salt water, dried by anhydrous sodium sulfate, and concentrated under reduced pressure. The obtained residue was further separated and purified by silica gel column chromatography (eluent: system A) to obtain tert-butyl (1-(3-bromo-4-cyano-1H-pyrazolo[3,4-d]pyrimidin-6-yl)-4-ethylpiperidin-4-yl)carbamate 58b (250 mg) with a yield of 96%.
MS m/z (ESI): 449.9 [M+1]

### Step 2

### Tert-butyl (1-(3-(2,3-dichlorophenyl)-4-cyano-1H-pyrazolo[3,4-d]pyrimidine-6-yl)-4-ethylpiperidine-4-yl) carbamate

Tert-butyl (1-(3-bromo-4-cyano-1H-pyrazolo[3,4-d]pyrimidin-6-yl)-4-ethylpiperidin-4-yl)carbamate 58b (250 mg, 555 µmοl), (2,3-dichlorophenyl)boronic acid **1g** (424 mg, 2.22 mmol), methanesulfonato(2-dicyclohexylphosphino-2',6'-di-isopropoxy-1,1'-biphenyl)(2-amino-1,1'-biphenyl-2-yl)palladium (93 mg, 111 µmol), 2-dicyclohexylphosphino-2',6'-di-isopropoxy-1,1'-biphenyl (104 mg, 222 µmol) and potassium phosphate (471 mg, 2.22 mmol) were added to a mixed solution of 1,4-dioxane (3 mL) and water (0.3 mL), subjected to argon gas displacement thrice, heated to 130°C, and reacted for 24 hours. After the reaction was completed, the reaction solution was concentrated under reduced pressure, quenched with a saturated aqueous ammonium chloride solution (30 mL), then added with ethyl acetate (30 mL), and aqueous phases were washed with ethyl acetate (30 mL×2). Oorganic phases were combined, and washed with saturated salt water, dried by anhydrous sodium sulfate, and concentrated under reduced pressure. The obtained residue was further separated and purified by silica gel column chromatography (eluent: system A) to obtain tert-butyl (1-(3-(2,3-dichlorophenyl)-4-cyano-1H-pyrazolo[3,4-d]pyrimidine-6-yl)-4-ethylpiperidine-4-yl) carbamate 58c (250 mg) with a yield of 87%.
MS m/z (ESI): 516.2 [M+1]

### Step 3

### 6-(4-amino-4-ethylpiperidin-1-yl)-3-(2,3-dichlorophenyl)-1H-pyrazolo[3,4-d]pyrimidine-4-carbonitrile

Tert-butyl (1-(3-(2,3-dichlorophenyl)-4-cyano-1H-pyrazolo[3,4-d]pyrimidine-6-yl)-4-ethylpiperidine-4-yl) carbamate 58c (250 mg, 484 µmol) was dissolved in dichloromethane (5 mL), slowly added with a trifluoroacetic acid (5.00 g, 43.9 mmol), and stirred overnight at room temperature. After the reaction was completed, the reaction solution was added with ethyl acetate (80 mL), and extracted with saturated sodium bicarbonate (100 mL×3). Organic phases were dried with anhydrous sodium sulfate, filtered, and concentrated under reduced pressure to obtain 6-(4-amino-4-ethylpiperidin-1-yl)-3-(2,3-dichlorophenyl)-1H-pyrazolo[3,4-d]pyrimidine-4-carbonitrile 58d (140 mg) with a yield of 69%, which was directly used for the next reaction without purification.
MS m/z (ESI): 416.1 [M+1]

### Step 4

### 6-(4-amino-4-ethylpiperidin-1-yl)-3-(2,3-dichlorophenyl)-1H-pyrazolo[3,4-d]pyrimidine-4-carboxamide

6-(4-amino-4-ethylpiperidin-1-yl)-3-(2,3-dichlorophenyl)-1H-pyrazolo[3,4-d]pyrimidine-4-carbonitrile 58d (140 mg, 336 µmol) and potassium hydroxide (38 mg, 673 µmol) were added to dimethyl sulfoxide (4 mL) in turn, and then slowly dropwise added with hydrogen peroxide(30%, 1 mL), and continuously stirred at room temperature for 1 hour. After the reaction was completed, a small amount of water was added to precipitate a faint yellow solid, a solid was obtained by filtration, and subjected to liquid phase separation (separation column: AKZONOBEL Kromasil; 250×21.2 mm I.D.; 5 µm, 20 mL/min; mobile phase A: 0.05% TFA + H₂O, mobile phase B: CH₃CN) to obtain 6-(4-amino-4-ethylpiperidin-1-yl)-3-(2,3-dichlorophenyl)-1H-pyrazolo[3,4-d]pyrimidine-4-carboxamide 58 (15 mg) with a yield of 10.3%.
MS m/z (ESI): 433.9 [M+1]
¹H NMR (400MHz, CD3OD) δ 7.57-7.59 (m, 1H), 7.33-7.40 (m, 2H), 4.37-4.41 (m, 2H), 3.76-3.82 (m, 2H), 2.66 (s, 4H), 1.90-1.99 (m, 4H), 1.79-1.86 (m, 2H), 1.06 (t, *J=* 7.6 Hz, 3H).

### Biological evaluation

### Test Example 1 Determination of allosteric inhibition activities of the compounds of the present invention on SHP2

The following method was used to determine the inhibition degrees of the compounds of the present invention on the activity of recombinant human full-length SHP2 in vitro. SHP2 was allosterically activated by the binding of di-tyrosyl-phosphorylated peptide to a Src homologous 2(SH2) domain of SHP2. An activation step of the latter leads to the self-inhibitory interface release of SHP2, and then activates SHP2 protein tyrosine phosphatase (PTP), which might be used for substrate recognition and reaction catalysis.

The experimental process was briefly described as follows: Test compounds were first dissolved in DMSO to prepare storage solutions. The reaction was carried out in a 384-well Small VolumeTM HiBase microplate (Greiner, 784075). Firstly, SHP2 (signalchem, P38-20G-10ug) and SHP-2 Activating Peptide (IRS1_pY1172(dPEG8)pY1222) BPS, 79319-1) were added to the wells till the final concentrations were 0.5 nM and 0.5 uM, respectively. Then, the compounds to be tested were added in a concentration range of 0.00004-10uM and incubated at 25°C for 60 minutes. Then DiFMUP (Thermo, D6567) was added in the reaction and incubated at 25°C for 30 minutes. After incubation, readings were taken using a microplate reader (BMG) with excitation and emission wavelengths of 340 nm and 450 nm, respectively. Compared with the fluorescence intensity ratio of a control group (0.1% DMSO), the percentage inhibition rates of the compounds at each concentration were calculated, and the IC₅₀ values of the compounds were obtained by performing nonlinear regression analysis with logarithmic value of the compound concentration - inhibition rate by GraphPad Prism 5 software, which was shown in Table 1.

**Table 1 IC₅₀ data of the compounds of the present invention on activity inhibition of full-length SHP2 enzyme**

| Compound No. | SHP2/ IC₅₀ (nM) |
|---|---|
| SHP-099 | 128 |
| 2 | 29 |
| 5 | 1 |
| 6 | 4 |
| 7 | 14 |
| 8 | 5 |
| 9 | 1 |
| 10 | 7 |
| 11 | 17 |
| 12 | 8 |
| 13 | 10 |
| 15 | 24 |
| 17 | 4 |
| 23 | 2 |
| 24 | 5 |
| 25 | 5 |
| 27 | 7 |
| 29 | 3 |
| 30 | 3 |
| 32 | 10 |
| 38 | 27 |
| 46 | 3 |
| 51 | 3 |
| 53 | 11 |

Conclusion: it can be seen from Table 1 that the compounds of the present invention have preferable allosteric inhibition effects on SHP2 enzyme.

Remarks: the structure of SHP-099 (prepared according to WO2015107493) is as follows:

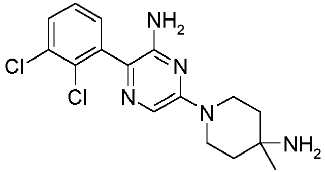

### Test Example 2 Determination of the compounds of the present invention on inhibiting NCI-H23 cell proliferation

The following method was used to determine the effects of the compounds of the present invention on NCI-H23 cell proliferation. NCI-H23 cells (containing KRAS G12C mutation) were purchased from Cell Resource Center of Shanghai Institutes for Biological Sciences, Chinese Academy of Sciences, and cultured in RPMI 1640 mediums containing 10% fetal bovine serum, 100 U penicillin, 100 µg/mL streptomycin and 1 mM Sodium Pyruvate. The activities of the cells were determined by CellTiter-Glo^{®} Luminescent Cell Viability Assay kit (Promega, article number: G7573).

The experimental method was operated according to the steps in the kit instruction, and was briefly described as follows: test compounds were first dissolved in DMSO to prepare storage solutions of 10mM, and then diluted in mediums, prepared as test samples. The final concentrations of the test compounds were 10,000 nM to 1.52 nM. The cells in logarithmic phase were inoculated in a 96-well cell culture plate with 1,000 cells per well, after cultured overnight in 5%CO₂ incubator at 37°C, the test compounds were added to the incubator to continue the culture for 120 hours. After the culture, 50 µL of CellTiter-Glo detection solution was added to each well, shaken for 5 minutes, and then stood for 10 minutes. Then, a Luminescence mode was used to read the luminescence values of each well of the samples on a microplate reader. Compared with the numerical value of a control group (0.3% DMSO), the percentage inhibition rates of the compounds at each concentration were calculated, and the IC₅₀ values of the compounds inhibiting cell proliferation were obtained by performing nonlinear regression analysis with logarithmic values of the compound concentration - inhibition rate by GraphPad Prism 5 software, which was shown in Table 2.

**Table 2 IC₅₀ data of the compounds of the present invention on inhibiting NCI-H23 cell proliferation**

| Example No. | IC₅₀(nM)/NCI-H23 |
|---|---|
| RMC-4550 | 240 |
| 5 | 143 |
| 6 | 61 |
| 9 | 13 |
| 27 | 167 |

It can be seen from Table 2 that the compounds of the present invention have preferable inhibition effects on NCI-H23.

Remarks: the structure of RMC-4550 (prepared according to WO2018013597) is as follows:

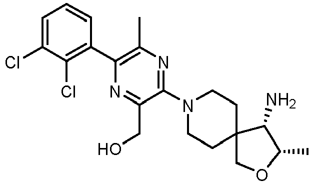

### Test Example 3 Determination of the compounds of the present invention on inhibiting NCI-H358 cell proliferation

The following method was used to determine the effects of the compounds of the present invention on NCI-H358 cell proliferation. NCI-H358 cells (containing KRAS G12C mutation) were purchased from Cell Resource Center of Shanghai Institutes for Biological Sciences, Chinese Academy of Sciences, and cultured in RPMI 1640 mediums containing 10% fetal bovine serum, 100 U penicillin, 100 µg/mL streptomycin and 1 mM Sodium Pyruvate. The activities of the cells were determined by CellTiter-Glo^{®} Luminescent Cell Viability Assay kit (Promega, article number: G7573).

The experimental method was operated according to the steps in the kit instruction, and was briefly described as follows: test compounds were first dissolved in DMSO to prepare storage solutions of 10mM, and then diluted in mediums, prepared as test samples. The final concentrations of the test compounds were 10,000 nM to 1.52 nM. The cells in logarithmic phase were inoculated in a 96-well cell culture plate with 1,000 cells per well, after cultured overnight in 5%CO₂ incubator at 37°C, the test compounds were added to the incubator to continue the culture for 120 hours. After the culture, 50 µL of CellTiter-Glo detection solution was added to each well, shaken for 5 minutes, and then stood for 10 minutes. Then, a Luminescence mode was used to read the luminescence values of each well of the samples on a microplate reader. Compared with the numerical value of the control group (0.3% DMSO), the percentage inhibition rates of the compounds at each concentration were calculated, and the IC₅₀ values of the compounds inhibiting cell proliferation were obtained by performing nonlinear regression analysis with logarithmic values of the compound concentration - inhibition rate by GraphPad Prism 5 software, which was shown in Table 3.

**Table 3 IC₅₀ data of the compounds of the present invention on inhibiting NCI-H23 and NCI-H358 cell proliferation**

| Example No. | IC₅₀(nM)/NCI-H358 |
|---|---|
| RMC-4550 | 80 |
| 5 | 27 |
| 6 | 19 |
| 27 | 37 |

It can be seenb from Table 3 that the compounds of the present invention have preferable inhibition effects on NCI-H358.

### Test Example 4 Pharmacokinetic test

### 1. Experimental purpose

ICR mice were used as test animals, and LC/MS/MS methods was used to determine the drug concentrations at different moments in plasma of mice administered by intragastric injection with the compound 5 and the compound 6 of the present invention, and to study the pharmacokinetic characteristics of the compounds of the present invention in mice.

### 2. Experimental solution

### 2.1 Experimental drugs and animals:

### Compound 5 and compound 6

ICR mice, male, 29.0 g to 33.8 g, purchased from Beijing Charles River Laboratory Animal Technology Co., Ltd.

### 2.2 Drug preparation

An appropriate amount of drug was weighed, added with an appropriate amount of sodium carboxymethylcellulose (CMC-Na, containing 0.5% Tween 80), vortexed, and ultrasonically prepared into 1 mg/kg suspension.

### 2.3 Administration

The ICR mice in the intragastric group of each compound to be tested (9 mice in each group) were fasted overnight and then administered by intragastric injection (PO, administration dose of 1 mg/kg, and administration volume of 10 mL/kg), and ate 4 hours after administration.

### 3. Operation

About 0.2 mL of blood was collected via jugular vein before administration and 0.083 hour, 0.25 hour, 0.5 hour, 1 hour, 2 hours, 4 hours, 8 hours and 24 hours after administration, and heparin sodium was used for anticoagulation. The collected blood samples were placed on ice, and plasma was separated by centrifugation (centrifugation condition: 1,500 g, 10 minutes). The collected plasma samples were stored at-40°C to -20°C before analysis.

LC-MS/MS was used to determine the contents of the compounds to be tested in mouse plasma after intragastric administration.

### 4. Results of pharmacokinetic parameters

The pharmacokinetic parameters of the compounds of the present invention were shown in Table 4.

**Table 4 Pharmacokinetic parameters of mice administrated with compounds**

| Compound No. | Pharmacokinetic experiment | | |
|---|---|---|---|
| | Administration mode and administration dose | Blood concentration Cₘₐₓ (ng/mL) | Area under curve AUC_{0-∞} (ng·h/mL) |
| Compound 5 | PO (10 mg/kg) | 2,113 | 31,264 |
| Compound 6 | PO (10 mg/kg) | 1,192 | 18,471 |

Conclusion: the compound **5** and the compound **6** of the present invention have high blood concentrations and areas under curve, and have good pharmacokinetic properties.

### Test Example 5 Pharmacokinetic test of mice

### 1. Experimental purpose

ICR mice were used as test animals, and LC/MS/MS methods was used to determine the drug concentrations at different moments in plasma of mice administered by intragastric injectiion with the compound 27 of the present invention, and to study the pharmacokinetic characteristics of the compound of the present invention in mice.

### 2. Experimental solution

### 2.1 Experimental drugs and animals:

### Compound 27

ICR mice, male, 29.0 g to 33.8 g, purchased from Beijing Charles River Laboratory Animal Technology Co., Ltd.

### 2.2 Drug preparation

An appropriate amount of drug compound 27 was weighed, added with an appropriate amount of sodium carboxymethylcellulose (CMC-Na, containing 0.5% Tween 80), vortexed, and ultrasonically prepared into 0.5 mg/kg suspension.

### 2.3 Administration

The ICR mice in the intragastric group of each compound to be tested (9 mice in each group) were fasted overnight and then administered by intragastric injection (PO, administration dose of the compound 27 was 5 mg/kg, and administration volume of the compound 27 was 10 mL/kg), and ate 4 hours after administration.

### 3. Operation

About 0.2 mL of blood was collected via jugular vein before administration and 0.083 hour, 0.25 hour, 0.5 hour, 1 hour, 2 hours, 4 hours, 8 hours, 12 hours and 24 hours after administration, and heparin sodium was used for anticoagulation. The collected blood samples were placed on ice, and plasma was separated by centrifugation (centrifugation condition: 1,500 g, 10 minutes). The collected plasma smaples were stored at-40°C to -20°C before analysis.

LC-MS/MS was used to determine the content of the compound to be tested in mouse plasma after intragastric administration.

### 4. Results of pharmacokinetic parameters

The pharmacokinetic parameters of the compound of the present invention were shown in Table 5.

**Table 5 Pharmacokinetic parameters of mice administrated with compound**

| Compound No. | Pharmacokinetic experiment | | | |
|---|---|---|---|---|
| | Administration mode and administration dose | Blood concentration Cₘₐₓ (ng/mL) | Area under curve AUC_{0-∞} (ng·h/mL) | Peak time Tmax (h) |
| Compound 27 | PO (5 mg/kg) | 285 | 2,720 | 8 |

Conclusion: the compound 27 of the present invention has high blood concentration and area under curve, and has good pharmacokinetic properties.

### Test Example 6 Test of growth inhibition effect of the compounds of the present invention in subcutaneous transplanted tumors of NCI-H358 tumor-bearing BALB/c nude mice

### 1. Experimental purpose

To evaluate antitumor effects and safety of the compounds of Example 27 in an animal model of BALB/c nude mice with subcutaneous transplanted NCI-H358 cell lines

### 2. Subject preparation

Solvent control group given DMA: Solutol HS 15: Saline = 5: 10: 85 (v/v/v);

An approximate amount of the compound of Example 27 was weighed, added with an approximate amount of DMA (dimethylacetamide) to fully dissolve the compound, then added with Solutol HS 15 and Saline (DMA: Solutol HS 15: Saline = 5: 10: 85 (v/v/v)) in turn, and mixed evenly by vortex, and the configuration concentration was 3 mg/mL.

### 3. Experimental animals

BALB/c nude mice, female, 6-7 weeks old (the age of mice when tumor cells were inoculated), 12 mice, purchased from Jiangsu GemPharmatech.

### 4. Cell culture

NCI-H358 cells were cultured in RPMI 1640 mediums containing 10% fetal bovine serum, 1% sodium pyruvate and 1% glutamine. NCI-H358 cells in exponential growth period were colllected, and resuspend in PBS to a suitable concentration for subcutaneous tumor inoculation in nude mice.

### 5. Animal modeling and random grouping

12 female BALB/c nude mice were subcutaneously inoculated with about 3×10⁶ NCI-H358 cells on the right sides of their backs. When the average volume of tumors reaches about 100 mm³ to 200 mm³, the mice were randomly divided according to tumor sizes with 6 mice in each group. The animals in each group were given the subjects once a day (qd) according to the animal body weights at a fixed time every day according to the table below, administered orally (po) for 10 consecutive days, and the daily body weights were recorded.

### 6. Animal administration and observation

After tumor inoculation, routine monitoring included the effects of tumor growth and treatment on the normal behaviors of the animals, specifically the mobility, feeding and drinking, weight gain or loss, eyes, coat and other abnormalities of the experimental animals.

Calculation formulae of a relative tumor volume (RTV), a relative tumor inhibition rate (T/C) and a tumor inhibition percentage (IR) were as follows:
(1) TV (tumor volume)= 1/2×a×b², wherein a and b represent the length and the width of the tumor respectively;
(2) RTV (relative tumor volume)=Vt/Vo, wherein Vo is the tumor volume measured at the time of grouping administration (i.e., d0), and Vt is the tumor volume at each measurement;
(3) Relative tumor proliferation rate T/C(%)=T_{RTV}/C_{RTV}×100%, wherein T_{RTV} is the RTV of the treatment group and C_{RTV} is the RTV of the control group;
(4) Tumor growth inhibition rate TGI (%) = (1-T/C)×100%; wherein, T and C are the relative tumor volumes of the treatment group and the control group at a specific time point. 7. Results

**Table 6 Inhibition rate (TGI%) of the compound of the present invention on tumor growth of human non-small cell lung cancer cell NCI-H358 tumor-bearing mice**

| Group | Tumor volume (mm³, mean value ±standard error) | | TGI% |
|---|---|---|---|
| | Day 0 | Day 10 | Day 10 |
| Solvent control group | 138 ± 14 | 347 ± 38 | - |
| Example 27 30 mg/kg | 138 ± 14 | 155 ± 22 | 55.6% |

At the dose of 30 mg/kg, the compound of Example 27 of the present invention has obvious growth inhibition effects on NCI-H358 tumor-bearing BALB/c nude mice, with no obvious change in body weight, and has high safety.

## Claims

1. A compound represented by a general formula (AI) or a stereoisomer or a tautomer thereof, or a pharmaceutically acceptable salt thereof: 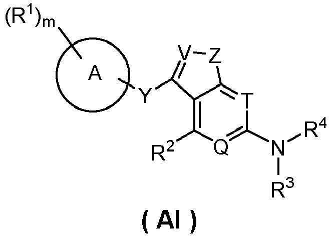 wherein:
Y is selected from a chemical bond or -S-;
when Z is selected from -NH-, V is selected from -N- or -CH-; alternatively, when Z is selected from -O-, V is selected from -N-;
Q and T are each independently selected from N or CH; wherein at least one of Q and T is selected from N;
ring A is selected from aryl, heteroaryl or bicyclic fused ring, wherein the aryl is monocyclic aryl, the heteroaryl is a 5-6 membered monocyclic heteroaryl, and the bicyclic fused ring is preferably a fused ring of aryl or heteroaryl with monocyclic heterocyclyl or monocyclic cycloalkyl;
R¹ are the same or different, and are each independently selected from hydrogen atom, alkyl, alkenyl, alkynyl, cyano, halogen, nitro, cycloalkyl, heterocyclyl, -OR⁵, -C(O)R⁵, -SO₂R⁵, -NR⁶R⁷, -SO₂NR⁶R⁷, -NHSO₂R⁵ or -C(O)NR⁶R⁷, wherein the alkyl, alkenyl, alkynyl, cycloalkyl or heterocyclyl is optionally further substituted by one or more substituents selected from halogen, nitro, cyano, alkyl, cycloalkyl, heterocyclyl, aryl, heteroaryl, -OR⁵, -C(O)R⁵, -C(O)OR⁵, - OC(O)R⁵, -SO₂R⁵, -NR⁶R⁷, -SO₂NR⁶R⁷, -NHSO₂R⁵ or -C(O)NR⁶R⁷;
R² is selected from cyano, tetrazolyl, -C(O)R⁵, -C(O)OR⁵ or -C(O)NR⁶R⁷;
R³ and R⁴ together with the N atom bound therewith form a 4-11 membered heterocyclyl, preferably a 5-11 membered heterocyclyl, wherein the heterocyclyl internally contains one or more N, O, S or SO₂ atoms, and the heterocyclyl is optionally further substituted by one or more substituents selected from halogen, nitro, cyano, alkyl, alkenyl, alkynyl, cycloalkyl, heterocyclyl, aryl, heteroaryl, -CH₂R⁵, -CH(OH)R⁵, -CH₂OR⁵, =O, -OR⁵, -SR⁵, -SOR⁵, -C(O)R⁵, -C(O)OR⁵, - OC(O)R⁵, -SO₂R⁵, -NR⁶R⁷, -SO₂NR⁶R⁷, -NHC(=NH)NH₂, -NHSO₂R⁵ or -C(O)NR⁶R⁷, wherein the alkyl, alkenyl, alkynyl, cycloalkyl, heterocyclyl, aryl or heteroaryl is optionally further substituted by one or more substituents selected from hydroxy, amino, halogen, nitro, cyano, alkyl, haloalkyl, alkoxy, cycloalkyl, heterocyclyl, aryl, heteroaryl, -C(O)R⁸, -C(O)OR⁸, -OC(O)R⁸, - SO₂R⁸, -NR⁹R¹⁰, -C(O)NR⁹R¹⁰, -SO₂NR⁹R¹⁰ or -NR⁹C(O)R¹⁰;
alternatively, R³ and R⁴ together with the N atom bound therewith form a group: 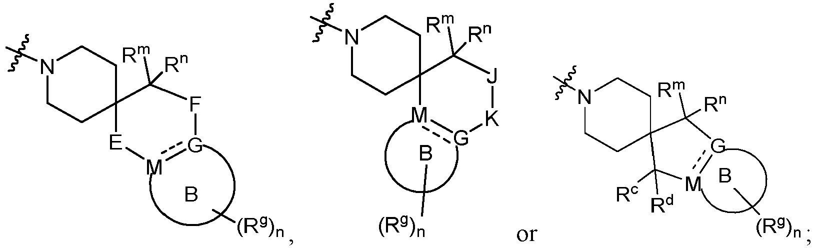 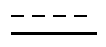
is a single bond or double bond;
when 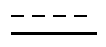 represents a single bond, G and M are each independently selected from N or CR^{j};
when 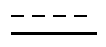 represents a double bond, G and M are each independently selected from C; ring B is selected from cycloalkyl, heterocyclyl aryl or heteroaryl;
E is selected from NR^{k}, (CR^{p}R^{q})ₚ, O or S;
F is selected from (CR^{p}R^{q})_{q};
the condition is that when E is selected from (CR^{p}R^{q})ₚ, p is 1 and q is 1; alternatively, p is 2 and q is 0; and when E is selected from NR^{k}, O or S, q is 1;
J is selected from CR^{p}R^{q};
K is selected from NRk, (CR^{p}R^{q})ᵣ, O or S;
r is 0 or 1;
R^{m}, Rⁿ, R^{p} and R^{q} are the same or different, and are each independently selected from R^{A};
alternatively, R^{p} and R^{q} together with the carbon atom bound therewith form R^{B};
R^{c} and R^{d} are the same or different, and are each independently selected from hydrogen atom, halogen, alkyl or -OR⁵, wherein the alkyl is optionally further substituted by a substituent of hydroxy, halogen, alkoxy, cycloalkyl or -NR⁶R⁷;
alternatively, R^{c} and R^{d} together with the carbon atom bound therewith form R^{B};
R^{g} are the same or different, and are each independently selected from hydrogen atom, halogen, nitro, alkyl, alkenyl, alkynyl, cyano, cycloalkyl, heterocyclyl, aryl, heteroaryl, -OR⁵, - C(O)R⁵, -C(O)OR⁵, -OC(O)R⁵, -SO₂R⁵, -NR⁶R⁷, -SO₂NR⁶R⁷, -NHC(=NH)NH₂, -NHSO₂R⁵ or - C(O)NR⁶R⁷, wherein the alkyl, alkenyl, alkynyl, cycloalkyl, heterocyclyl, aryl or heteroaryl is optionally further substituted by a substituent of hydroxy, halogen, alkyl, alkoxy, cycloalkyl or - NR⁶R⁷;
alternatively, two R^{g} together with the same carbon atom bound therewith form C=O;
R^{j} and R^{k} are the same or different, and are each independently selected from hydrogen atom or alkyl;
R^{A} are the same or different, and are each independently selected from hydrogen atom, halogen, nitro, alkyl, alkenyl, alkynyl, cyano, cycloalkyl, heterocyclyl, aryl, heteroaryl, -OR⁵, - C(O)R⁵, -C(O)OR⁵, -OC(O)R⁵, -SO₂R⁵, -NR⁶R⁷, -SO₂NR⁶R⁷, -NHC(=NH)NH₂, -NHSO₂R⁵ or - C(O)NR⁶R⁷, wherein the alkyl, alkenyl, alkynyl, cycloalkyl, heterocyclyl, aryl or heteroaryl is optionally further substituted by a substituent of hydroxy, halogen, alkyl, alkoxy, cycloalkyl or - NR⁶R⁷;
R^{B} are the same or different, and are each independently selected from 3-10 membered cycloalkyl or 3-10 membered heterocyclyl, wherein the cycloalkyl or heterocyclyl is optionally further substituted by one or more substituents selected from halogen, cyano, nitro, alkyl, cycloalkyl, heterocyclyl, aryl, heteroaryl, =O, -OR⁵, -C(O)R⁵, -C(O)OR⁵, -OC(O)R⁵, -SO₂R⁵, - NR⁶R⁷, -SO₂NR⁶R⁷, -NHC(=NH)NH₂, -NHSO₂R⁵ or -C(O)NR⁶R⁷;
R⁵, R⁶ and R⁷ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, amino, halogen, nitro, cyano, alkyl, alkoxy, cycloalkyl, heterocyclyl, aryl, heteroaryl, -C(O)R⁸, -C(O)OR⁸, - OC(O)R⁸, -SO₂R⁸, -NR⁹R¹⁰, -C(O)NR⁹R¹⁰, -SO₂NR⁹R¹⁰ or -NR⁹C(O)R¹⁰;
alternatively, R⁶ and R⁷ together with the N atom bound therewith form a 3-8 membered heterocyclyl, wherein the 3-8 membered heterocyclyl internally contains one or more N, O, S or SO₂ atoms, and the 3-8 membered heterocyclyl is optionally further substituted by one or more substituents selected from hydroxy, halogen, amino, alkyl or alkoxy;
R⁸, R⁹ and R¹⁰ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, halogen, nitro, cyano, alkyl, alkoxy, cycloalkyl, heterocyclyl, aryl, heteroaryl, carboxyl or carboxylate;
m is 0, 1, 2, 3, 4 or 5;
n is selected from 0, 1, 2, 3 or 4; and
p is selected from 1 or 2.

2. The compound or the stereoisomer or the tautomer thereof, or the pharmaceutically acceptable salt thereof according to claim 1, which is a compound represented by general formula (AII) or a stereoisomer or a tautomer thereof, or a pharmaceutically acceptable salt thereof: 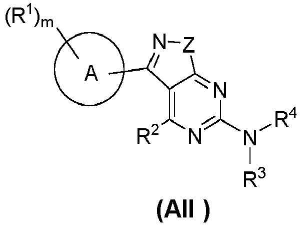 wherein: ring A, m, Z and R¹-R⁴ are defined as in claim 1.

3. The compound or the stereoisomer or the tautomer thereof, or the pharmaceutically acceptable salt thereof according to claim 1, which is a compound represented by general formula (I) or a stereoisomer or a tautomer thereof, or a pharmaceutically acceptable salt thereof: 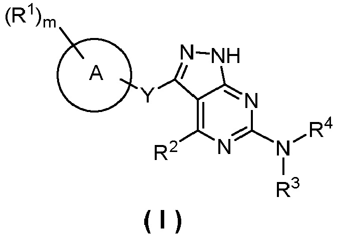 wherein: ring A, Y, m and R¹-R⁴ are defined as in claim 1.

4. The compound or the stereoisomer or the tautomer thereof, or the pharmaceutically acceptable salt thereof according to claim 1, which is a compound represented by general formula (II) or a stereoisomer or a tautomer thereof, or a pharmaceutically acceptable salt thereof: 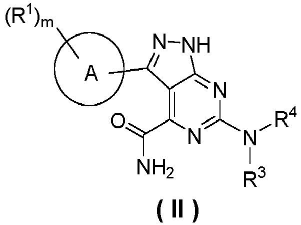 wherein: ring A, m, R¹, R³ and R⁴ are defined as in claim 1.

5. The compound or the stereoisomer or the tautomer thereof, or the pharmaceutically acceptable salt thereof according to any one of claims 1-4, wherein:
R³ and R⁴ together with the N atom bound therewith form a 4-8 membered monocyclic heterocyclyl, preferably a 5-6 membered monocyclic heterocyclyl, more preferably piperidinyl, wherein the monocyclic heterocyclyl is optionally further substituted by one or more substituents selected from methyl, amino, cycloalkyl, phenyl, halophenyl, heteroaryl, -CH₂NH₂, -CH₂OH, - NHC(=NH)NH₂, =O or -OR⁵; wherein the methyl, cycloalkyl, phenyl or heteroaryl is optionally further substituted by substituents selected from one or more of mesyl, hydroxy, amino, halogen, haloalkyl, alkoxy, haloalkoxy, pyridinyl, or pyrimidinyl; wherein the heteroaryl is preferably pyridinyl, pyrimidinomethylbenzopyrazolyl, pyrrolyl, furanyl, thienyl, pyrazolyl, imidazolyl, thiazolyl, benzimidazolyl, benzofuranyl or benzoxazolyl; and
R⁵ is defined as in claim 1.

6. The compound or the stereoisomer or the tautomer thereof, or the pharmaceutically acceptable salt thereof according to any one of claims 1-4, wherein:
R³ and R⁴ together with the N atom bound therewith form a 7-11 membered spiroheterocyclyl, wherein the spiroheterocyclyl is optionally further substituted by one or more substituents selected from methyl, amino, -CH₂NH₂, -CH₂OH, -NHC(=NH)NH₂, =O or -OR⁵; and
R⁵ is defined as in claim 1.

7. The compound or the stereoisomer or the tautomer thereof, or the pharmaceutically acceptable salt thereof according to claim 6, wherein the spiroheterocyclyl is selected from: 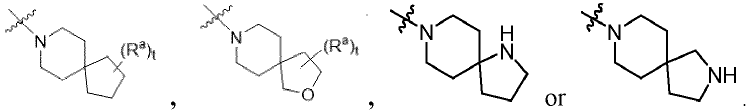
R^{a} are the same or different, and are each independently selected from methyl, amino, - CH₂NH₂, -CH₂OH, -NHC(=NH)NH₂ or -OR⁵;
alternatively, two R^{a} together with the same carbon atom bound therewith form C=O;
t is 1, 2 or 3; and
R⁵ is defined as in claim 1.

8. The compound or the stereoisomer or the tautomer thereof, or the pharmaceutically acceptable salt thereof according to any one of claims 1-4, wherein:
R³ and R⁴ together with the N atom bound therewith form a 7-11 membered bridged heterocyclyl, wherein the bridged heterocyclyl is optionally further substituted by one or more substituents selected from methyl, amino, -CH₂NH₂, -CH₂OH, -NHC(=NH)NH₂, =O or -OR⁵; and
R⁵ is defined as in claim 1.

9. The compound or the stereoisomer or the tautomer thereof, or the pharmaceutically acceptable salt thereof according to any one of claims 1-4, wherein:
R³ and R⁴ together with the N atom bound therewith form a 7-11 membered fused heterocyclyl, wherein the fused heterocyclyl is optionally further substituted by one or more substituents selected from methyl, amino, -CH₂NH₂, -CH₂OH, -NHC(=NH)NH₂, =O or -OR⁵; and
R⁵ is defined as in claim 1.

10. The compound or the stereoisomer or the tautomer thereof, or the pharmaceutically acceptable salt thereof according to claim 3, which is a compound represented by general formula (III) or a stereoisomer or a tautomer thereof, or a pharmaceutically acceptable salt thereof: 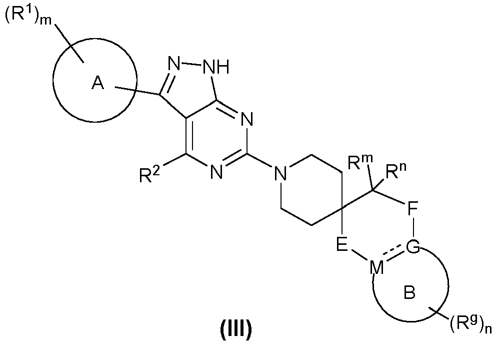 wherein:
ring B is selected from phenyl, 3-8 membered cycloalkyl, 4-8 membered heterocyclyl or 5-6 membered heteroaryl;
E is selected from NR^{k}, (CR^{p}R^{q})ₚ, O or S;
F is selected from ((CR^{p}R^{q})_{q};
the condition is that when E is selected from (CR^{p}R^{q})ₚ, p is 1 and q is 1; alternatively, p is 2 and q is 0; and when E is selected from NR^{k}, O or S, q is 1;
R^{m} is selected from amino, -CH₂NH₂ or -NHC(=NH)NH₂;
Rⁿ is selected from hydrogen atom, methyl or -CH₂OH;
R^{p} and R^{q} are each independently selected from hydrogen atom, halogen, amino, C₁-C₄ alkyl, hydroxy C₁-C₄ alkyls, amino C₁-C₄ alkyls or -OR⁵; and 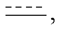
ring A, G, M, m, n, R¹-R², R⁵, R^{k} and R^{g} are defined as in claim 3.

11. The compound or the stereoisomer or the tautomer thereof, or the pharmaceutically acceptable salt thereof according to claim 3, which is a compound represented by general formula (IV) or a stereoisomer or a tautomer thereof, or a pharmaceutically acceptable salt thereof: 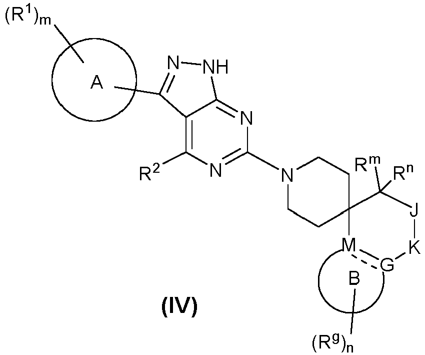 wherein:
ring B is selected from phenyl, 3-8 membered cycloalkyl, 4-8 membered heterocyclyl or 5-6 membered heteroaryl;
J is selected from CR^{p}R^{q};
K is selected from NR^{k}, (CR^{p}R^{q})ᵣ, O or S;
r is 0 or 1;
R^{m} is selected from amino, -CH₂NH₂ or -NHC(=NH)NH₂;
Rⁿ is selected from hydrogen atom, methyl or -CH₂OH;
R^{p} and R^{q} are each independently selected from hydrogen atom, halogen, amino, C₁-C₄ alkyls, hydroxy C₁-C₄ alkyls, amino C₁-C₄ alkyls or -OR⁵; and 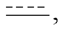 ring A, G, M, m, n, R¹-R², R⁵, R^{k} and R^{g} are defined as in claim 3.

12. The compound or the stereoisomer or the tautomer thereof, or the pharmaceutically acceptable salt thereof according to claim 3, which is a compound represented by general formula (V) or a stereoisomer or a tautomer thereof, or a pharmaceutically acceptable salt thereof: 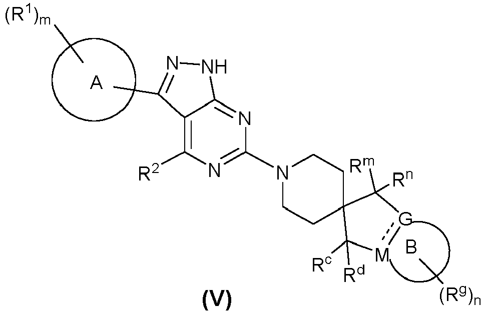 wherein:
ring B is selected from phenyl, 3-8 membered cycloalkyl, 4-8 membered heterocyclyl or 5-6 membered heteroaryl;
R^{c} and R^{d} together with the atom bound therewith form a 3-8 membered cycloalkyl;
R^{m} is selected from amino, -CH₂NH₂ or -NHC(=NH)NH₂;
Rⁿ is selected from hydrogen atom, methyl or -CH₂OH; and 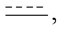
ring A, G, M, m, n, R¹-R² and R^{g} are defined as in claim 3.

13. The compound or the stereoisomer or the tautomer thereof, or the pharmaceutically acceptable salt thereof according to claim 1, which is a compound represented by general formula (VI) or a stereoisomer or a tautomer thereof, or a pharmaceutically acceptable salt thereof: 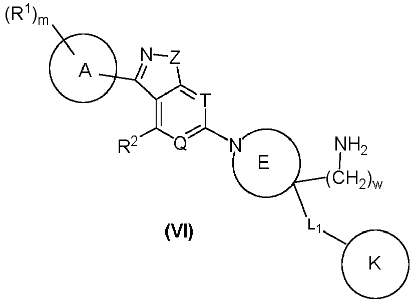 wherein:
L₁ is absent, or selected from -(C=O)- and -(CR^{w}R^{v})ᵤ-, wherein any one of -(CR^{w}R^{v})- is optionally further replaced by -N(R^{z})-, -O-, -S-, -SO- and -SO₂-;
each R^{w} and R^{v} are the same or different, and are each independently selected from hydrogen atom, halogen, hydroxy, alkyl or alkoxy;
each R^{z} are the same or different, and are each independently selected from hydrogen atom or alkyl;
ring E is selected from 4-11 membered monocyclic heterocyclyl containing N, 4-11 membered fused heterocyclyl containing N or 4-11 membered bridged heterocyclyl containing N, wherein the monocyclic heterocyclyl, fused heterocyclyl or bridged heterocyclyl is optionally further substituted by one or more substituents selected from halogen, alkyl, -OR⁵ or =O;
ring K is absent, or selected from cycloalkyl, aryl or heteroaryl, wherein the cycloalkyl, aryl or heteroaryl is optionally further substituted by one or more substituents selected from hydroxy, amino, halogen, nitro, cyano, alkyl, alkoxy, haloalkyl, cycloalkyl, heterocyclyl, aryl, heteroaryl, - C(O)R⁸, -C(O)OR⁸, -OC(O)R⁸, -SO₂R⁸, -NR⁹R¹⁰, -C(O)NR⁹R¹⁰, -SO₂NR⁹R¹⁰ or -NR⁹C(O)R¹⁰;
wherein -L₁-ring K and -(CH₂)_{W}-NH₂ are bound to the same carbon atom of ring E;
w is 0, 1 or 2;
u is 0, 1, 2 or 3; and
ring A, Z, Q, T, m, n, R¹-R², R⁵, and R⁸-R¹⁰ are defined as in claim 1.

14. The compound or the stereoisomer or the tautomer thereof, or the pharmaceutically acceptable salt thereof according to any one of claims 1-4 and 10-13, wherein R¹ is selected from hydrogen atom, F, Cl, Br, amino, hydroxy, cyano, nitro, methoxy, ethoxy, methyl, ethyl, trifluoromethyl, cyclopropyloxy, ethynyl, ethenyl, -NHCH₃ or -N(CH₃)₂.

15. The compound or the stereoisomer or the tautomer thereof, or the pharmaceutically acceptable salt thereof according to any one of claims 10-13, wherein R² is selected from - C(O)NH₂ or -C(O)OH.

16. The compound or the stereoisomer or the tautomer thereof, or the pharmaceutically acceptable salt thereof according to any one of claims 1-15, wherein R⁵ is selected from hydrogen atom or alkyl.

17. The compound or the stereoisomer or the tautomer thereof, or the pharmaceutically acceptable salt thereof according to any one of claims 1-4 and 10-13, wherein ring A is selected from phenyl, pyridinyl or pyrimidinyl.

18. The compound or the stereoisomer or the tautomer thereof, or the pharmaceutically acceptable salt thereof according to any one of claims 10-12, wherein ring B is selected from:

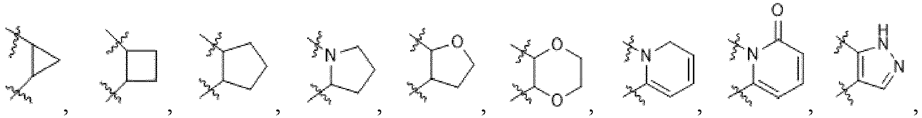

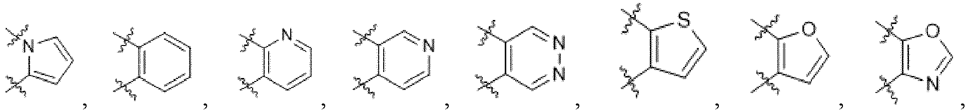

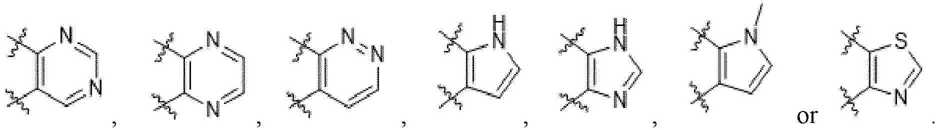

19. The compound or the stereoisomer or the tautomer thereof, or the pharmaceutically acceptable salt thereof according to any one of claims 10-12, wherein R^{g} are the same or different, and are each independently selected from hydrogen atom, F, Cl, Br, amino, hydroxy, cyano, nitro, methoxy, ethoxy, methyl, ethyl, ethynyl, ethenyl, -NHCH₃ or -N(CH₃)₂; and
alternatively, two R^{g} together with the same carbon atom bound therewith form C=O.

20. The compound or the stereoisomer or the tautomer thereof, or the pharmaceutically acceptable salt thereof according to claim 13, wherein ring E is selected from:

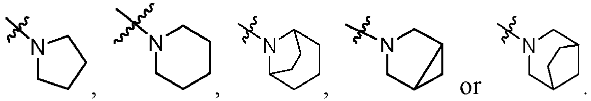

21. The compound or the stereoisomer or the tautomer thereof, or the pharmaceutically acceptable salt thereof according to any one of claims 1-20, wherein the compound is selected from:

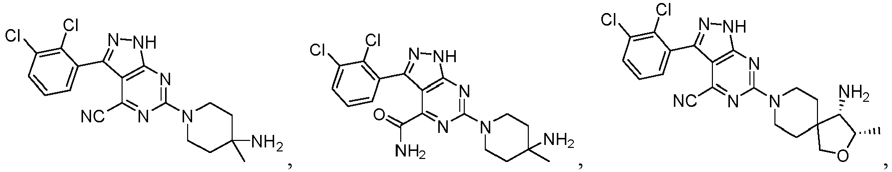

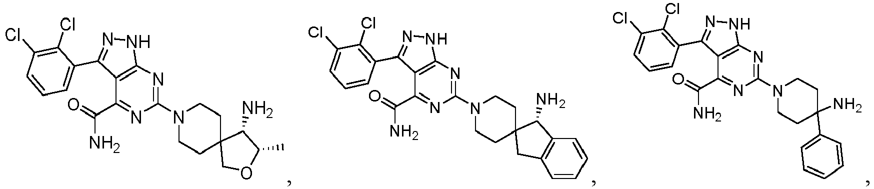

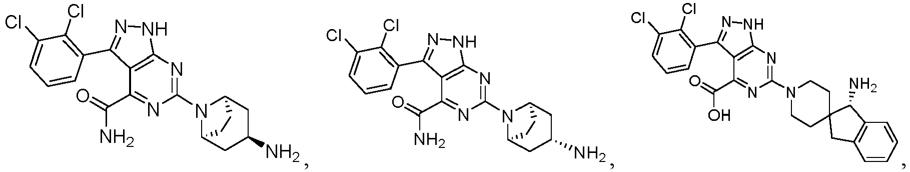

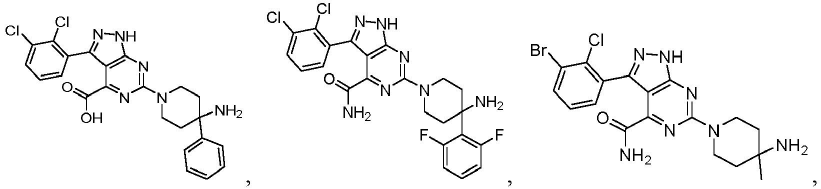

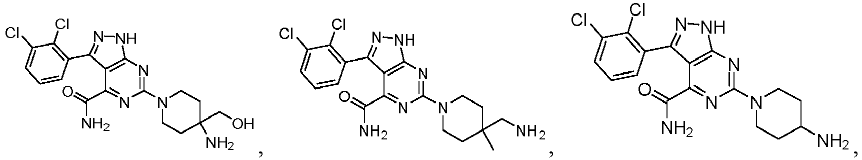

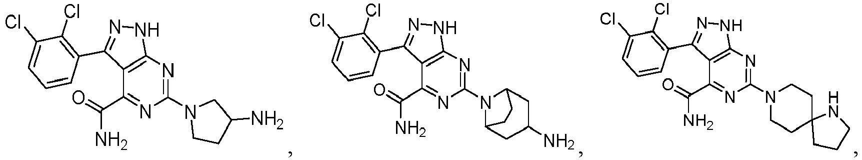

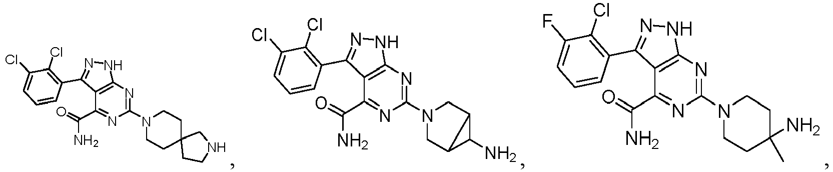

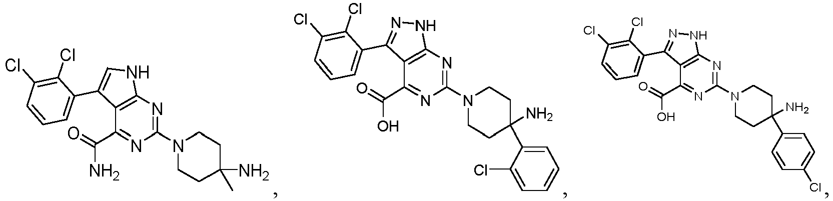

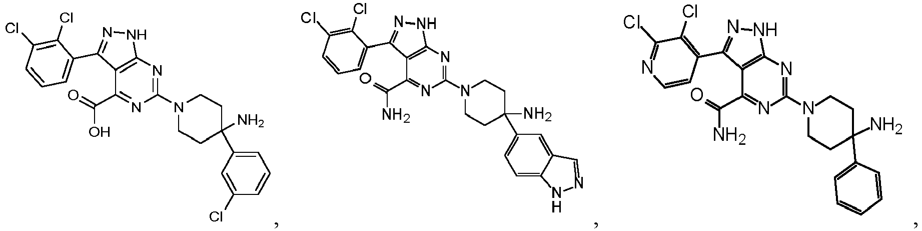

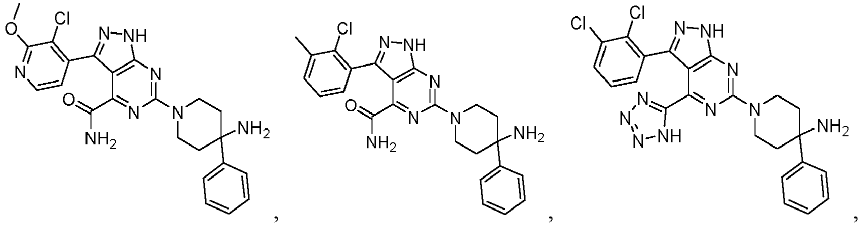

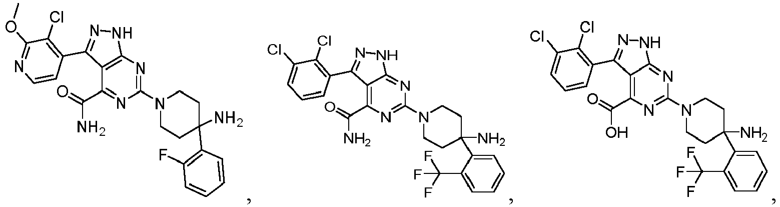

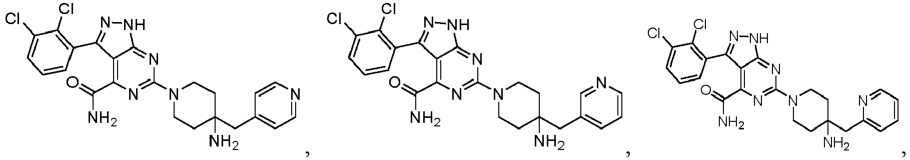

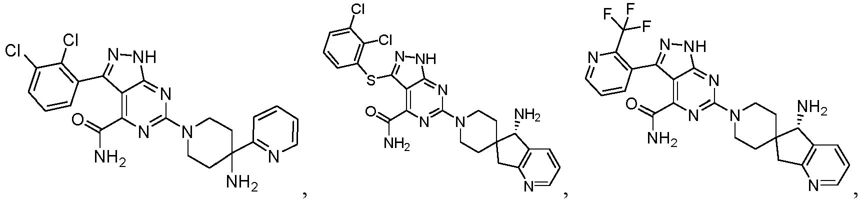

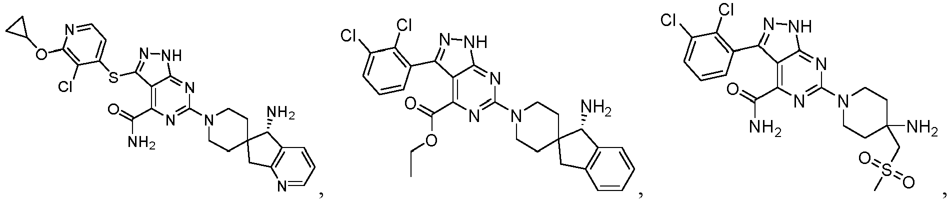

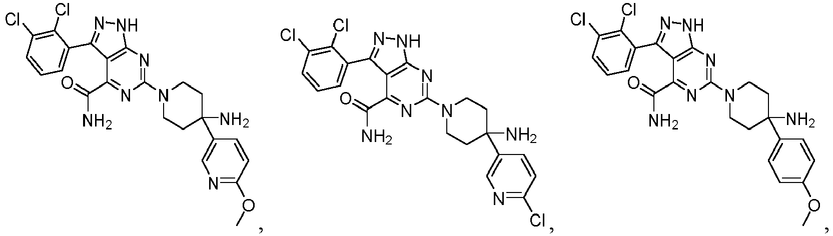

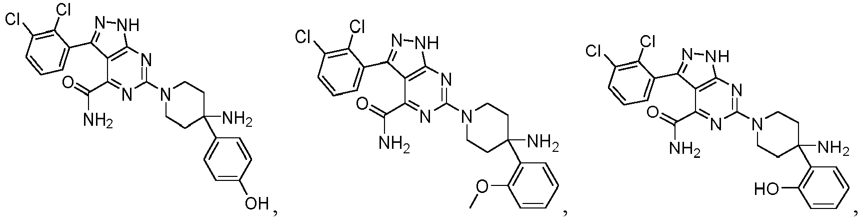

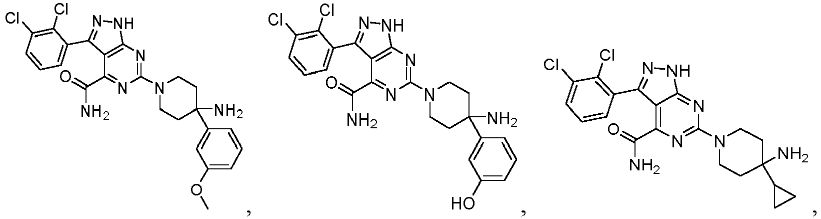

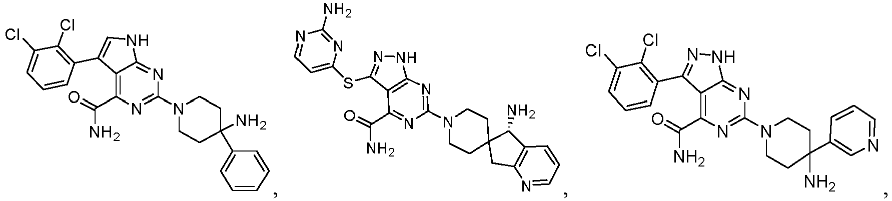

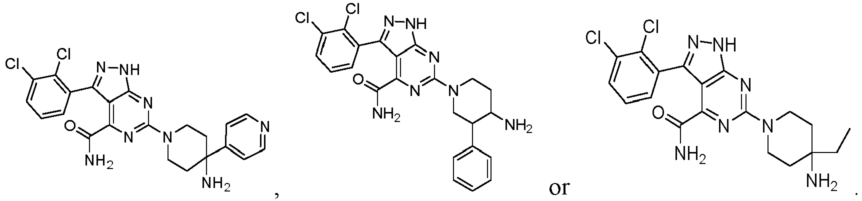

22. A preparation method for the compound represented by general formula (I) or the stereoisomer or the tautomer thereof according to claim 3, wherein the method comprises: 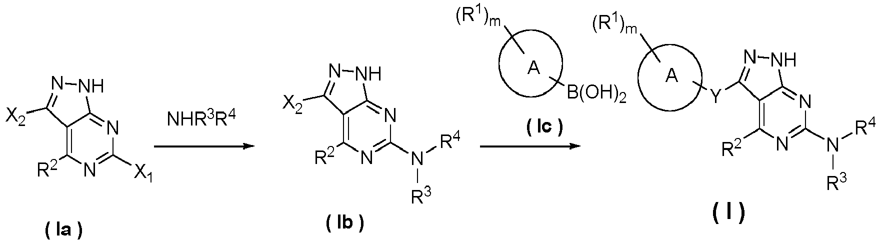 subjecting the compound represented by general formula (Ia) and NHR³R⁴ to a nucleophilic substitution reaction under alkaline condition to obtain the compound represented by general formula (Ib); and subjecting the compound represented by general formula (Ib) and the compound represented by general formula (Ic) to a Suzuki reaction in the presence of palladium catalyst and alkaline condition, and optionally further removing a protecting group of the obtained compound to obtain the compound represented by general formula (I); wherein:
Y is selected from chemical bond;
X₁ is selected from leaving group, wherein the leaving group is selected from halogen or - SO₂R^{t};
X₂ is selected from halogen;
R^{t} is selected from alkyl; and
ring A, m, and R¹-R⁴ are defined as in claim 3.

23. A preparation method for the compound represented by general formula (I) or the stereoisomer or the tautomer thereof according to claim 3, wherein the method comprises: 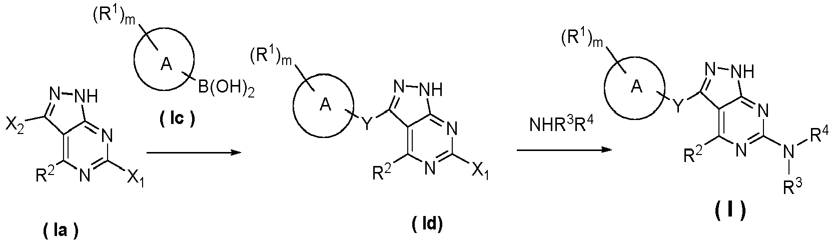 subjecting the compound represented by general formula (Ia) and the compound represented by general formula (Ic) to a Suzuki reaction in the presence of palladium catalyst and alkaline condition, to obtain the compound represented by general formula (Id); and subjecting the compound represented by general formula (Id) and NHR³R⁴ to a nucleophilic substitution reaction under alkaline condition to obtain the compound represented by general formula (I); wherein:
Y is selected from chemical bond;
X₁ is selected from leaving group, wherein the leaving group is selected from halogen or SO₂R^{t};
X₂ is selected from halogen;
R^{t} is selected from alkyl; and
ring A, m, and R¹-R⁴ are defined as in claim 3.

24. A compound represented by general formula (Ia) or a stereoisomer or a tautomer thereof, which is an intermediate for preparing a compound represented by general formula (I): 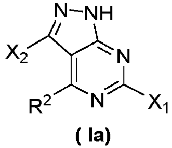 wherein:
X₁ is selected from leaving group, wherein the leaving group is selected from halogen or SO₂R^{t};
X₂ is selected from halogen;
R^{t} is selected from alkyl; and
R² is selected from cyano, tetrazolyl, -C(O)R⁵, -C(O)OR⁵ or -C(O)NR⁶R⁷;
R⁵, R⁶ and R⁷ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, amino, halogen, nitro, cyano, alkyl, alkoxy, cycloalkyl, heterocyclyl, aryl, heteroaryl, -C(O)R⁸, -C(O)OR⁸, - OC(O)R⁸, -SO₂R⁸, -NR⁹R¹⁰, -C(O)NR⁹R¹⁰, -SO₂NR⁹R¹⁰ or -NR⁹C(O)R¹⁰;
alternatively, R⁶ and R⁷ together with the N atom bound therewith form a 3-8 membered heterocyclyl, wherein the 3-8 membered heterocyclyl internally contains one or more N, O, S or SO₂ atoms, and the 3-8 membered heterocyclyl is optionally further substituted by one or more substituents selected from hydroxy, halogen, amino, alkyl or alkoxy; and
R⁸, R⁹ and R¹⁰ are each independently selected from hydrogen atom, alkyl, cycloalkyl, heterocyclyl, aryl or heteroaryl, wherein the alkyl, cycloalkyl, heterocyclyl, aryl or heteroaryl is optionally further substituted by one or more substituents selected from hydroxy, halogen, nitro, cyano, alkyl, alkoxy, cycloalkyl, heterocyclyl, aryl, heteroaryl, carboxyl or carboxylate.

25. A preparation method for the compound represented by general formula (Ia) or the stereoisomer or the tautomer thereof according to claim 24, wherein the method comprises: 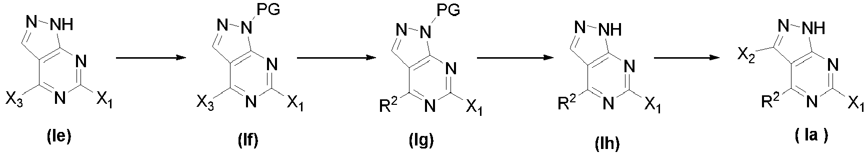 protecting the amino of the compound represented by general formula (Ie) to obtain the compound represented by general formula (If); subjecting the compound represented by general formula (If) to a coupling reaction under the action of palladium catalysts to obtain the compound represented by general formula (Ig); removing the protecting group PG from the compound represented by general formula (Ig) to obtain the compound represented by general formula (Ih); and subjecting the compound represented by general formula (Ih) to a halogenating reaction to obtain the compound represented by general formula (Ia); wherein:
PG is the protecting group, preferably 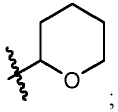
X₃ is selected from halogen; and
X₁, X₂ and R² are as defined in claim 24.

26. A pharmaceutical composition, wherein the pharmaceutical composition comprises an effective dose of the compound or the stereoisomer or the tautomer thereof, or the pharmaceutically acceptable salt thereof according to any one of claims 1-21, and a pharmaceutically acceptable carrier, an excipient or a combination thereof.

27. Use of the compound or the stereoisomer or the tautomer thereof, or the pharmaceutically acceptable salt thereof according to any one of claims 1-21, or the pharmaceutical composition according to claim 26 in preparing a SHP2 allosteric inhibitor.

28. Use of the compound or the stereoisomer or the tautomer thereof, or the pharmaceutically acceptable salt thereof according to any one of claims 1-21, or the pharmaceutical composition according to claim 26 in preparing a medicament for treating a disease mediated by SHP2, wherein the disease mediated by SHP2 is preferably cancer, cancerometastasis, cardiovascular disease, immune disorder, fibrosis or visual disorder.

29. The use according to claim 28, wherein the disease mediated by SHP2 is selected from Noonan syndrome, Leopard spot syndrome, juvenile myelomonocytic leukemia, neuroblastoma, melanoma, acute myeloid leukemia, breast cancer, esophagus cancer, lung cancer, colon cancer, head cancer, neuroblastoma, squamous cell carcinoma of head and neck, gastric cancer, anaplastic large cell lymphoma and glioblastoma.
